# EUROPEAN PATENT SPECIFICATION

(11) **EP 3 149 176 B1**
(45) Date of publication and mention of the grant of the patent: **23.10.2019**
(21) Application number: 15799855.0
(22) Date of filing: 01.06.2015
(51) Int. Cl.: C12N 15/10, C12N 15/67, G16B 5/00, G16B 45/00

(54) **METHODS FOR ALTERING POLYPEPTIDE EXPRESSION**
VERFAHREN ZUR ÄNDERUNG EINER POLYPEPTIDEXPRESSION
PROCÉDÉ DE MODIFICATION DE L'EXPRESSION D'UN POLYPEPTIDE

(30) Priority: 30.05.2014 US 201462005571 P; 03.09.2014 US 201462045507 P
(43) Date of publication of application: 05.04.2017
(73) Proprietor: The Trustees of Columbia University in the City of New York, New York, NY 10027 (US); Hunt, John, Francis, III, New York, NY 10025 (US); Aalberts, Daniel, Williamstown, MD 01267 (US); Boel, Gregory, P., New York, NY 10032 (US)
(72) Inventor: HUNT, John, Francis, III, New York, NY 10025 (US); AALBERTS, Daniel, Williamstown, MD 01267 (US); BOEL, Gregory, P., 75005 Paris (FR)
(74) Representative: Barker Brettell LLP
(86) International application number: PCT/US2015/033622
(87) International publication number: WO 2015/184466

(56) References cited:
- WO-A1-89/00604
- WO-A2-01/55342
- WO-A2-01/68835
- WO-A2-02/098443
- WO-A2-02/099105
- WO-A2-2004/066183
- WO-A2-2006/046132
- WO-A2-2011/100369
- US-A1- 2005 244 847
- US-A1- 2008 260 706
- US-A1- 2010 293 625
- US-A1- 2012 053 333
- US-A1- 2012 255 069
- ZHENG HUABAO ET AL: "Improving Escherichia coli FucO for furfural tolerance by saturation mutagenesis of individual amino acid positions.", APPLIED AND ENVIRONMENTAL MICROBIOLOGY MAY 2013, vol. 79, no. 10, May 2013 (2013-05), pages 3202-3208, XP002774098, ISSN: 1098-5336
- HARRY A STERN ET AL: "Accelerating calculations of RNA secondary structure partition functions using GPUs", ALGORITHMS FOR MOLECULAR BIOLOGY, BIOMED CENTRAL LTD, LO, vol. 8, no. 1, 1 November 2013 (2013-11-01), page 29, XP021166799, ISSN: 1748-7188, DOI: 10.1186/1748-7188-8-29
- EINERT THOMAS R ET AL: "Theory for RNA folding, stretching, and melting including loops and salt.", BIOPHYSICAL JOURNAL 08 JUN 2011, vol. 100, no. 11, 8 June 2011 (2011-06-08) , pages 2745-2753, XP28230163, ISSN: 1542-0086
- REUTER JESSICA S ET AL: "RNAstructure: software for RNA secondary structure prediction and analysis", BMC BIOINFORMATICS, BIOMED CENTRAL, LONDON, GB, vol. 11, no. 1, 15 March 2010 (2010-03-15) , page 129, XP021071478, ISSN: 1471-2105, DOI: 10.1186/1471-2105-11-129
- JIA M ET AL: "The relationship among gene expression, folding free energy and codon usage bias in Escherichia coli", FEBS LETTERS, ELSEVIER, AMSTERDAM, NL, vol. 579, no. 24, 10 October 2005 (2005-10-10), pages 5333-5337, XP027697304, ISSN: 0014-5793 [retrieved on 2005-10-10]
- GRÉGORY BOËL ET AL: "Codon influence on protein expression in E. coli correlates with mRNA levels", NATURE, vol. 529, no. 7586, 13 January 2016 (2016-01-13), pages 358-363, XP55409519, ISSN: 0028-0836, DOI: 10.1038/nature16509
- MATHEWS DAVID H ET AL: "Incorporating chemical modification constraints into a dynamic programming algorithm for prediction of RNA secondary structure.", PROCEEDINGS OF THE NATIONAL ACADEMY OF SCIENCES OF THE UNITED STATES OF AMERICA 11 MAY 2004, vol. 101, no. 19, 11 May 2004 (2004-05-11) , pages 7287-7292, XP55574868, ISSN: 0027-8424

## Description

This application claims the benefit of and priority to U.S. Provisional Application No. 62/005,571, filed on May 30, 2014 and U.S. Provisional Application No. 62/045,507, filed on September 3, 2014.

This patent disclosure contains material that is subject to copyright protection. The copyright owner has no objection to the facsimile reproduction by anyone of the patent document or the patent disclosure as it appears in the U.S. Patent and Trademark Office patent file or records, but otherwise reserves any and all copyright rights.

### BACKGROUND OF THE INVENTION

Overexpression of recombinant polypeptides is a central method in contemporary biochemistry, structural biology, and biotechnology. Many recombinant polypeptides express at low levels or not at all when produced in expression systems. Industrial applications, such as drug discovery and vaccine preparation, frequently require that large amounts of polypeptide be prepared.

Many types of expression systems can be used to synthesize proteins, including mammalian, fungal and bacterial expression systems. However, over-expression of a target recombinant polypeptide can be problematic where low expression yields arise from poor transcription and translation. This inherent limitation to recombinant polypeptide expression presents a problem for the use of such systems where the goal of an expression strategy is to obtain useful yields of a given recombinant polypeptide. Despite the existence of experimental and computational methods for addressing this variability, the physiochemical parameters and processes that influence polypeptide expression remain poorly understood and the expression of recombinant polypeptides remains a significant experimental challenge, as indicated by the large amount of prior art in this field (Makrides (1996) Microbiology and Molecular Biology Reviews 60:512; Sorensen and Mortensen (2005) Journal of Biotechnology 115:113-128; Christen et al. (2009) Polypeptide Expression and Purification). For example, WO20168835 A2 discloses methods for increasing the rate at which a nucleic acid can be translated by modifying the polynucleotide such that the rate at which the polynucleotide is translated is altered. Also disclosed are methods for modulating the translated of an mRNA by adding a nucleic acid that increases translation of the mRNA. WO0155342 A discloses a synthetic nucleic acid sequence comprising a non-naturally occurring polymer of nucleic acids and having a difference in sequence of at least about 5 % between the nucleic acids of the synthetic sequence and the starting sequence. The difference between the nucleic acid sequences results in a different free energy of folding for the synthetic sequence as compared to the starting sequence, such that the synthetic sequence would be expressed better in a selected heterologous host cell than the starting sequence would be if expressed in the same heterologous host cell. WO8900604 A discloses a method for increasing the translation efficiency of a mRNA sequence is provided. Zheng Huabao et al. (2013) Applied and Environmental Microbiology 79: 3202-3208; discloses how to improve E. coli FucO for furfural tolerance by saturation mutagenesis of individual amino acid positions. Harry A Stern and David H. Mathews (2013) Algorithms for Molecular Biology 8:29, 1-9; disclose how to accelerate calculations of RNA secondary structure partition functions using GPUs. Einert Thomas and Roland R. Netz (2011) 100:2745-2753; disclose a theory for RNA folding, stretching and melting, including loops and salt. Reuter Jessica and David M. Mathews (2010) BMC Bioinformatic 11:129, 1-9; disclose software for RNA secondary structure predictions and analysis. Jia M and Yanda Li (2005) FEBS Letters 579:5333-5337; consider the relationship among gene expression, folding free energy and codon usage bias in *E. Coli.* There is, therefore, a need for methods for identifying polypeptides that have a high probability of being expressed at high levels in cellular expression systems. There is also a need for methods suitable for increasing the expression of polypeptides. This invention addresses these needs.

### SUMMARY OF THE INVENTION

In one aspect of the invention, there is provided a method to increase the expression of a recombinant polypeptide in an in vitro or in vivo expression system, comprising making one or more synonymous substitutions in the protein-coding nucleic acid sequence by selecting a good codon for every amino acid according to a generalized linear multiparameter model of the influence of a comprehensive set of RNA sequence parameters on measurable experimental values correlated with protein expression level, wherein the comprehensive set of RNA sequence parameters includes (i) in-frame single codon frequencies, (ii) in-frame ATA-ATA dicodon frequency, (iii) linear, parabolic, and inverse-linear functions of nucleotide base composition codons 2-6, (iv) nucleotide base composition in the remainder of the protein-coding sequence, (v) the partition-function free-energy of RNA folding calculated by the program RNAstructure with default parameters for the first 48 nucleotides in the protein-coding sequence plus the 5'-UTR sequence if that sequence is consistent in the modeled dataset, (vi) the average value of the partition-function free-energy of RNA folding calculated by the program RNAstructure with default parameters for 50% overlapping windows of 96 nucleotides after nucleotide 48 in the protein-coding sequence, (vii) the length in nucleotides of the protein-coding sequence, (viii) the amino acid repetition rate, and (ix) the codon repetition rate. In another aspect, there is provided a method to increase the expression of a recombinant polypeptide in an in vitro or in vivo expression system that comprises providing a nucleic acid sequence with a protein-coding sequence functionally linked to a 5'-untranslated region (5'-UTR) containing a ribosome-binding site, making one or more synonymous substitutions in codons 2, 3, 5, 4, and 6 of the protein-coding sequence that lower guanine content or raise adenine content, and making synonymous substitutions in the resulting coding sequence that produce a partition-function free-energy of RNA folding calculated by the program RNAstructure with default parameters that is as close as achievable to being greater than -10 kcal/mol for the first 48 nucleotides in the protein-coding sequence and, if using a pET vector 5'-UTR, that is -30.0 kcal/mol or higher for the first 48 nucleotides in the coding sequence plus the 5'-UTR. In a further aspect, there is provided a method to increase the expression of a recombinant polypeptide in an in vitro or in vivo expression system by making one or more synonymous substitutions so that the partition-function free-energy of RNA folding as calculated by the program RNAstructure with default parameters is as close as achievable to being in the range from 10 kcal/mol less than to 5 kcal/mol greater than (-0.32^{∗}(W-18)) kcal/mol where W is the number of nucleotides in the tail sequence window downstream of nucleotide 48 in the protein-coding sequence, with W typically being 96 nucleotides. The disclosure also relates to a method for increasing the expression of a recombinant polypeptide in an expression system by introducing one or more synonymous substitutions, the method comprising providing a nucleic acid sequence comprising a coding sequence encoding the polypeptide and a 5' UTR comprising a ribosome binding site and wherein the 5' UTR is functionally linked to said coding sequence, and (a) introducing one or more substitutions in the 5'UTR or one or more synonymous nucleic acid substitutions in a head sequence consisting essentially of the first 48 nucleic acids of the coding sequence, wherein the one or more substitutions in the 5'UTR and the one or more synonymous nucleic acid substitutions increase the predicted free energy of folding of the RNA sequence corresponding to the head sequence and the 5' UTR functionally linked to said coding sequence (i.e., decrease the stability of its folding), (b) introducing one or more synonymous nucleic acid substitutions in a tail sequence consisting essentially of the coding sequence downstream of the head sequence, wherein the one or more synonymous nucleic acid substitutions alter the predicted free energy of folding of the RNA sequence corresponding to each of one or more tail sequence windows within the tail sequence to be in a range of about (-0.32^{∗}(W-18)) kcal/mol minus 10 kcal/mol or plus 5 kcal/mol where W is the number of nucleotides in the tail sequence window, (c) introducing one or more synonymous nucleic acid substitutions in the first 18 nucleic acids of the head sequence so as to replace, where possible each of codons 2, 3, 4, 5 and 6 with a synonymous codon having a lower guanine content or a higher adenine content, (d) optimizing codons in the coding sequence according to a sub method selected from any of: a 6AA method, a 31C-FO method, a Model M method, a CHGlir method, or a BLOGIT method, (e) introducing one or more substitutions in the coding sequence so as to replace pairs of identical repeating codons separated by 0, 1, 2, 3, 4, 5, 6, 7, 8, 9, 10, 11, 12, 13, 14, or 15 intervening codons so as to change at least one of the repeating codons to a different synonymous codon, (f) substituting at least one nucleic acid in a ATA ATA dicodon repeat within the coding sequence so as to introduce a synonymous dicodon repeat that is not an ATA ATA sequence, and (g) substituting at least one codon in the coding sequence ending with a G or C with a synonymous codon ending with a A or T.

The disclosure also relates to a method for increasing the expression of a recombinant polypeptide in an expression system by introducing one or more synonymous substitutions, the method comprising providing a nucleic acid sequence comprising a coding sequence encoding the polypeptide and a 5' UTR comprising a ribosome binding site and wherein the 5' UTR is functionally linked to said coding sequence, **and further comprising one or more of:** (a) introducing one or more substitutions in the 5'UTR or one or more synonymous nucleic acid substitutions in a head sequence consisting essentially of the first 48 nucleic acids of the coding sequence, wherein the one or more substitutions in the 5'UTR and the one or more synonymous nucleic acid substitutions increase the predicted free energy of folding of the RNA sequence corresponding to the head sequence and the 5' UTR functionally linked to said coding sequence, (b) introducing one or more synonymous nucleic acid substitutions in a tail sequence consisting essentially of the coding sequence downstream of the head sequence, wherein the one or more synonymous nucleic acid substitutions alter the predicted free energy of folding of the RNA sequence corresponding to each of one or more tail sequence windows within the tail sequence to be in a range of about (-0.32^{∗}(W-18)) kcal/mol minus 10 kcal/mol or plus 5 kcal/mol where W is the number of nucleotides in the tail sequence window, (c) introducing one or more synonymous nucleic acid substitutions in the first 18 nucleic acids of the head sequence so as to replace, where possible each of codons 2, 3, 4, 5 and 6 with a synonymous codon having a lower guanine content or a higher adenine content, (d) optimizing codons in the coding sequence according to a sub method selected from any of: a 6AA method, a 31C-FO method, a Model M method, a CHGlir method, or a BLOGIT method, (e) introducing one or more substitutions in the coding sequence so as to replace pairs of identical repeating codons separated by 0, 1, 2, 3, 4, 5, 6, 7, 8, 9, 10, 11, 12, 13, 14, or 15 intervening codons so as to change at least one of the repeating codons to a different synonymous codon, (f) substituting at least one nucleic acid in a ATA ATA dicodon repeat within the coding sequence so as to introduce a synonymous dicodon repeat that is not an ATA ATA sequence, and (g) substituting at least one codon in the coding sequence ending with a G or C with a synonymous codon ending with a A or T.

The disclosure relates to a method for increasing the expression of a recombinant polypeptide in an expression system by introducing one or more synonymous substitutions, the method comprising providing a nucleic acid sequence comprising a coding sequence encoding the polypeptide, and (a) introducing one or more substitutions in a head sequence consisting essentially of the first 48 nucleic acids of the coding sequence, wherein the one or more synonymous nucleic acid substitutions increase the predicted free energy of folding of the RNA sequence corresponding to the head sequence, (b) introducing one or more synonymous nucleic acid substitutions in a tail sequence consisting essentially of the coding sequence downstream of the head sequence, wherein the one or more synonymous nucleic acid substitutions alter predicted free energy of folding of the RNA sequence corresponding to each of one or more tail sequence windows within the tail sequence to be in a range of about (-0.32^{∗}(W-18)) kcal/mol minus 10 kcal/mol or plus 5 kcal/mol where W is the number of nucleotides in the tail sequence window, (c) introducing one or more synonymous nucleic acid substitutions in the first 18 nucleic acids of the head sequence so as to replace, where possible each of codons 2, 3, 4, 5 and 6 with a synonymous codon having a lower guanine content or a higher adenine content, (d) optimizing codons in the coding sequence according to a sub method selected from any of: a 6AA method, a 31C-FO method, a Model M method, a CHGlir method, or a BLOGIT method, (e) introducing one or more substitutions in the coding sequence so as to replace pairs of identical repeating codons separated by 0, 1, 2, 3, 4, 5, 6, 7, 8, 9, 10, 11, 12, 13, 14, or 15 intervening codons so as to change at least one of the repeating codons to a different synonymous codon, (f) substituting at least one nucleic acid in a ATA ATA dicodon repeat within the coding sequence so as to introduce a synonymous dicodon repeat that is not an ATA ATA sequence, and (g) substituting at least one codon in the coding sequence ending with a G or C with a synonymous codon ending with a A or T.

The disclosure relates to a method for increasing the expression of a recombinant polypeptide in an expression system by introducing one or more synonymous substitutions, the method comprising providing a nucleic acid sequence comprising a coding sequence encoding the polypeptide, **and further comprising one or more of:** (a) introducing one or more substitutions in a head sequence consisting essentially of the first 48 nucleic acids of the coding sequence, wherein the one or more synonymous nucleic acid substitutions increase the predicted free energy of folding of the RNA sequence corresponding to the head sequence, (b) introducing one or more synonymous nucleic acid substitutions in a tail sequence consisting essentially of the coding sequence downstream of the head sequence, wherein the one or more synonymous nucleic acid substitutions alter the predicted free energy of folding of the RNA sequence corresponding to each of one or more tail sequence windows within the tail sequence to be in a range of about (-0.32^{∗}(W-18)) kcal/mol minus 10 kcal/mol or plus 5 kcal/mol where W is the number of nucleotides in the tail sequence window, (c) introducing one or more synonymous nucleic acid substitutions in the first 18 nucleic acids of the head sequence so as to replace, where possible each of codons 2, 3, 4, 5 and 6 with a synonymous codon having a lower guanine content or a higher adenine content, (d) optimizing codons in the coding sequence according to a sub method selected from any of: a 6AA method, a 31C-FO method, a Model M method, a CHGlir method, or a BLOGIT method, (e) introducing one or more substitutions in the coding sequence so as to replace pairs of identical repeating codons separated by 0, 1, 2, 3, 4, 5, 6, 7, 8, 9, 10, 11, 12, 13, 14, or 15 intervening codons so as to change at least one of the repeating codons to a different synonymous codon, (f) substituting at least one nucleic acid in a ATA ATA dicodon repeat within the coding sequence so as to introduce a synonymous dicodon repeat that is not an ATA ATA sequence, and (g) substituting at least one codon in the coding sequence ending with a G or C with a synonymous codon ending with a A or T.

The method may comprise of any of: step a; step b; step c; step; step d; step e; step f; step g; steps ab; steps ac; steps ad; steps ae; steps af; steps ag; steps bc; steps bd; steps be; steps bf; steps bg; steps cd; steps ce; steps cf; steps cg; steps de; steps df; steps dg; steps ef; steps eg; steps fg; steps abc; steps abd; steps abe; steps abf; steps abg; steps acd; steps ace; steps acf; steps acg; steps ade; steps adf; steps adg; steps aef; steps aeg; steps afg; steps bcd; steps bce; steps bcf; steps bcg; steps bde; steps bdf; steps bdg; steps bef; steps beg; steps bfg; steps cde; steps cdf; steps cdg; steps cef; steps ceg; steps cfg; steps def; steps deg; steps dfg; steps efg; steps abcd; steps abce; steps abcf; steps abcg; steps abde; steps abdf; steps abdg; steps abef; steps abeg; steps abfg; steps acde; steps acdf; steps acdg; steps acef; steps aceg; steps acfg; steps adef; steps adeg; steps adfg; steps aefg; steps bcde; steps bcdf; steps bcdg; steps bcef; steps bceg; steps bcfg; steps bdef; steps bdeg; steps bdfg; steps befg; steps cdef; steps cdeg; steps cdfg; steps cefg; steps defg; steps abcde; steps abcdf; steps abcdg; steps abcef; steps abceg; steps abcfg; steps abdef; steps abdeg; steps abdfg; steps abefg; steps acdef; steps acdeg; steps acdfg; steps acefg; steps adefg; steps bcdef; steps bcdeg; steps bcdfg; steps bcefg; steps bdefg; steps cdefg; steps abcdef; steps abcdeg; steps abcdfg; steps abcefg; steps abdefg; steps acdefg; steps bcdefg; or steps abcdefg.

The optimizing codons in the coding sequence may comprise (i) substituting at least one codon in the head sequence with a synonymous codon having a higher CHGlir slope, (ii) substituting all codons in the head sequence with a synonymous codon having a higher CHGlir slope, (iii) substituting at least one codon in the head sequence with a synonymous codon having a lower CHGlir slope and at least one codon in the head sequence with a synonymous codon having a higher CHGlir slope, (iv) substituting at least one codon in the head sequence with a synonymous codon having a higher BLOGIT coefficient, (v) substituting all codons in the head sequence with a synonymous codon having a higher BLOGIT coefficient, (vi) substituting at least one codon in the head sequence with a synonymous codon having a lower BLOGIT coefficient and at least one codon in the head sequence with a synonymous codon having a higher BLOGIT coefficient, (vii) substituting at least one codon in the tail sequence with a synonymous codon having a higher CHGlir slope, (viii) substituting all codons in the tail sequence with a synonymous codon having a higher CHGlir slope, (ix) substituting at least one codon in the tail sequence with a synonymous codon having a lower CHGlir slope and at least one codon in the tail sequence with a synonymous codon having a higher CHGlir slope, (x) substituting at least one codon in the tail sequence with a synonymous codon having a higher BLOGIT coefficient, (xi) substituting all codons in the tail sequence with a synonymous codon having a higher BLOGIT coefficient, (xii) substituting at least one codon in the tail sequence with a synonymous codon having a lower BLOGIT coefficient and at least one codon in the tail sequence with a synonymous codon having a higher BLOGIT coefficient.

The substitutions of step (a) may not change the ribosome binding site of the 5'UTR.

The ribosome binding site may be a Kozak sequence or a Shine Dalgarno sequence.

The 5'UTR further may comprise a 5' cap sequence.

The substitutions of step (a) may not change the 5' cap sequence.

The substitutions of step (a) may not interfere with functional processing of the RNA corresponding to the coding sequence or the 5'UTR.
Step (a) may comprise increasing the predicted free energy of folding to at least about -35 kcal/mol
Step (a) may comprise increasing predicted free energy of folding to at least about -39 kcal/mol
Step (a) may comprise increasing predicted free energy of folding to at least about -5 kcal/mol
Step (a) may comprise maximizing a predicted free energy of folding.

The predicted free energy of folding of step (b) may be in the range of about -20 kcal/mol to about -40 kcal/mol when the tail sequence window in 96 nucleic acids.

The predicted free energy of folding may be computed with RNA structure using default parameters.

The predicted free energy of folding may be computed with UNAFOLD, ViennaRNA, mFold, Sfold, Bindigo or Bindigonet using default parameters.

The one or more synonymous nucleic acid substitutions of step (a) or step (b) may be selected from the list comprising (i) substituting a GCT codon with a GCA codon, or substituting a GCA codon with a GCT codon; (ii) substituting a CGT codon with a CGA codon, or substituting a CGA codon with a CGT codon; (iii) substituting a CAA codon with a CAG codon, or substituting a CGA codon with a CAA codon; (iv) substituting a CAT codon with a CAC codon, or substituting a CAC codon with a CAT codon; (v) substituting a ATT codon with a ATC codon, or substituting a ATC codon with a ATT codon; (vii) substituting a TTA codon to either a TTG codon or a CTA codon, or substituting a TTG codon to either a TTA codon or a CTA codon, or substituting a CTA codon to either a TTA codon or a TTG codon; (viii) substituting a CCT codon with a CCA codon, or substituting a CCA codon with a CCT codon; (ix) substituting a AGT codon with a TCA codon, or substituting a TCA codon with a AGT codon; (x) substituting a ACA codon with a ACT codon, or substituting a ACT codon with a ACA codon; (xi) substituting a GTT codon with a GTA codon, or substituting a GTA codon with a GTT codon.

The one or more tail sequence windows within the tail sequence of step (b) may be overlapping sequence windows. The one or more overlapping sequence windows of step (b) may overlap by 25 nucleic acids. The one or more tail sequence windows within the tail sequence of step (b) do not overlap.

The one or more tail sequence windows within the tail sequence of step (b) may have a length of 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, or 144 nucleic acids.

The one or more tail sequence windows within the tail sequence of step (b) may have a length of at least about 145 nucleic acids, at least about 150 nucleic acids, at least about 160 nucleic acids, at least about 170 nucleic acids, at least about 180 nucleic acids, at least about 190 nucleic acids, at least about 200 nucleic acids, at least about 220 nucleic acids, at least about 240 nucleic acids, at least about 260 nucleic acids, at least about 280 nucleic acids, at least about 300 nucleic acids, at least about 340 nucleic acids, at least about 380 nucleic acids, at least about 420 nucleic acids, at least about 460 nucleic acids, at least about 500 nucleic acids, at least about 600 nucleic acids, at least about 700 nucleic acids, at least about 800 nucleic acids, at least about 900 nucleic acids, at least about 1000 or more nucleic acids.

The one or more tail sequence windows within the tail sequence of step (b) may have a length of 47 nucleic acids or less.

The one or more tail sequence windows within the tail sequence of step (b) may have a length of 145 nucleic acids or more.

The 6AA method may comprise: (i) altering all codons in the coding sequence encoding an arginine residue to CGT; (ii) altering all codons in the coding sequence encoding aspartic acid to GAT; (iii) altering all codons in the coding sequence encoding glutamine to CAA; (iv) altering all codons in the coding sequence encoding glutamic acid to GAA; (v) altering all codons in the coding sequence encoding histidine residue to CAT; and (vi) altering all codons in the coding sequence encoding isoleucine to ATT.

The 6AA method may comprise any of: (i) altering at least one of any codon in the coding sequence encoding an arginine residue to CGT; (ii) altering at least one of any codon in the coding sequence encoding aspartic acid to GAT; (iii) altering at least one of any codon in the coding sequence encoding glutamine to CAA; (iv) altering at least one of any codon in the coding sequence encoding glutamic acid to GAA; (v) altering at least one of any codon in the coding sequence histidine residue to CAT; or (vi) altering at least one of any codon in the coding sequence encoding isoleucine to ATT.

The 31C-FO method may comprise substituting at least one codon with a synonymous codon having a higher binary logistic regression slope. In certain embodiments, the 31C-FO method comprises substituting at all codons with a synonymous codon having a higher binary logistic regression slope. In certain embodiments, the 31C-FO method comprises substituting at least one codon with a synonymous codon having a lower binary logistic regression slope and at least one codon with a synonymous codon having a higher binary logistic regression slope. In certain embodiments, the 31C-FO method comprises substituting at least one codon with a synonymous codon having a higher ordinal logistic regression slope. In certain embodiments, the 31C-FO method comprises substituting at all codons with a synonymous codon having a higher ordinal logistic regression slope. In certain embodiments, the 31C-FO method comprises substituting at least one codon with a synonymous codon having a lower ordinal logistic regression slope and at least one codon with a synonymous codon having a higher ordinal logistic regression slope.

The 31C-FO method may comprise any of: (i) altering at least one of any codon in the coding sequence encoding alanine to either GCT or GCA; (ii) altering at least one of any codon in the coding sequence encoding arginine to either CGT or CGA; (iii) altering at least one of any codon in the coding sequence encoding asparagine to be AAT; (iv) altering at least one of any codon in the coding sequence encoding aspartic acid to GAT; (v) altering at least one of any codon in the coding sequence encoding cysteine to TGT; (vi) altering at least one of any codon in the coding sequence encoding glutamine to either CAA or CAG; (vii) altering at least one of any codon in the coding sequence encoding glutamic acid to GAA; (viii) altering at least one of any codon in the coding sequence encoding glycine to GGT; (ix) altering at least one of any codon in the coding sequence encoding histidine to either CAT or CAC; (x) altering at least one of any codon in the coding sequence encoding isoleucine to ATT or ATC; (xi) altering at least one of any codon in the coding sequence encoding leucine to any of TTA, TTG, or CTA; (xii) altering at least one of any codon in the coding sequence encoding lysine to AAA; (xiii) altering at least one of any codon in the coding sequence encoding methionine to ATG; (xiv) altering at least one of any codon in the coding sequence encoding phenylalanine to TTT; (xv) altering at least one of any codon in the coding sequence encoding proline to either CCT or CCA; (xvi) altering at least one of any codon in the coding sequence encoding serine to AGT or TCA; (xvii) altering at least one of any codon in the coding sequence encoding threonine to either ACA or ACT; (xviii) altering at least one of any codon in the coding sequence encoding tryptophan to TGG; (xix) altering at least one of any codon in the coding sequence encoding tyrosine to TAT; or (xx) altering at least one of any codon in the coding sequence encoding valine to either GTT or GTA.

The 31C-FO method may comprise (i) altering all codons in the coding sequence encoding alanine to either GCT or GCA; (ii) altering all codons in the coding sequence encoding arginine to either CGT or CGA; (iii) altering all codons in the coding sequence encoding asparagine to be AAT; (iv) altering all codons in the coding sequence encoding aspartic acid to GAT; (v) altering all codons in the coding sequence encoding cysteine to TGT; (vi) altering all codons in the coding sequence encoding glutamine to either CAA or CAG; (vii) altering all codons in the coding sequence encoding glutamic acid to GAA; (viii) altering all codons in the coding sequence encoding glycine to GGT; (ix) altering all codons in the coding sequence encoding histidine to either CAT or CAC; (x) altering all codons in the coding sequence encoding isoleucine to ATT or ATC; (xi) altering all codons in the coding sequence encoding leucine to any of TTA, TTG, or CTA; (xii) altering all codons in the coding sequence encoding lysine to AAA; (xiii) altering all codons in the coding sequence encoding methionine to ATG; (xiv) altering all codons in the coding sequence encoding phenylalanine to TTT; (xv) altering all codons in the coding sequence encoding proline to either CCT or CCA; (xvi) altering all codons in the coding sequence encoding serine to AGT or TCA; (xvii) altering all codons in the coding sequence encoding threonine to either ACA or ACT; (xviii) altering all codons in the coding sequence encoding tryptophan to TGG; (xix) altering all codons in the coding sequence encoding tyrosine to TAT; and (xx) altering all codons in the coding sequence encoding valine to either GTT or GTA.

The Model M method may comprise any of: (i) making synonymous codon changes that increase the value of the equation for Model M $θ = 4.38 + 0.0451 {G}_{UH} + 23.6 / < {G}_{T} {>}_{96} - 0.00117 L - 489 / L + 6.55 {A}_{H} - 6.30 {{A}_{H}}^{2} + 0.753 {U}_{3 ⁢ H} - 1.85 {{G}_{H}}^{2} - 1.50 \left({G}_{UH}∗<-39\right) \left({GC}_{H}>10/15\right) - 11.7 r - 1.82 i + 0.077 {s}_{7 - 16} + 0.059 {s}_{17 - 32} + 0.878 {\sum }_{c} {β}_{c} {f}_{c} ,$ (ii) increasing the mean value of CHGlir slope calculated for some set of the codons downstream of codon 6 in the coding sequence, (iii) increasing the mean value of CHGlir slope calculated for all of the codons downstream of codon 6 in the coding sequence, (iv) increasing the mean value of CHGlir slope calculated for some set of the codons downstream of codon 6 in the coding sequence, (v) increasing the mean value of CHGlir slope calculated for all of the codons downstream of codon 6 in the coding sequence.

The methods described herein can be used for optimization of gene sequences for protein expression in any organism. Output from the computational approach used to generate model "M" or derivatives thereof can be applied to protein-expression profiling data or mRNA profiling data from that organism.

The BLOGIT method may comprise any of: (i) increasing the mean value of BLOGIT slope calculated for all of the codons downstream of codon 6 in the coding sequence, (ii) increasing the mean value of BLOGIT slope calculated for some set of the codons downstream of codon 6 in the coding sequence, or (iii) increasing the mean value of BLOGIT slope calculated for all of the codons downstream of codon 6 in the coding sequence

The BLOGIT method may comprise (i) altering all codons downstream of codon 6 in the coding sequence encoding alanine to either GCT or GCA; (ii) altering all codons downstream of codon 6 in the coding sequence encoding arginine to either CGT or CGA; (iii) altering all codons downstream of codon 6 in the coding sequence encoding asparagine to be AAT; (iv) altering all codons downstream of codon 6 in the coding sequence encoding aspartic acid to GAT; (v) altering all codons downstream of codon 6 in the coding sequence encoding cysteine to TGT; (vi) altering all codons downstream of codon 6 in the coding sequence encoding glutamine to either CAA or CAG; (vii) altering all codons downstream of codon 6 in the coding sequence encoding glutamic acid to GAA; (viii) altering all codons downstream of codon 6 in the coding sequence encoding glycine to GGT; (ix) altering all codons downstream of codon 6 in the coding sequence encoding histidine to either CAT or CAC; (x) altering all codons downstream of codon 6 in the coding sequence encoding isoleucine to ATT or ATC; (xi) altering all codons downstream of codon 6 in the coding sequence encoding leucine to any of TTA, TTG, or CTA; (xii) altering all codons downstream of codon 6 in the coding sequence encoding lysine to AAA; (xiii) altering all codons downstream of codon 6 in the coding sequence encoding methionine to ATG; (xiv) altering all codons downstream of codon 6 in the coding sequence encoding phenylalanine to TTT; (xv) altering all codons downstream of codon 6 in the coding sequence encoding proline to either CCT or CCA; (xvi) altering all codons downstream of codon 6 in the coding sequence encoding serine to AGT or TCA; (xvii) altering all codons downstream of codon 6 in the coding sequence encoding threonine to either ACA or ACT; (xviii) altering all codons downstream of codon 6 in the coding sequence encoding tryptophan to TGG; (xix) altering all codons downstream of codon 6 in the coding sequence encoding tyrosine to TAT; and (xx) altering all codons downstream of codon 6 in the coding sequence encoding valine to either GTT or GTA, (xxi) substituting at least one codon encoding an leucine residue with a CTC codon, a CTG codon, or possibly a TTA codon, (xxii) substituting at least one codon encoding an isoleucine residue with a ATT codon or possibly a ATC codon, (xxiii) substituting at least one codon encoding an glutamate residue with a GAA codon, or (xxiv) substituting at least one codon encoding an aspartate residue with a GAT codon.

The CHGlir method may comprise substituting at least one codon with a synonymous codon having a higher CHGlir slope. The CHGlir method may comprise substituting all codons with a synonymous codon having a higher CHGlir slope. The CHGlir method may comprise substituting at least one codon with a synonymous codon having a lower CHGlir slope and at least one codon with a synonymous codon having a higher CHGlir slope.

The CHGlir method comprises: (i) substituting at least one codon encoding an alanine residue with a GCG codon; (ii) substituting at least one codon encoding an arginine residue with a CGC codon, a AGA codon, or a AGG codon; (iii) substituting at least one codon encoding a glutamine residue with a CAA codon; (iv) substituting at least one codon encoding a phenylalanine residue with a TTT codon; (v) substituting at least one codon encoding a proline residue with a CCG codon or a CCC codon; (vi) substituting at least one codon encoding a serine residue with a AGC codon or a TCA codon; (vii) substituting at least one codon encoding a threonine residue with a ACA codon or a ACC codon; (viii) substituting at least one codon encoding a tyrosine residue with a TAT codon; (ix) substituting at least one codon encoding a valine residue with a GTT codon, a GTG codon or GTA codon, (x) substituting at least one codon encoding an leucine residue with a CTC codon, a CTG codon, or possibly a TTA codon, (xi) substituting at least one codon encoding an isoleucine residue with a ATT codon or possibly a ATC codon, (xii) substituting at least one codon encoding an glutamate residue with a GAA codon, (xiii) substituting at least one codon encoding an histidine residue with a CAT codon, (xiv) substituting at least one codon encoding an aspartate residue with a GAT codon, (xv) substituting at least one codon encoding an asparagine residue with a AAC codon, or (xvi) substituting at least one codon encoding an glycine residue with a GGA or GGT codon.

The CHGlir method comprises: (i) substituting all codons encoding an alanine residue with a GCG codon; (ii) substituting all codons encoding an arginine residue with a CGC codon, a AGA codon, or a AGG codon; (iii) substituting all codons encoding a glutamine residue with a CAA codon; (iv) substituting all codons encoding a phenylalanine residue with a TTT codon; (v) substituting all codons encoding a proline residue with a CCG codon or a CCC codon; (vi) substituting all codons encoding a serine residue with a AGC codon or a TCA codon; (vii) substituting all codons encoding a threonine residue with a ACA codon or a ACC codon; (viii) substituting all codons encoding a tyrosine residue with a TAT codon; (ix) substituting all codons encoding a valine residue with a GTT codon, a GTG codon or GTA codon, (x) substituting at least one codon encoding an leucine residue with a CTC codon, a CTG codon, or possibly a TTA codon, (xi) substituting at least one codon encoding an isoleucine residue with a ATT codon or possibly a ATC codon, (xii) substituting at least one codon encoding an glutamate residue with a GAA codon, (xiii) substituting at least one codon encoding an histidine residue with a CAT codon, (xiv) substituting at least one codon encoding an aspartate residue with a GAT codon, (xv) substituting at least one codon encoding an asparagine residue with a AAC codon, or (xvi) substituting at least one codon encoding an glycine residue with a GGA or GGT codon.

The BLOGIT method comprises substituting at least one codon with a synonymous codon having a higher BLOGIT coefficient. The BLOGIT method comprises substituting all codons with a synonymous codon having a higher BLOGIT coefficient. The BLOGIT method comprises substituting at least one codon with a synonymous codon having a lower BLOGIT coefficient and at least one codon with a synonymous codon having a higher BLOGIT coefficient.

The BLOGIT method comprises:
(i) substituting all codons encoding an alanine residue with a GCT codon, or substituting all codons encoding an alanine residue with a substitution selected from:
   GCC to any of GCG, GCA, or GCT;
   GCG to GCA or GCT; or
   GCA to GCT;
(ii) substituting all codons encoding an asparagine residue with a AAT codon;
(iii) substituting all codons encoding an arginine residue with a CGT codon, or substituting all codons encoding an arginine residue with a substitution selected from:
   CGG to any of AGG, CGC, AGA, CGA or CGT;
   AGG to any of CGC, AGA, CGA or CGT;
   CGC to any of AGA, CGA or CGT;
   AGA to CGA or CGT; or
   CGA to CGT;
(iv) substituting all codons encoding an aspartic acid residue with a GAT codon;
(v) substituting all codons encoding a cysteine residue with a TGT codon;
(vi) substituting all codons encoding a glutamine residue with a CAA codon;
(vii) substituting all codons encoding a glutamic acid residue with a GAA codon;
(viii) substituting all codons encoding a glycine residue with a GGT codon, or substituting all codons encoding a glycine residue with a substitution selected from:
   GGG to any of GGC, GGA or GGT;
   GGC to GGA or GGT; or
   GGA to GGT;
(ix) substituting all codons encoding a histidine residue with a CAT codon;
(x) substituting all codons encoding an isoleucine residue with a ATT codon, or substituting all codons encoding an isoleucine residue with a substitution selected from:
   ATA to ATC or ATT; or
   ATC to ATT;
(xi) substituting all codons encoding a leucine residue with a TTA codon, or substituting all codons encoding a leucine residue with a substitution selected from:
   CTC to any of CTG, CTA, CTT, TTG, or TTA;
   CTG to any of CTA, CTT, TTG, or TTA;
   CTA to any of CTT, TTG, or TTA;
   CTT to TTG or TTA; or
   TTG to TTA;
(xii) substituting all codons encoding a lysine residue with a AAA codon;
(xiii) substituting all codons encoding a phenylalanine residue with a TTT codon;
(xiv) substituting all codons encoding a proline residue with a CCA codon, or substituting all codons encoding a proline residue with a substitution selected from:
   CCC to any of CCG, CCT, or CCA;
   CCG to CCT or CCA; or
   CCT to CCA;
(xv) substituting all codons encoding a serine residue with a TCA codon, or substituting all codons encoding a serine residue with a substitution selected from:
   TCC to any of TCG, AGC, TCT, AGT, or TCA;
   TCG to any of AGC, TCT, AGT, or TCA;
   AGC to any of TCT, AGT, or TCA;
   TCT to AGT or TCA; or
   AGT to TCA;
(xvi) substituting all codons encoding a threonine residue with a ACA codon, or substituting all codons encoding a threonine residue with a substitution selected from:
   ACC to any of ACG, ACT, or ACA;
   ACG to ACT or ACA; or
   ACT to ACA;
(xvii) substituting all codons encoding a tyrosine residue with a TAT codon;
(xviii) substituting all codons encoding a valine residue with a GTA codon, or substituting all codons encoding a valine residue with a substitution selected from:
   GTG to any of GTC, GTT, or GTA;
   GTC to GTT or GTA; or
   GTT to GTA; and
(xviii) substituting all codons encoding a stop codon with a TGA codon, or substituting all codons encoding a stop codon with a substitution selected from:
   TAG to TAA or TGA; or
   TAA to TGA.

Step (e) may comprise: (i) altering a GCTGCT repeat codon in the coding sequence to a GCTGCA or a GCAGCT sequence; (ii) altering a GCAGCA repeat codon in the coding sequence to a GCTGCA or a GCAGCT sequence; (iii) altering a CGTCGT repeat codon in the coding to a CGTCGA or a CGACGT sequence; (iv) altering a CGACGA repeat codon in the coding to a CGTCGA or a CGACGT sequence; (v) altering a CAACAA repeat codon in the coding to a CAACAG or a CAGCAA sequence; (vi) altering a CAGCAG repeat codon in the coding to a CAACAG or a CAGCAA sequence; (vii) altering a CATCAT repeat codon in the coding to a CATCAC or a CACCAT sequence; (viii) altering a CACCAC repeat codon in the coding to a CATCAC or a CACCAT sequence; (ix) altering a ATTATT repeat codon in the coding to a ATTATC or a ATCATT sequence; (x) altering a ATCATC repeat codon in the coding to a ATTATC or a ATCATT sequence; (xi) altering a TTATTA repeat codon in the coding to any of a TTATTG, TTACTA, TTGTTA, TTGCTA, CTATTA, or a CTATTG sequence; (xii) altering a TTGTTG repeat codon in the coding to any of a TTATTG, TTACTA, TTGTTA, TTGCTA, CTATTA, or a CTATTG sequence; (xiii) altering a CTACTA repeat codon in the coding to any of a TTATTG, TTACTA, TTGTTA, TTGCTA, CTATTA, or a CTATTG sequence; (xiv) altering a CCTCCT repeat codon in the coding to a CCTCCA or a CCACCT sequence; (xv) altering a CCACCA repeat codon in the coding to a CCTCCA or a CCACCT sequence; (xvi) altering a AGTAGT repeat codon in the coding to a AGTTCA or a TCAAGT sequence; (xvii) altering a TCATCA repeat codon in the coding to a AGTTCA or a TCAAGT sequence; (xviii) altering a ACAACA repeat codon in the coding to a ACAACT or a ACTACA sequence; (xix) altering a ACTACT repeat codon in the coding to a ACAACT or a ACTACA sequence; (xx) altering a GTTGTT repeat codon in the coding to a GTTGTA or a GTAGTT sequence; or (xxi) altering a GTAGTA repeat codon in the coding to a GTTGTA or a GTAGTT sequence.

Step (e) may comprise: (i) where a first and a second GCT codon are separated by one to five intervening codons, replacing the first or second GCT codon with a GCA codon; (ii) where a first and a second GCA codon are separated by one to five intervening codons, replacing the first or second GCA codon with a GCT codon; (iii) where a first and a second CGT codon are separated by one to five intervening codons, replacing the first or second CGT codon with a CGA codon; (iv) where a first and a second CGA codon are separated by one to five intervening codons, replacing the first or second CGA codon with a GCT codon; (v) where a first and a second CAA codon are separated by one to five intervening codons, replacing the first or second CAA codon with a CAG codon; (vi) where a first and a second CAG codon are separated by one to five intervening codons, replacing the first or second CAG codon with a CAA codon; (vii) where a first and a second CAT codon are separated by one to five intervening codons, replacing the first or second CAT codon with a CAC codon; (viii) where a first and a second CAC codon are separated by one to five intervening codons, replacing the first or second CAC codon with a CAT codon; (ix) where a first and a second ATT codon are separated by one to five intervening codons, replacing the first or second ATT codon with a ATC codon; (x) where a first and a second ATC codon are separated by one to five intervening codons, replacing the first or second ATC codon with a ATT codon; (xi) where a first and a second TTA codon are separated by one to five intervening codons, replacing the first or second TTA codon with a TTG codon or a CTA codon; (xii) where a first and a second TTG codon are separated by one to five intervening codons, replacing the first or second TTG codon with a TTA codon or a CTA codon; (xiii) where a first and a second CTA codon are separated by one to five intervening codons, replacing the first or second CTA codon with a TTA codon or a TTG codon; (xiv) where a first and a second CCT codon are separated by one to five intervening codons, replacing the first or second CCT codon with a CCA codon; (xv) where a first and a second CCA codon are separated by one to five intervening codons, replacing the first or second CCA codon with a CCT codon; (xvi) where a first and a second AGT codon are separated by one to five intervening codons, replacing the first or second AGT codon with a TCA codon; (xvii) where a first and a second TCA codon are separated by one to five intervening codons, replacing the first or second TCA codon with a AGT codon; (xviii) where a first and a second ACA codon are separated by one to five intervening codons, replacing the first or second ACA codon with a ACT codon; (xix) where a first and a second ACT codon are separated by one to five intervening codons, replacing the first or second ACT codon with a ACA codon; (xx) where a first and a second GTT codon are separated by one to five intervening codons, replacing the first or second GTT codon with a GTA codon; or (xxi) where a first and a second GTA codon are separated by one to five intervening codons, replacing the first or second GTA codon with a GTT codon.

The coding sequence may be functionally linked to a 5'UTR.

The coding sequence may be functionally linked to a 3'UTR.

The nucleic acid may be an RNA sequence.

The nucleic acid sequence may comprise a coding sequence encoding the polypeptide is a bacterial sequence.

The nucleic acid sequence may comprise a coding sequence encoding the polypeptide is a archaeal sequence.

The nucleic acid sequence may comprise a coding sequence encoding the polypeptide is a eukaryotic sequence.

The nucleic acid sequence may comprise a coding sequence encoding the polypeptide is a sequence of synthetic origin.

The expression system may be an *in vitro* expression system.

The expression system may be a bacterial expression system.

The expression system may be a eukaryotic expression system.

The *in vitro* expression system may be a cell-free transcription/translation system.

The expression system may be an *in vivo* expression system.

The *in vivo* expression system may be a bacterial expression system or a eukaryotic expression system.

The *in vivo* expression system may be an E. coli cell.

The *in vivo* expression system may be a mammalian cell.

The recombinant polypeptide may be a human polypeptide, or a fragment thereof.

The recombinant polypeptide may be a viral polypeptide, or a fragment thereof.

The recombinant polypeptide may be an antibody, an antibody fragment, an antibody derivative, a diabody, a tribody, a tetrabody, an antibody dimer, an antibody trimer or a minibody.

The antibody fragment may be a Fab fragment, a Fab' fragment, a F(ab)2 fragment, a Fd fragment, a Fv fragment, or a ScFv fragment.

The recombinant polypeptide may be a cytokine, an inflammatory molecule, a growth factor, a cytokine receptor, an inflammatory molecule receptor, a growth factor receptor, an oncogene product, or any fragment thereof.

The disclosure relates to a recombinant polypeptide produced according to the methods described herein.

The disclosure relates to a pharmaceutical composition comprising the recombinant polypeptide produced according to the methods described herein.

The disclosure relates to an immunogenic composition comprising the recombinant polypeptide produced according to the methods described herein.

### BRIEF DESCRIPTION OF THE FIGURES

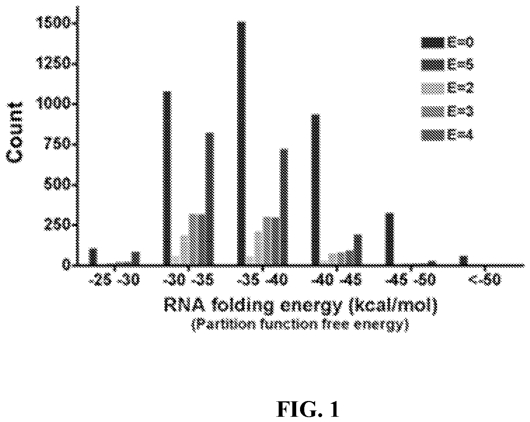

**Figure 1** shows calculation windows containing the 5'-UTR plus the first 50 bases of the coding sequence

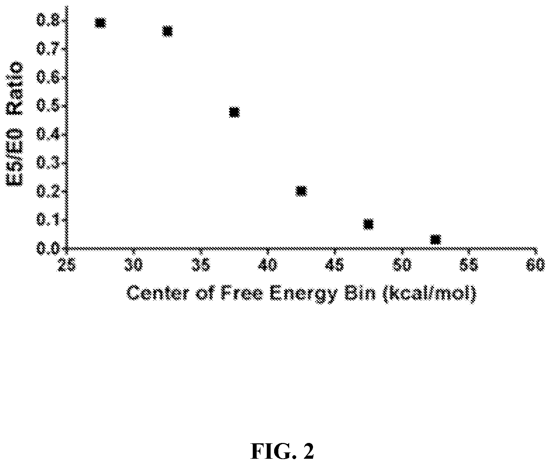

**Figure 2** shows folding energy threshold correlated to polypeptide expression levels

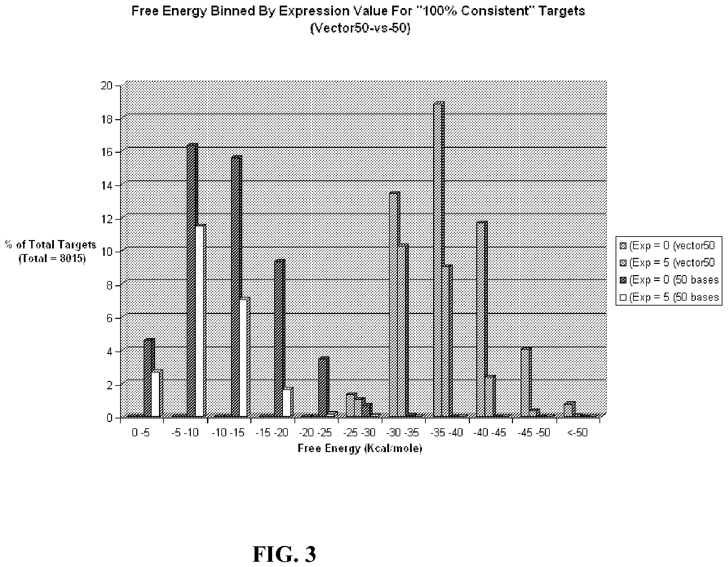

**Figure 3** shows free energy binned by expression value for "100% consistent" targets (Vector50 vs 50)

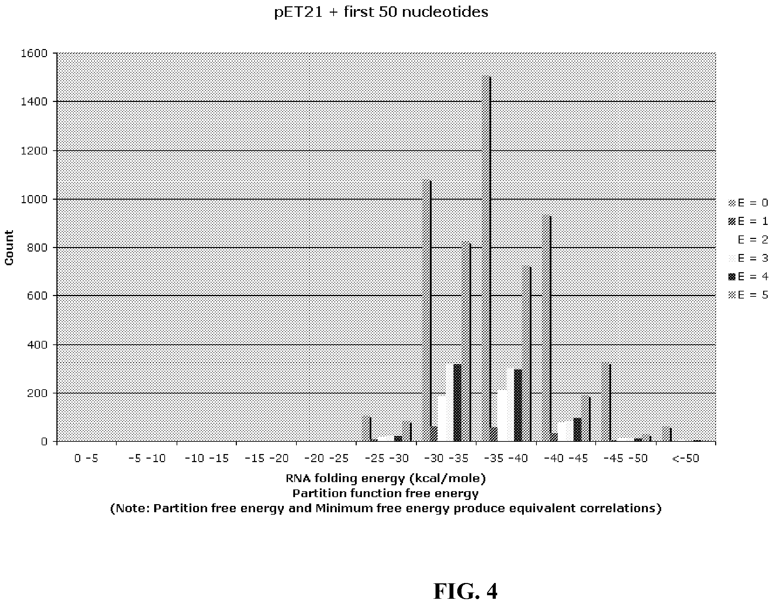

**Figure 4** shows RNA folding energies for pET21 + first 50 nucleotides

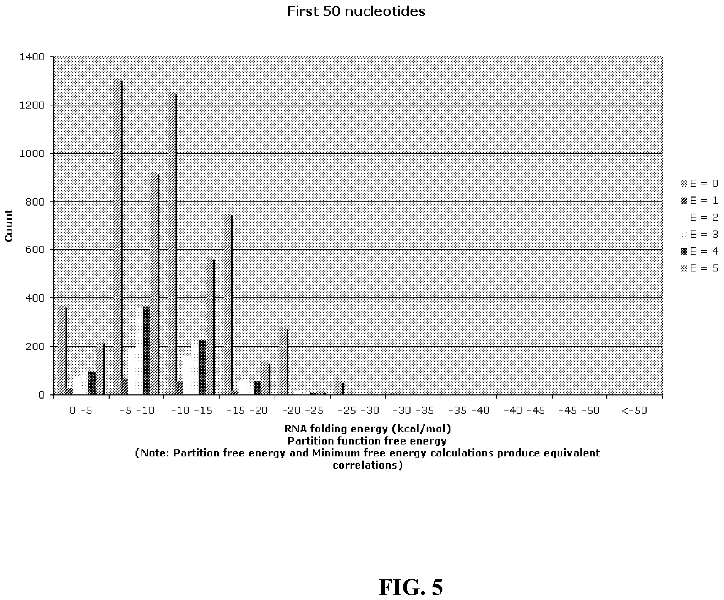

**Figure 5** shows RNA folding energies for first 50 nucleotides

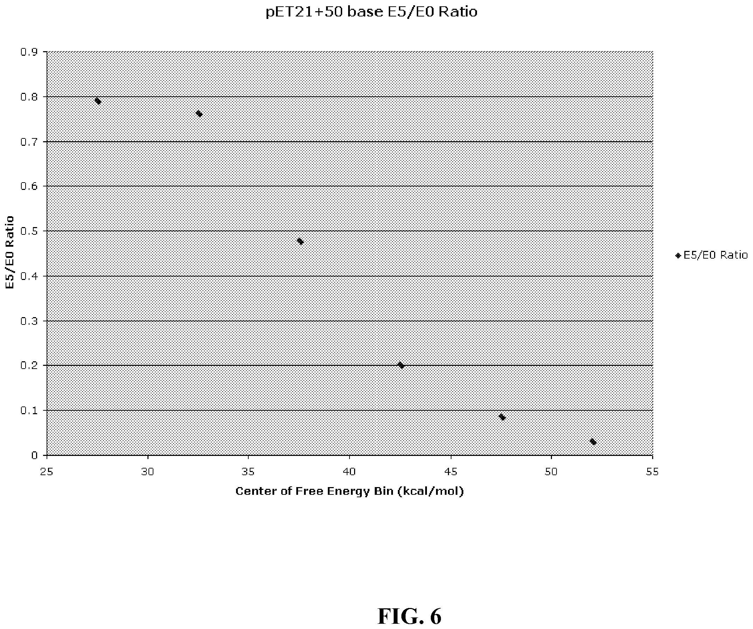

**Figure 6** shows the E5/E0 ratio for pET21+50 base

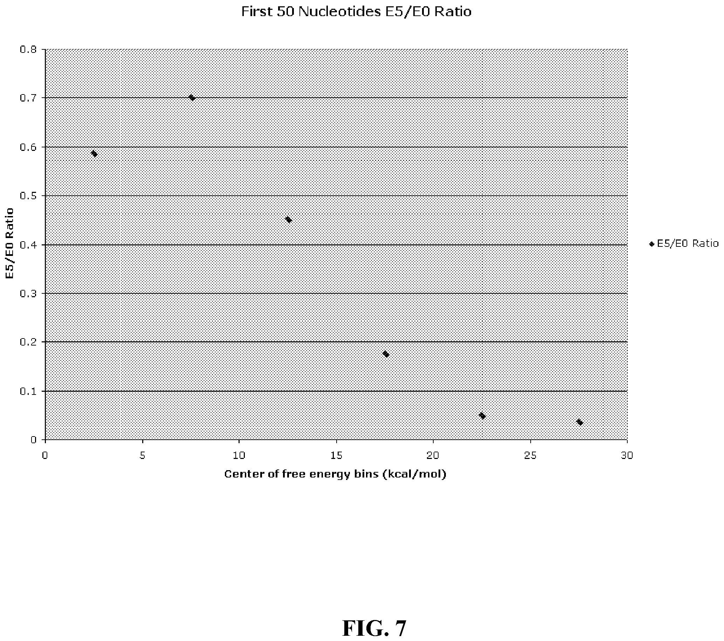

**Figure 7** shows the E5/E0 ratio for first 50 nucleotides

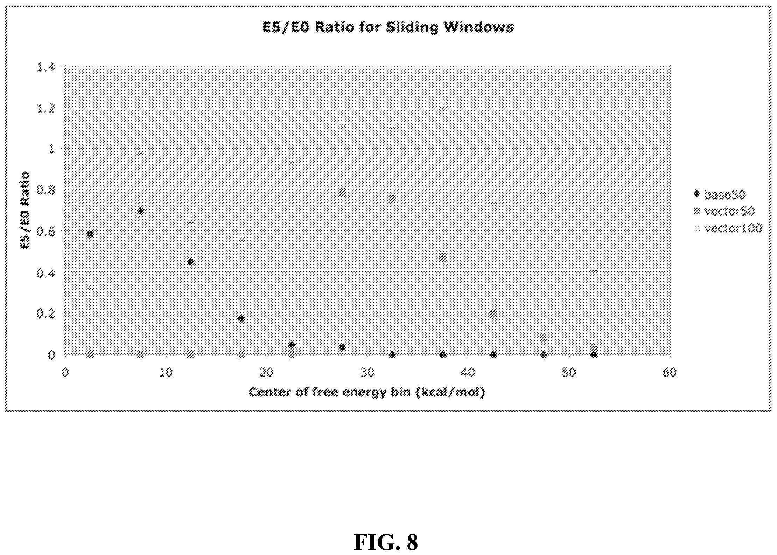

**Figure 8** shows E5/E0 ration for sliding windows

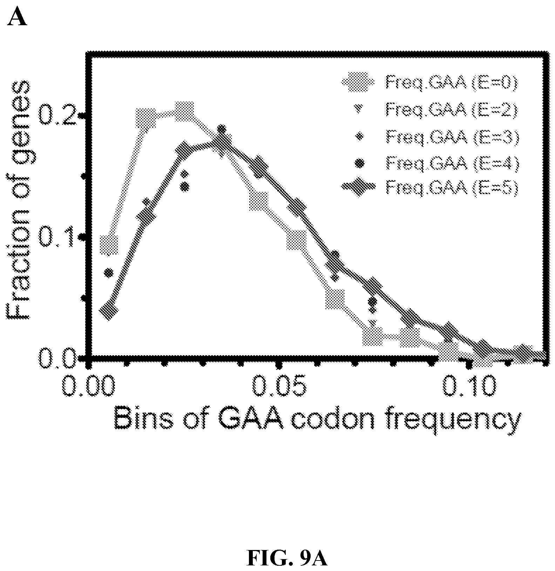

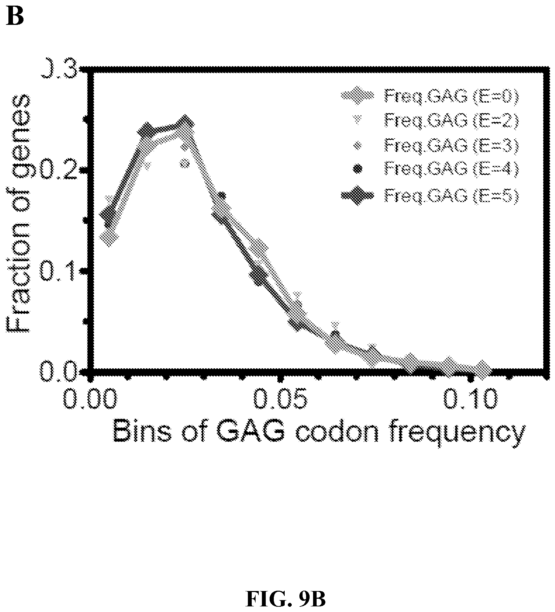

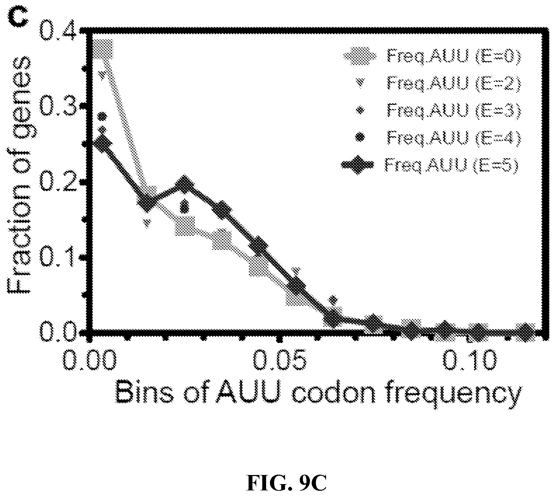

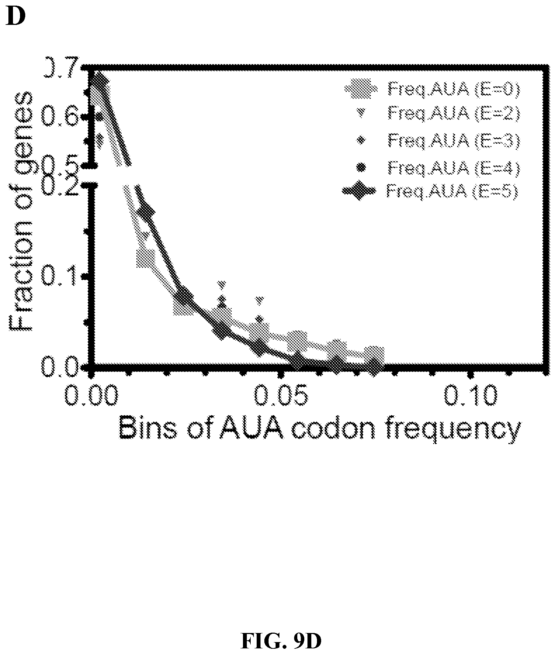

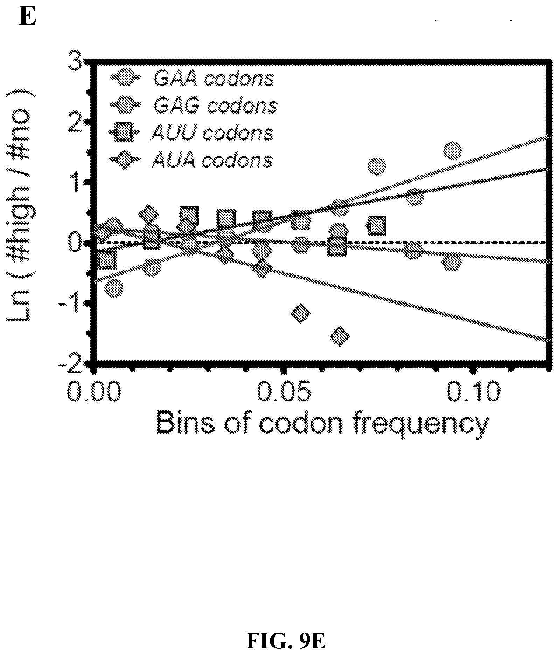

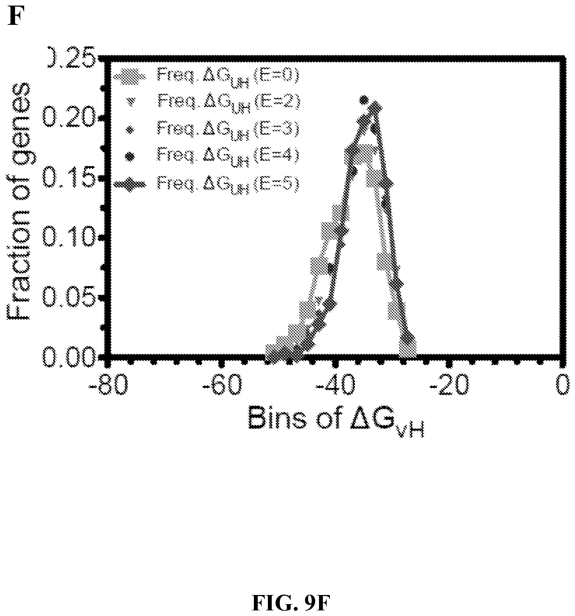

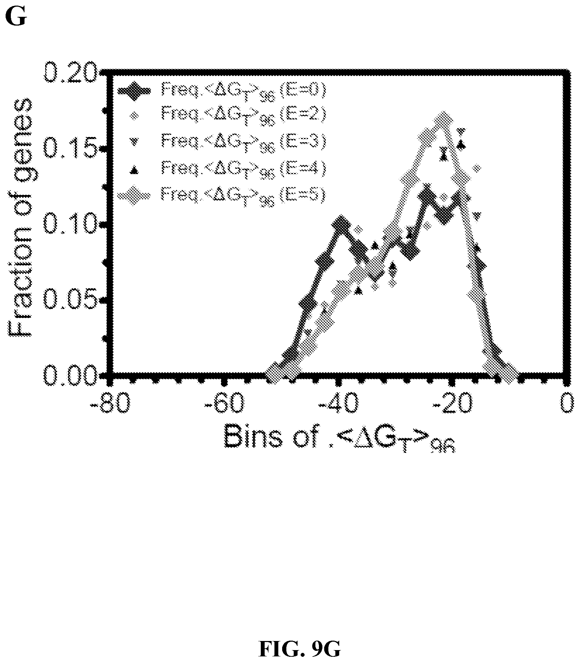

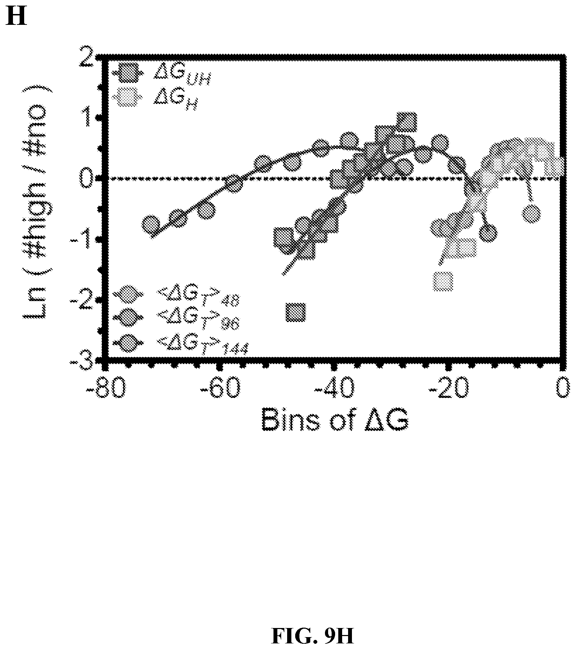

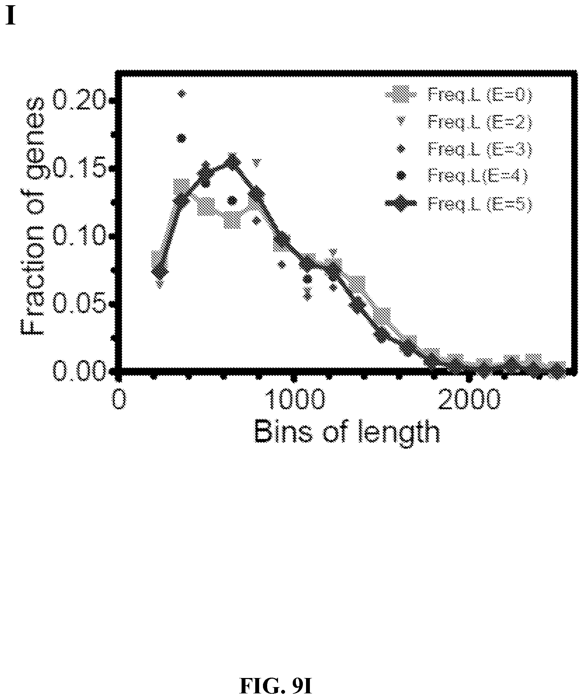

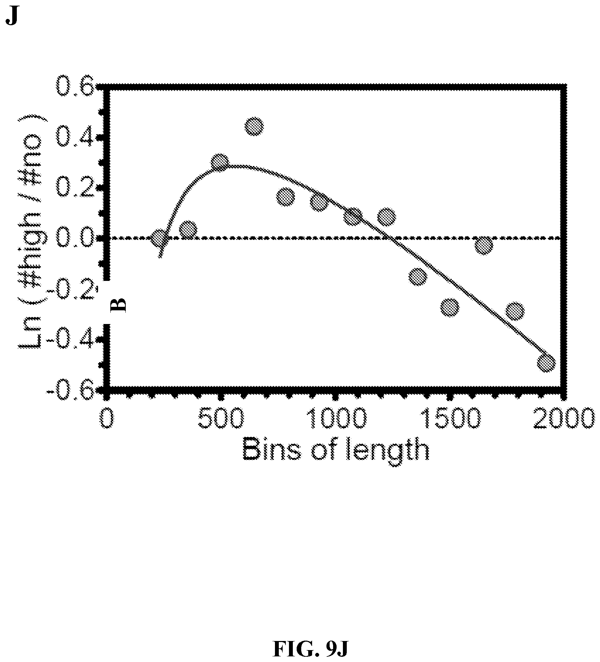

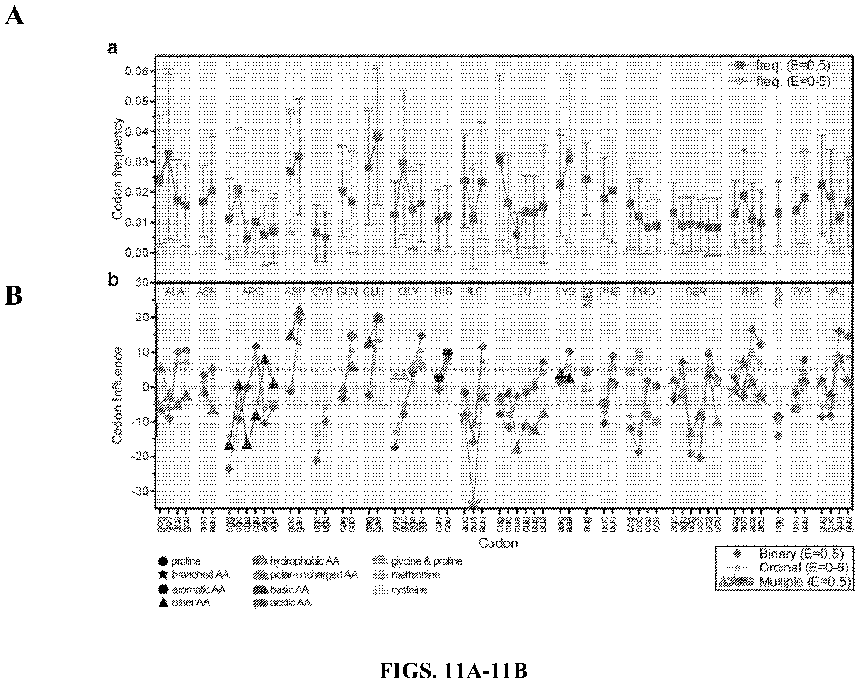

**Figures 9A-9J** show the distributions of representative RNA sequence parameters in different protein-expression categories in the large-scale dataset. **Figure 9A** and **Figure 9B** are histograms showing the frequencies of two Glu codons (GAA in **Figure 9A** and GAG in **Figure 9B****).** **Figure 9C** and **Figure 9D** are histograms showing the frequencies of two Ile codons (AUU in **Figure 9C** and AUA in **Figure 9D****).** **Figure 9F** is a histogram showing the partition-function free energy of folding in the 5'-UTR from the expression vector plus the initial 16 codons or "head" of each gene (ΔG_{UH}). **Figure 9G** is a histogram showing the average partition-function free energy of folding in the remainder or "tail" of each gene in 50% overlapping windows with widths of length *w* (<ΔG_{T}>₉₆). **Figure 9I** is a histogram showing the length of the protein-coding sequences in nucleotides. The parameter distributions in the E = 0 and E = 5 categories are shown in light and dark color respectively, while those in the E = 1-4 bins are shown in shades of gray. **Figures 9E****,** **9H** **and** **9J** show "log-odds" plots of the logarithm of the ratio of the number of proteins in the E5 *vs.* E0 categories in bins of parameter values. The solid lines show the results of single-variable binary logistic regression (*i*.*e*., linear least-squares fitting of the data in this format), which yields the codon slope values shown in **Figure 11B****.**

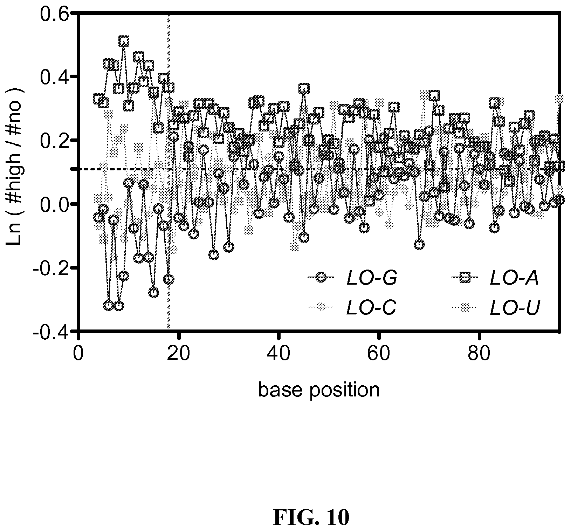

**Figure 10** shows the logarithm of the ratio of the number of proteins in the E5 vs. E0 categories for proteins encoded by the indicated nucleotide base at positions 3-96 in their coding sequences. G, C, A, and U represent guanine, cytidine, adenine, and uracil bases, respectively. Positions are numbered starting from the A of the AUG initiation codon. The gray dotted line indicates the approximate region protected by the ribosome in the 70S initiation complex.

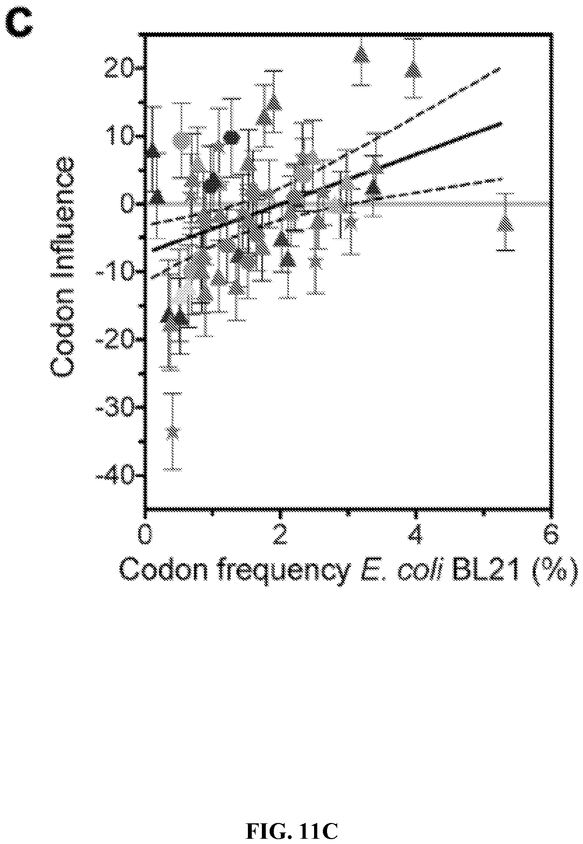

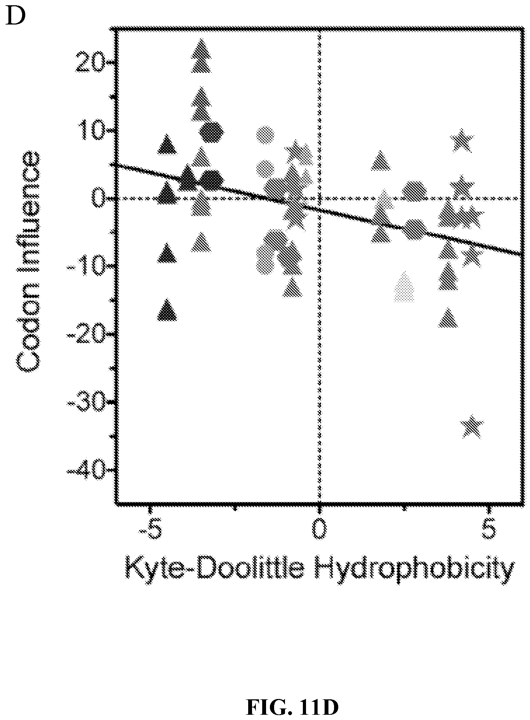

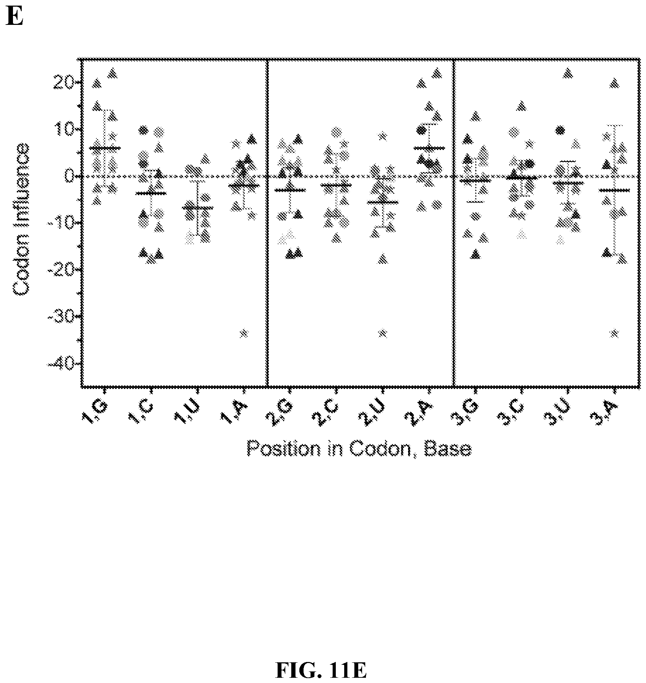

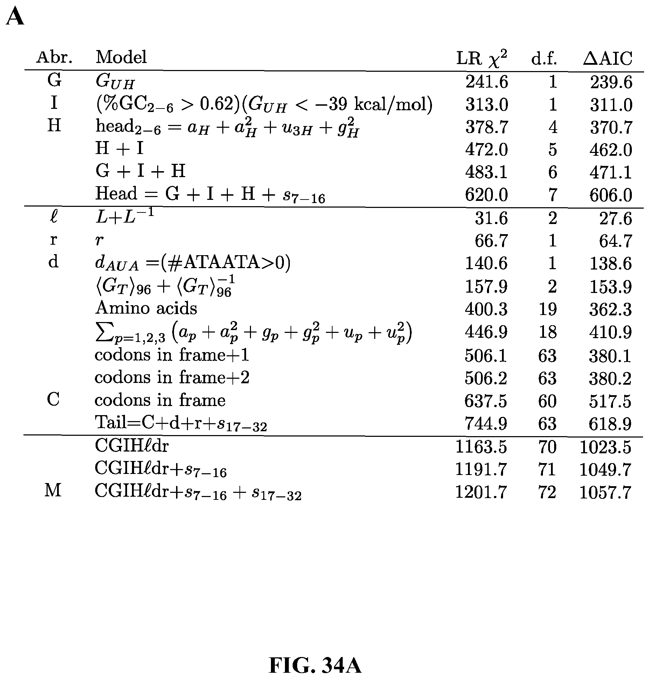

**Figures 11A-11E** show the codon influence on protein expression in the large-scale dataset. **Figure 11A** shows a plot of the frequencies of each non-stop codon in the genes in the E = 0 plus E = 5 categories (dark gray) and in the E = 0-5 categories (light gray). Error bars represent the sample variance of the frequency distributions. **Figure 11B** shows the slopes for every non-stop codon from single parameter binary logistic regression analyses of proteins in the E = 0 vs. E = 5 categories (dark gray), single parameter ordinal logistic regression analyses of proteins in the E = 0-5 categories (light gray), and simultaneous multi-parameter binary logistic regression analysis of proteins in the E = 0 vs. E = 5 categories from model M in **Figures 34A-34B** (colored symbols). Blue symbols represent basic residues, red symbols represent acidic residues, magenta symbols represent polar uncharged residues, dark green symbols represent hydrophobic residues, light green symbols represent glycine and proline residues, the orange symbol represents methionine, and the yellow symbol represents cysteine. Stars (★) represent β-branched residues, hexagons represent aromatic residues, circles represent proline (•), and triangles (Δ) represent all other residues. **Figures 11C****,** **11D** **and** **11E** show the codon slopes from the multi-parameter binary logistic regression analysis plotted against codon usage frequency in the genome of *E. coli* BL21 (**Figure 11C**), the Kyte-Doolittle hydrophobicity of the corresponding amino acid (**Figure 11D**), and the nucleotide base at each of the three positions in the codon (**Figure 11E**).

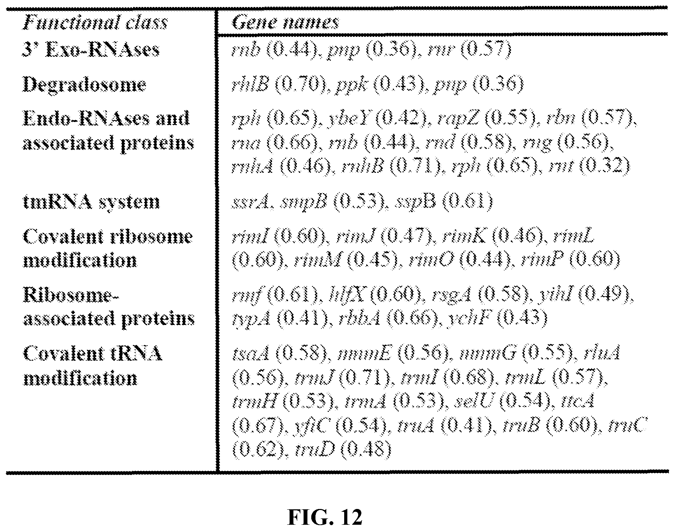

**Figure 12** shows representative candidate genes that will be evaluated for their influence on synonymous codon usage effects. Knockout strains for these non-essential genes are available in the KEIO Collection, which is distributed by the E. coli Genetic Stock Center at Yale University. The number in parentheses following the gene name gives the log-phase growth rate of the corresponding knockout strain in LB liquid medium, expressed as a fraction of the rate of the matched wild-type strain under the same conditions.

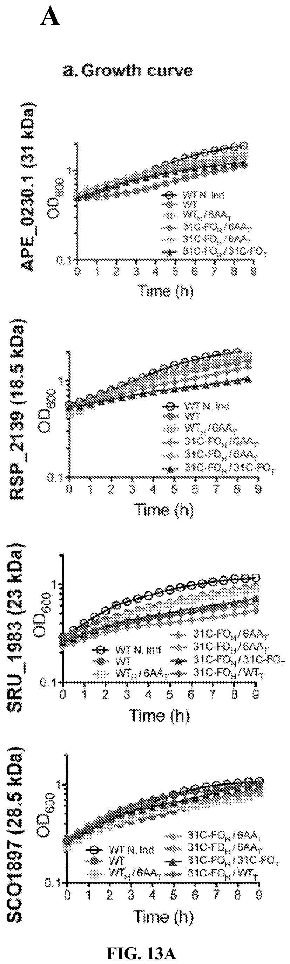

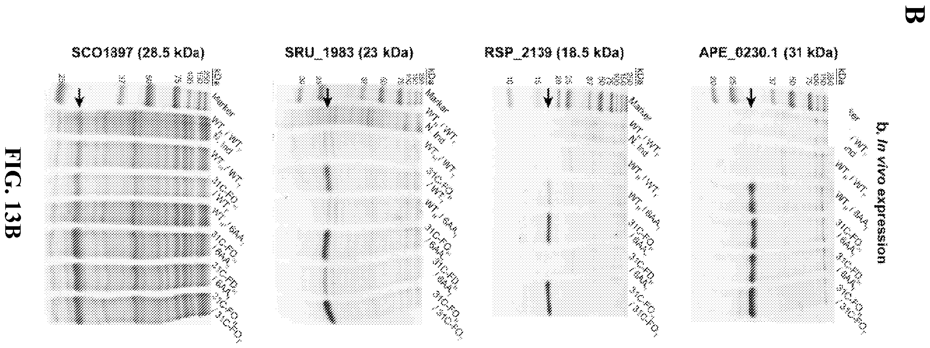

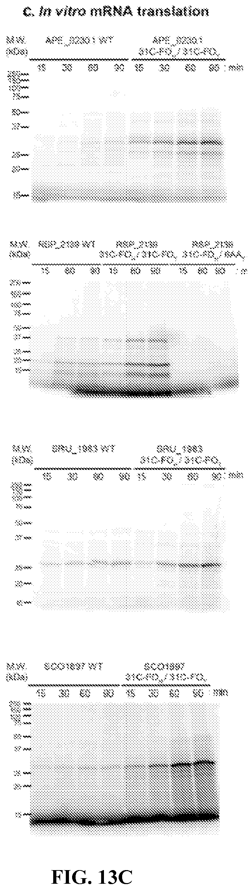

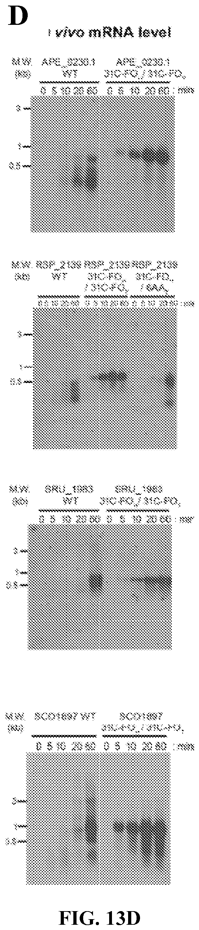

**Figures 13A-13D** shows the experimental evaluation of the expression of synthetic genes designed to enhance protein expression. **Figures 13A-13D** show comparisons of the *in vivo* and *in vitro* expression properties of inefficiently translated native (WT) genes and synonymous genes redesigned in the head or the tail or both using the 6AA, 31C folding optimized (31C-FO), or 31C folding deoptimized (31C-FD) methods. The type of sequence in the head (subscript H) is indicated separately from that in the tail (subscript T), and the name of the target protein is indicated on the left on each row. Non-induced controls for *in vivo* experiments are labeled "N. Ind.". **Figure 13A** shows *E. coli* BL21(DE3) host cell growth curves at room temperature after induction of the target gene at time zero. **Figure 13B** shows Coomasie Blue stained SDS-PAGE gels of whole cells following overnight induction at 18 °C. The amount loaded in each lane was normalized to the OD₆₀₀ of the culture at the time of harvest. The black arrow in the left-most lane with the molecular weight markers indicates the migration position of the target protein. **Figure 13C** shows autoradiographs of SDS-PAGE gels of *in vitro* translation reactions using fully purified translation components in the presence of [³⁵S]-methionine. Each reaction contained an equal amount of purified mRNA that was transcribed *in vitro* using T7 RNA polymerase. The bands at higher molecular weight than the target protein represent SDS-resistant oligomers. **Figure 13D** shows northern blot analyses of the mRNA for the target protein after induction of expression *in vivo.* An equal amount of total RNA was loaded in each lane, and the blots were hybridized with a probe matching the 5'UTR.

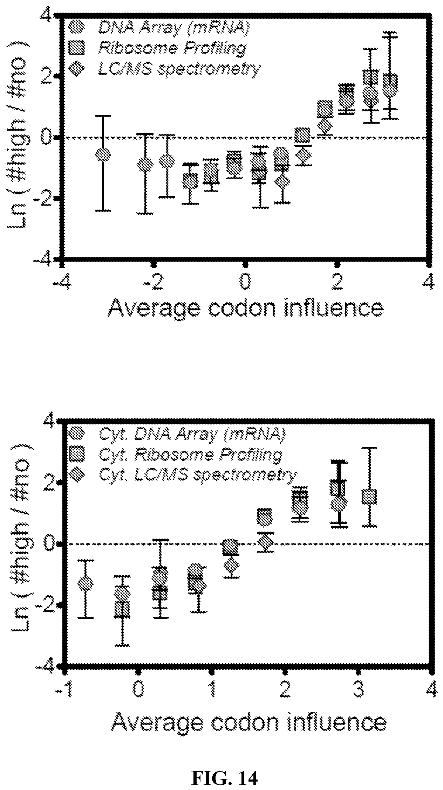

**Figure 14** shows the correlation between codon influence from logistic regression analyses and both endogenous mRNA and protein levels in E. coli. The average value of the codon influence (logistic-regression slope shown in **Figure 11A**) was calculated for all genes in *E. coli,* which were binned according to this value. For each resulting bin, the plots show the natural logarithm of the ratio of the number of genes/proteins in the top vs. bottom thirds of the levels observed in previous genome-scale *in vivo* profiling studies conducted on log-phase *E. coli* cells growing in chemically defined liquid media. The cyan, magenta, and red traces show data from a microarray analysis of mRNA concentration, a deep-sequencing analysis of ribosome occupancy of mRNA sequences, and a mass spectrometric analysis of protein concentration, respectively. The plot on the left shows data for all proteins encoded in the *E. coli* genome while that on the right is restricted to those predicted by the program LipoP to be localized in the cytosol.

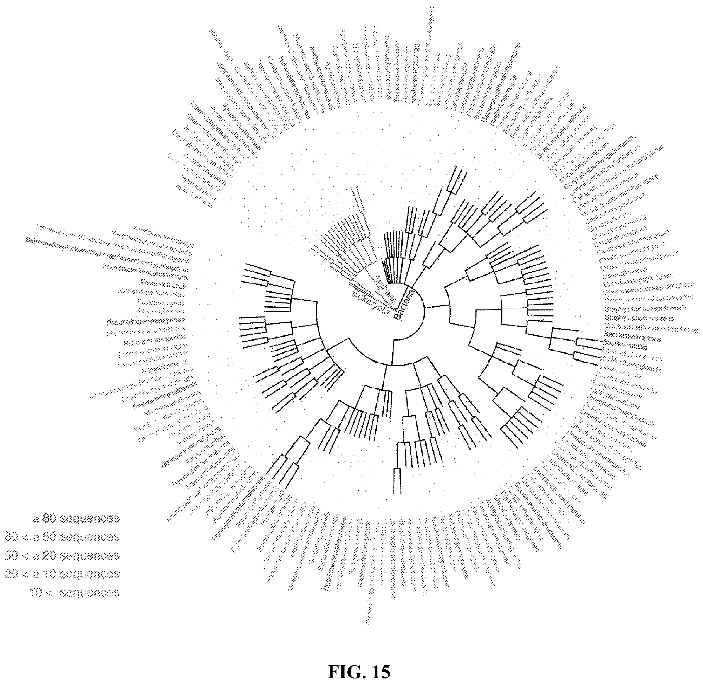

**Figure 15** shows the phylogenic distributions of the proteins present in in the large-scale protein expression dataset.. The colors in the cladogram encode the number of proteins from each organism, as indicated by the legend. The dataset includes 47 from eukaryotes (45 from humans and 2 from mouse), 809 from archaebacteria, and 96 from E. *coli,* with the remainder coming from other eubacteria. The organism contributing the largest number of proteins to the dataset is the eubacterium *Bacteroides thetaiotaomicron* (150 proteins).

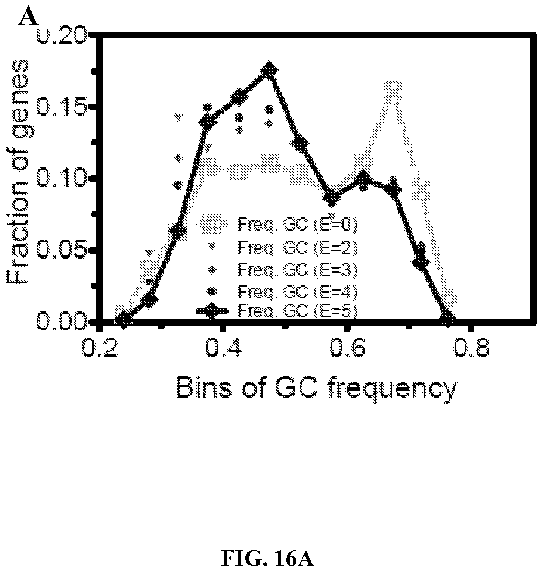

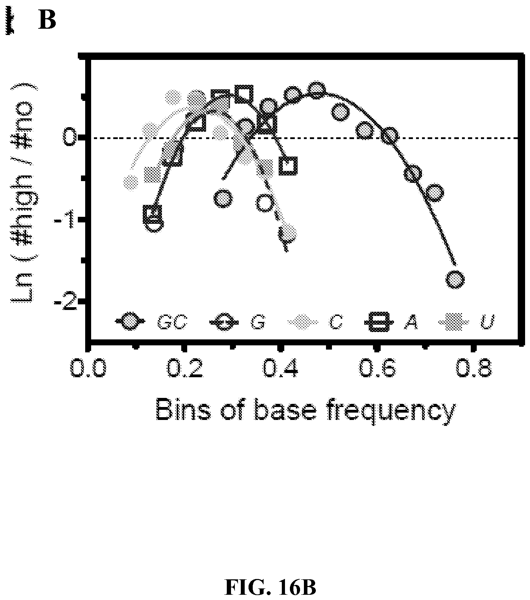

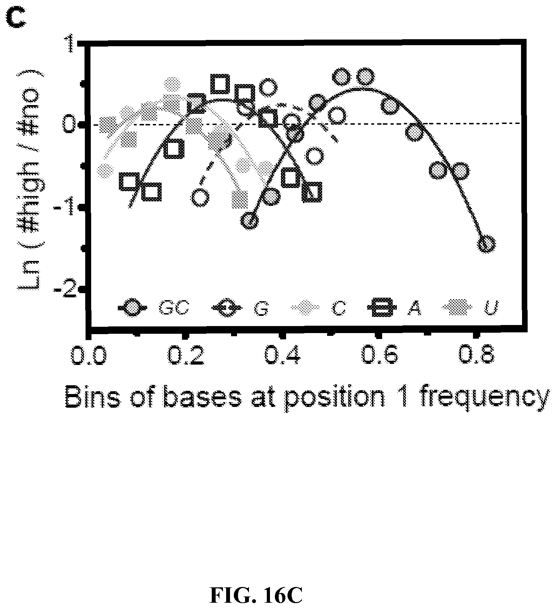

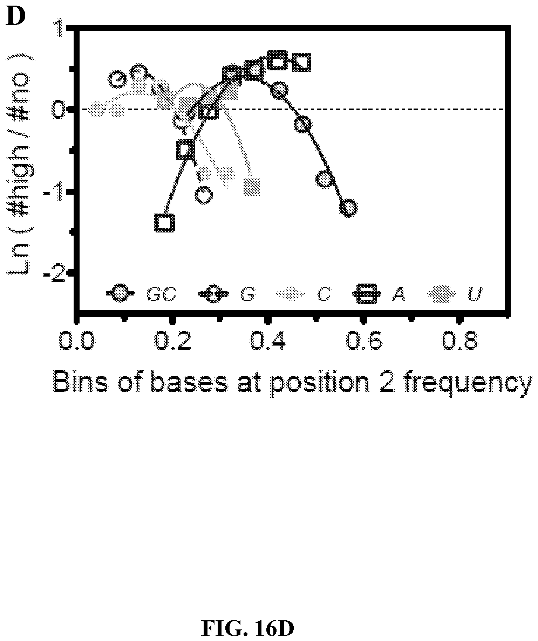

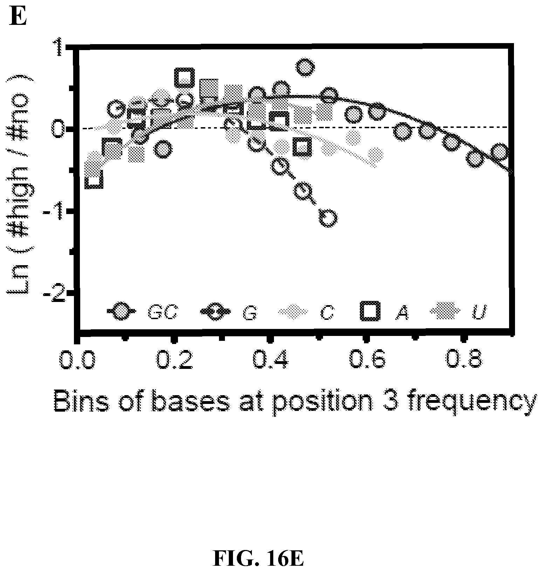

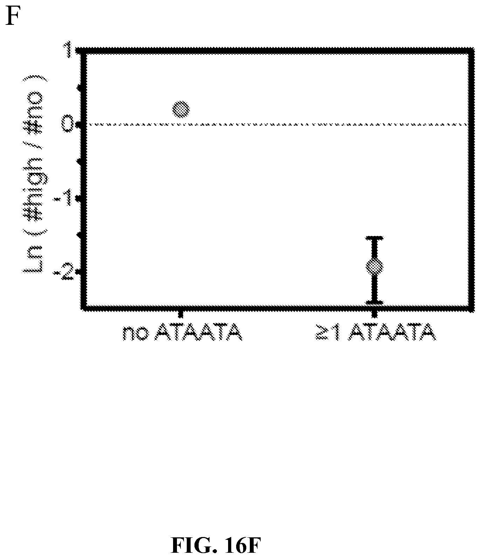

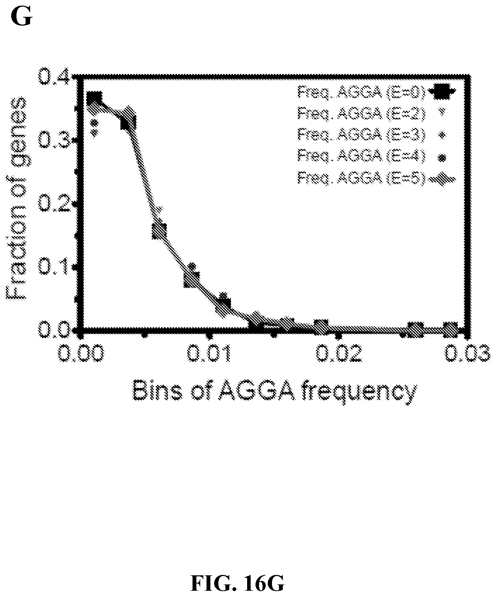

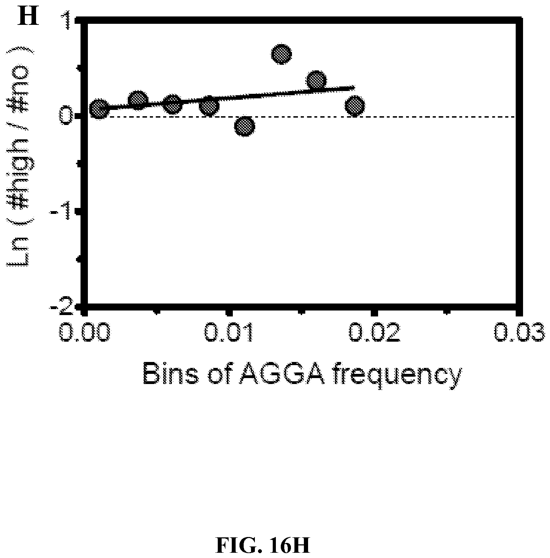

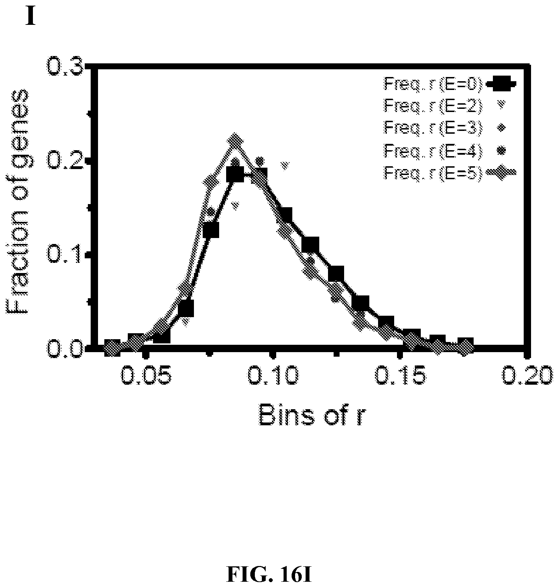

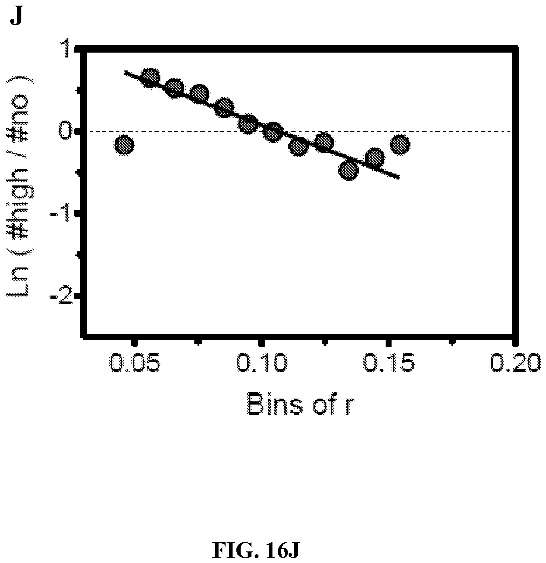

**Figures 16A-16J** show the distributions of additional mRNA sequence parameters at different expression levels in the large-scale protein expression dataset. Parameter distributions were calculated from the 6,348 genes included in the dataset. **Figure 16A** is a histogram showing the overall G+C frequency, **Figure 16G** is a histogram showing the AGGA sequence frequency in all reading frames, and **Figure 16I** is a histogram showing the frequency of codon repetition rate r. The parameter distributions in the E = 0 and E = 5 categories are shown in **Figure 16A** in dark and light blue, respectively, and in **Figure 16G** and **Figure 16I** in red and black, respectively. The symbols used for the histograms for the intermediate expression scores are indicated in the legend for each figure. **Figures 16B-16F**, **16H****, and** **Figure16** **J** show the logarithm of the ratio of the number of proteins in the E5 vs. E0 categories in bins of parameter values. **Figure 16B** shows data for the overall frequencies of the four individual nucleotide bases as well as the combined G+C frequency (labeled GC), while **Figures 16C-16E** show respectively the equivalent data separately for the 1^{st}, 2^{nd}, and 3^{rd} positions in the codons in the genes. **Figure 16F** shows data for genes either not containing or containing at least one occurrence of the ATA·ATA dicodon. The error bars in this figure represent 95% confidence limits calculated from bootstrapping (with the error bars for the genes without any occurrence of this di-codon being smaller than the size of the symbol). **Figure 16J** shows data for the codon-repetition rate *r*.

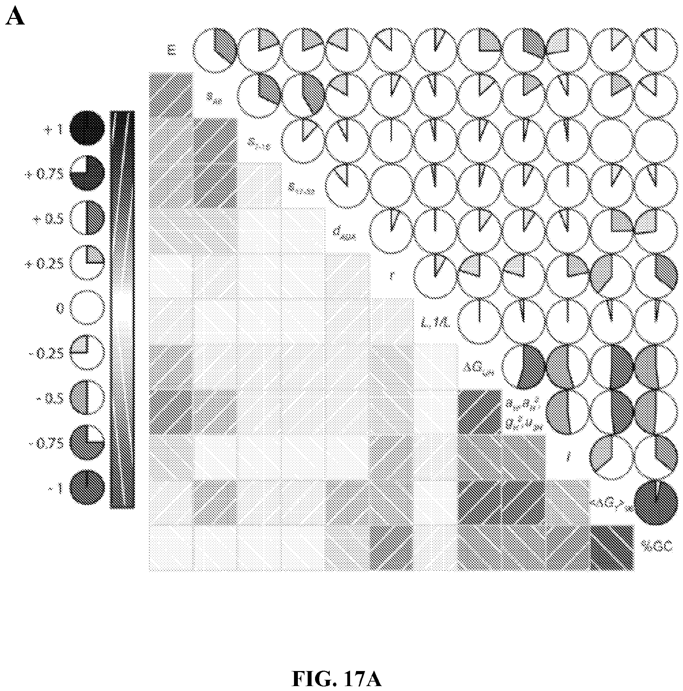

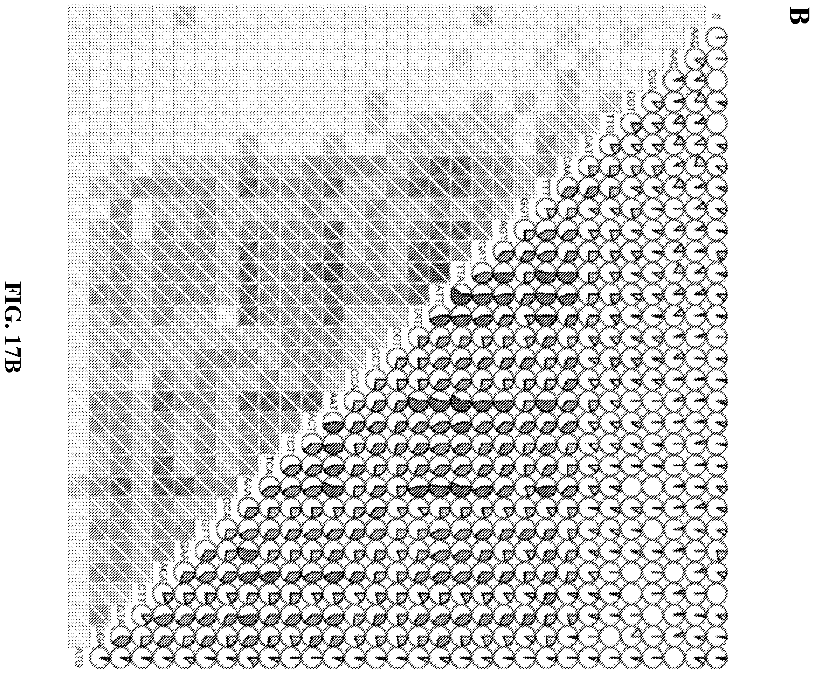

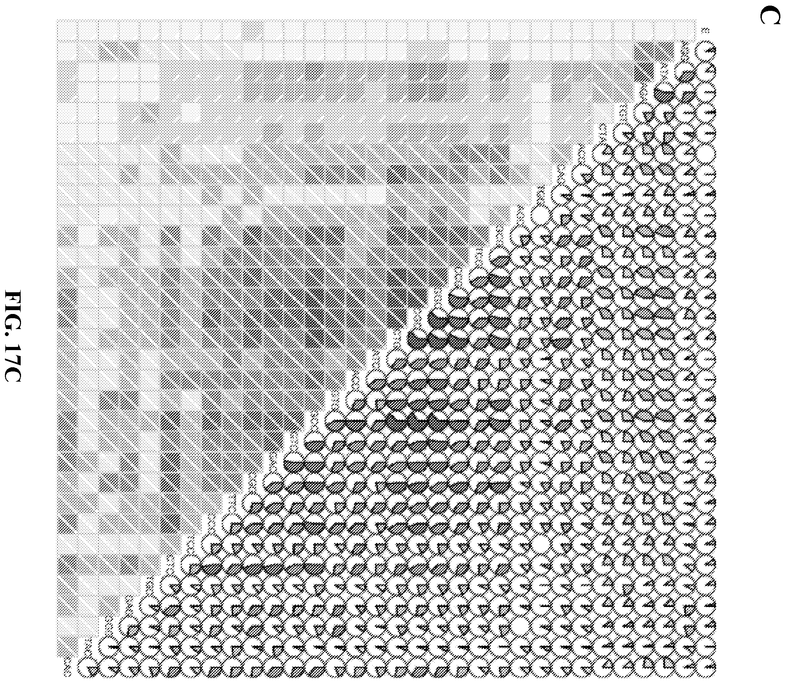

**Figures 17A-17C** show correlations between sequence parameters in the genes included in the large-scale protein expression dataset. Corrgrams representing the signed Pearson correlations coefficients between different mRNA sequence parameters in the genes included in the dataset. The color-coding is defined schematically on the left of **Figure 17A****,** with blue being used for positively correlated variables, red for negatively correlated variables, and white for uncorrelated variables. In **Figures 17A****,** E represents the expression score in the binary categories (0,5), *s_{All}* represents the mean value of the new codon-influence metric (colored symbols in **Figure 11B**) over the entire gene (without the LEHHHHHH tag), *s*₇₋₁₆ and *s*₁₇₋₃₂ represent the mean values of this metric for codons 7-16 and 17-32, respectively, Δ*G_{UH}* represents the predicted free energy of mRNA folding for the 5'-UTR from the pET21 expression vector plus the first 48 nucleotides in the gene, <Δ*G_{T}*>₉₆ represents the mean value in the remainder of the gene of the predicted free energy of folding in 50% overlapping windows of 96 nucleotides, *I* represents an indicator variable that assumes a value of 0 or 1 if (Δ*G_{UH}* < -39 kcal/mol) and (%GC₂₋₆ > 0.65), *d_{AUA}* assumes a value of 0 or 1 if there is at least one occurrence of the ATA.ATA dicodon, r represents the codon repetition rate (see *Online Methods*), and %GC represents the percentage content of G plus C bases in the gene. The variables *a_{H}*, *a_{H}²*, *g_{H}²*, and *u_{3H}* represent monomial functions of the A, G, and U base content in codons 2-6. **Figure 17B** shows data for the frequencies of the codons positively correlated with E, whereas **Figure 17C** shows data for the frequencies of the codons positively correlated with E.

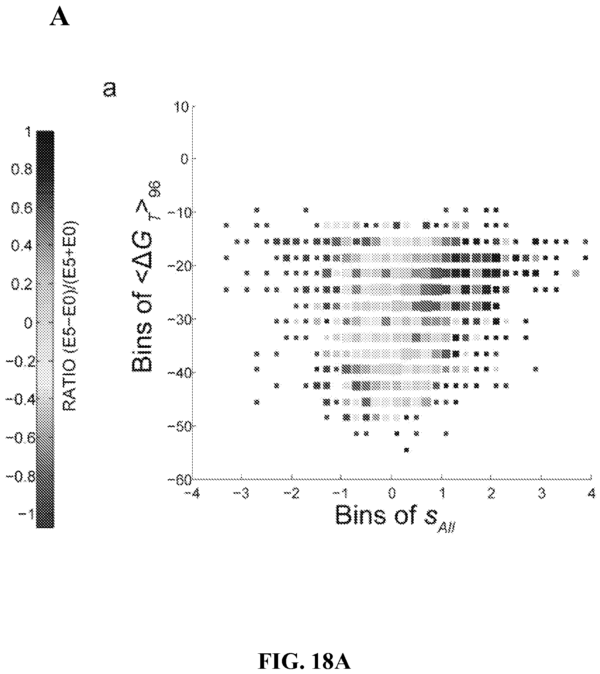

**Figures 18A-18D** show two-dimensional histograms to illustrate the dependence of outcome in the large-scale protein-expression dataset on pairs of sequence parameters. The color of each square encodes the fractional excess of proteins in the E = 5 *vs.* E = 0 categories in that bin (*i*.*e*., (#E5-#E0)/ (#E5+#E0)), as calibrated by the scale bar on the right. The area of each square is proportional to the square root of the number of proteins included in each bin, which approximately tracks the statistical significance of the data points. The variables *s_{All}*, s₇₋₁₆, and sTail represent the mean values of the new codon-influence metric (colored symbols in **Figure 11B**) for the entire gene, for codons for 7 through 16, and for all of the remaining codons downstream in the gene. Δ*G_{UH}* represents the predicted free energy of mRNA folding for the 5'-UTR from the pET21 expression vector plus the first 48 nucleotides in the gene, <Δ*G_{T}*>₉₆ represents the mean value in the remainder of the gene of the predicted free energy of folding in 50% overlapping windows of 96 nucleotides, and r represents the codon repetition rate.
   2D distributions of the protein present in the high-through protein-expression dataset.

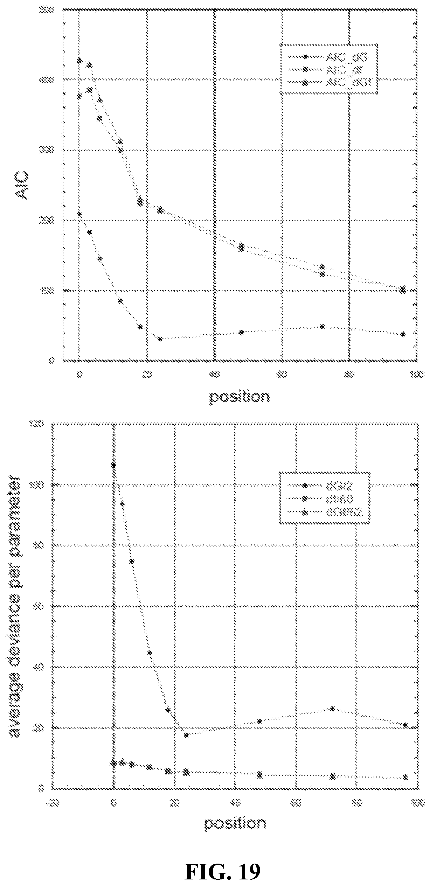

**Figure 19** shows the parameters influence by position in the mRNA.

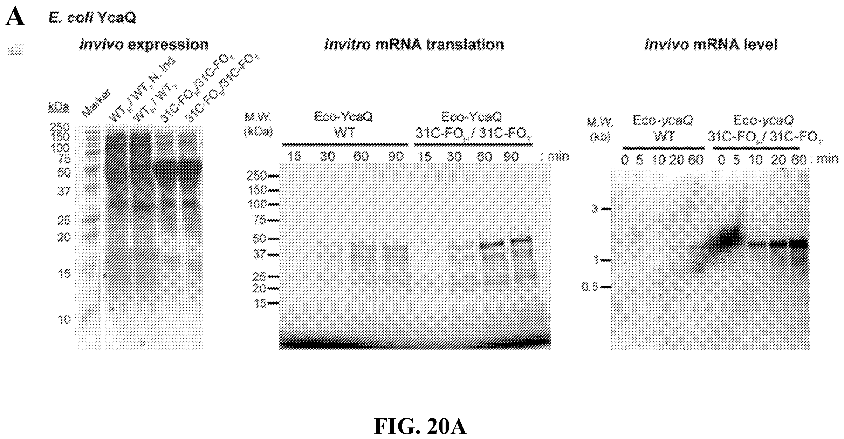

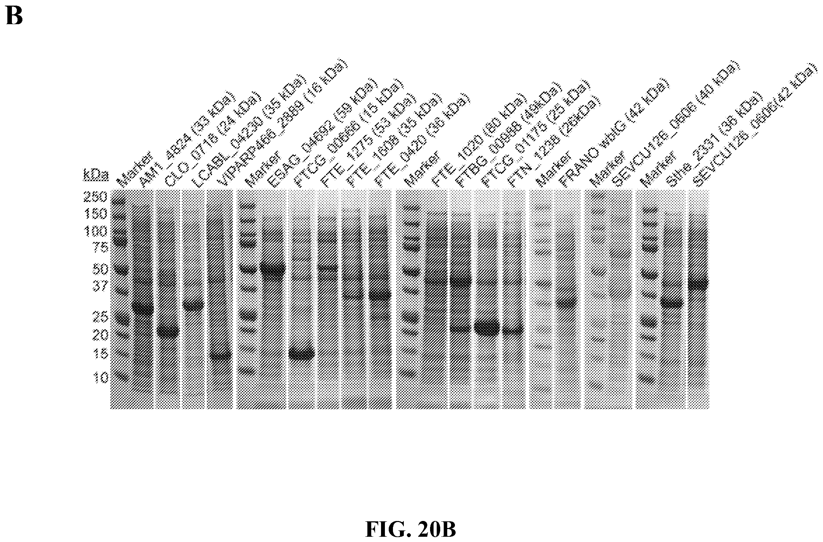

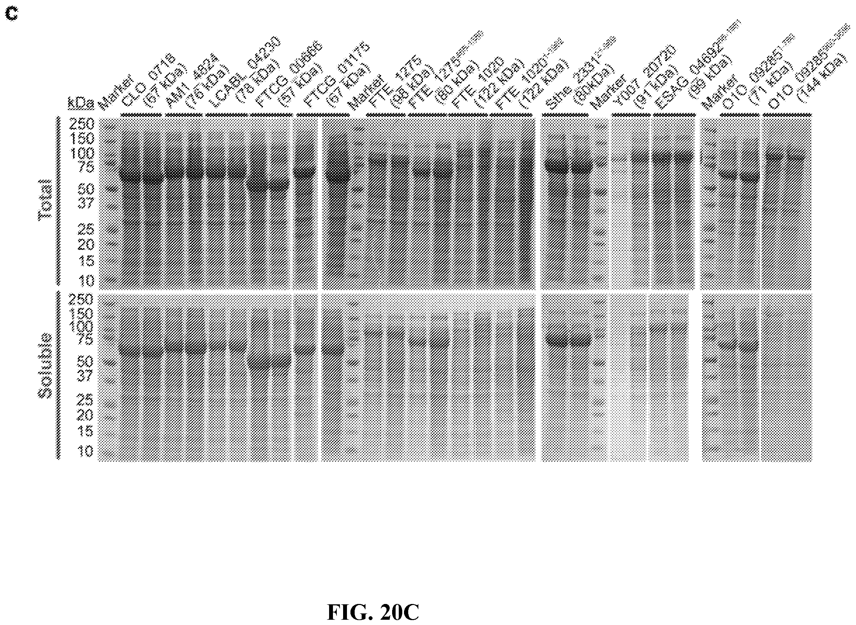

**Figures 20A-20C** show in vivo expression of synthetic genes with sequences optimized using the 31C-FO method. (**Figure 20A**) Comparison of expression properties of WT (WT_{H}/WT_{T}) *vs*. optimized (31C-FO_{H}/31C-FO_{T}) variants of the *E. coli yacQ* gene. The left panel shows a Coomasie Blue stained SDS-PAGE gel of whole *E. coli* BL21(DE3) pMGK cells following overnight induction at 18 °C; the volume of cell extract loaded on the gel was normalized to the OD₆₀₀ of the culture at the time of harvest. The center panel shows an autoradiograph of an SDS-PAGE gel of *in vitro* translation reactions using fully purified translation components in the presence of [³⁵S]-methionine; each reaction contained an equal amount of purified mRNA transcribed *in vitro* using T7 RNA polymerase. The right panel shows a Northern blot of the mRNA for the target protein after induction of expression *in vivo*; an equal amount of total RNA was loaded in each lane, and the blots were hybridized with a probe matching the 5 'UTR. (**Figure 20B**) Coomasie Blue stained SDS-PAGE gels of whole cell extracts following overnight induction at 18 °C of synthetic genes designed using the 31C-FO_{H} method for 17 different proteins. All genes were cloned in-frame with a C-terminal hexahistidine tag in the same pET21 plasmid derivative used to generate the large-scale protein-expression dataset (Acton, T. B. et al. (2005) Methods Enzymol 394, 210-243). Equal volumes of induced cultures were loaded in all lanes. **Figure 20C** Coomasie Blue stained SDS-PAGE gels of whole cell extracts (top) and the corresponding soluble fractions (bottom) following overnight induction at 18 °C of 14 of the same synthetic genes fused in-frame at the C-terminus of the gene for the *E. coli* maltose-binding protein (MBP). The protein sequences expressed in **Figures 20B-20C** come from the following source organisms: LCABL_04230 from *Lactobacillus casei BL23**;*** VIPARP466_2889 from *Vibrio parahaemolyticus**;*** AM1_4824 from *Acaryochloris marina* MBIC11017; CLO_0718 from *Clostridium botulinum El*; ESAG 04692 from *Escherichia sp.* 3_2_53FAA; FTCG_00666 and FTCG_01175 from *Francisella tularensis subsp. novicida* GA99-3549; FTE_1275, FTE_1608, FTE_0420, and FTE_1020 from *Francisella tularensis subsp. novicida* FTE; FRANO wbtG and A1DS62_FRANO from *Francisella novicida*l; FTBG_00988 and A7JEH2_FRATL from *Francisella tularensis subsp. tularensis* FSC033; FTN_1238 from *Francisella tularensis subsp. novicida* U112; O1O_09285 from *Pseudomonas aeruginosa* MPA01/P1; Sthe_2331 from *Sphaerobacter thermophilus* DSM20745/S6022; SEVCU126_0606 from *Staphylococcus epidermidis* VCU126; and Y007_20720 from *Salmonella enterica subsp. enterica serovar Montevideo* 507440-20 **-C**

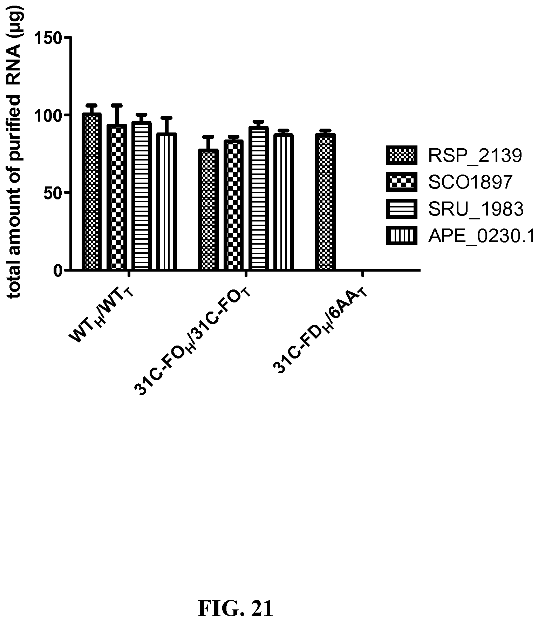

**Figure 21** shows the yield of pure mRNA obtained after T7 *in vitro* translation. **Figure 21** is a column graph representing the average yield of pure mRNA obtained for 2 independents *in vitro* T7 translation synthesis for each native or optimized genes.

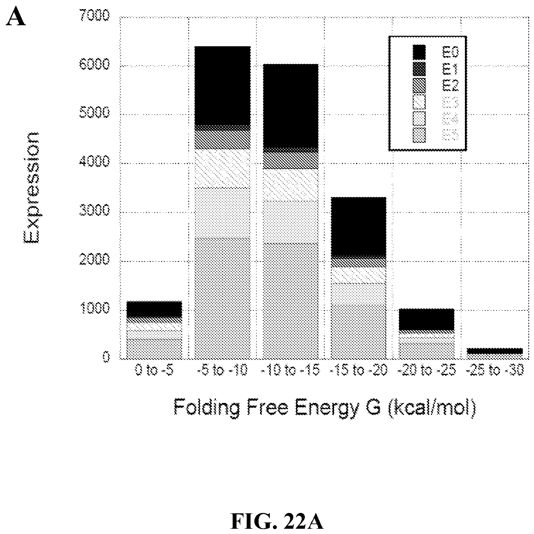

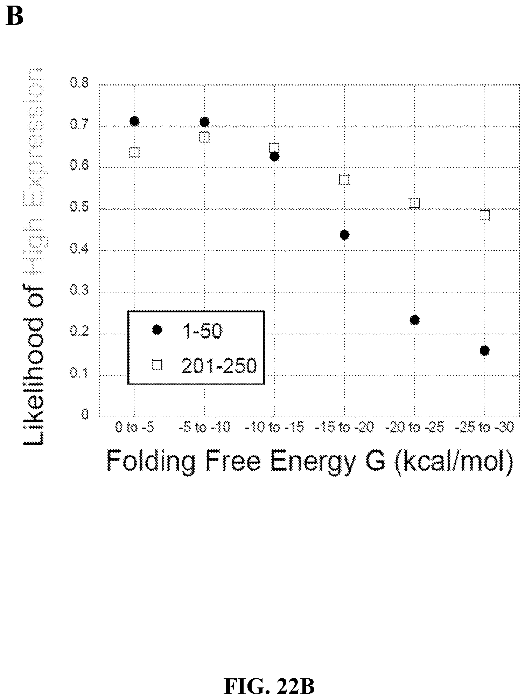

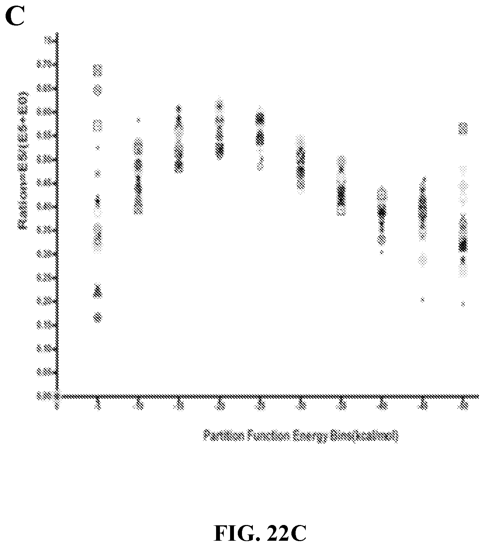

**Figures 22A-22C** **show** the Northeast Structural Genomics (NESG) Consortium dataset wherein expression was scored from E0 (none) to E5 (highest). In **Figure 22A****,** the free energy of the first 50 coding bases is computed. The high free energy bins (with relatively little secondary structure) have a greater fraction of high expression than do lower free energy bins. In **Figure 22B****,** the probability of high expression (E3+E4+E5) is plotted as a function of free energy for the first 50 coding bases and for coding bases 201-250. There is less variation in expression levels for the later windows, but the peak observed for -10 kcal/mol ≤ G ≤ -5 kcal/mol in **Figure 22B** and the parabolic trend is observed in a series of 96mer windows in **Figure 22C** suggests that too little structure may also be deleterious.

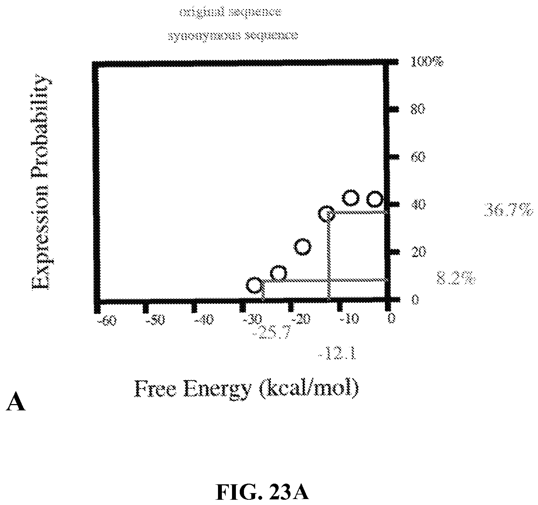

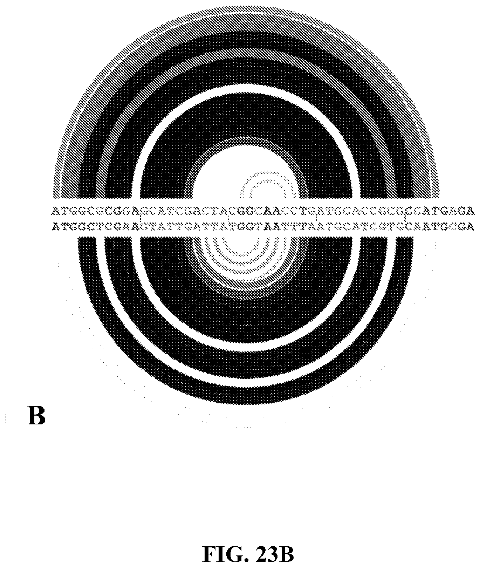

**Figures 23A-23B** show comparisons between an original sequence (in red) and an engineered synonymous sequence generated using the methods described herein (in blue). **Figure 23A** shows sample output from a prototype web application wherein increasing the free energy of the first 50 coding bases increases the probability that the gene will be highly expressed E5 / (E0+E1+...+E5). In **Figure 23B** the differences in secondary structure are depicted with a RNAbow diagram. Unique bases and base pairs are colored red or blue; common bases and pairs are in black.

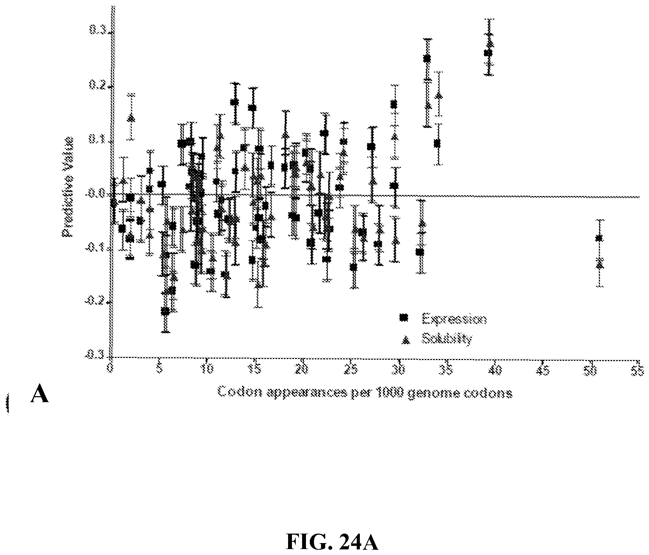

**Figures 24A-24B** shows (24A) codon effects are uncorrelated to genomic codon usage frequency and (24B) codon effects are unrelated to tRNA levels or "codon adaptation index.

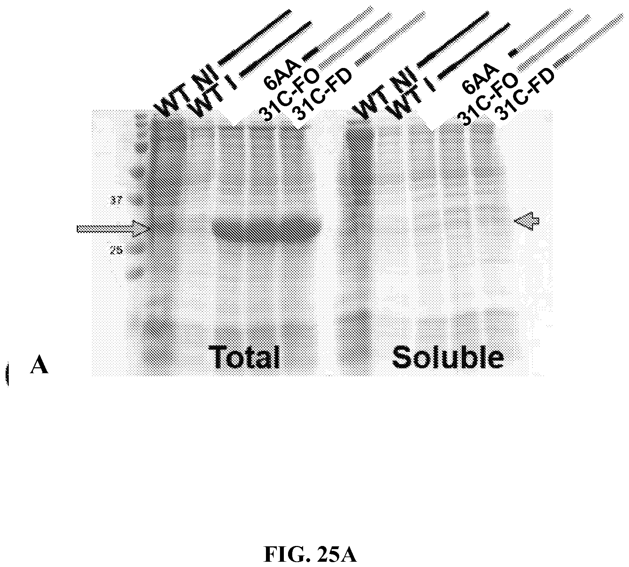

**Figures 25A-25D** shows experiments on (a) APE_0230.1, (b) RSP_2139, (c) SRU_1983, and (d) SCO1897 genes, removing the worst codons from the tail (6AA, green) increases the expression relative to WT (black). WT Not Induced and Induced are controls. In the head, codon optimization increases expression in all cases. In SCO1897, a 31C-FD head with low free energy can shut off expression. In other genes the 31C-FD free energy is not very low. WT: wildtype sequences; 6AA: optimizing the six most important codons (D → GAT, E → GAA, H → CAT, I → ATT, Q → CAA, R → CGT); 31C-FO: in which the free energy is optimized using only good codons; 31C-FD: in which the free energy is made as stable as possible using only good codons.

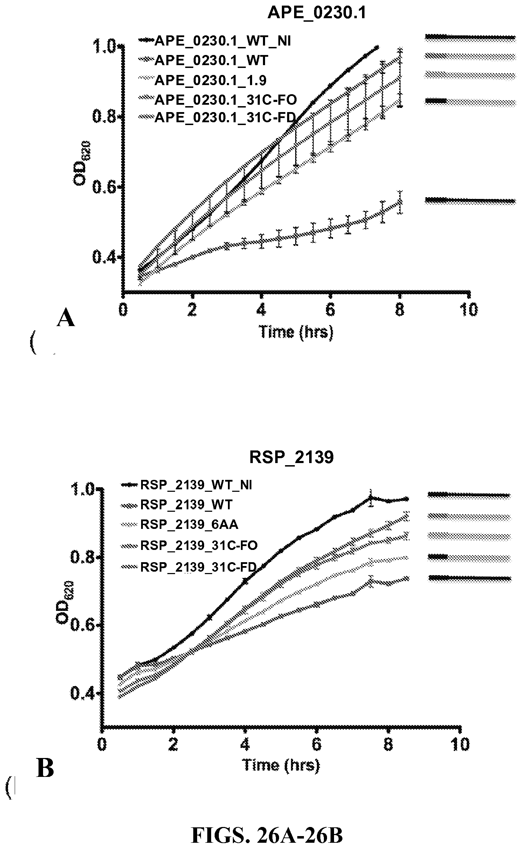

**Figures 26A-26B** shows 6AA (green) tails decrease toxicity in (26A) APE_0230.1 and (26B) RSP_2139. The gain of cell mass means a gain in protein production.

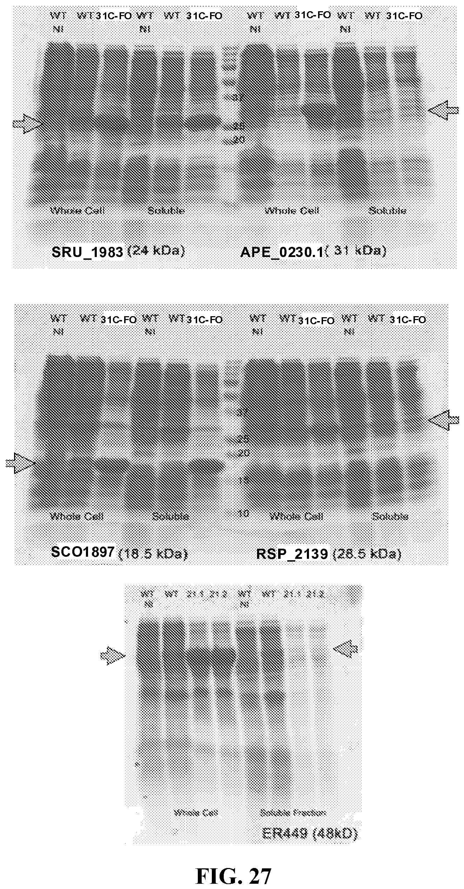

**Figure 27** shows Combining 31C-FO optimized heads and tails produces large increases in expression in all four genes previously studied. Endogenous E. coli protein ER449 with 31C-FO optimization (lanes 21.1 & 21.2) shows increased expression over wild type (WT).

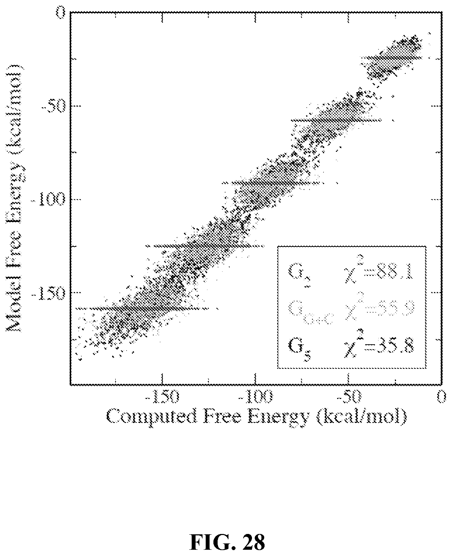

**Figure 28** shows the minimum free energy of 1000 pseudorandom sequences with mRNA dinucleotide correlations of length 100, 200, 300, 400, or 500 computed with RNAstructure are compared with: (28A) a two-parameter model G2 and (28B) a five-parameter model G5 that depends on base composition. The squared residuals in (kcal/mol)² units are given.

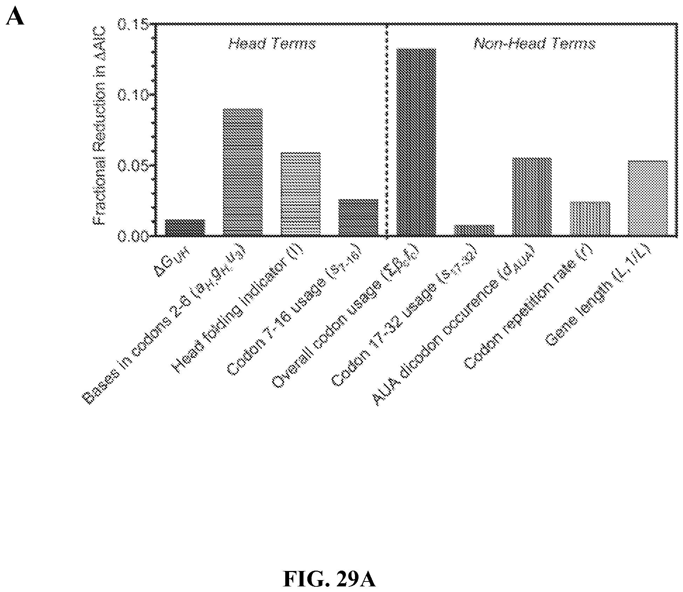

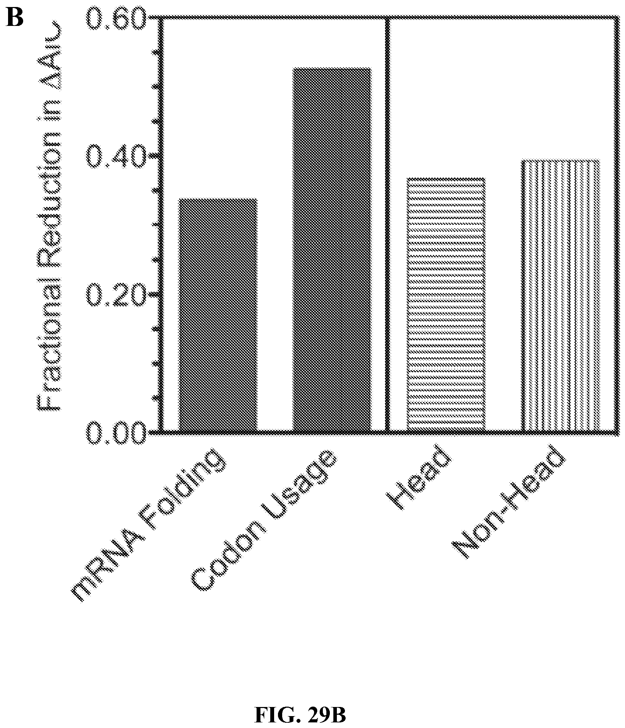

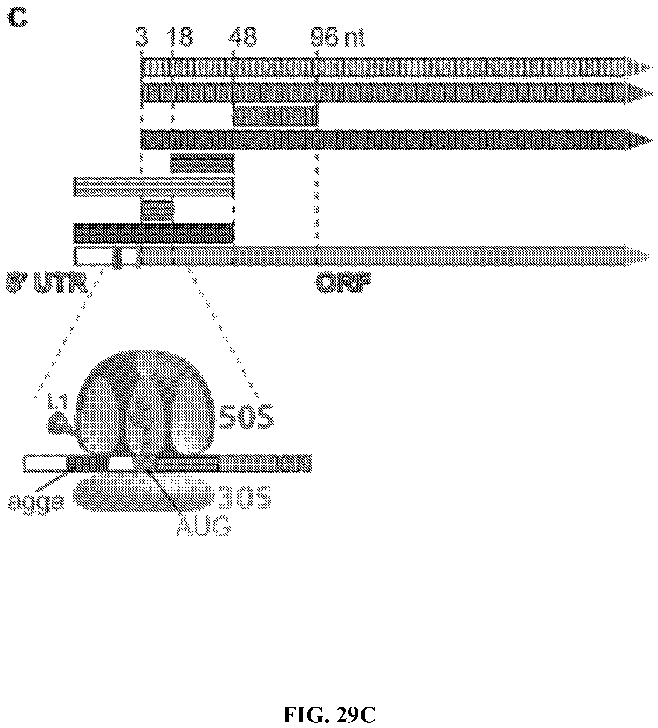

**Figures 29A-29C** shows the contributions of physicochemical factors and regions of the protein-coding sequence to the multi-parameter binary logistic regression model of protein-expression level. The magnitudes of the contributions of different factors were quantified using drop-out calculations in which individual terms or sets of terms were omitted prior to re-optimization of the remaining terms in the final model M (**Figures 34A-****34B**). The bar graphs show the resulting fractional reduction in the magnitude of ΔAIC (change in the Akaike Information Criterion), which quantifies the predictive power of the model compared to random expectation based on its number of degrees of freedom (see *Online Methods).* **Figure 29A** shows the summary of calculations dropping out each individual term. **Figure 29B** shows the summary of calculations dropping out combinations of terms. Those related to mRNA folding stability are shown in blue and cyan in **Figure 29A**, whereas those related to codon usage are shown in red, orange, yellow, and magenta. Head vs. non-head terms are shown on the left and right, respectively, in **Figure 29A****.** **Figure 29C** shows a schematic diagram in which the colors in **Figure 29A** are used to represent the regions of the protein-coding sequence included when calculating the corresponding sequence parameter. The AUG start codon begins at nucleotide (nt) position 1.

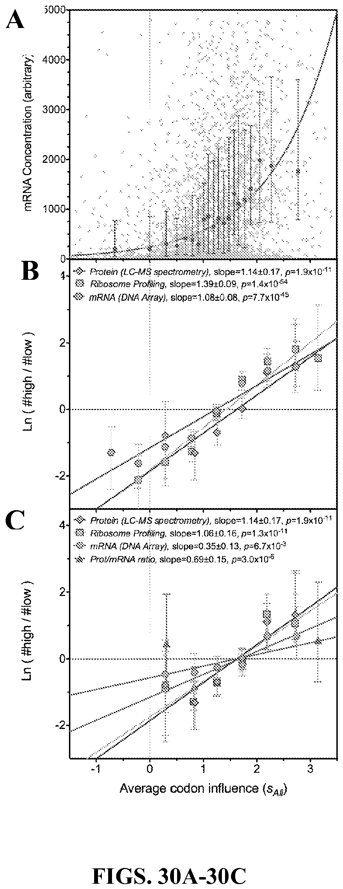

**Figures 30A-30C** show the mean codon influence from the multi-parameter binary logistic regression model correlates with endogenous mRNA and protein levels in E. coli. **Figure 30A** shows the levels of the mRNAs for every predicted cytoplasmic protein in *E. coli* detected in a previous microarray analysis are plotted as a function of *s_{All}*, the average value of the new codon-influence metric (colored symbols in **Figure 11B**). The cyan dots represent individual genes, while the blue symbols and vertical bars indicate the median and the 25^{th} through 75^{th} percentiles in 20 bins of *s_{All}* with equal population. **Figures 30B-30C** show the log-odds plots showing the natural logarithm of the ratio of the number of *E. coli* genes/proteins in the top vs. bottom 30% of the population in previous genome-scale *in vivo* profiling studies as a function of *s_{All}*. The red, magenta, and cyan curves in **Figure 30B** represent data from, respectively, a mass spectrometric analysis of protein concentration (Ishihama, Y. et al. (2008) BMC Genomics 9, 102) (*n* = 825), a deep-sequencing analysis of ribosome distribution on mRNAs (Li, G. W., Burkhardt, D., Gross, C. & Weissman, J. S. (2014) Cell 157, 624-635) (*n* = 2,597), and the same microarray analysis of mRNA concentration shown in **Figure 30A** (*n* = 2,817). **Figure 30B** shows the results for all predicted cytoplasmic proteins in *E. coli* (identified as described in the examples), while **Figure 30C** shows these results restricted to the proteins detected in the mass spectrometric analysis (*n* = 825). The green curve in **Figure 30C** shows the protein-to-mRNA ratio for these proteins, calculated as the quotient of the values from the mass spectrometric and microarray analyses. All profiling studies were conducted on log-phase cells growing in chemically defined medium

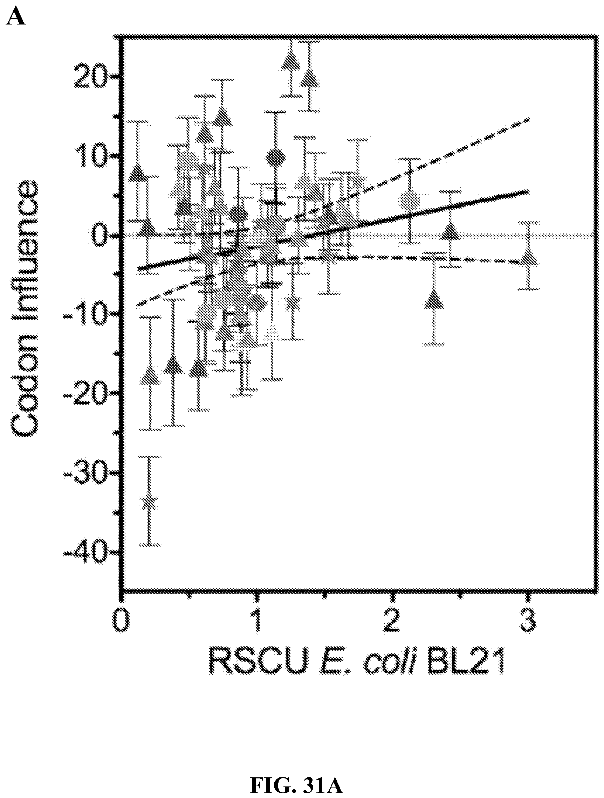

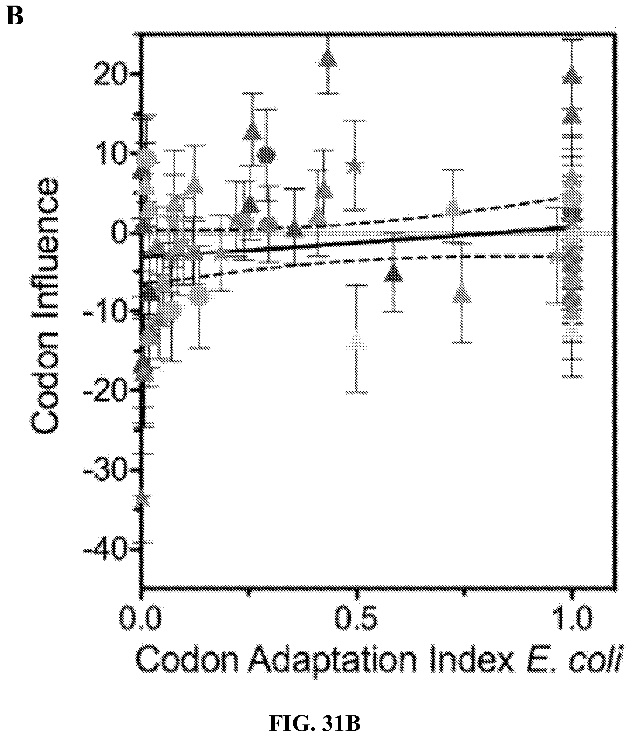

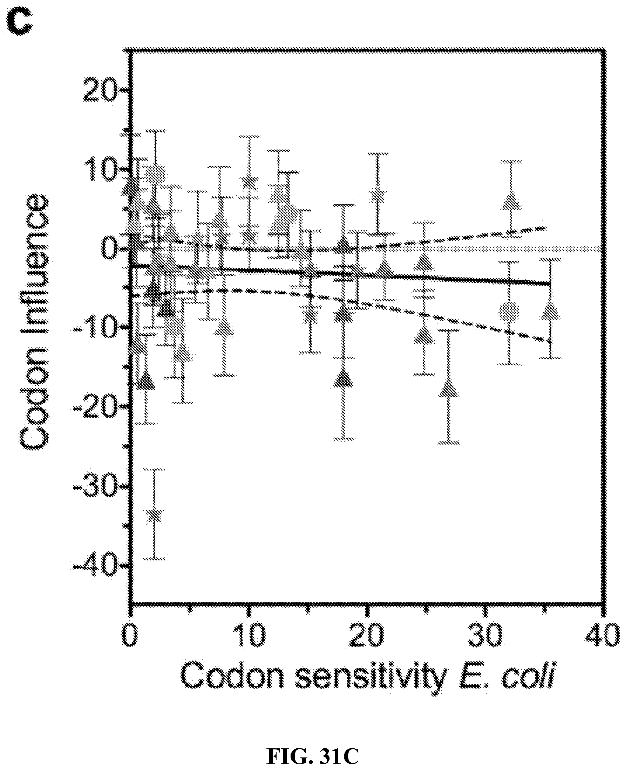

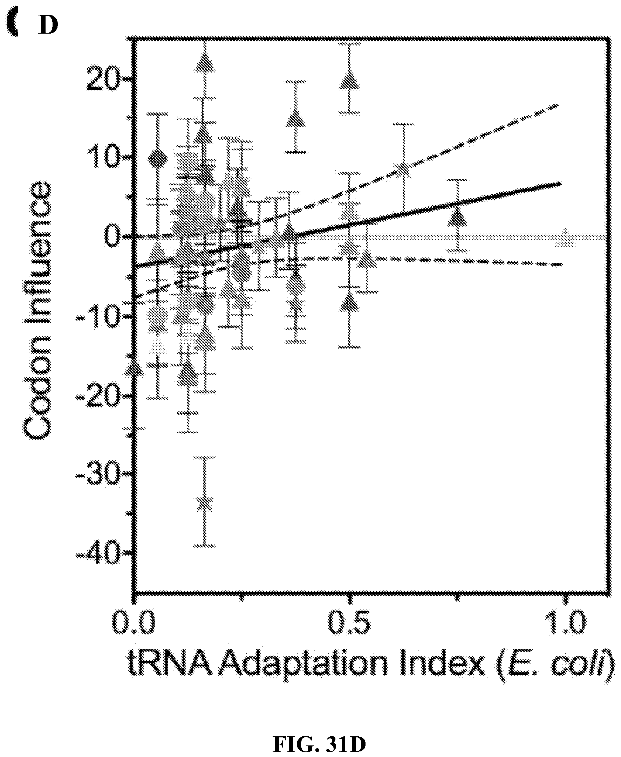

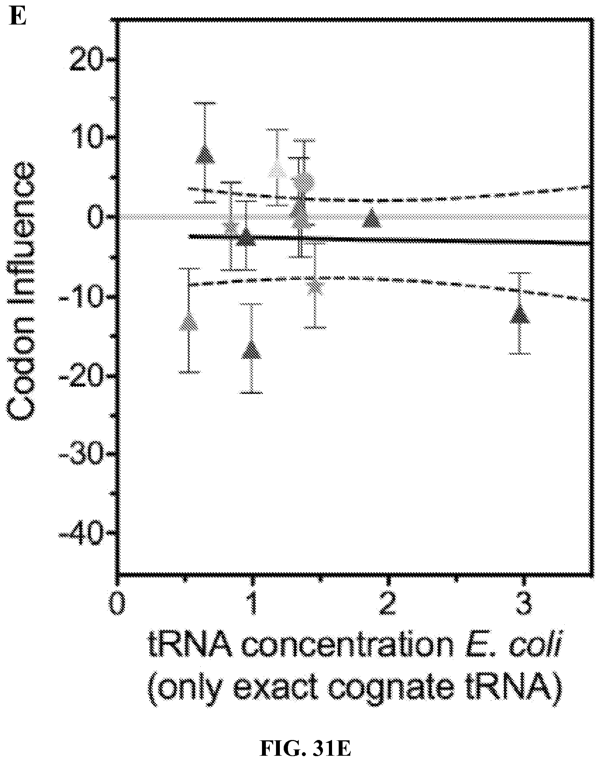

**Figures 31A-31E** show the relationship of the codon-influence metric to parameters assumed to influence translation efficiency in prior literature. The codon slopes from the simultaneous multi-parameter binary logistic regression analysis (colored symbols in **Figure 11B**) are plotted on the ordinate in all of these graphs. The color-coding and the shapes of the symbols are the same as in **Figures 11B-11E****.** **Figure 31A** shows a plot vs. the relative synonymous codon usage (RSCU) in *E. coli* BL21. **Figure 31B** shows a plot *vs.* the codon adaptation index (Sharp, P. M. & Li, W. H. (1987) Nucleic Acids Res 15, 1281-1295) in E. *coli* K12. **Figure 31C** shows a plot vs. the codon sensitivity (Elf, J., Nilsson, D., Tenson, T. & Ehrenberg, M. (2003) Science 300, 1718-1722) in E. *coli* K12. **Figure 31D** shows a plot *vs.* the tRNA Adaptation Index (Tuller, T. et al. (2010) Cell 141, 344-354) in E. *coli K12.* **Figure 31E** shows a plot *vs*. the concentration of exactly cognate tRNAs (Dong, H., Nilsson, L. & Kurland, C. G. (1996) Journal of Molecular Biology 260, 649-663) *in E. coli* K12.

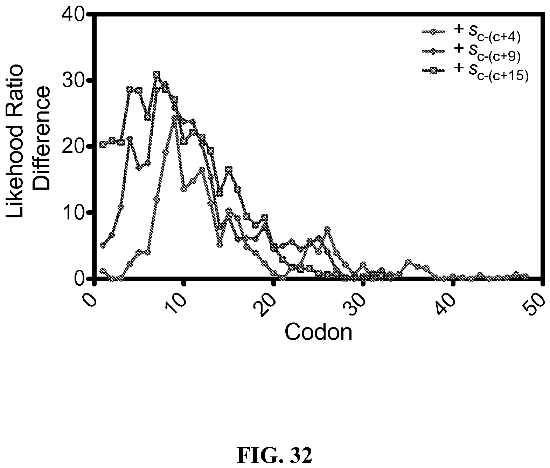

**Figure 32** shows the variation in codon influence as a function of position in the coding sequence. Plots showing the reduction in the deviance of the computational model resulting from adding a term representing the mean value of the codon slope (colored symbols in **Figure 11B**) in a window 5 (blue), 10 (red), or 16 (magenta) codons wide starting at the position indicated on the abscissa. The reduction in deviance was calculated relative to a base model containing codon frequencies, head nucleotide composition terms (*a_{H}*, *a_{H}²*, *u_{3H}*, *g_{H}²*), the predicted free energy of RNA folding in the head plus the 5'-UTR (Δ*G_{UH}*), the binary indicator variable for head folding effects *I*, the binary variable indicating the occurrence of an AUAAUA di-codon *d_{AUA}*, and the codon repetition rate *r*. The mean slope of codons 2-6 presumably does not improve the model because the head-composition terms rather than codon content dominate the influence of this region on protein-expression level. This effect also likely accounts for the peaks in the *s*_{c-(c+9)} and *s*_{c-(c+15)} plots for windows starting at codon 7. For reference, adding *s*₇₋₁₆ and *s*₁₆₋₃₂ terms to Model M contributes 30 points (p=5x10⁻⁸) and 10 points (p=0.001) of model deviance, respectively (**Figures 34A-34B** and **Figure 29A**). Individual codons at positions 7-16 in the head are ~2.3 times more influential than those downstream in the tail, based on comparing the total reduction in deviance attributable to the codons in this region divided by their number [(30 + (2.4^{∗}10))/10 = 5.4 per codon] to the average reduction in deviance per codon throughout the gene [(637.5/270) = 2.4 per codon].

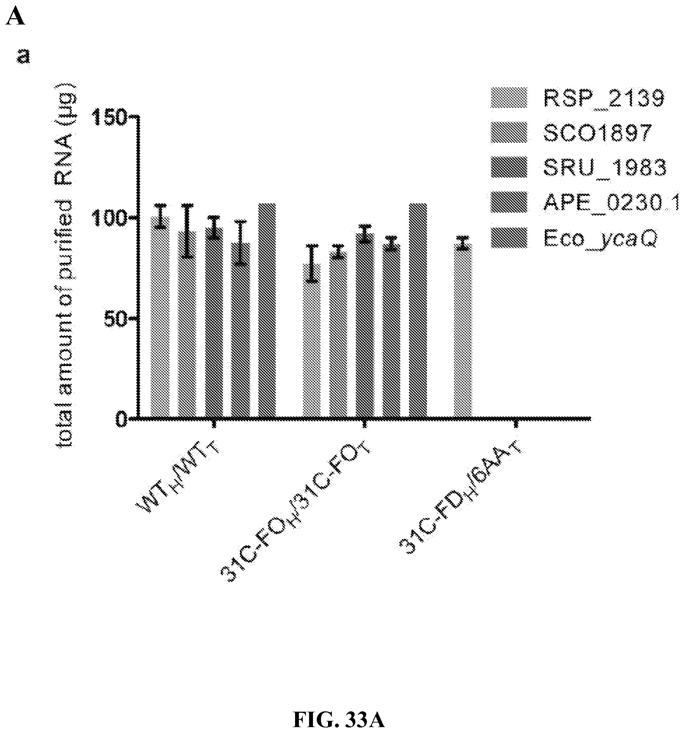

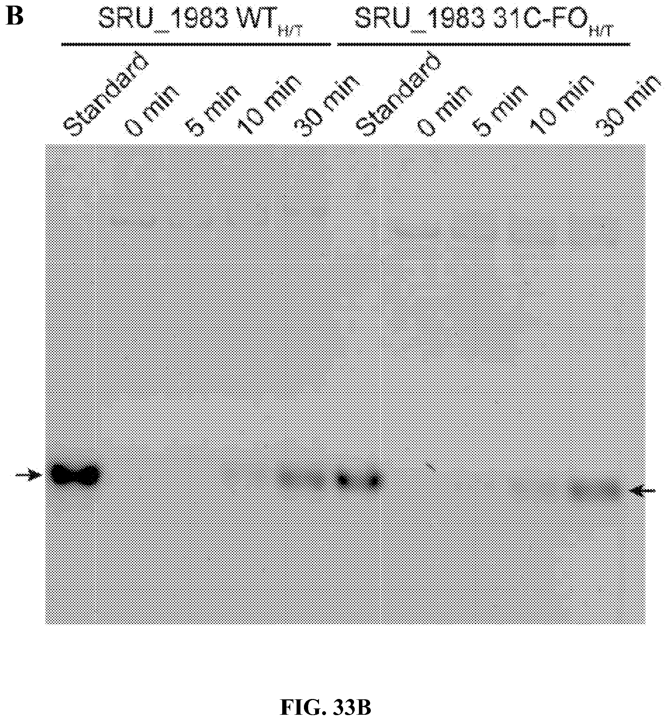

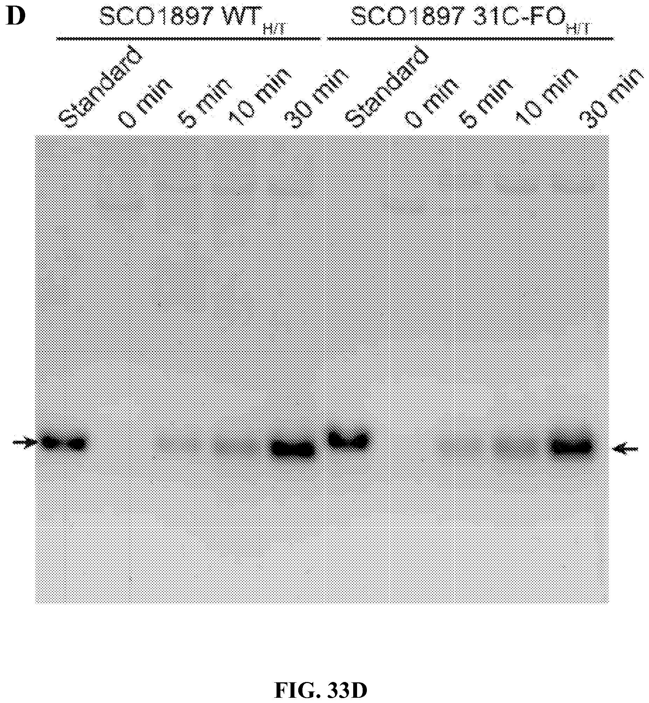

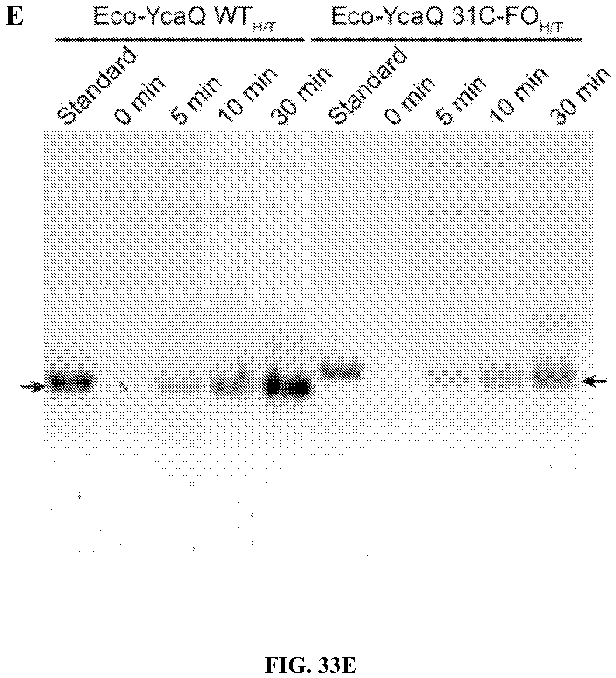

**Figures 33A-33E** show the yield of mRNA from in vitro transcription using purified T7 RNA polymerase. **Figure 33A** show the mRNAs were purified as described below and their final yields were quantified based on the optical density at 260 nm. (**Figures 33B-33D**) Time point samples of the T7 *in vitro* transcription reactions at 0, 5, 10 and 30 minutes run on denaturing formaldehyde-agarose gel. Reaction where started by addition of the WT or 31C-FO_{H}/31C-FO_{T} (31 C-FO_{H}/_{T}) linearized plasmid for: SRU_1983 (**Figure 33B**), APE_0230.1 (**c**), SCO1897 (**Figure 33D**) and Eco-YcaQ (**Figure 33E**). For each reaction a 1 µg of corresponding purified mRNA was loaded on the gel as standard to asset the ethidium bromide staining of each mRNA.

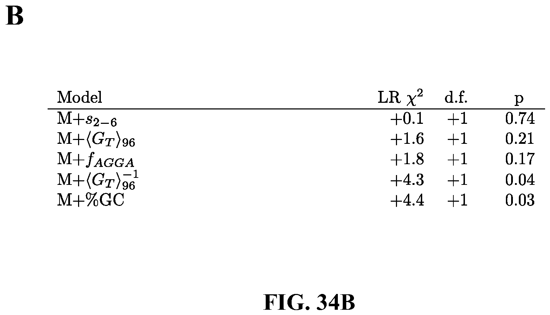

**Figures 34A-34B** show tables for Model development and the effects of adding terms to the final computation model M. **Figure 34A** shows table for Model development. The Likelihood Ratio (LR) χ² measures the difference in deviance relative to that of the null model (5153.8). The deviance is defined below. The ΔAIC, given by (LR χ2 - 2*d.f.), represents the change in the Akaike Information Criterion for a given number of degrees of freedom (d.f.) added to the model. The best model M is a sum of the indicated parameters, which are defined above in this table. Having considered many compositional, free energy, and other terms, a factor of 100 was used to correct for multiple-hypothesis testing and only included parameters in the final model if significant at a Bonferroni-corrected level of p < 0.05/100 (5 x 10⁻⁴). **Figure 34B** shows the effects of adding terms to the final computation model M.

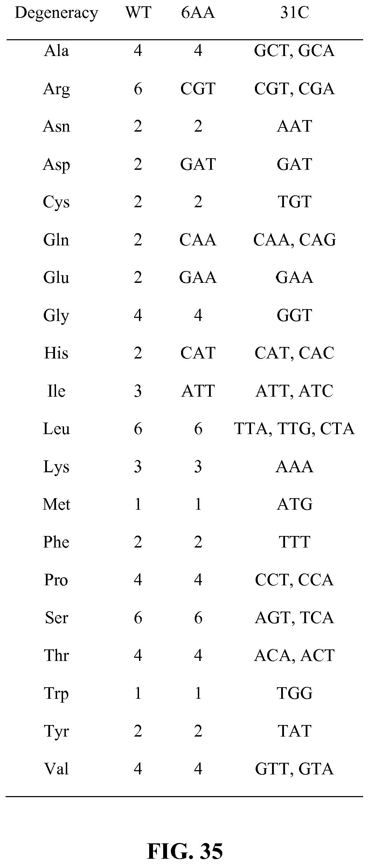

**Figure 35** shows a table for codons used for gene design. In the design of synonymous sequences, the native degeneracy of the genetic code was reduced to eliminate bad codons and eliminate the worst codons. In the 6AA approach, a specific codon was used for six amino acids while the other 14 were not changed from their wild type sequence. In the 31C-FO (and FD) approaches, the free energy was optimized (or deoptimized) using only the indicated subset of codons

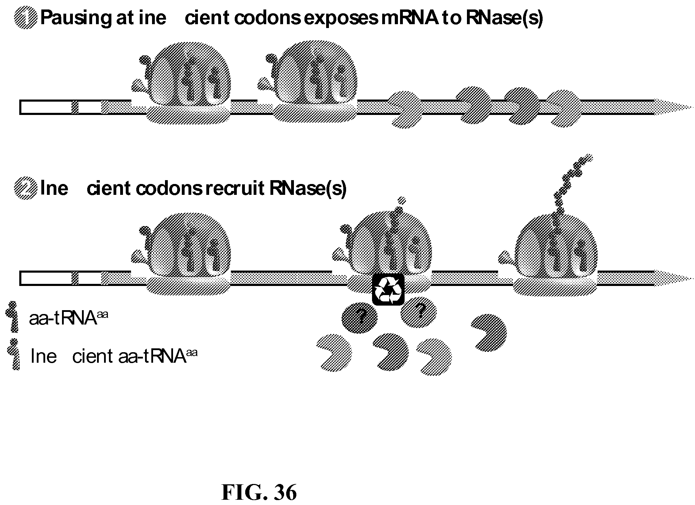

**Figure 36** shows models for the mechanism by which synonymous codons alter mRNA degradation. The tRNA translating an inefficient codon is illustrated here as occupying the A-site on the ribosome because the concentration of charged cognate tRNA can influence translational efficiency under some circumstances. However, effects at the P-site and E-site are also possible.

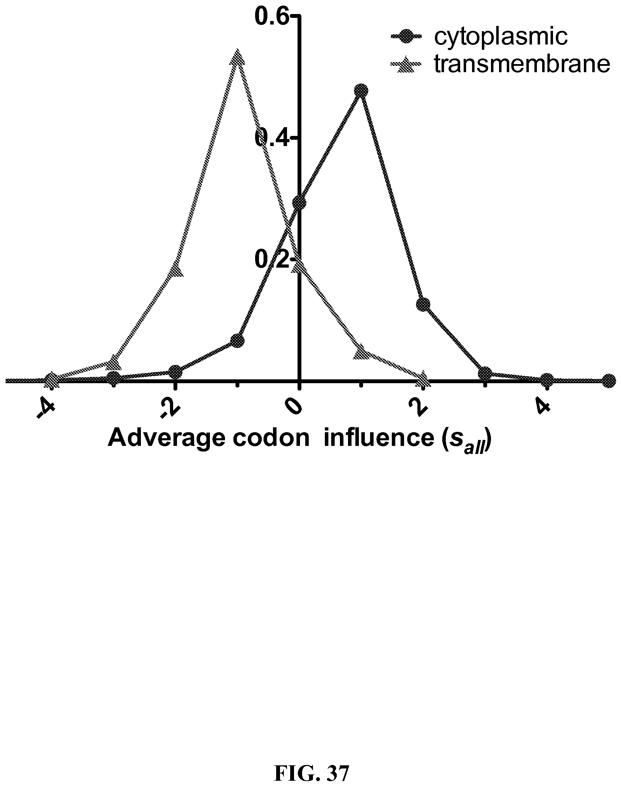

**Figure 37** shows the mean value of the new codon-influence metric for all predicted cytoplasmic vs. membrane proteins encoded in the E. coli genome. The programs LipoP and TMHMM were used to analyze all protein-coding sequences. Proteins not predicted to have a signal sequence or a transmembrane α-helix were designated cytoplasmic, while those predicted to have at least two transmembrane α -helices were designated transmembrane.

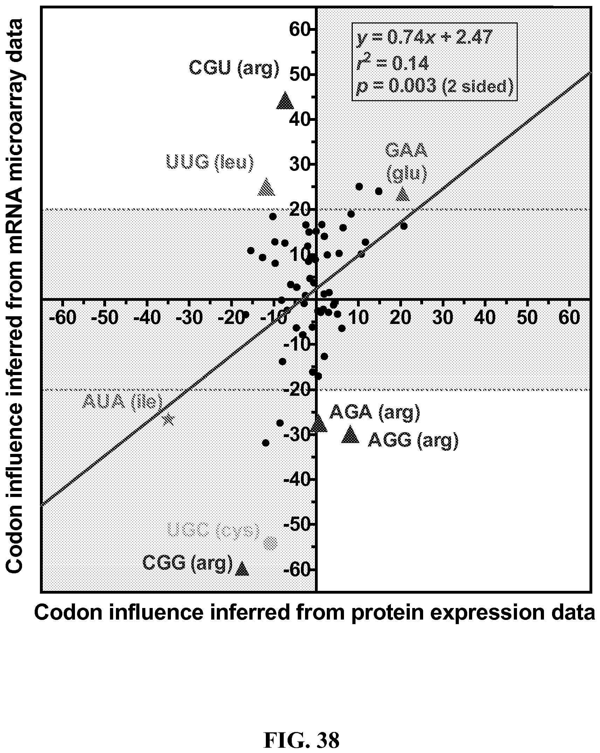

**Figure 38** shows comparison of codon influence inferred from 6,348 independent protein-expression experiments to that inferred from a single mRNA microarray using equivalent multiparameter logistic regression modeling methods. The white background highlights codons going from positive to strongly negative influence or vice-versa.

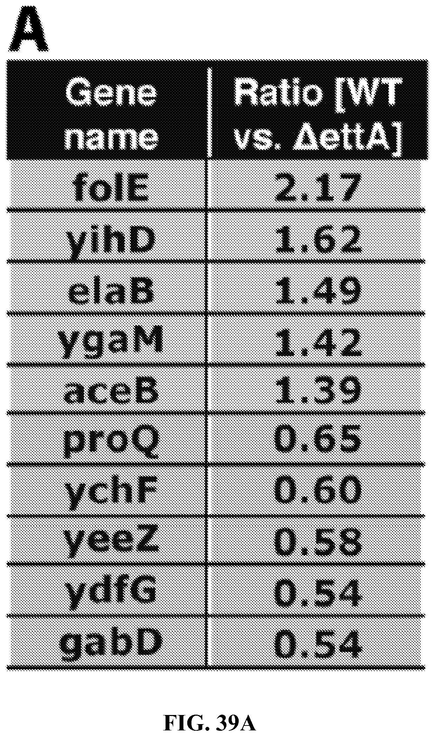

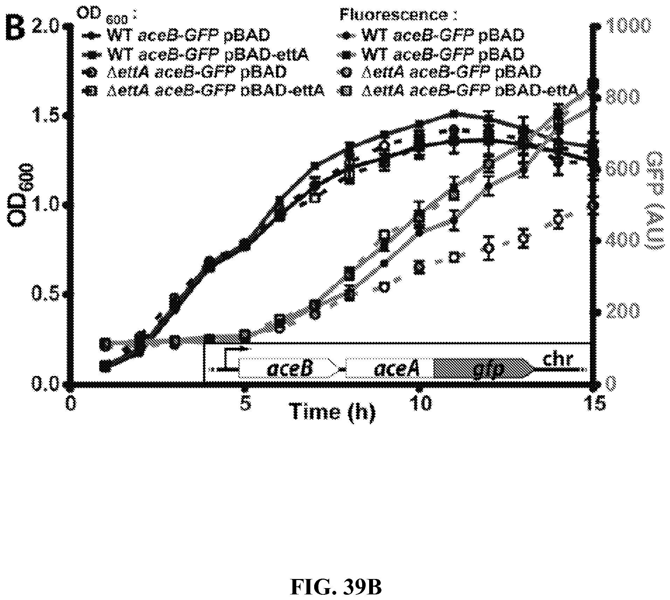

**Figures 39A-39B** show the Influence of ΔettA on in vivo protein expression in log-phase E. coli MG1655 in glucose minimal medium. (**Figure 39A**) Table showing proteins most strongly altered in differential proteomics assays comparing WT to ΔettA. (**Figure 39B**) Real time assays of OD₆₀₀ (black) and YFP fluorescence (green) from strains harboring an in-frame fusion of YFP to the C-terminus of the chromosomal gene encoding AceB; cells contained an EttA-expressing plasmid or empty control plasmid.

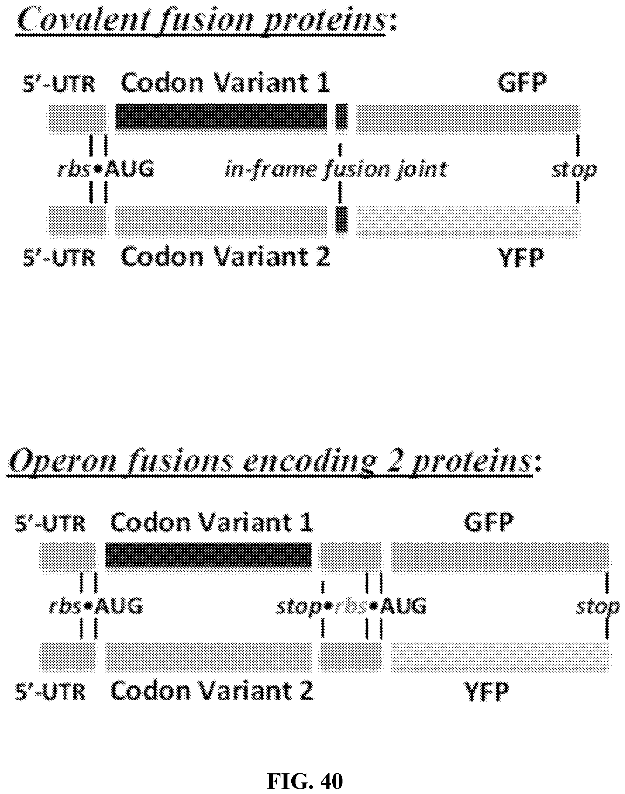

**Figure 40** shows a schematic diagram of proposed reporter-gene structures. AUG is the start-codon, and rbs stands for ribosome-binding site.

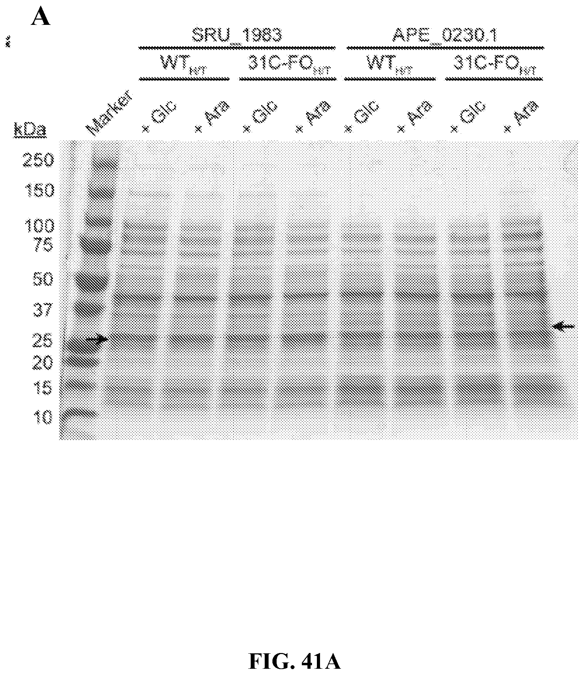

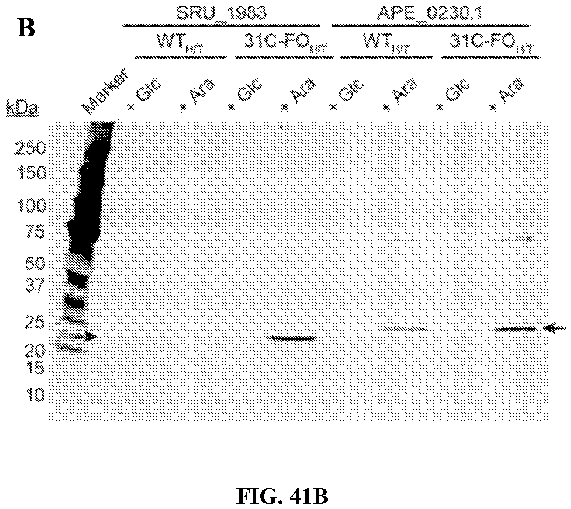

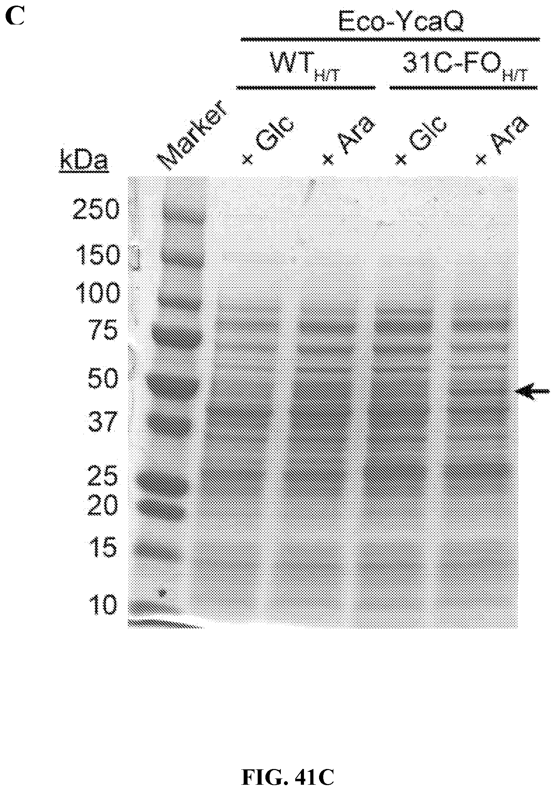

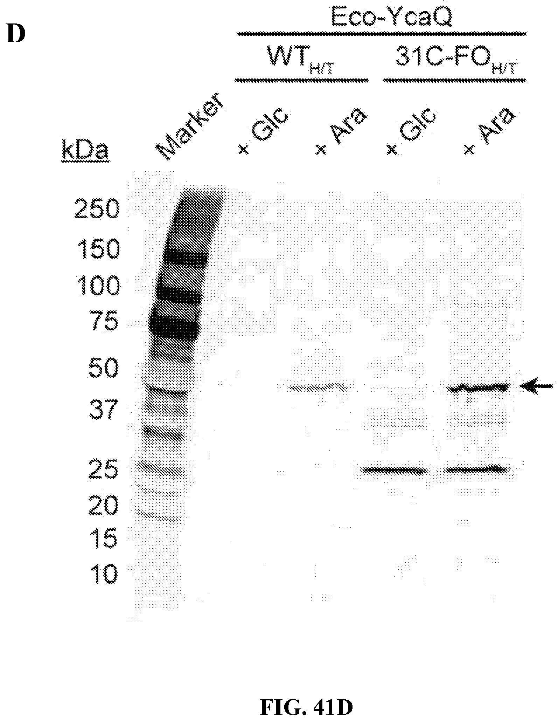

**Figures 41A-41D** show the effect of gene optimization at physiological expression levels. The WT and 31C-FO_{H}/31C-FO_{T} (31C-FO_{H}/_{T}) genes for SRU_1983, APE_0230.1 and Eco-YcaQ were re-cloned in a pBAD plasmid (Life Technologies) with a ₆His-tag in 5' of the ORF. Genes cloned in this plasmid are expressed by the native *E. coli*'s RNA polymerase with an arabinose inducible promoter. BL21 pMGK cell carrying the pBAD plasmids were grown in LB media with 100 µg/ml of Ampicillin and 30 µg/ml of Kanamycin. Non-induced controls were grown in media with 0.4% glucose (lanes +Glc). At an OD₆₀₀ of 0.6 cells were induced with arabinose at a final concentration of 0.001% for APE_0230.1 and 0.01% for SRU_1983 and Eco-YcaQ for1 hour (lanes +Ara). (**Figures 41A**,**41C**) Induced and non-induced cells were processed as described in the *online method* and run on SDS-PAGE gels. Parallel gels were run for western blot analysis. (**Figures 41B**,**41D**) Western-blots incubated with a 1:2,000 dilution of an tetra-His antibody (34670, Qiagen), developed with a donkey anti-rabbit secondary antibodies conjugate to IRDye 680 (926-32223, Li-cor) and scanned on an Odyssey CLx scanner (Li-cor). Black arrows show the location of the induced protein on the gel. For YcaQ_31C-FO_{H}/_{T} (**Figure 41D**) samples other proteins of smaller molecular weight are reacting with the tetra-His antibody, more likely they are due to internal transcription/translation initiation in the YcaQ_31C-FO_{H}/_{T} sequence that are independent of the arabinose inducible promoter.

### DETAILED DESCRIPTION OF THE INVENTION

The singular forms "a," "an," and "the" include plural references unless the content clearly dictates otherwise. Thus, for example, reference to a "virus" includes a plurality of such viruses.

Overexpression of recombinant polypeptides is an important step in a variety of biotechnology applications, however poor expression of recombinant polypeptides can be problematic for polypeptide related applications. For example, industrial and commercial applications such as food production, drug discovery and drug production often require that the polypeptides be expressed at high levels.

The methods described herein are based in part on large-scale statistical data mining from thousands of unique polypeptides expressed in more than 6,348 expression experiments. The invention described herein relates to a codon efficiency metric that can qualitatively and quantitatively describe the influence of individual codons on protein expression level.

The methods described herein may relate to the use of logistic regression to analyze 6,348 protein-expression experiments employing bacteriophage T7 polymerase to drive mRNA synthesis in *E. coli.* The methods described herein show that (a) the head (initial ~16 codons), and (b) the tail (remainder) of a gene exert about the same influence on protein expression. The methods described herein show that while mRNA folding effects dominate the influence of the head, codon usage contributes to its influence and dominates that of the tail. Without wishing to be bound by theory, the codon-efficiency metric analyses described herein can show a weak correlation with genomic codon-usage frequency in *E. coli* and a strong correlation with both protein and mRNA concentrations measured in genome-scale profiling studies. Genes redesigned based on the methods described herein can be transcribed *in vitro* with unaltered efficiency and yet yield mRNAs translated *in vitro* with substantially higher efficiency. The methods described herein can be used to yield greater increases in protein expression *in vivo.* The increase in protein expression obtained according to the methods described herein may be due in part to increased mRNA level. The methods described here can be used to identify biophysical factors influencing protein translation. Without wishing to be bound to theory, the methods described herein relate to the finding that translation efficiency is a major but heretofore unappreciated determinant of physiological mRNA level in *E. coli.*

The invention described herein may relate to a quantitative method developed useful for predicting the effect of mRNA folding energy on protein expression level.

The methods described herein relate to the use of statistical analyses of a large-scale experimental protein-expression dataset. The methods described herein may focus on simultaneous evaluation of the influence of a wide variety of local and global mRNA sequence properties.

The methods described herein may involve testing the mechanistic inferences (for example inferences resulting from the influence of a wide variety of local and global mRNA sequence properties) through biochemical analysis. As described herein, these combined computational and experimental studies can be used to determine and identify the influence of mRNA sequence features on protein expression level. In some aspects, the methods described herein can be used to determine the relative influence of codon translation efficiency *versus* mRNA-folding energy as well as the variation in the influence of these factors in different regions of the protein-coding sequence. The methods described herein also provide a codon-efficiency metric. In certain aspects, the methods described herein relate to the finding that sequence-dependent bottlenecks to translation initiation and elongation can reduce steady-state mRNA levels. In certain aspects, the reduction of steady-state mRNA levels due to sequence-dependent bottlenecks to translation initiation and elongation amplifies their influence on protein expression.

The inventions described herein are also based in part on the finding that low expression can be a strong correlate to low folding free energy at the start of the coding region of a nucleic acid sequence encoding a polypeptide. Thus, the methods described herein can be used to a evaluate whether for a given gene, it can evaluate whether a polypeptide encoded by a nucleic acid sequence is likely to be poorly expressed due to strong folding effects of the nucleic acid. Thus, in certain aspects, the method described herein can make use of the degeneracy of the genetic code to generate synonymous nucleic acid sequences capable of encoding a same polypeptide and wherein the synonymous nucleic acid sequence comprises synonymous alterations to generate a nucleic acid sequence with a high predicted free energy of folding of the corresponding RNA sequence relative to the unaltered sequence, and thereby produce higher protein expression.

While DNA is built from Watson-Crick complementary base pairs, the base composition of RNA is not constrained by universal complementarity so more sophisticated approximations than (G+C) content should be made for RNA. The four bases have different mean folding free energies, a fact that can be exploited for designing sequences with optimal properties.

Accordingly, the methods and compositions described herein can be useful for identifying polypeptides that have a higher or lower probability of being expressed at a high level in a gene expression system, improving the expression of a given gene. These methods can have the benefit of reducing the cost of protein expression for a variety of applications, including research, biotechnological and commercial applications. Thus, the findings described herein can be used to provide improved expression of a protein that does not otherwise express well from its native sequence by introducing synonymous alterations to the nucleic acid sequence that improve translational efficiency of a polypeptide encoded therefrom.

In certain aspects, the methods described herein relate to the finding that the influence of the base composition in codons 2-6 combined with the predicted free energy of folding of the RNA sequence corresponding to the head region of a nucleic acid encoding a polypeptide influence the expression of a polypeptide encoded therefrom. The methods described herein may involve assessing the base pair composition of the first codon of a nucleic acid sequence encoding a polypeptide in combination with the predicted free energy of folding of the RNA sequence corresponding to the head region of the nucleic acid encoding the polypeptide to determine whether a polypeptide is likely to be expressed well. The methods described herein may involve assessing the base pair composition of the first two codons of a nucleic acid sequence encoding a polypeptide in combination with the predicted free energy of folding of the RNA sequence corresponding to the head region of the nucleic acid encoding the polypeptide to determine whether a polypeptide is likely to be expressed well. The methods described herein may involve assessing the base pair composition of the first three codons of a nucleic acid sequence encoding a polypeptide in combination with the predicted free energy of folding of the RNA sequence corresponding to the head region of the nucleic acid encoding the polypeptide to determine whether a polypeptide is likely to be expressed well. The methods described herein may involve assessing the base pair composition of the first four codons of a nucleic acid sequence encoding a polypeptide in combination with the predicted free energy of folding of the RNA sequence corresponding to the head region of the nucleic acid encoding the polypeptide to determine whether a polypeptide is likely to be expressed well. The methods described herein involve assessing the base pair composition of the first five codons of a nucleic acid sequence encoding a polypeptide in combination with the predicted free energy of folding of the RNA sequence corresponding to the head region of the nucleic acid encoding the polypeptide to determine whether a polypeptide is likely to be expressed well. The methods described herein involve assessing the base pair composition of the first six codons of a nucleic acid sequence encoding a polypeptide in combination with the predicted free energy of folding of the RNA sequence corresponding to the head region of the nucleic acid encoding the polypeptide to determine whether a polypeptide is likely to be expressed well.

In certain aspects, the methods described herein relate to the finding that the tail region of a nucleic acid sequence can have an effect on a polypeptide sequence encoded therefrom. The free energy terms used to assess the effect of the head region on polypeptide expression is subsumed by determining the effect of "codon slopes" and a "codon repetition rate" term (r). Minimal codon repetition in the tail region of a nucleic acid encoding a polypeptide (as determined by the codon repetition rate term) indicates that a polypeptide encoded by the nucleic acid is likely to be expressed at a higher level than a polypeptide encoded from a nucleic acid sequence having a higher amount of codon repetition in its tail region. Expression of a polypeptide can be improved by eliminating codons that reduce expression (e.g. ATA, CGG, CGA, CUA, UUG) prior to optimizing the sequence.

Thus, in certain aspects, the invention relates to a method for improving the expression of a polypeptide encoded from a nucleic acid, the method comprising (a) generating a list to evaluate the potential benefit to improve expression that can be obtained by changing each codon as a function (i) of codon slope and, (ii) the impact on the codon repetition rate; (b) sorting the list and substituting in the codon predicted to cause the largest increase in expression of the polypeptide encoded from the nucleic acid; and (c) repeating steps (a) and (b) until no further improvement of polypeptide expression is possible or desired. The codon predicted to cause the second largest increase in protein production can be employed in place of the codon predicted to cause the largest increase in expression of the polypeptide encoded from the nucleic acid. The repeating of step (c) is performed while retaining the codon repetition rate within a desired range.

The methods described herein can be applied to global mRNA profiling data from E. coli to generate an equivalent gene-optimization algorithm, as indicated in **Figure 30****.** The methods described herein can include, but are not limited to, the computational approach used to generate Model M described herein. Thus, the methods described herein can be applied to global mRNA profiling data from any organism to generate a gene-optimization algorithm specific to that organism and can be applied to any organism for which a global mRNA profile can be generated. The methods described herein, (e.g. the computational approach used to generate Model "M") can be used to generate an equivalent gene-optimization algorithm for E. COLI from any mRNA profiling data from E. coli. The methods described herein, (e.g. the computational approach used to generate Model "M") can be used to generate an equivalent gene-optimization algorithm for any organism from any mRNA profiling data or protein-expression profiling data from that organism, including, but not limited to, bacterial organisms, archaeal organisms, or eukaryotic organisms, including, but not limited to the organisms shown in Figure 15.

The organism suitable for use with the methods described herein, (e.g. Model "M" or output from the computational approach used to generate Model "M" applied to protein-expression profiling data or mRNA profiling data) can be a transgenic or genetically engineered organism comprising one or more genes from a different organism or from a synthetic origin. An expression system suitable for use with the methods described herein, (e.g. Model "M" or output from the computational approach used to generate model "m" applied to protein-expression profiling data or mRNA profiling data) can be an in-vitro expression system or an reconstituted expression system comprising one or more transcription or translation components from a bacteria, an archaea or a eukaryote. An expression system suitable for use with the methods described herein, (e.g. Model "M" or output from the computational approach used to generate model "m" applied to protein-expression profiling data or mRNA profiling data) can be an in-vitro expression system or a reconstituted expression comprising one or more transcription or translation components from an organism shown in Figure 15. An expression system suitable for use with the methods described herein, (e.g. Model "M" or output from the computational approach used to generate model "m" applied to protein-expression profiling data or mRNA profiling data) can be an in-vitro expression system or a reconstituted expression comprising one or more transcription or translation components from an organism shown in Figure 15.

Model M can be a multiparameter generalized linear logistic regression model. In certain embodiments, application of the methods described herein to mRNA profiling data can be logistic or non-logistic. Thus, application of the methods described herein to mRNA profiling data can be a multiparameter generalized linear linear regression model.

The degeneracy in the genetic code, the fact that 61 different nucleotide triplet codons direct polymerization of just 20 different amino acids, enables the same protein sequence to be encoded by a vast number of different but synonymous mRNA sequences. Synonymous changes in protein-coding sequences (single-nucleotide polymorphisms) can alter human susceptibility to a wide range of diseases (Kimchi-Sarfaty, C. et al. (2007) Science 315, 525-528; Hunt RC et al., (2014) Trends in genetics : TIG, doi:10.1016/j.tig.2014.04.006). Molecular biological studies have provided many examples of synonymous changes in mRNA sequence that produce both subtle and dramatic alterations in protein expression level (Steinthorsdottir V et al., (2007) Nature genetics 39, 770-775; Hunt RC et al., (2014) Trends in genetics : TIG, doi:10.1016/j.tig.2014.04.006; Zhang F. et al. (2010) Science 329, 1534-1537). Variations in mRNA sequence can play an important role in regulating protein expression in organisms ranging from *E. coli* to humans, and a variety of different mechanistic factors have been implicated in mediating these effects in different experimental systems (Spencer PS et al., (2012) J Mol Biol 422, 328-335; Plotkin JB et al., (2011) Nature reviews. Genetics 12, 32-42; Gingold, H. (2011) Mol Syst Biol 7, 481). However, there is limited understanding of relative contribution of the different factors in controlling protein expression level in any given system, and conflicting reports concerning the influence of some of these factors remain unresolved.

mRNA features have been implicated in controlling the translation efficiency of mRNA. Stable mRNA folding in the 5' region, but not downstream in the protein-coding sequence, can attenuate translation in *E. coli* (Goodman DB et al., (2013) Science, doi:10.1126/science.1241934; Kudla G et al., (2009) Science 324, 255-258; Bentele K et al., (2013) Molecular systems biology 9, 675; Tuller, T. et al. (2010) Proc Natl Acad Sci U S A 107, 3645-3650). This effect may reflect inhibition of the assembly of the 70S ribosomal initiation complex onto the AUG start codon in the mRNA. Although there are cases where modulation of stable mRNA folding overlapping the start codon mediates physiologically important regulation of protein translation (Shakin-Eshleman SH et al., (1988) Biochemistry 27, 3975-3982 (1988); Kozak M (2005) Gene 361, 13-37; Castillo-Mendez, M. A. et al. (2012) Biochimie 94, 662-672), the relationship between mRNA folding energy and the efficiency of protein translation remains undefined. In certain aspects, the methods and compositions described herein relate to the relationship between mRNA folding energy and the efficiency of protein translation.

Differences in the translation efficiency of synonymous codons may influence protein expression level, but a systematic quantification of these effects is also lacking. Much of the literature on codon usage focuses on inefficient translation of a set of infrequently used codons in the *E. coli* genome, especially the AUA codon for isoleucine (Caskey CT et al., (1968) J Mol Biol 37, 99-118; Muramatsu T et al., (1988) Nature 336, 179-181) and the AGA, AGG, and CGG codons for arginine (Chen GT et al., (1994) Genes & development 8, 2641-2652; Vivanco-Dominguez S et al., (2012) J Mol Biol 417, 425-439).

Uncertainty exists concerning the influence of synonymous codons on translation efficiency (Goodman DB et al., (2013) Science, doi:10.1126/science.1241934; Kudla, G. et al. (2009) Science 324, 255-258; Bentele, K. et al. (2013) Mol Syst Biol 9, 675; Cannarozzi, G. et al. (2010) Cell 141, 355-367; Li, G. W. et al. (2014) Cell 157, 624-635; Chen, G. T. et al. (1994) Genes Dev 8, 2641-2652; Caskey, C. T. et al. (1968) J Mol Biol 37, 99-118, Price, W. N. et al. (2011) Microbial Informatics and Experimentation 1, 6; Wallace, E. W. et al. (2013) Mol Biol Evol 30, 1438-1453; Li, G.-W. et al. (2012) Nature 484, 538-541; Elf, J. et al. (2003) Science 300, 1718-1722; Ran, W. et al. (2014) MBio 5, e00956-00914; Quax, T. E. et al. (2013) Cell Rep 4, 938-944), the mechanistic basis of such effects, and their relationship to mRNA folding effects (Shakin-Eshleman SH et al., (1988) Biochemistry 27, 3975-3982 (1988); Kozak M (2005) Gene 361, 13-37; Castillo-Mendez, M. A. et al. (2012) Biochimie 94, 662-672; Goodman DB et al., (2013) Science, doi:10.1126/science.1241934; Kudla G et al., (2009) Science 324, 255-258; Bentele K et al., (2013) Molecular systems biology 9, 675; Tuller, T. et al. (2010) Proc Natl Acad Sci U S A 107, 3645-3650). A ribosome-profiling study (Ingolia, N. T. et al. (2009) Science 324, 218-223) concluded that the net translation-elongation rate is effectively constant for *E. coli* mRNAs, irrespective of codon usage (Li, G. W. et al. (2014) Cell 157, 624-635; Li, G.-W. et al. (2012) Nature 484, 538-541). This finding challenges the assumption that differences in the translation rate of synonymous codons influence protein expression, an assumption underlying much of the codon-usage literature (Zhang, F. et al. (2010) Science 329, 1534-1537; Spencer, P. S. et al. (2012) J Mol Biol 422, 328-335; Gingold, H. et al. (2011) Mol Syst Biol 7, 481; Tuller, T. et al. (2010) Proc Natl Acad Sci USA 107, 3645-3650; Quax, T. E. et al. (2013) Cell Rep 4, 938-944; Dana, A. et al. (2014) Nucleic Acids Res 42, 9171-9181; Sharp, P. M. et al. (1987) Nucleic Acids Res 15, 1281-1295) but no alternative mechanism has been proposed to explain the many experiments in which changes in codon usage produce dramatic alterations in protein expression (Gingold, H. et al. (2011) Mol Syst Biol 7, 481).

Uncertainty furthermore exists concerning which codon-related properties are beneficial vs. detrimental for protein expression (Gingold, H. et al. (2011) Mol Syst Biol 7, 481). For example, more homogeneous codon usage has been proposed alternatively to enhance (Cannarozzi, G. et al. (2010) Cell 141, 355-367; Quax, T. E. et al. (2013) Cell Rep 4, 938-944) or reduce (Zhang, G. et al. (2010) Nucleic Acids Res 38, 4778-4787) translation efficiency. Much of the codon-usage literature focuses on inefficient translation of a set of rare codons (Zhang, S.P. et al. (1991) Gene 105, 61-72) in the *E. coli* genome (Sharp, P.M. et al. (1987) Nucleic Acids Res 15, 1281-1295; Zhang, S.P. et al. (1991) Gene 105, 61-72; Ikemura, T. et al. (1981) J Mol Biol 151, 389-409), especially the AUA codon for ile (Caskey, C. T. et al. (1968) J Mol Biol 37, 99-118; Muramatsu, T. et al. (1988) Nature 336, 179-181) and the AGA, AGG, and CGG codons for arg (Chen, G.T. et al. (1994) Genes Dev 8, 2641-2652; Vivanco-Dominguez, S. et al. (2012) J Mol Biol 417, 425-439). On this basis, it is widely assumed that genomic codon-usage frequency, which parallels tRNA pool level (Ikemura, T. et al. (1981) J Mol Biol 151, 389-409; Dong, H. et al. (1996) Journal of Molecular Biology 260, 649-663), influences translation efficiency and that infrequent codons are translated inefficiently (Chen, G. T. et al. (1994) Genes Dev 8, 2641-2652; Caskey, C. T. et al. (1968) J Mol Biol 37, 99-118). However, the expression of a fluorescent reporter protein is increased when the head of the gene contains the rare codons most cited as a barrier to translation (Goodman DB et al., (2013) Science, doi:10.1126/science.1241934). This effect was interpreted to reflect tolerance for inefficient codon usage in the head to prevent stable mRNA folding that would attenuate translation (Goodman DB et al., (2013) Science, doi:10.1126/science.1241934). However, no experiments were performed manipulating either parameter to verify this inference or to dissect their interplay, and alternative theories suggest that rare codons can enhance translation efficiency (Elf, J. et al. (2003) Science 300, 1718-1722; Dittmar, K. A. et al. (2005) EMBO Rep 6, 151-157; Tuller, T. et al. (2010) Cell 141, 344-354). The evolutionary biology literature focuses on a different correlate of genomic codon-usage frequency, which is accuracy in protein synthesis (Wallace, E. W. et al. (2013) Mol Biol Evol 30, 1438-1453; Bulmer, M. (1991) Genetics 129, 897-907; Akashi, H. (1994) Genetics 136, 927-935). Biochemical studies suggest that more frequent codons should be translated more accurately because the levels of their cognate tRNAs are systematically higher, and competition from near-cognate tRNAs is the major cause of translational errors (Ikemura, T. et al. (1981) J Mol Biol 151, 389-409; Dong, H. et al. (1996) Journal of Molecular Biology 260, 649-663; Kramer, E. B. et al. (2007) RNA 13, 87-96; Zaher, H. S. et al. (2011) Cell 147, 396-408). Usage of more frequent codons is enhanced at more conserved sites in proteins (Ran, W. et al. (2014) MBio 5, e00956-00914; Akashi, H. (1994) Genetics 136, 927-935), presumably because more accurate translation (Ninio, J. (1986) FEBS Lett 196, 1-4) at such sites promotes greater evolutionary fitness (Wallace, E. W. et al. (2013) Mol Biol Evol 30, 1438-1453; Drummond, D. A. et al. (2008) Cell 134, 341-352). While lower frequency codons also can be translated less efficiently (Dana, A. et al. (2014) Nucleic Acids Res 42, 9171-9181; Rocha, E. P. (2004) Genome Res 14, 2279-2286), a systematic correlation between these parameters has yet to be demonstrated

One factor complicating investigations of the influence of mRNA sequence on protein expression is that synonymous changes in sequence can simultaneously influence multiple mechanistic factors related to protein translation - codon identity, codon homogeneity, and mRNA folding as well as other potentially influential local and global sequence features that range from codon-pair effects to overall A/U/C/G content. Previous experimental and theoretical studies have focused on individual parameters or pairs of parameters in a local region of the mRNA (Goodman DB et al., (2013) Science, doi:10.1126/science.1241934; Kudla G et al., (2009) Science 324, 255-258; Bentele K et al., (2013) Molecular systems biology 9, 675; Cannarozzi G et al., (2010) Cell 141, 355-367; Li, GW et al., (2012) Nature 484, 538-541), and few mechanistic inferences from these studies have been tested using biochemical methods. For example, several publications have examined the relationship between translation efficiency, and (a) codon usage frequency, (b) the accuracy of protein translation, (c) the concentration of charged cognate tRNAs, (d) homogeneity and inhomogeneity (diversity) in codon usage within a gene, (e) genomic-scale studies, (f) local concentration of cognate tRNAs and aminoacyl tRNA synthetases near ribosomes (Goodman DB et al., (2013) Science, doi:10.1126/science.1241934; Elf J et al., (2003) Science 300, 1718-1722; Bulmer M et al., (1991) Genetics 129, 897-907; Cannarozzi G et al., (2010) Cell 141, 355-367)

In certain aspects, the methods described herein relate to the finding that codons for arginine, aspartate, glutamate, glutamine, histidine, and isoleucine can be substituted with synonymous codons that have high "codon slopes", as determined by linear regression analysis of codon frequencies and protein expression levels

In certain aspects, the methods described herein relate to the finding that codon-slopes determined using single-parameter logistic regressions show that codons ending in A or U are systematically enriched in the genes giving the highest level of protein expression in the current dataset, while the synonymous codons ending in G or C are systematically depleted in these genes. Thus, in certain aspects, the findings provide guidance for engineering synthetic genes that enhance protein expression by emulating the properties of the best-expressed genes in the current dataset.

In certain aspects, the methods described herein relate to the finding that the in-frame codon model is superior to non-reading frame models or to a parabolic model for the overall base compositions at each codon position. The number of degrees of freedom (d.f.) may be one less than the number of non-stop codons because the sum of frequencies equals one.

In certain aspects, the methods described herein relate to the finding that for codons 2-6 (the ribosome initiation site), the base composition variables are more descriptive than codon frequencies. The interaction term with composition and the predicted free energy of folding of the RNA sequence corresponding to the head highlights the importance of unstable folding in this region. In the methods described herein, expression may increase if extra weight is given for the mean slopes of codons 7-16 and to a lesser extent 16-32 even where adding a mean codon slope variable for codons 2-6 is statistically insignificant. In certain aspects of the disclosure, including a variable for the frequency of the Shine-Dalgarno consensus AGGA in any frame does not improve the model at the 5% significance level.

In certain aspects of the disclosure, the head and tail regions are of similar overall importance in the models described herein. Codons 1-6 (initiation) may be influential to protein expression and are governed by their composition and secondary structure propensity. Codon 7-32 slopes may be about three times as influential as slopes of codons later in the tail. Iterative application of the methods described herein can be used to increase or reduce the expression of a polypeptide in an expression system, including, but not limited to in vivo expression systems and in-vitro expression systems.

In certain aspects, the methods described herein relate to the finding that reducing the RNA unfolding energy of an RNA sequence within a computational window comprising about the first 48 nucleotides of the coding sequence immediately 3' to the 5'UTR can be used to improve expression of a polypeptide encoded by the RNA when the polypeptide is expressed in an expression system. In certain aspects, the methods described herein relate to the finding that reducing the RNA unfolding energy of an RNA sequence within a computational window comprising the 5'UTR and a region comprising about the first 48 nucleotides of the coding sequence immediately 3' to the 5'UTR can be used to improve expression of a polypeptide encoded by the RNA when the polypeptide is expressed in an expression system.

Thus, in certain aspects, the methods described herein provide a predictive quantitative metric useful for determining when RNA secondary structure affects protein translation in an expression system (e.g. in an E. coli cell).

Iterative application of the methods described herein can be used to increase or reduce the expression of a polypeptide in an expression system, including, but not limited to in vivo expression systems and in vitro expression systems.

Proteins may have been selected from a wide variety of source organisms based on structural uniqueness. No sequence with greater than 30% amino acid identity had an experimentally determined structure deposited into the Protein Data Bank at the time of selection. The dataset was filtered to reduce the amino acid identity between any two proteins to be less than 60%. The analyzed dataset included 6,348 genes from 171 organisms, as detailed in the cladogram in **Figure 15****.** It contained 95 endogenous *E. coli* genes, including *ycaQ* that was examined in biochemical experiments, and 6,253 genes from heterologous sources, including 47 from mammals, 809 from archaeabacteria, and the remainder from 151 different eubacterial organisms.

The predominance of heterologous genes in the dataset has several advantages relative to the use of large-scale experimentation to probe biochemical mechanism. The central premise may be that one way to understand the fundamental mechanisms underlying physiological processes is to challenge the biochemical apparatus in a given organism with sequences that have NOT evolved under selective pressure in that organism. Evolutionary processes will tend to exert parallel selective effects on sequential steps in a physiological pathway, which can create surrogate effects - significant sequence correlations that do not reflect a direct mechanistic effect. Regulation of protein expression minimally involves the interplay of transcription, translation, RNA degradation, and protein degradation. Endogenous *E. coli* genes are likely to have sequence features influencing some of these interconnected processes but not others, which can produce surrogate effects, and their expression can also be influenced by gene/protein-specific regulatory systems. These problems were circumvented with endogenous *E. coli* genes by evaluating the expression of heterologous proteins without *E. coli* orthologs that were encoded by synthetic gene sequences designed using a well-defined computational algorithm. However, some starting point for the development of a gene-design algorithm was needed, and it was concluded that genes from heterologous organisms provide more effective reagents than endogenous *E. coli* genes for interrogation of the fundamental biochemical properties of the physiological systems in *E. coli.*

To the extent that there is divergence in the biochemical and physiological properties of the source organisms compared to *E. coli,* evaluating the expression of genes from heterologous sources reduces the extent of the evolutionary cross-correlations and surrogate effects discussed above. Only biochemical effects that are universally conserved among the diverse source organisms can produce strong surrogate effects due to parallel selection for sequence features influencing sequential steps in the expression pathway. Universally conserved biochemical mechanisms will influence statistical analyses performed on any dataset examining net protein expression level, irrespective of the source of the gene sequences. However, the experimental design employing heterologous proteins from diverse phylogenetic sources can suppress surrogate effects of this kind the statistical analyses described herein.

Genes from heterologous organisms have the additional advantage of reducing or eliminating effects from gene/protein-specific regulatory systems.

Genes from heterologous sources have the additional advantage of providing greater diversity in sampling of codon-space than would be possible using exclusively genes from *E. coli* or any other single organism. Furthermore, it has provided greater diversity than achieved in previous studies using synthetic genes to examine codon-usage effects.

It is important to verify that some endogenous *E. coli* genes exhibit behavior consistent with inferences derived from experiments on heterologous genes. The *E. coli* gene *ycaQ* may have been included in the mechanistically resolved studies. This endogenous gene/protein behaved similarly in all assays to the genes/proteins from heterologous sources. Another way to address this issue is to compare the performance of the computational model predicting high *vs.* no expression when applied to the *E. coli* genes or heterologous genes in the large-scale protein-expression dataset (**Figure 41**). This analysis shows that the computational model performs similarly on both sets of genes, supporting the validity of the approach using heterologous gene sequences to interrogate the fundamental biochemical properties of the physiological systems *in E. coli.*

Indirect evolutionary couplings and parallel selection operating on sequential steps in a pathway can create significant sequence correlations that do not reflect a direct mechanistic effect. The predominance of heterologous genes in large-scale dataset should reduce but may not eliminate the influence of surrogate effects. These considerations highlight the importance of the *in vitro* transcription and translation assays using purified components that are presented herein. Assays represent the most rigorous approach possible to verifying that the strong codon effects identified in the statistical analyses discussed herein have a mechanistic effect on protein translation efficiency.

In contrast, the codon-efficiency metrics used in the extensive prior literature on this topic were never validated in biochemical experiments of this kind, meaning that they can potentially derive in part or even entirely from indirect correlations and parallel selective effects. One example of this phenomenon is provided by a paper published by Presnyak et al. (Cell 160:1 111). These authors claim that protein translation efficiency in the yeast *Saccharomyces cerevisiae* strongly influences mRNA stability. While it is possible that this claim is accurate because of its strong resonance with an important conclusion from the studies in *E. coli* presented herein, their claim is based on a theoretical metric for translational efficiency called the tRNA Adaptation Index (tAI) that has never been validated to influence protein translation efficiency in prior literature on any organism. The tAI for *E. coli* correlates only weakly with the codon metric (**Fig 31D**). This is demonstrated to influence protein translation efficiency strongly both *in vivo* and *in vitro.* Therefore, the tAI itself as well as the effects reported by Presnyak *et al.* can potentially derive in whole or in part from parallel selection phenomena. Presnyak *et al.* furthermore present single-variable regression analyses of the relationship between mRNA lifetime and codon frequency, but **Figure 17** demonstrates that single-variable analyses of this kind on the dataset yield misleading conclusions concerning the effects of individual codons, because they are dominated by cross-correlations in the codon content of the genes - *i.e.,* an indirect evolutionary correlation. In this context, the codon metric reported by Presnyak *et al*., which has not been demonstrated experimentally to influence protein translation efficiency *in vitro,* can measure primarily mRNA degradation effects, which is all that they have measured, and its apparent dependence on reading frame can derive from parallel evolutionary selection.

The native and redesigned genes were explicitly subjected to *in vitro* transcription assays and *in vitro* translation assays. In contrast, this shows that the sequence features that were inferred to influence mRNA translation into protein directly modulate this biochemical process. Mechanistically resolved *in vitro* experimentation of this kind is essential to demonstrate rigorously that sequence features inferred from analyses of naturally evolved genes influence a specific biochemical process and do not derive from surrogate effects attributable to parallel selective pressures. The *in vitro* assays described herein showing that genes redesigned based on the computational model may have the predicted influence on translation represent a fundamentally important component of the invention described herein. Reliable conclusions regarding biochemical mechanism would not have been possible without them.

Despite these advantages in experimental design, complicated evolutionary and physiological factors can influence results from such statistical analyses on naturally occurring genes. Thus, experiments were performed to directly evaluate the experimental behavior of synthetic genes with sequences designed based on statistical inferences. The results obtained from these sequences using mechanistically resolved biochemical assays have been significantly reinforced by the new *in vivo* analyses that were performed at physiological expression level under the control of *E. coli* RNA polymerase.

As used herein, a folded RNA molecule can be an RNA molecule in a native conformation in the absence of denaturing conditions. A folded RNA can also be an RNA molecule in its lowest Gibbs free energy state. A folded RNA can also be an RNA molecule in a collection of structure in thermal equilibrium with relative probabilities as determined by partition function based methods. Without wishing to be bound by theory, RNA molecules may exhibit one or more alternative folded states of identical or similar Gibbs free energy states. Such states can depend upon environmental and experimental conditions of analysis, including, but not limited to buffer, temperature, presence of ligands, and the like. One of skill in the art will readily be capable of accounting for differences in environmental and experimental conditions when calculating or comparing RNA folding patterns.

One of skill in the art will appreciate that there are an exponential number of ways for an RNA molecule to fold. This exponential number can be expressed as 1.8^{N}, where N is the number of nucleic acids in the molecule. The folded state of an RNA molecule is determined by intramolecular base-pairing patterns as well as well as higher-order structures stabilized by covalent or non-covalent bonding. The folding of RNA molecules occurs in a hierarchical process wherein the folding of secondary structure elements dictates the formation of tertiary contacts within the RNA molecule (Brion et al., "Hierarchy and Dynamics of RNA Folding," Annu. Rev. Biophys. Biomol. Struct. 26:113-137 (1997)). RNA molecules comprise four different heterocyclic aromatic base residues. Although RNA Watson-Crick G-C and A-U pairs are strong, it is known that G U Wobble-base pairs can form. Secondary structure formation in RNA molecules is driven in part by stacking between contiguous base pairs. This stacking process involves greater energies than those involved in the formation of tertiary interactions (Tinoco et al., "How RNA Folds," J. Mol. Biol. 293:271-281 (1999)). RNA folding energies depend in part on the existence of secondary structures in an RNA molecule (Flamm et al., "RNA Folding at Elementary Step Resolution," RNA 6:325-338 (2000)).

Algorithms designed to determine global minimum and near optimal structures as well as the quantification of folding energies can be used in connection with the methods described herein (Zuker, M. (1989) Science 244, 48-52). Several software platforms have been developed for predicting the tertiary structure of nucleic acid molecules. As such, methods for calculating the RNA folding energies suitable for use with the methods described herein can be any method known in the art, including, but not limited, algorithms useful for determining the minimum free Gibbs energy of a given structure and/or algorithms useful for determining a partition function for a given RNA molecule structure. Many tools have been developed for predicting the secondary structure of RNA by using thermodynamic methods (the Gibbs free energy). Without wishing to be bound by theory, thermodynamics-based structure prediction relies on the presumption that the Minimum Gibbs Free Energy (MFE) structure (i.e. the structure in which the RNA molecule has the lowest free energy) is the most likely conformation for that RNA molecule even though suboptimal folds for the RNA molecule may otherwise exist in nature. For example, thermodynamics computational methods may not always accurately account for potential tertiary interactions and thus the true structure of an RNA molecule may be a suboptimal folding pattern. There are two thermodynamic-based algorithmic approaches: (1) identify the one structure that has the minimum free energy (MFE) according to the Turner model (Mathews et al, J. Mol. Biol., 288, 911-940 (1999); Turner and Mathews, Nucleic Acids Research, 38, D280-D283 (2009)), or (2) compute the partition function which involves all of the structures. In accordance with the methods described herein, the minimum free energy structure of an RNA molecule (i.e. the most stable structure), is used to represent the overall conformational energetics of an given RNA sequence. In accordance with the methods described herein, the partition function approach is used to represent the overall conformational energetics of an given RNA sequence.

In the minimum free energy approach, the minimum free energy can be computed recursively. Because the Turner model is additive, the total free energy is the sum of free energies for substructures. Thus, the minimum free energy of sub-structures can be computed and assembled to find the minimum free energy of bigger substructures recursively. Minimum free energy structures for RNA molecules can be calculated using any method known in the art, including, but not limited to the Mfold algorithm. The Mfold program determines the minimum free energy conformation (most stable) by exploring all possible base pairings in a nucleic acid sequence (Zuker and Stiegler, Nucleic Acids Res. 9 (1) (1981), 133-148; Zuker, Science, 244,48-52, (1989); Jaeger et al., Proc. Natl. Acad. Sci. USA, Biochemistry, 86:7706-7710 (1989); Jaeger et al., Predicting Optimal and Suboptimal Secondary Structure for RNA. in "Molecular Evolution: Computer Analysis of Protein and Nucleic Acid Sequences", R. F. Doolittle ed., Methods in Enzymology, 183, 281-306 (1989)).

Other methods for assessing RNA folding suitable for use with the methods described herein include partition function based methods. The partition function gives base-pairing probabilities for a Boltzmann ensemble of secondary structures. In partition function based methods, all possible secondary structure conformations and each of their respective energies are calculated to determine the most prevalent conformation by generating a probability of a given base-pair based on the partition function calculation. Thus, the most prevalent conformation for an RNA molecule may not be the same as the Minimum Gibbs Free Energy (MFE) structure where multiple suboptimal confirmations exist. If a given RNA molecule did not have suboptimal folds, the partition structure will be equivalent to the Minimum Gibbs Free Energy structure. In the partition function approach, the free energies of all of the states (not just the one MFE state) contribute. $G = - kT Log \left[Sum_s Exp\left\{-G_s/kT\right\}\right] .$

The exponentials are Boltzmann weights which relate to the thermal probability of each state. The sum of all of the Boltzmann weights is called the partition function. The average thermal energy kT=(Boltzmann's factor)(absolute temperature). The partition function G accounts for the entropy of mixing of all of the states. The partition function computation can rely on the same dynamic programming algorithmic approach as was used to compute MFE (McCaskill (1990)).

The total predicted free energy of folding of the RNA sequence according to the methods described herein may be calculated by partition function based methods. Exemplary partition function based methods include those describe in McCaskill Biopolymers, 29, 1105-1119 (1990). Another partition function based method suitable for use with the methods described herein includes, the RNA secondary structure prediction program RNAStructure (see Proc. Natl. Acad. Sci., 101, 7287-7292 (2004)). RNAStructure is a folding algorithm that uses empirical energy values measured in vitro to predict RNA conformations and their relative free energy. Both MFE and partition function methods are implemented in the RNAstructure code. The algorithm can be used to predict lowest free energy structures and base pair probabilities for a RNA sequence and can be constrained using experimental data, including SHAPE, enzymatic cleavage, and chemical modification accessibility. Another partition function based method suitable for use with the methods described herein includes the SFold algorithm (Ding and Lawrence (2003) Nucleic Acids Res. 31 (24): 7280-301; Ding et al., (2004) Nucleic Acids Res. 32 (Web Server issue): W135-41; Ding et al., (2005) RNA. 11 (8): 1157- 66; Chan et al., Bioinformatics 21 (20): 3926-8). The Sfold algorithm employs statistical sampling of all possible structures weighted by partition function probabilities that is not dependent upon free energy minimization.

Algorithms capable of computing both Minimum Gibbs Free Energy (MFE) structures and partition function based structures are also known in the art. For example, the Vienna RNA package predicts secondary structure by using two kinds of dynamic programming algorithms: the minimum free energy algorithm of Zuker and Stiegler (Nucl. Acid. Res. 9: 133-148 (1981)) and the *partition function* algorithm of McCaskill (Biopolymers 29, 1105-1119 (1990)). See Hofacker et al., J Mol Biol 319, 1059 (Jun. 21, 2002).

Other RNA folding algorithms suitable for use with the methods described herein include, but are not limited to, Kinefold (Xayaphoummine et al., (2003) Proc. Natl. Acad. Sci. U.S.A. 100(26): 15310-5; Xayaphoummine et al., (2005) Nucleic Acids Res. 33 (Web Server issue): W605-10), CentroiFold (Hamada *et al.* (2009)), CONTRAfold (Do et al., (2006) Bioinformatics 22 (14): 90-8), CyloFold (Bindewald et al., (2010) Nucleic Acids Res. Suppl (W): 368-72); PknotsRG (Reeder et al., (2007) Nucleic Acids Res. 35 (Web Server issue): W320-4; Bompfünewerer et al., (2008) J. Math Biol., 56 (1-2): 129-144), RNAshapes (Giegerich et al., (2004) Nucleic Acids Res. 32 (16): 4843-4851; Voß B et al., (2006). BMC Biol. 4: 5), and UNAFold (Markham NR and Zuker M (2008) Methods Mol. Biol 453: 3-31. Other RNA folding algorithms suitable for use with the methods described herein include those described in Dirks and Pierce (2003) J. Comput. Chem. 24, 1664-1677; Dirks and Pierce (2004) J. Comput. Chem. 25, 1295-1304; Han and Byun (2003) Nucleic Acid Res. 31, 3432-3440.

In certain aspects, RNA folding algorithms can be used to calculate the folding energy of part or all of an RNA molecule. For example, the methods described herein relate to the finding that a greater stability of secondary structures in a calculation window at or near the 5' end of an mRNA encoding a polypeptide is correlated with reduced expression of the polypeptide in an expression system. Thus, the RNA folding algorithms described herein can be applied to a calculation window of an RNA sequence to determine whether expression of a polypeptide encoded by the RNA can be increased by reducing the stability of RNA structures within the calculation window. The calculation window can be of any size and folding energies can be calculated for multiple calculation windows for a given RNA sequence. Where multiple calculation windows are employed, the windows can be successive, non-successive or overlapping along the RNA sequence.

One of skill in the art will appreciate that the methods described herein can be adapted to any expression system, polypeptide or expression vector and that the quantitative threshold for other expression system, polypeptide or expression vector can differ from the quantitative thresholds described herein.

In certain aspects, the invention relates to the finding that the predicted folding energy of an RNA sequences is determinative of reduced expression of a polypeptide encoded by the RNA sequence when the folding energy is below a threshold level. Thus, the methods described herein may be useful for predicting when RNA unfolding energy inhibits expression of a polypeptide encoded by the RNA. The methods described herein are also useful for determining when reducing RNA unfolding energy of a RNA encoding a polypeptide can be useful for increasing expression of a polypeptide encoded by the RNA.

The stability of the secondary structure of an RNA molecule can be quantified as the amount of free energy that is released or used upon the formation of base pairs. Because free energies are additive, the total free energy of an RNA secondary structure can be determined by adding the component free energies in the structure. The unit of measurement of the free energy of an RNA molecule can be defined in units of kcal/mol.

A threshold the predicted free energy of folding of the RNA sequence may be about -39 kcal/mol or higher as measured over a calculation window consisting essentially of the first 48 bases of the coding sequence of an nucleic acid sequence encoding a polypeptide plus about 90 nucleic acids of a 5'UTR sequence functionally linked to the coding sequence, will be predictive that the polypeptide encoded by the nucleic acid will be expressed at a suitable level in an expression system. A threshold the predicted free energy of folding of the RNA sequence may be about -35 kcal/mol or higher as measured over a calculation window consisting essentially of the first 48 bases of the coding sequence of an nucleic acid sequence encoding a polypeptide plus about 90 nucleic acids of a 5'UTR sequence functionally linked to the coding sequence, will be predictive that the polypeptide encoded by the nucleic acid will be expressed at a suitable level in an expression system. A threshold the predicted free energy of folding of the RNA sequence may be about -30 kcal/mol or higher as measured over a calculation window consisting essentially of the first 48 bases of the coding sequence of an nucleic acid sequence encoding a polypeptide plus about 90 nucleic acids of a 5'UTR sequence functionally linked to the coding sequence, will be predictive that the polypeptide encoded by the nucleic acid will be expressed at a suitable level in an expression system. A threshold the predicted free energy of folding of the RNA sequence may be about -25 kcal/mol or higher as measured over a calculation window consisting essentially of the first 48 bases of the coding sequence of an nucleic acid sequence encoding a polypeptide plus about 90 nucleic acids of a 5'UTR sequence functionally linked to the coding sequence, will be predictive that the polypeptide encoded by the nucleic acid will be expressed at a suitable level in an expression system. In A threshold the predicted free energy of folding of the RNA sequence may be about -20 kcal/mol or higher as measured over a calculation window consisting essentially of the first 48 bases of the coding sequence of an nucleic acid sequence encoding a polypeptide plus about 90 nucleic acids of a 5'UTR sequence functionally linked to the coding sequence, will be predictive that the polypeptide encoded by the nucleic acid will be expressed at a suitable level in an expression system.

A threshold the predicted free energy of folding of the RNA sequence may be about -10 kcal/mol as measured over a calculation window consisting essentially of the first 48 bases of the coding sequence of a nucleic acid sequence encoding a polypeptide will be predictive that the polypeptide encoded by the nucleic acid will be expressed at a suitable level in an expression system

A threshold the predicted free energy of folding of the RNA sequence at least about -5 kcal/mol as measured over a calculation window consisting essentially of the first 48 bases of the coding sequence of an nucleic acid sequence encoding a polypeptide may be predictive that the polypeptide encoded by the nucleic acid will be expressed at a suitable level in an expression system.

A predicted free energy of folding of an RNA sequence range as measured over a nucleic acid sequence downstream of the first 48 bases of a coding sequence can be predictive that the polypeptide encoded by the nucleic acid will be expressed in an expression system. More specifically, the a predicted free energy of folding of an RNA sequence range of a nucleic acid sequence downstream of the first 48 bases of a coding sequence can be measured in one or more calculation windows so as to cover the length of the sequence downstream of the first 48 bases of a coding sequence.

Predicted free energy of folding of an RNA sequence thresholds calculated over one or more windows in a tail sequence can be predictive that a polypeptide encoded by the nucleic acid will be expressed at a suitable level in an expression system. The windows may be non-overlapping over the length of the tail sequence. The windows may be overlapping. Overlap of the windows in the tails sequence can be selected from an overlap of 1, 2, 3, 4, 5, 6, 7, 8, 9, 10, 11, 12, 13, 14, 15, 16, 17, 18, 19, 20, 21, 22, 23, 24, 25, 26, 27, 28 , 29 30, 31, 32, 33, 34, 35, 36, 37, 38, 39, 40, 41, 42, 43, 44, 45, 46, 47, 48, 49, 50, or more than 50 nucleic acids in length. The window may be 144 nucleic acids in length. The window may be 96 nucleic acids in length. The window may be 48 nucleic acids in length.

A predicted free energy of folding of an RNA sequence range corresponding to each of one or more tail sequence windows within the tail sequence a range of about $\left(-0.32∗\left(W-18\right)\right) kcal/mol minus 10 kcal / mol or plus 5 kcal / mol$
where W is the number of nucleotides in the tail sequence window, will be predictive that the polypeptide encoded by the nucleic acid will be expressed at a suitable level in an expression system. The methods described herein may involve increasing the predicted free energy of folding of an RNA sequence in a sequence window downstream of the first 48 nucleic acids in a coding sequence will be in the range of about -40 kcal/mol to about -20 kcal/mol when the window(s) for the tail region around about 96 nucleic acids long.

Thus, it will be appreciated that mutagenesis techniques to reduce the unfolding energy of a RNA calculation window comprising less than essentially the first 48 bases can be used to improve expression of a polypeptide encoded by the RNA.

In certain aspects, the present invention is directed to methods for generating modified RNA sequences capable of directing higher polypeptide expression as compared to the corresponding wild-type RNA sequence by reducing the stability of one or more RNA structure within a sequence window comprising about the first 48 nucleic acids in the coding sequence of the RNA. For example, the methods described herein can be implemented to predictively grade expression on the basis of RNA folding energy for RNA molecules encoding particular polypeptides. Alternatively, the methods described herein can be used to optimize or design improved expression vectors suitable for the production of polypeptides in an expression system.

In certain aspects, the methods described herein can be used to reduce RNA folding energies according to the correlation of the effect of RNA folding energy on the expression of a polypeptide encoded by the RNA. In one aspect, the present invention is directed to a nucleic acid encoding a recombinant polypeptide that has been mutated to reduce folding energy of 5' untranslated and/or coding region sequences of a nucleic acid sequence encoding the polypeptide. The methods described herein may be directed to methods of making such mutations.

One of skill in the art will appreciate that the methods for increasing expression of a polypeptide as described herein can be limited by certain structural features inherent to an RNA molecule encoding the polypeptide. For example, it is understood that functional integrity of Shine-Dalgarno and initiation codon sequences can be maintained for protein expression. Thus, modifications that increased the expression of a polypeptide according to the methods described herein may be performed exclusively on coding sequence regions in an RNA molecule. Modifications that increase the expression of a polypeptide according to the methods described herein may be performed on regions that do not include Shine-Dalgarno sequences. Modifications that increase the expression of a polypeptide according to the methods described herein may be performed on regions that do not include translation initiation sequences. Modifications that increase the expression of a polypeptide according to the methods described herein may be performed on regions that do not include transcription promoter sequences.

The predicted free energy of folding of an RNA structure depends on a number of parameters associated with the pairing configurations in the structure. Such parameters include, but are not limited to base pair stacks and internal base pairs, internal, bulge and hairpin loops, and defined motifs. The effect of each of these parameters on the stability of an RNA structure is also known in the art. For example, parameters that are known to affect the stability of an RNA structure include the number of GC versus AU and GU base pairs, the number of base pairs in a stem region, the number of base pairs in a hairpin loop region, the number of unpaired bases in interior loops and the number of unpaired bases in bulges. Thus, one of skill in the art will readily appreciate that the methods described herein can be used in conjunction with known methods for reducing the stability of an RNA structure within a RNA calculation window so as to increase the expression of a polypeptide encoded by the RNA in an expression system.

Thus, the methods described herein can be used to reduce the stability of an RNA structure in a RNA calculation window by reducing the number of GC base pairs relative to the number of AU and GU base pairs within the window or reducing the number of GC, down to, and including, zero GC pairs. The methods described herein can be used to reduce the stability of an RNA structure in a RNA calculation window by increasing the number of unpaired bases in an interior loop within the window. The methods described herein can be used to reduce the stability of an RNA structure in a RNA calculation window by increasing the number of unpaired bases in an bulge within the window. The methods described herein can be used to reduce the stability of an RNA structure in a RNA calculation window by decreasing the number of base pairs in a stem region within the window so as to generate larger loops or bulges. The methods described herein can be used to reduce the stability of an RNA structure in a RNA calculation window by increasing the number of base pairs in a loop region within the window. The stability of an RNA structure can be reduced by introducing loops or bulges having 8 or more bases.

The methods for improving the expression of a polypeptide described herein can also be combined with any other method known the art suitable for improving polypeptide production. For example, the methods described herein can be used to improve the expression of a polypeptide by introducing one or more modifications with the coding sequence of an RNA encoding the polypeptide. In such cases, it can be useful to do so without altering amino acid sequence of the polypeptide. In embodiments where the expression altering modification is in a coding region of an RNA sequence, the expression altering modification can replace a codon sequence such that the modification does not alter the amino acid(s) encoded by the nucleic acid. For example, in the event that the expression increasing modification is a CTG codon, and the coding sequence being replaced by the mutation can be any of AGA, AGG, CGA, CGC or CGG codon, each of which also encode arginine. In the event that the expression increasing modification is a GCG codon, and the coding sequence being replaced by the mutation can be any of GCT, GCA, or GCC codon, each of which also encode alanine. In the event that the expression increasing modification is a GGG codon, and the coding sequence being replaced by the mutation can be any of GGT, GGA, or GGC codon, each of which also encode glycine. One of skill in the art can readily determine how to change one or more of the nucleotide positions within a codon without altering the amino acid(s) encoded, by referring to the genetic code, or to RNA or DNA codon tables. Canonical amino acids and their three letter and one-letter abbreviations are Alanine (Ala) A, Glutamine (Gln) Q, Leucine (Leu) L, Serine (Ser) S, Arginine (Arg) R, Glutamic Acid (Glu) E, Lysine (Lys) K, Threonine (Thr) T, Asparagine (Asn) N, Glycine (Gly) G, Methionine (Met) M, Tryptophan (Trp) W, Aspartic Acid (Asp) D, Histidine (His) H, Phenylalanine (Phe) F, Tyrosine (Tyr) Y, Cysteine (Cys) C, Isoleucine (Ile) I, Proline (Pro) P, Valine (Val) V.

The methods described herein may be useful for altering the expression of a recombinant polypeptide by making one or more conservative substitutions in the amino acid sequence of the polypeptide. Such mutations may result in one or more different amino acids being encoded, or may result in one or more amino acids being deleted or added to the amino acid sequence. If the expression altering modification does affect the amino acid(s) encoded, it is possible to make one of more amino acid changes that do not adversely affect the structure, function or immunogenicity of the polypeptide encoded. For example, the mutant polypeptide encoded by the mutant nucleic acid can have substantially the same structure and/or function and/or immunogenicity as the wild-type polypeptide. It is possible that some amino acid changes may lead to altered immunogenicity and artisans skilled in the art will recognize when such modifications are or are not appropriate.

It is known to one skilled in the art that a polypeptide having one or more conservative amino acid substitutions will not necessarily result in the polypeptide having a significantly different activity, function or immunogenicity relative to a wild type polypeptide. A conservative amino acid substitution occurs when one amino acid residue is replaced with another that has a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine), aromatic side chains (e.g., tyrosine, phenylalanine, tryptophan, histidine), aliphatic side chains (e.g., glycine, alanine, valine, leucine, isoleucine), and sulfur-containing side chains (methionine, cysteine). Substitutions can also be made between acidic amino acids and their respective amides (e.g., asparagine and aspartic acid, or glutamine and glutamic acid). For example, replacement of a leucine with an isoleucine may not have a major effect on the properties of the modified recombinant polypeptide relative to the non-modified recombinant polypeptide.

The methods described herein can also be used in conjunction with methods disclosed in International Patent Application PCT/US11/24251 entitled Methods for Altering Polypeptide Expression and Solubility. PCT/US11/24251 describes methods for altering the expression or solubility of a polypeptide by using a codon replacement strategy based on the finding that synonymous codons can have differential effects on protein production. Thus, the methods described herein can be used to increase the expression of a polypeptide encoded by an RNA by reducing the secondary structure of the RNA molecule according to the methods described herein and altering one or more codons in the coding sequence of the RNA so as to further increase solubility or expression of the protein.

The generation of a mutation for the purpose of decreasing the stability of an RNA structure in a coding sequence according to the methods described herein can be performed by biasing the mutagenesis strategy to select a solubility or expression increasing codon as set forth in International Patent Application PCT/US11/24251. For example, in a mutagenesis strategy designed to reduce the stability of an RNA structure according to the methods described herein wherein the method involves any of (a) reducing the number of GC base pairs relative to the number of AU and GU base pairs (b) reducing the number of base pairs in a stem region, (c) altering the number of base pairs in a hairpin loop region, (d) introducing hairpin loops of greater than 8 nucleotides, (e) increasing the number of unpaired bases in an interior loop, or (f) increasing the number of unpaired bases in an bulge in an RNA calculation window comprising an coding sequence of the RNA, the mutagenesis strategy can involve replacing an arginine codon selected from any of AGA, AGG, CGA, or CGC with a CTG codon if mutagenesis of the codon also reduces the stability of an RNA structure within the sequence window. Other expression and solubility increasing codon substitutions provided in PCT/US11/24251 can be used in conjunction with the methods described herein.

Also suitable for use with the methods described herein is any technique known in the art for altering the expression of a recombinant polypeptide in an expression system (e.g. expression of a human polypeptide in a bacterial cell), including methods for increasing or decreasing expression or solubility of a polypeptide as described in International Patent Application PCT/US11/24251. Techniques that have been developed to facilitate expression generally focus on optimization of factors extrinsic to the target polypeptide itself (Makrides (1996) Microbiology and Molecular Biology Reviews 60:512; Sorensen and Mortensen (2005) Journal of biotechnology 115:113-128). Techniques for altering expression are known in the art, include, but are not limited to, co-expression of fusion partners (including MBP (Kapust and Waugh (1999) PRS 8:1668-1674), smt (Lee et al. (2008) Polypeptide Sci. 17:1241-1248), and Mistic (Kefala et al. (2007) Journal of Structural and Functional Genomics 8:167-172)), codon enhancement (Carstens (2003) Methods in Molecular Biology 205:225-234; Christen et al. (2009) Polypeptide Expression and Purification), or optimization (Gustafsson et al. (2004) Trends in biotechnology 22:346-353; Kim et al. (1997) Gene 199:293-301; Hatfield GW, Roth DA (2007) Biotechnol Annu Rev 13:27-42) (including removal of 5' RNA secondary structure (Etchegaray and Inouye (1999) Journal of Biological Chemistry 274:10079-10085)), and the use of protease deficient strains (Gottesman (1990) Methods in enzymology 185:119). Techniques that have been developed specifically to improve solubility of recombinant polypeptides include chaperone co-expression (Tresaugues et al. (2004) Journal of Structural and Functional Genomics 5:195-204; Mogk et al. 2002 Chembiochem 3, 807; Buchner, Faseb J. 1996 10, 10; Beissinger and Buchner, 1998. J. Biol. Chem. 379, 245)), fusion to solubility-enhancing tags or polypeptide domains (Kapust and Waugh (1999) PRS 8:1668-1674; Davis et al. (1999) Biotechnology and bioengineering 65), expression at lower temperature (Makrides (1996) Microbiology and Molecular Biology Reviews 60:512), heat shock (Chen et al. (2002) Journal of molecular microbiology and biotechnology 4:519-524), expression in a different growth medium (Makrides (1996) Microbiology and Molecular Biology Reviews 60:512; Georgiou and Valax (1996) Current Opinion in Biotechnology 7:190-197), reduction of polypeptide expression level (e.g., by using less inducer or a weaker promoter (Wagner et al. (2008) Proc. Natl. Acad. Sci. U.S.A 105:14371-14376)), directed evolution (Pédelacq et al. (2002) Nature biotechnology 20:927-932; Waldo (2003) Current opinion in chemical biology 7:33-38), and rational mutagenesis (Dale et al. (1994) Polypeptide Engineering Design and Selection 7:933-939).

E. coli has served as a model system for characterizing basic cellular biochemistry for more than 50 years, and significant insight into the biochemistry of other organisms including humans derives from studies conducted in E. coli. Therefore, results obtained from the E. coli data mining studies described herein can also be applied to protein expression in any living cell or in ribosome-based in vitro translation systems. In addition, the methods also relate to methods for the design of synthetic genes, de novo, and for enhanced accumulation a of its encoded polypeptide or the polypeptide product in a host cell.

The methods described herein can be used to increase or decrease the expression of a polypeptide expressed in any type of expression system known in the art. Expression systems suitable for use with the methods described herein include, but are not limited to in vitro expression systems and in vivo expression systems. Exemplary in vitro expression systems include, but are not limited to, cell-free transcription/translation systems (e.g., ribosome based protein expression systems). Several such systems are known in the art (see, for example, Tymms (1995) In vitro Transcription and Translation Protocols: Methods in Molecular Biology Volume 37, Garland Publishing, NY).

Exemplary in vivo expression systems include, but are not limited to prokaryotic expression systems such as bacteria (e.g., E. coli and B. subtilis), and eukaryotic expression systems including yeast expression systems (e.g., Saccharomyces cerevisiae), worm expression systems (e.g. Caenorhabditis elegans), insect expression systems (e.g. Sf9 cells), plant expression systems, amphibian expression systems (e.g. melanophore cells), vertebrate including human tissue culture cells, and genetically engineered or virally infected whole animals.

The disclosure relates to a mutant cell having a genome that has been mutated to comprise one or more one or more expression altering modifications as described herein. The disclosure also relates to a recombinant cell (e.g. a prokaryotic cell or a eukaryotic cell) that contains a nucleic acid sequence comprising one or more expression altering modifications as described herein.

The methods described herein can be useful for producing a polypeptide for commercial applications which include, but are not limited to the production of vaccines, pharmaceutically valuable recombinant polypeptides (e.g. growth factors, or other medically useful polypeptides), reagents that may enable advances in drug discovery research and basic proteomic research.

Polypeptides produced according to the methods described herein may contain one or more modified amino acids. Modified amino acids may be included in a polypeptide produced according to the methods described herein to (a) increase serum half-life of the polypeptide, (b) reduce antigenicity or the polypeptide, (c) increase storage stability of the polypeptide, or (d) alter the activity or function of the polypeptide. Amino acids can be modified, for example, co-translationally or post-translationally during recombinant production (e.g., N-linked glycosylation at N-X-S/T motifs during expression in mammalian cells) or modified by synthetic means. Examples of modified amino acids suitable for use with the methods described herein include, but are not limited to, glycosylated amino acids, sulfated amino acids, prenylated (e.g., farnesylated, geranylgeranylated) amino acids, acetylated amino acids, PEG-ylated amino acids, biotinylated amino acids, carboxylated amino acids, phosphorylated amino acids, and the like. Exemplary protocol and additional amino acids can be found in Walker (1998) Protein Protocols on CD-ROM Human Press, Towata, N.J.

The present invention encompasses any and all nucleic acids encoding a recombinant polypeptide which have been mutated to comprise an expression altering modification as described herein and any and all methods of making such mutations, regardless of whether that nucleic acid is present in a virus, a plasmid, an expression vector, as a free nucleic acid molecule, or elsewhere. The present invention encompasses any and all types of recombinant polypeptides that encoded by a nucleic acid comprising one or more expression altering modifications as described herein.

The present invention is not limited to any specific types of recombinant polypeptide described herein. Instead, it encompasses any and all recombinant polypeptides encoded by a nucleic acid comprising one or more expression modifications as described herein. Polypeptides that can be produced using the methods described herein can be from any source or origin and can include a polypeptide found in prokaryotes, viruses, and eukaryotes, including fungi, plants, yeasts, insects, and animals, including mammals (e.g., humans). Polypeptides that can be produced using the methods described herein include, but are not limited to any polypeptide sequences, known or hypothetical or unknown, which can be identified using common sequence repositories. Examples of such sequence repositories, include, but are not limited to GenBank EMBL, DDBJ and the NCBI. Other repositories can easily be identified by searching on the internet. Polypeptides that can be produced using the methods described herein also include polypeptides have at least about 30% or more identity to any known or available polypeptide (e.g., a therapeutic polypeptide, a diagnostic polypeptide, an industrial enzyme, or portion thereof, and the like).

Polypeptides that can be produced using the methods described herein also include polypeptides comprising one or more non-natural amino acids. As used herein, a non-natural amino acid can be, but is not limited to, an amino acid comprising a moiety where a chemical moiety is attached, such as an aldehyde- or keto-derivatized amino acid, or a non-natural amino acid that includes a chemical moiety. A non-natural amino acid can also be an amino acid comprising a moiety where a saccharide moiety can be attached, or an amino acid that includes a saccharide moiety.

Exemplary polypeptides that can be produced using the methods described herein include but are not limited to, cytokines, inflammatory molecules, growth factors, their receptors, and oncogene products or portions thereof. Examples of cytokines, inflammatory molecules, growth factors, their receptors, and oncogene products include, but are not limited to e.g., alpha-1 antitrypsin, Angiostatin, Antihemolytic factor, antibodies (including an antibody or a functional fragment or derivative thereof selected from: Fab, Fab', F(ab)2, Fd, Fv, ScFv, diabody, tribody, tetrabody, dimer, trimer or minibody), angiogenic molecules, angiostatic molecules, Apolipopolypeptide, Apopolypeptide, Asparaginase, Adenosine deaminase, Atrial natriuretic factor, Atrial natriuretic polypeptide, Atrial peptides, Angiotensin family members, Bone Morphogenic Polypeptide (BMP-1, BMP-2, BMP-3, BMP-4, BMP-5, BMP-6, BMP-7, BMP-8a, BMP-8b, BMP-10, BMP-15, etc.); C--X--C chemokines (e.g., T39765, NAP-2, ENA-78, Gro-a, Gro-b, Gro-c, IP-10, GCP-2, NAP-4, SDF-1, PF4, MIG), Calcitonin, CC chemokines (e.g., Monocyte chemoattractant polypeptide-1, Monocyte chemoattractant polypeptide-2, Monocyte chemoattractant polypeptide-3, Monocyte inflammatory polypeptide-1 alpha, Monocyte inflammatory polypeptide-1 beta, RANTES, 1309, R83915, R91733, HCC1, T58847, D31065, T64262), CD40 ligand, C-kit Ligand, Ciliary Neurotrophic Factor, Collagen, Colony stimulating factor (CSF), Complement factor 5a, Complement inhibitor, Complement receptor 1, cytokines, (e.g., epithelial Neutrophil Activating Peptide-78, GRO alpha/MGSA, GRO beta, GRO gamma , MIP-1 alpha , MIP-1 delta, MCP-1), deoxyribonucleic acids, Epidermal Growth Factor (EGF), Erythropoietin ("EPO", representing a preferred target for modification by the incorporation of one or more non-natural amino acid), Exfoliating toxins A and B, Factor IX, Factor VII, Factor VIII, Factor X, Fibroblast Growth Factor (FGF), Fibrinogen, Fibronectin, G-CSF, GM-CSF, Glucocerebrosidase, Gonadotropin, growth factors, Hedgehog polypeptides (e.g., Sonic, Indian, Desert), Hemoglobin, Hepatocyte Growth Factor (HGF), Hepatitis viruses, Hirudin, Human serum albumin, Hyalurin-CD44, Insulin, Insulin-like Growth Factor (IGF-I, IGF-II), interferons (e.g., interferon-alpha, interferon-beta, interferon-gamma, interferon-epsilon, interferon-zeta, interferon-eta, interferon-kappa, interferon-lambda, interferon-T, interferon-zeta, interferon-omega), glucagon-like peptide (GLP-1), GLP-2, GLP receptors, glucagon, other agonists of the GLP-1R, natriuretic peptides (ANP, BNP, and CNP), Fuzeon and other inhibitors of HIV fusion, Hurudin and related anticoagulant peptides, Prokineticins and related agonists including analogs of black mamba snake venom, TRAIL, RANK ligand and its antagonists, calcitonin, amylin and other glucoregulatory peptide hormones, and Fc fragments, exendins (including exendin-4), exendin receptors, interleukins (e.g., IL-1, IL-2, IL-3, IL-4, IL-5, IL-6, IL-7, IL-8, IL-9, IL-10, IL-11, IL-12, etc.), I-CAM-1/LFA-1, Keratinocyte Growth Factor (KGF), Lactoferrin, leukemia inhibitory factor, Luciferase, Neurturin, Neutrophil inhibitory factor (NIF), oncostatin M, Osteogenic polypeptide, Parathyroid hormone, PD-ECSF, PDGF, peptide hormones (e.g., Human Growth Hormone), Oncogene products (Mos, Rel, Ras, Raf, Met, etc.), Pleiotropin, Polypeptide A, Polypeptide G, Pyrogenic exotoxins A, B, and C, Relaxin, Renin, ribonucleic acids, SCF/c-kit, Signal transcriptional activators and suppressors (p53, Tat, Fos, Myc, Jun, Myb, etc.), Soluble complement receptor 1, Soluble I-CAM 1, Soluble interleukin receptors (IL-1, 2, 3, 4, 5, 6, 7, 9, 10, 11, 12, 13, 14, 15), soluble adhesion molecules, Soluble TNF receptor, Somatomedin, Somatostatin, Somatotropin, Streptokinase, Superantigens, i.e., Staphylococcal enterotoxins (SEA, SEB, SEC1, SEC2, SEC3, SED, SEE), Steroid hormone receptors (such as those for estrogen, progesterone, testosterone, aldosterone, LDL receptor ligand and corticosterone), Superoxide dismutase (SOD), Toll-like receptors (such as Flagellin), Toxic shock syndrome toxin (TSST-1), Thymosin a 1, Tissue plasminogen activator, transforming growth factor (TGF- alpha, TGF- beta), Tumor necrosis factor beta (TNF beta), Tumor necrosis factor receptor (TNFR), Tumor necrosis factor- alpha (TNF alpha), transcriptional modulators (for example, genes and transcriptional modular polypeptides that regulate cell growth, differentiation and/or cell regulation), Vascular Endothelial Growth Factor (VEGF), virus-like particle, VLA-4/VCAM-1, Urokinase, signal transduction molecules, estrogen, progesterone, testosterone, aldosterone, LDL, corticosterone.

Additional polypeptides that can be produced using the methods described herein include but are not limited to enzymes (e.g., industrial enzymes) or portions thereof. Examples of enzymes include, but are not limited to amidases, amino acid racemases, acylases, dehalogenases, dioxygenases, diarylpropane peroxidases, epimerases, epoxide hydrolases, esterases, isomerases, kinases, glucose isomerases, glycosidases, glycosyl transferases, haloperoxidases, monooxygenases (e.g., p450s), lipases, lignin peroxidases, nitrile hydratases, nitrilases, proteases, phosphatases, subtilisins, transaminase, and nucleases.

Other polypeptides that that can be produced using the methods described herein include, but are not limited to, agriculturally related polypeptides such as insect resistance polypeptides (e.g., Cry polypeptides), starch and lipid production enzymes, plant and insect toxins, toxin-resistance polypeptides, Mycotoxin detoxification polypeptides, plant growth enzymes (e.g., Ribulose 1,5-Bisphosphate Carboxylase/Oxygenase), lipoxygenase, and Phosphoenolpyruvate carboxylase.

Polypeptides that that can be produced using the methods described herein include, but are not limited to, antibodies, immunoglobulin domains of antibodies and their fragments. Examples of antibodies include, but are not limited to antibodies, antibody fragments, antibody derivatives, Fab fragments, Fab' fragments, F(ab)2 fragments, Fd fragments, Fv fragments, single-chain Fv fragments (scFv), diabodies, tribodies, tetrabodies, dimers, trimers, and minibodies.

Polypeptides that that can be produced using the methods described herein can be a prophylactic vaccine or therapeutic vaccine polypeptides. A prophylactic vaccine is one administered to subjects who are not infected with a condition against which the vaccine is designed to protect. A preventive vaccine will prevent a virus from establishing an infection in a vaccinated subject, i.e. it will provide complete protective immunity. However, even if it does not provide complete protective immunity, a prophylactic vaccine may still confer some protection to a subject. For example, a prophylactic vaccine may decrease the symptoms, severity, and/or duration of the disease. A therapeutic vaccine, is administered to reduce the impact of a viral infection in subjects already infected with that virus. A therapeutic vaccine may decrease the symptoms, severity, and/or duration of the disease.

As described herein, vaccine polypeptides include polypeptides, or polypeptide fragments from infectious fungi (e.g., Aspergillus, Candida species) bacteria (e.g. E. coli, Staphylococci aureus), or Streptococci (e.g., pneumoniae); protozoa such as sporozoa (e.g., Plasmodia), rhizopods (e.g., Entamoeba) and flagellates (Trypanosoma, Leishmania, Trichomonas, Giardia, etc.); viruses such as (+) RNA viruses (examples include Poxviruses e.g., vaccinia; Picornaviruses, e.g., polio; Togaviruses, e.g., rubella; Flaviviruses, e.g., HCV; and Coronaviruses), (-) RNA viruses (e.g., Rhabdoviruses, e.g., VSV; Paramyxovimses, e.g., RSV; Orthomyxovimses, e.g., influenza; Bunyaviruses; and Arenaviruses), dsDNA viruses (Reoviruses, for example), RNA to DNA viruses, i.e., Retroviruses, e.g., HIV and HTLV, and certain DNA to RNA viruses such as Hepatitis B.

The methods described herein relate to a method for immunizing a subject against a virus comprising administering to the subject an effective amount of a recombinant polypeptide encoded by a nucleic acid sequence comprising one or more expression altering modifications as described herein. The disclosure relates to a method for immunizing a subject against a virus, comprising administering to the subject an effective amount of recombinant polypeptide encoded by a nucleic acid sequence comprising one or more expression altering modifications as described herein.

The disclosure relates to a composition comprising a recombinant polypeptide encoded by a nucleic acid sequence comprising one or more expression altering modifications as described herein, and an additional component selected from the group consisting of pharmaceutically acceptable diluents, carriers, excipients and adjuvants.

Polypeptides that that can be produced using the methods described herein can also further comprise a chemical moiety selected from the group consisting of: cytotoxins, pharmaceutical drugs, dyes or fluorescent labels, a nucleophilic or electrophilic group, a ketone or aldehyde, azide or alkyne compounds, photocaged groups, tags, a peptide, a polypeptide, a polypeptide, an oligosaccharide, polyethylene glycol with any molecular weight and in any geometry, polyvinyl alcohol, metals, metal complexes, polyamines, imidizoles, carbohydrates, lipids, biopolymers, particles, solid supports, a polymer, a targeting agent, an affinity group, any agent to which a complementary reactive chemical group can be attached, biophysical or biochemical probes, isotypically-labeled probes, spin-label amino acids, fluorophores, aryl iodides and bromides.

The nucleic acid sequences comprising one or more expression altering modifications as described herein may also be incorporated into a vector suitable for expressing a recombinant polypeptide in an expression system. The nucleic acid sequences comprising one or more expression altering modifications as described herein can be operatively linked to any type of recombinant polypeptide, including, but not limited to immunogenic polypeptides, antibodies, hormones, receptors, ligands and the like as well as fragments, variants, homologues and derivatives thereof.

The expression altering modifications may be made by any suitable gene synthesis or mutagenesis method known in the art, including, but are not limited to, site-directed mutagenesis, oligonucleotide-directed mutagenesis, positive antibiotic selection methods, unique restriction site elimination (USE), deoxyuridine incorporation, phosphorothioate incorporation, and PCR-based mutagenesis methods. Details of such methods can be found in, for example, Lewis et al. (1990) Nucl. Acids Res. 18, p3439; Bohnsack et al. (1996) Meth. Mol. Biol. 57, p1; Vavra et al. (1996) Promega Notes 58, 30; Altered SitesII in vitro Mutagenesis Systems Technical Manual #TM001, Promega Corporation; Deng et al.. (1992) Anal. Biochem. 200, p81; Kunkel et al. (1985) Proc. Natl. Acad. Sci. USA 82, p488; Kunke et al. (1987) Meth. Enzymol. 154, p367; Taylor et al. (1985) Nucl. Acids Res. 13, p8764; Nakamaye et al. (1986) Nucl. Acids Res. 14, p9679; Higuchi et al. (1988) Nucl. Acids Res. 16, p7351; Shimada et al. (1996) Meth. Mol. Biol. 57, p157; Ho et al. (1989) Gene 77, p51; Horton et al. (1989) Gene 77, p61; and Sarkar et al. (1990) BioTechniques 8, p404. Numerous kits for performing site-directed mutagenesis are commercially available, such as the QuikChange II Site-Directed Mutagenesis Kit from Stratgene Inc. and the Altered Sites II in vitro mutagenesis system from Promega Inc. Such commercially available kits may also be used to mutate AGG motifs to non-AGG sequences. Other techniques that can be used to generate nucleic acid sequences comprising one or more expression altering modifications as described herein are well known to those of skill in the art. See for example Sambrook et al. (2001) Molecular Cloning: A Laboratory Manual, 3rd Ed., Cold Spring Harbor Laboratory, Cold Spring Harbor, N.Y ("Sambrook").

Any plasmid or expression vector may be used to express a recombinant polypeptide as described herein. One skilled in the art will readily be able to generate or identify a suitable expression vector that contains a promoter to direct expression of the recombinant polypeptide in the desired expression system. For example, if the polypeptide is to be produced in bacterial or human cells, a promoter capable of directing expression in, respectively, bacterial or human cells can be used. Commercially available expression vectors which already contain a suitable promoter and a cloning site for addition of exogenous nucleic acids may also be used. One of skill in the art can readily select a suitable vector and insert the mutant nucleic acids of the invention into such a vector. The mutant nucleic acid can be under the control of a suitable promoter for directing expression of the recombinant polypeptide in an expression system. A promoter that is already present in the vector may be used. Alternatively, an exogenous promoter may be used. Examples of suitable promoters include any promoter known in the art capable of directing expression of a recombinant polypeptide in an expression system. For example, in bacterial systems, any suitable promoter, including the T7 promoter, pL of bacteriophage lambda, plac, ptrp, ptac (ptrp-lac hybrid promoter) and the like may be used. Other elements important for expression of a recombinant polypeptide from an expression vector include, but are not limited to the presence of least origin of replication on the expression vector, a transcription termination element (e.g. G-C rich fragment followed by a poly T sequence in prokaryotic cells), a selectable marker (e.g., ampicillin, tetracycline, chloramphenicol, or kanamycin for prokaryotic host cells), a ribosome binding element (e.g. a Shine-Dalgarno sequence in prokaryotes). One skilled in the art will readily be able to construct an expression vector comprising elements sufficient to direct expression of a recombinant polypeptide in an expression system.

Methods for transforming cells with an expression vector are well characterized, and include, but are not limited to calcium phosphate precipitation methods and or electroporation methods. Exemplary host cells suitable for expressing the recombinant polypeptides described herein include, but are not limited to any number of E. coli strains (e.g., BL21, HB101, JM109, DH5alpha, DH10, and MC1061) and vertebrate tissue culture cells.

The methods described herein can be implemented in hardware or software, or a combination of both. The folding energy calculation methods described herein can be implemented in computer programs executing on programmable computers each comprising a processor, a data storage system (including volatile and non-volatile memory and/or storage elements), at least on input device, and at least one output device. Program code can be applied to input data to perform the functions described herein and generate output information. The output information can be applied to one or more output devices, in known fashion. The computer can be, for example, a personal computer, microcomputer, workstation, cluster or mainframe of conventional design or arrangement of those.

The methods described herein can be implemented in a procedural or object oriented programming language to communicate with a computer system. The methods described herein can also be implemented in assembly or machine language. The methods described herein can be stored on a storage media or device (e.g., ROM, ZIP, or magnetic diskette) readable by a general or special purpose programmable computer, for configuring and operating the computer when the storage media or device is read by the computer to perform the methods described herein. Data generated by the methods described herein can also be included in a computer-readable memory and can be administrated in databases. The methods described herein can also be processed in parallel_computers or processors to allow reduction of processing time and facilitate high throughput application of the methods.

The following examples illustrate the present invention, and are set forth to aid in the understanding of the invention.

### Example 1: mRNA Features Controlling Protein Expression Level in E. coli

Expression of 6,348 protein-coding genes from a wide variety of phylogenetic sources was evaluated **(****Figure 15****).** The protein coding genes were transcribed from the bacteriophage T7 promoter in pET21, a 5.4 kb pBR322-derived plasmid harboring an ampicillin resistance marker (Acton, T. B. et al. (2005) Methods Enzymol 394, 210-243). This dataset provides broad sampling of codon-space due to variations in codon-usage frequency in different organisms. A bacteriophage polymerase was used to drive transcription to minimize potentially confounding effects from the coupling of translation to transcription by the native *E. coli* RNA polymerase (Iost, I. et al. (1995) Embo j 14, 3252-3261; Iost, I. et al. (1992) J Bacteriol 174, 619-622). Protein expression (Acton, T. B. et al. (2005) Methods Enzymol 394, 210-243) was induced overnight at 18° C in *E. coli* strain BL21λ(DE3). The *E. coli* strain BL21λ(DE3) encodes in its chromosome a single copy of the gene for T7 polymerase under control of an IPTG -inducible promoter. This strain also contains pMGK, a 5.4 kb pACYC177-derived plasmid that harbors a kanamycin resistant marker, a single copy of the *lacI* gene, and a single copy of the *argU* gene encoding the tRNA cognate to the AGA codon for arginine. All proteins were expressed with the same eight-residue C-terminal extension (an affinity tag with sequence LEHHHHHH). This DNA sequence encoding this extension was omitted from computational analyses.

The proteins included in the large-scale expression dataset described herein share less than 60% sequence identity. Protein expression level from two isolates of the same plasmid was scored on an integer scale from 0 (no expression) to 5 (highest expression). The scoring was based on visual inspection of a Coomasie-blue-stained SDS-PAGE gel of a whole cell lysate. Scoring can also be performed by any suitable method known in the art, including but not limited to measured densitomery, colorimetry, fluorescence, or radioactivity. Scores rarely varied by more than ±1 between the two isolates. Roughly 30% of the proteins gave a score of 0 (1,754 protein) or 5 (1,973 proteins), while roughly 40% gave an intermediate score (2,621 proteins) (Price, W. N. et al. (2011) Microbial Informatics and Experimentation 1, 6).

The distributions of a variety of mRNA sequence parameters in the genes giving each expression score in the large-scale dataset were evaluated **(****Figures 9** **&** **16****).** This evaluation revealed many systematic differences between the genes giving high *vs*. low protein expression. Histograms of the parameter distributions for the genes giving each score were examined **(****Figures 9A-D****,****F****,****G-I** **&** **16A****,****G****,****I****).** The histograms of the parameter distributions showed relatively monotonic changes with increasing score. "Log-odds-ratio" plots of the natural logarithm of the ratio of the numbers of genes giving scores of 5 *vs*. 0 as a function of each parameter value were also examined **(****Figures 9E****,H,J & 16B-F,H,J).** This examination can be used to provide a graphical summary of the trends observed in the histograms. These plots can also be used for logistic-regression modeling of the relationship between mRNA sequence parameters and protein expression level in the large-scale dataset, as done below.

While the most highly expressed proteins are encoded by mRNAs with approximately equal content of A, U, G, and C bases **(****Figure 16B****),** the optimal base content varies at the three different positions in the codons in the genes **(****Figures 16C-E****).** This reading-frame dependency demonstrates that codon translation properties significantly influence protein expression level. Increasing frequency of some codons correlates with higher or lower protein-expression levels. The codon showing the strongest expression-enhancing effect is the GAA codon for glutamate. The synonymous GAG codon shows an equivalent frequency distribution for all expression scores **(****Figures 9A****,B,E).** The codon showing the one of the strongest expression-attenuating effects is the AUA codon for isoleucine. The synonymous AUC and AUU codons show neutral and slight expression-enhancing effects, respectively **(****Figures 9C-E****).** The synonymous AUC codon shows an equivalent frequency distribution for all expression scores. While these trends can otherwise be indicative of differences between the translation efficiencies of these codons, the multivariate statistical analyses and biochemical analyses presented herein indicate that their origin is more complex.

Adjacent pairs of AUA codons for isoleucine have a very strong expression-attenuating effect **(****Figure 16F****)** that is likely to reflect inefficient translation of this sequence based on the analyses presented below. In contrast, the frequency of the AGGA motif (Ingolia, N. T. et al. (2009) Science 324, 218-223) **(****Figures 16G-H****),** which matches the Shine-Dalgarno sequence, does not appear to have a significant influence on protein expression level. The distributions of the predicted partition-function free energies of folding (Reuter, J. S. et al. (2010) BMC Bioinformatics 11, 129) of the mRNA transcripts also show systematic differences between proteins with different expression scores. Expression is attenuated by increasingly stable folding *(i.e.,* decreasing free energy of folding) in the first 48 nucleotides in the protein-coding sequence **(****Figure 9H****)** (Shakin-Eshleman SH et al., (1988) Biochemistry 27, 3975-3982 (1988); Kozak M (2005) Gene 361, 13-37; Castillo-Mendez, M. A. et al. (2012) Biochimie 94, 662-672).

The results described herein provide robust calibration of the probability of attenuating expression as a function of predicted free-energy of folding in the head (ΔG_{H}). The results described herein show an <1/*e* reduction in the odds of high expression when ΔG_{H} < -15 kcal/mol. The strength of the correlation with expression level is increased modestly by including the 5' untranslated region (UTR) of the mRNA when calculating the free energy of folding of the head, ΔG_{UH} **(****Figures 9F****,****H****).** This parameter can be used for the global modeling of the expression results described herein.

Unexpectedly, the mean value of the predicted free energy of folding in the tail of the gene (nucleotides 49 through the stop codon) shows a non-linear influence on expression level, with both very high and very low values of <ΔG_{T}> systematically attenuating expression **(****Figures 9G****,****H****).** Equivalent trends are observed when the mean is calculated in 50% overlapping windows with widths of 48, 96, or 144 nucleotides. While these observations indicate that excessively stable or unstable mRNA folding in the tail both attenuate protein expression. The results described herein also indicate these effects also have more complex origins.

The methods described herein relate to the finding that several additional global sequence parameters were observed to have a systematic relationship to protein expression level. An increasing value of the codon repetition rate (e.g. the average frequency at which the same codon occurs again in the mRNA sequence) may correlate with lower expression level **(****Figures 16I-J****).** In certain embodiments, higher statistical entropy in the sequence correlates with lower expression level. Of these two mutually correlated parameters, the repetition rate is more influential than entropy, indicating that redundant use of the same codon can attenuate protein expression.

The methods described herein may relate to the finding that the length of the target mRNA/protein shows a non-linear influence on expression level, with very long and very short sequences showing systematically lower expression levels **(****Figures 9I-J****).**

The influence of nucleotide identity at individual positions at the start of the protein coding sequence on the log-odds-ratio of genes giving scores of 5 *vs.* 0 was examined **(****Figure 10****).** It was observed that the nucleotide composition in this region has a strong influence on protein expression. The magnitude of this influence may decline substantially after the sixth codon, which corresponds to the region of the mRNA physically protected by the ribosome in the 70S initiation complex (IC) in which the start codon is docked into its peptidyl-tRNA binding (P) site. Within the region of protection, G bases consistently reduce the probability of high expression, while A bases consistently increase it, and C and U bases have intermediate effects **(****Figure 10****).** The rank-order of these effects matches the probability of base-pairing for each nucleotide in large ensembles of folded RNA structures, suggesting the observed trend can reflect a requirement for the mRNA bases in this region to be unpaired for efficient ribosome docking. (The periodicity of three in **Figure 10** is related to the parameter cross-correlations in AT-rich genes

The relative influence of different mRNA sequence parameters on protein expression level was examined using logistic regression. The logistic regression can employ a generalized linear model to quantify the influence of continuous variables on either binary or ordinal results. Binary results can be modeled assuming that the log-odds-ratio for two mutually exclusive outcomes (*e.g*., 5 *vs.* 0 scores in the dataset) increases linearly with the value of some function of a continuous variable (*e.g*., codon frequency). Ordinal results may be modeled assuming that the logs-odds-ratio between all successive integer outcomes (*e.g*., 5-0 scores in the dataset) increases in exactly the same manner. **Figure 9E** illustrates the simplest form of a binary logistic regression, in which the logs-odds-ratio is assumed to be a linear function of the continuous variable. The solid lines in this figure show the most probable slopes if there is a linear relationship between the codon frequencies and the log-odds-ratio of proteins with 5 vs. 0 expression scores. This simple linear model accurately describes the beneficial influence of the GAA codon on protein expression (green in **Figure 9E**), while it is less accurate in describing the more complex deleterious influence of the AUA codon.

Logistic regression can be performed using different mathematical functions of the continuous variable to model more complex behavior of this kind, which is done below. Nonetheless, "codon slopes" from linear logistic regression analyses such as these provide a qualitatively and quantitatively useful metric to describe the influence of individual codons on protein expression level.

Single-variable analyses was performed on all 61 non-stop codons using either binary (5 *vs.* 0 scores) or ordinal (5-0 scores) linear logistic regression dark and light gray, respectively, in in **Figure 11B**). The relatively uniform variance in codon frequencies in the genes in the dataset **(****Figure 11A****)** enables regression parameters for all codons to be determined with similar precision. The binary and ordinal regressions yield equivalent codon-slopes, indicating that codon content has a generally monotonic influence on protein expression level in the dataset. Furthermore, the equivalence of the results observed when comparing proteins with just 0 *vs.* 5 expression scores to those observed when also including proteins with intermediate scores indicates that the same mRNA features that partially attenuate expression can completely stop it. This effect, which is also apparent when examining parameter histograms for the proteins giving different expression scores **(****Figures 9A-D****,****F-G****,****I** **&** **16A****,****I****),** can be due to factors that impede translation that also lead to mRNA degradation.

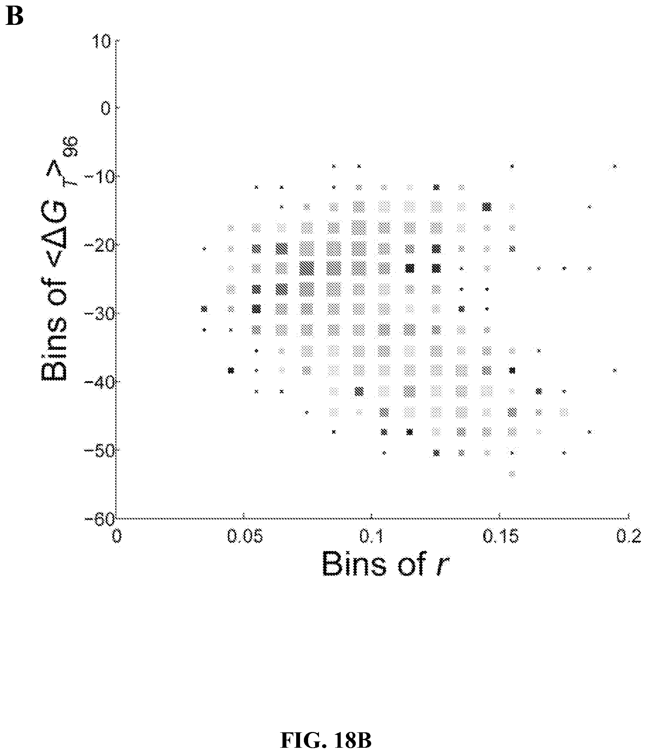

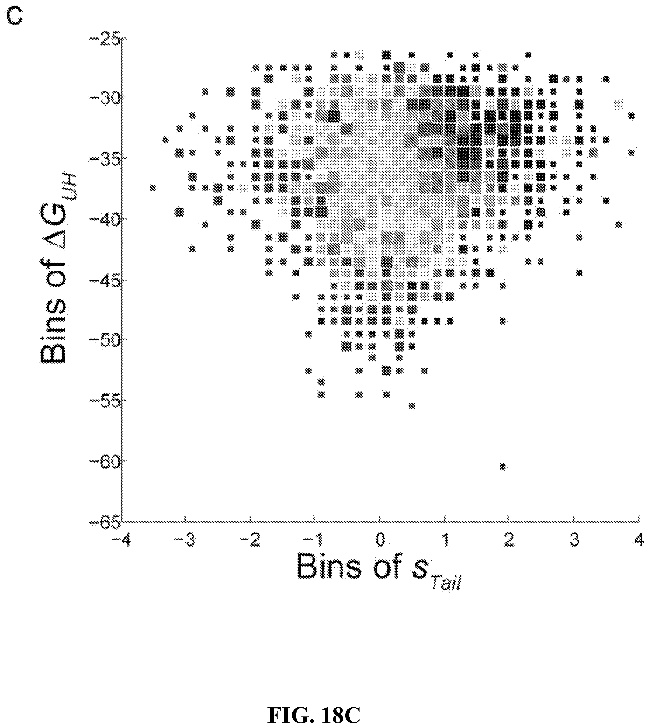

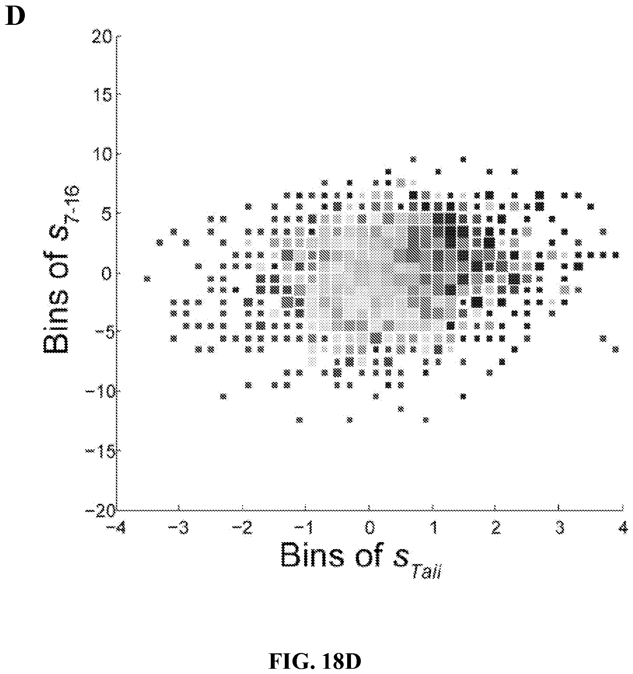

The codon-slopes determined using single-parameter logistic regressions **(****Figures 11B****,****E****)** show that codons ending in A or U are systematically enriched in the genes giving the highest level of protein expression in the dataset, while the synonymous codons ending in G or C are systematically depleted in these genes. These results provide guidance for engineering synthetic genes that enhance protein expression by emulating the properties of the best-expressed genes in the dataset. However, this computational approach does not provide reliable information on the mechanistic influence of each codon because the frequencies of most codons ending in A or U are strongly correlated with one another in the genes in the dataset **(****Figures 17A-C****),** due at least in part to substantial variations in AT *vs*. GC frequency in the DNA of the genomes of the source organisms. Many parameters that vary systematically between genes giving different protein expression levels, including <Δ*G_{T}*>₉₆ and the codon repetition rate r, are also mutually correlated **(****Figures 17A** **and** **18****).** A parameter that does not directly influence outcome can nonetheless appear influential in a single-parameter regression when its value is correlated with that of a directly influential parameter. Therefore, to develop insight into the relative mechanistic contributions of the different parameters, multiple-parameter logistic regression modeling of the expression dataset was performed. This approach simultaneously analyses all correlated parameters to delineate their relative influence on outcome. The reliability with which differences can be quantified may depend on the extent to which the two parameters vary independently in the genes in the dataset despite their overall mutual correlation.

The invention relates to a binary logistic-regression model that combines the explanatory variables explored individually in **Figures 9****,** **10****, &** **16** after eliminating those whose influence is captured by other correlated variables. (See examples.) The logarithm of the odds of observing the highest level of expression *vs*. no expression is given by $θ = 3.8 + 0.046 ⁢ Δ {G}_{UH} - 1.5 ⁢ I + 6.6 {a}_{H} - 6.3 {a}_{H}^{2} - 1.9 {g}_{H}^{2} + 0.76 {u}_{3 ⁢ H} + 0.077 {s}_{7 - 16} + 0.059 {s}_{17 - 32} + 0.86 {\sum }_{c} {β}_{c} {f}_{c} - 18 {d}_{AUA} - 13 ⁢ r - 0.011 ⁢ L - 490 / L$ In this equation, Δ*G_{UH}* is the predicted free energy of folding of the head of the gene plus the 5'-UTR (in kcal/mol), *I* is a binary indicator variable that is 1 if Δ*G_{UH}* < -39 kcal and the GC content of codons 2-6 is greater than 62% (and is otherwise zero), *a_{H}* and *g_{H}* are respectively the frequencies of adenine and guanine in codons 2-6, *u_{3H}* is the frequency of uridine at 3^{rd} position in codons 2-6, *s*₇₋₁₆ and *s*₁₇₋₃₂ are respectively the mean slopes **(****Figure 11B****)** for codons 7-16 and 17-32, *β_{c}* and *ƒ_{c}* are respectively the slopes and frequencies of each non-termination codon in the gene, *d_{AUA}* is a binary variable that assumes a value of 1 if there are any AUA-AUA di-codons (and is otherwise zero), *r* is the codon repetition rate, and *L* is the sequence length.

Calculating the loss in the predictive power when one or more terms is omitted gives the best estimate of the relative influence of different terms in the model and of different regions in the genes **(****Figures 29A-B****).** The influence of the head is captured by the combination of the folding-energy and base-composition terms, which likely reflect the accessibility of the translation initiation site for ribosome docking (Duval, M. et al. (2013) PLoS Biol 11, e1001731), together with the *s*₇₋₁₆ term. The influence of the tail is captured by the *s*₁₇₋₃₂ term together with the global terms, because the tail dominates these parameters (overall codon influence, *d_{AUA}*, *r*, and *L*). Computation modeling indicates that the influential mRNA-folding energy effects are restricted to the head and that these effects are significant but weaker in their overall influence than codon-related effects **(****Figure 29B****).** The codon-related effects are ∼2.3 times stronger near the 5' end of the coding sequence and decline to a constant level after codon ∼32 **(****Figure 32****),** which roughly matches the number of residues required to fill the ribosomal exit channel (Lu, J. et al. (2008) J Mol Biol 384, 73-86). However, because the genes in dataset have tails that are much longer than the head, codon content in the average tail is ∼7 times more influential than that in the head. Calculations described in the examples show that in-frame codon models are superior to out-of-frame codon models or a model with parabolic base-composition at each codon position. They also show that the mean predicted free energy of mRNA folding in the tail (*i.e*., <*G_{T}*>₉₆) makes an insignificant contribution to the model when the codon slopes and codon-repetition rate r are included, indicating that the apparent influence of <*G_{T}*>₉₆ on expression is likely attributable to its correlation with these more influential parameters.

The codon slopes from the best multiple logistic regression model (red in the bottom graph in **Figure 11B**) provide insight into the influence of the individual codons on the efficiency of protein translation in *E. coli.* The AUA codon for isoleucine, which is decoded by an unusual non-cognate tRNA (Wallace, E. W. et al. (2013) Mol Biol Evol 30, 1438-1453; Vivanco-Dominguez, S. et al. (2012) J Mol Biol 417, 425-439), has by far the strongest expression-attenuating effect, and adjacent pairs of AUA codons have a significantly stronger expression-attenuating effect than two non-adjacent AUA codons **(****Figure 16F****).** The other two codons for isoleucine have an approximately neutral influence on expression, indicating that the expression-suppressing effect of AUA is attributable to codon structure rather than amino acid structure. Similarly, the CGG and CGA codons for arginine have a strong expression-suppressing effect, while the four synonymous codons have a weakly positive or negative influence on expression. Among the eight codons emphasized in previous literature to be deleterious for protein expression (Price, W. N. et al. (2011) Microbial Informatics and Experimentation 1, 6; Wallace, E. W. et al. (2013) Mol Biol Evol 30, 1438-1453; Quax, T. E. et al. (2013) Cell Rep 4, 938-944; Muramatsu, T. et al. (1988) Nature 336, 179-181; Duval, M. et al. (2013) PLoS Biol 11, e1001731; Lu, J. (2008) J Mol Biol 384, 73-86), only four attenuate expression in the dataset (the AUA/CGG/CGA codons cited above and the CUA codon for leu), while the other four are either neutral (the AGA codon for arg and the GGA codon for glycine) or weakly enhance expression (the AGG codon for arg and the CCC codon for pro). The apparent influence of AGA and possibly that of AGG may be biased by overexpression of the ArgU tRNA cognate to AGA. Ignoring these two codons, which have the lowest frequencies in *E. coli,* the next three least frequent codons attenuate expression **(****Figures 11C** **&** **31A****).** However, there is a wide variation in the magnitude of their influence, and codons with slightly higher frequencies are neutral or weakly enhance expression. Furthermore, there is no significant correlation between the frequencies of the remaining 56 non-stop codons and their influence on expression **(****Figures 11C** **&** **31A****).** Similarly, there is no significant correlation between the influence of all 61 non-stop codons and either the codon adaptation index (Sharp, P. M. et al. (1987) Nucleic Acids Res 15, 1281-1295) **(****Figure 31B****),** the codon sensitivity (Elf, J. et al. (2003) Science 300, 1718-1722) **(****Figure 31C****),** the tRNA adaptation index (Tuller, T. et al. (2010) Cell 141, 344-354) **(****Figure 31D****),** or an estimate of cognate tRNA concentration (Dong, H. et al. (1996) Journal of Molecular Biology 260, 649-663) **(****Figure 31E****).**

The most strongly expression-enhancing codons in **Figure 11B** correspond to the three amino acids with sidechains that can act as general base catalysts (glutamate, aspartate, and histidine). For these three amino acids, the codons ending in A or U have a stronger expression-enhancing effect than the synonymous codons ending in G or C, indicating that codon structure is likely to modulate the efficiency of their translation. However plotting the codon slopes in the multiple logistic regression model against amino acid hydrophobicity reveals a strong correlation **(****Figure 11D****),** with charged amino acids having systematically higher slopes than polar or hydrophobic amino acids. The analyses suggest that translation efficiency varies systematically with amino acid structure. Analyzing the codon slopes as a function of the identity of the nucleotide base at each codon position reveals some systematic trends **(****Figure 11E****)**. However, these trends seem likely to reflect the conservation of the physicochemical properties of the amino acids encoded by codons with the same bases at their first two positions. Differences in the translation efficiency of synonymous codons **(****Figure 11B****)** are unlikely to have a systematic relationship to base content.

The validity and predictive value of the analyses presented above was tested by evaluating the expression properties of a set of synthetic genes **(****Figures 13** **&** **20****).** Sequences were designed using two different methods that emulate the codon-usage and mRNA-folding properties of the genes giving the highest level of protein expression in the large-scale dataset. In the "six amino acid" (6AA) method, all codons for arginine, aspartate, glutamate, glutamine, histidine, and isoleucine were substituted with the synonymous codon with the highest slope in the single-variable logistic regressions in **Figure 11B****.** The resulting mRNAs are enriched in codons ending in A or U bases, which have lower mean folding energies than G or C bases, and they tend to have mRNA-folding properties and other properties that match those of the genes giving the highest level of protein expression in the dataset, providing a concrete example of the origin of the parameter cross-correlations shown in **Figures 17A-C****.** In the "31 codon folding optimization" (31C-FO) method, the calculated free energy of mRNA folding was optimized using just 31 codons with the highest slopes for each amino acid in the single-variable logistic regressions in **Figure 11B****;** the folding energy in the head (ΔG_{UH}) was maximized (*i.e*., minimizing the stability of folded structures), while the folding energy in the tail (<ΔG_{T}>₄₈) was adjusted to be near -10 kcal/mole. In some experiments, the head but not the tail sequence of the gene was engineered, or *vice versa*, to evaluate the reliability of these inferences from multi-parameter computational modeling concerning their relative contributions to expression.

Genes optimized in both the head and the tail using the 31C-FO method were synthesized for five bacterial proteins that were poorly expressed in the large-scale dataset **(****Figure 13** **and** **Figure 20****)** and 17 additional proteins unrelated to those previously characterized **(****Figure 20B****).** These genes give uniformly high protein expression (scores of 4 or 5 for all proteins < 500 amino acids in length). While some of them yield insoluble protein products using the standard induction protocol, they uniformly yield high levels of soluble protein when fused in-frame at the C-terminus of the *E. coli* maltose-binding protein **(****Figure 20C****).**

To investigate whether codon usage in the tail can influence protein expression, the native head sequences were retained and the codons in the tails were exclusively optimized for four genes using the 6AA method (WT_{H}/6AA_{T} in **Figure 13B**)**.** Tail optimization increases expression of all four of these target proteins, although the extent of improvement varies substantially.

Also tested was the relative influence of codon usage *vs*. mRNA folding in the head. This testing was performed by constructing genes with identical tails but different heads that were codon-optimized using the 31C method while either optimizing (31C-FO_{H} with maximized ΔG_{UH}) or deoptimizing (31C-FD_{H} with minimized ΔG_{UH}) their calculated free energies of folding **(****Figure 13B****).** The gene-optimization experiments demonstrate that folding effects in the head, codon usage in the head, and codon usage in the tail all have a significant influence on protein expression, supporting the validity of our computational inferences **(****Figure 29****).**

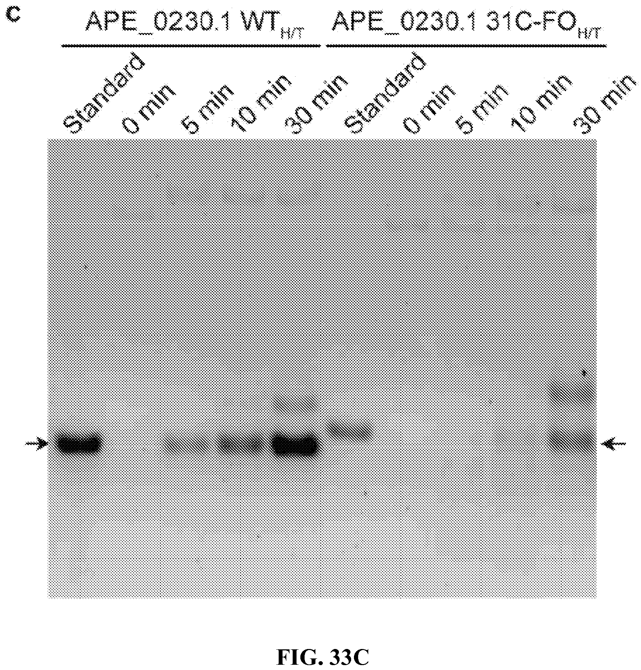

For the native bacterial genes from the large-scale dataset and their optimized counterparts, cellular growth-rates **(****Figure 13A****),** protein expression levels **(****Figure 13B****),** and mRNA levels **(****Figure 13D****)** were compared after induction *in vivo* in *E. coli.* Also compared were the products of *in vitro* transcription **(****Figure 33****)** and translation **(****Figure 13C****)** reactions. For one target (APE_0230.1), inhibition of cell growth upon induction of protein expression is eliminated by optimization of the gene sequence even though it greatly increases protein expression **(****Figures 13A-B****),** This result indicated that some mRNA sequence features impeding translation cause physiological toxicity in *E. coli.* Although *in vitro* transcription of the native or optimized genes using purified T7 RNA yields equivalent amounts of mRNA **(****Figure 33****),** *in vitro* translation of the resulting mRNAs using purified ribosomes and translation factors yields substantially higher levels of protein synthesis for all of the optimized sequences **(****Figure 13C****).** Notably, the sites of internal translational pausing are different in some of the optimized mRNAs compared to the corresponding native mRNAs (*e.g*., for APE_0230.1). These observations demonstrate that protein translation efficiency in *E. coli* is improved by the codon-optimization methods derived from the computational analyses of the large-scale protein expression dataset **(****Figures 11** **&** **29****).**

Given these *in vitro* biochemical results, the dramatically lower levels of mRNA observed *in vivo* after induction of the inefficiently translated native sequences compared to the optimized genes **(****Figure 13D****)** indicates that at least some mRNA-sequence-dependent translational obstacles can strongly influence steady-state mRNA level. It was noted that 5 min after induction, full-length mRNA is detected for all of the optimized but none of the native genes. This suggests the inefficiently translated native mRNAs are rapidly degraded, because T7 polymerase transcribes them with equivalent efficiency *in vitro* **(****Figure 33****).** To evaluate further the physiological relevance of the coupling between translation efficiency and mRNA stability observed in these experiments, the multivariate binary logistic regression results (red in **Figure 11B**) were used to calculate *s_{ALL}*, the average codon-slope for all endogenous *E. coli* genes encoding cytoplasmic proteins. This parameter derived from the large-scale expression dataset correlates strongly with the *in vivo* protein levels in *E. coli* quantified using mass spectrometry **(****Figure 30B****),** supporting the validity of the new codon-influence metric. Strikingly, *s_{ALL}* correlates almost as strongly with the *in vivo* mRNA levels of all predicted cytoplasmic proteins **(****Figures 30A-B****),** indicating that codon content significantly influences steady-state mRNA concentration.. For the set of proteins detected in mass spectrometric profiling, which are generally more abundant, *s_{ALL}* correlates with both their mRNA levels and protein/mRNA ratios **(****Figure 30C****),** which can reflect translation efficiency. These global correlations support codon content exerting an important influence not only on the efficiency of mRNA translation but also on mRNA stability. As described herein, simultaneous multiparameter computational modeling of results from 6,348 independent protein-expression experiments was used to dissect the mRNA sequences features that control protein-expression level in *E*. *coli* **(****Figures 10****,** **11****,** **29****).** Also described herein is validation this computational studies in follow-up experiments using biochemical methods **(****Figure 13****),** including *in vitro* translation experiments using fully purified components **(****Figure 13C****).** The mRNAs that were redesigned based on the computational results are translated more efficiently **(****Figures 13B-C****),** validating inferences that codon usage throughout a gene and mRNA folding stability in the head (the first ~16 codons) both contribute to controlling translation **(****Figure 29****).** The redesigned genes yield much higher levels of mRNA *in vivo* than the inefficiently translated native genes **(****Figure 13D****),** which led to an examination of the relationship between the new codon-influence metric and genome-wide protein and mRNA concentrations in *E. coli.* The average value of the codon-influence metric (*s_{All}*) in endogenous *E. coli* genes correlates strongly with the concentrations of the corresponding proteins *in vivo* **(****Figures 30B-C****).** It also correlates strongly with mRNA concentration **(****Figures 30A-C****)** and protein/mRNA ratio **(****Figure 30C****).** These genome-scale correlations indicate that codon content is an important determinant of both the translation efficiency and stability of mRNA in *E. coli* and that these parameters are tightly coupled (Duval, M. et al. (2013) PLoS Biol 11, e1001731; Li, X. et al. (2007) Mol Microbiol 63, 116-126; Shoemaker, C. J. et al. (2012) Nat Struct Mol Biol 19, 594-601; Shoemaker, C. J. et al. (2010) Science 330, 369-372; Becker, T. et al. (2012) Nature 482, 501-506). While the effect on mRNA stability could explain how codon usage can change protein expression level without significantly modulating net protein-elongation rate, the simplest explanation for the observed correlation of the codon-influence metric with protein/mRNA ratio is that codon content has an important effect on this rate, contrary to the interpretation of recent ribosome-profiling experiment in *E. coli* (Li, G. W. et al. (2014) Cell 157, 624-635; Li, G.-W. et al. (2012) Nature 484, 538-541).

As described herein, the coupling of codon content to steady-state mRNA concentration could be explained by several molecular mechanisms. It is possibly mediated by a kinetic competition between protein elongation and mRNA degradation that is modulated by ribosomal elongation dynamics (*i.e*., the sequential binding and conformational processes involved in amino-acyl-tRNA selection, peptide-bond synthesis, and tRNA/mRNA translocation). The bacteriophage T7 RNA polymerase used in the experiments described herein synthesizes mRNA too rapidly for translating ribosomes to keep up, making the resulting transcripts insensitive to transcription-translation coupling but more sensitive to endonuclease cleavage (lost, I. et al. (1995) Embo j 14, 3252-3261; Cardinale, C. J. et al. (2008) Science 320, 935-938). Consequently, it is possible that the inefficiently translated mRNAs produced by T7 polymerase that are fragmented and have lower concentrations *in vivo* **(****Figure 13D****)** reflect enhanced degradation. This reasoning, as well as the tendency of expression-attenuating codons to eliminate protein expression entirely in the large-scale dataset **(****Figures 9A-D****),** indicates that mRNA degradation is controlled in part by ribosomal elongation dynamics (Zaher, H. S. et al. (2011) Cell 147, 396-408; Li, X. et al. (2007) Mol Microbiol 63, 116-126; Deana, A. et al. (1996) J Bacteriol 178, 2718-2720; Nogueira, T. et al. (2001) J Mol Biol 310, 709-722; Li, X. et al. (2006) RNA 12, 248-255; Leroy, A. et al. (2002) Molecular Microbiology 45, 1231-1243; dos Reis, M. (2003) Nucleic Acids Research 31, 6976-6985). Several biochemical systems mediate recycling of ribosomes stalled due to protein synthesis/folding problems (Li, X. et al. (2006) RNA 12, 248-255; Richards, J. et al. (2008) Biochim Biophys Acta 1779, 574-582) or mRNA truncation (Shoemaker, C. J. et al. (2012) Nat Struct Mol Biol 19, 594-601; Christensen, S. K. et al. (2003) Molecular Microbiology 48, 1389-1400). In eukaryotes, this "No-Go" decay pathway involves the Dom34, Hbs1 (Shoemaker, C. J. et al. (2012) Nat Struct Mol Biol 19, 594-601; Shoemaker, C. J. et al. (2010) Science 330, 369-372), and ABCE1 (Becker, T. et al. (2012) Nature 482, 501-506) proteins, whereas in *E. coli,* similar activities are mediated by unrelated systems including the tmRNA pathway (Vivanco-Dominguez, S. et al. (2012) J Mol Biol 417, 425-439; Richards, J. et al. (2008) Biochim Biophys Acta 1779, 574-582; Ivanova, N. et al. (2005) J Mol Biol 350, 897-905; Christensen, S. K. et al. (2003) Molecular Microbiology 48, 1389-1400), ArfA, YaeJ (Chadani, Y. et al. (2011) Mol Microbiol 80, 772-785), and RF3(Vivanco-Dominguez, S. et al. (2012) J Mol Biol 417, 425-439; Zaher, H. S. et al. (2011) Cell 147, 396-408). These prokaryotic mRNA quality-control systems (Shoemaker, C. J. et al. (2012) Nat Struct Mol Biol 19, 594-601) are candidates to participate in the mRNA decay process that is potentially coupled to codon-dependent variations in ribosomal elongation dynamics.

The codon-influence metric **(****Figure 11B****)** established by the multiparameter computational models described herein have substantial differences compared to previous inferences regarding the influence of synonymous codons on protein expression in *E. coli.* The results described herein show that amino-acid identity influences translation efficiency but that, despite longstanding assumptions (Li, G. W. et al. (2014) Cell 157, 624-635; Li, G.-W. et al. (2012) Nature 484, 538-541), genomic codon-usage frequency is not directly related. The 3^{rd}, 4^{th}, and 5^{th} least frequent codons in *E. coli* have the most deleterious influence on expression in the large-scale dataset **(****Figures 11C** **&** **31A****).** However, these codons attenuate expression to widely varying extents, and slightly more frequent codons have a neutral or expression-enhancing influence **(****Figure 11B****).** Furthermore, the frequencies of the other 58 non-stop codons are not significantly correlated with expression level **(****Figures 11C** **&** **31A****).** Codon-usage frequency has been assumed to influence translation *in vivo* because it is correlated with the concentration of the cognate tRNA (Caskey, C. T. et al. (1968) J Mol Biol 37, 99-118; Ikemura, T. (1981) J Mol Biol 151, 389-409; Muramatsu, T. et al. (1988) Nature 336, 179-181; Dong, H. et al. (1996) Journal of Molecular Biology 260, 649-663), which can clearly influence protein-elongation rate *in vitro* (Wallace, E. W. et al. (2013) Mol Biol Evol 30, 1438-1453; Spencer, P. S. et al. (2012) J Mol Biol 422, 328-335) and protein yield *in vivo* (Chen, G. T. et al. (1994) Genes Dev 8, 2641-2652; Vivanco-Dominguez, S. et al. (2012) J Mol Biol 417, 425-439; Deana, A. et al. (1996) J Bacteriol 178, 2718-2720; Li, X. et al. (2006) RNA 12, 248-255). Indeed, as described herein, ArgU tRNA was overexpressed to promote higher expression of proteins enriched in AGA/AGG codons (Chen, G. T. et al. (1994) Genes Dev 8, 2641-2652), which may bias the influence of these codons in the dataset **(****Figure 11B****).** Further research will be required to understand the factors determining when tRNA concentration influences ribosomal elongation dynamics. Nonetheless, the analyses described herein suggest that ribosomal elongation dynamics exert a stronger influence on protein expression than cognate tRNA concentration. This inference is consistent with the demonstration that the translation factor EFP aids elongation of proline-rich sequences (Ude, S. et al. (2013) Science 339, 82-85). Furthermore, it suggests that translational regulatory effects could operate via modification of ribosomal elongation dynamics, mediated for example by covalent modification of tRNAs or the ribosome (Muramatsu, T. et al. (1998) Nature 336, 179-181). Complicating related mechanistic studies (lost, I. et al. (1995) Embo j 14, 3252-3261; Deana, A. et al. (1996) J Bacteriol 178, 2718-2720; Nogueira, T. et al. (2001) J Mol Biol 310, 709-722; dos Reis, M. (2003) Nucleic Acids Research 31, 6976-6985), the results described herein also suggest that such regulatory effects could be manifested via alterations in mRNA levels.

### Example 2: Model M Predicting Probability of High Protein Expression Level from RNA Sequence

The codon repetition rate is defined as: *r* =< *d_i^* - 1 > where *dᵢ* is the distance to the next occurrence of codon *cᵢ*. For example, "AAA.CGT.CCG.CGT.AAA" *r*=average(1/4, 1/2, 0, 0, 0)=3/20. The binary multiple logistic regression is a linear model in explanatory variables *xᵢ* for the log odds of high expression, *θ* = log[*E*_5/*E*_0] = *A* + ∑*ᵢ βᵢxᵢ*. The predicted probability of high expression is: $π = \frac{{E}_{5}}{{E}_{0} + {E}_{5}} = \frac{exp \left\{θ\right\}}{1 + exp \left\{θ\right\}} .$ The number of degrees of freedom for codon variables is one fewer than the number of codons because of the constraint 1 = ∑ *ƒ_{c}.* In the multiple logistic analysis in **Figure 11****,** ATG is removed, making slope *β_{ATG}* = 0 with its contribution absorbed into the constant *A.* The R statistics program [R Core Team (2013). R is a language and environment for statistical computing. R Foundation for Statistical Computing, Vienna, Austria. http://www.R-project.org/] is used to compute the model parameters (A,***β***). Logistic regression slopes *β* > 0 indicate that the odds of high expression increase along with the associated variable. To optimize protein expression, synonymous mutations are made that increase the usage of good codons (toward those with larger slopes *β*) while also tuning the free energy toward the optimal value, ultimately trying to maximize *θ* and thus *π*. The final Model M is: *θ* = 4.38 + 0.0451 *G_{UH}*+23.6/<G_{T}>₉₆ - 0.00117 L -489/L +6.55 A_{H} -6.30 A_{H}² + 0.753 U_{3H} -1.85 G_{H}² -1.50 (G_{UH}*<-39)(GC_{H} >10/15) -11.7 r-1.82 i + 0.077 *s*₇₋₁₆ + 0.059 *s*₁₇₋₃₂ +0.878 ∑*_{c} β_{c}ƒ_{c}.*

### Example 3: Methods for Building Synonymous Sequences

Synonymous sequences were designed with two methods and then tested experimentally. In the 6AA approach, codons for six amino acids were changed to the specified codon in Table 1. Although no explicit free energy optimization was performed with the 6AA method, the average free energy density was also more favorable in the genes that were tested. In the 31C-FO approach, the free energy of the head + pET21 expression vector was optimized to be as high as possible (i.e., with the weakest mRNA secondary structure) and the free energy of the tail was optimized to be near -10 kcal/mol for 48mer nucleotide windows, using only the subset of codons listed in Table 1 below. With 31C-FD, the free energy was de-optimized to be as low as possible (with the strongest mRNA secondary structure) with a subset of codons.

**Table 1:**

| Degeneracy | WT | 6AA | 31C |
|---|---|---|---|
| Ala | 4 | 4 | GCT,GCA |
| Arg | 6 | CGT | CGT,CGA |
| Asn | 2 | 2 | AAT |
| Asp | 2 | GAT | GAT |
| Cys | 2 | 2 | TGT |
| Gln | 2 | CAA | CAA,CAG |
| Glu | 2 | GAA | GAA |
| Gly | 4 | 4 | GGT |
| His | 2 | CAT | CAT,CAC |
| Ile | 3 | ATT | ATT,ATC |
| Leu | 6 | 6 | TTA,TTG,CTA |
| Lys | 3 | 3 | AAA |
| Met | 1 | 1 | ATG |
| Phe | 2 | 2 | TTT |
| Pro | 4 | 4 | CCT,CCA |
| Ser | 6 | 6 | AGT,TCA |
| Thr | 4 | 4 | ACA,ACT |
| Trp | 1 | 1 | TGG |
| Tyr | 2 | 2 | TAT |
| Val | 4 | 4 | GTT,GTA |

### Example 4: Evaluating correlations between protein expression and mRNA folding free energy of the first ∼50 coding bases and of the rest of the gene

A data set of diverse polypeptide sequences (from the Northeast Structural Genomics Consortium) with quantified gene expression was studied. Polypeptides were quantified independently in categories E0 (no expression) to E5 (highest expression). The polypeptide sequence data set contains more than 7000 mRNA sequences with less than 60% amino acid identity. These polypeptide sequences were drawn from about 20,000 in the NESG (Northeast Structural Genomics Consortium) pipeline that were expressed and purified in a consistent manner. The polypeptides were evaluated for expression and solubility in order to determine the features that correlate with high expression (Acton TB et al. (2005) Robotic cloning and polypeptide production platform of the Northeast Structural Genomics Consortium. Methods in Enzymology 394:210-243; Price WN et al. (2009) Nat. Biotechnol 27:51-57).

The folding free energy was computed for the first 50 bases in the coding region, the *head*, and the 5'-UTR expression vector + the first 50 bases. Other window sizes ranging from 40 to 150 were likewise evaluated. The minimum free energy and partition-function free energy were both correlated with the expression level of each gene. Representative data shown in **Figure 22A** makes clear that the probability of high expression (E3+E4+E5) decreases when the folding free energy is most stable.

The folding free energy of the first 50 coding bases is very highly correlated with expression levels (Table 2). Including the 5'-UTR expression vector plus the first 50 may produce a stronger correlation, based on the p-value of an ordinal logistic regression. Ordered expression categories between E0 and E5 can be studied using ordinal logistic regression and binary outcomes can be studied using standard logistic regression (Brant R (1990) Biometrics 46:1171-1178; Hosmer DW and Lemeshow S (2004) Applied logistic regression (Wiley-Interscience)).

**Table 2:**

| Free Energy | Expression p-value |
|---|---|
| First 50 coding bases | 3.5E-105 |
| 5'-UTR + first 50 coding | 3.3E-119 |

The significance of the correlation in Table 2 is strong evidence for the importance of free energy in translational efficiency. Codon and free energy effects will be explored individually and in combination.

A free energy higher or lower than approximately -20 kcal/mol for the first 50 coding bases separates higher and lower expression regimes (**Figure 22B**). A monotonic decrease towards low expression with lowering the free energy of the first 50 bases is observed. This trend indicates that increasing the folding free energy of the first 50 using synonymous mutations can increase expression of polypeptides.

The free energies of the latter portion of genes, the *tails*, were computed. The parabolic shape of the expression versus free energy curve (**Figure 22C**), with a maximum at intermediate folding energy, was also observed for other window locations and sizes throughout the mRNA tail (i.e., the coding region after the ∼50 base head) and indicates that too little structure can be deleterious. The tail effects are less pronounced than in the first 50 coding bases. It may not be necessary that every window in the tail contains a bottleneck that limits high expression. Whether the worst window is rate limiting for global expression or whether it depends on the average free energy will be investigated.

In the tail, low free energy correlates with lower expression. Lower expression when the free energy is low is consistent with results from the first 50, and is consistent with the intuition that stable secondary structures will inhibit ribosome initiation or processivity.

Gene expression may be highest when the free energy for coding bases 201-250 is not too high (e.g., G is not above -5 kcal/mol for 50mers or G is not above -15 kcal/mol for 96mers). The feature that very high free energy (i.e., minimal secondary structure) can be sub-optimal for gene expression may offer novel insights into other biological processes.

The parabolic dependence observed in **Figure 22C** will be explored by testing the expression of synonymous sequences after constraining folding free energy densities to be in different ranges. Programs to engineer synonymous sequences with the desired properties will be written. These synthetic genes will be commissioned and contributed to the NESG pipeline to be evaluated for expression levels.

### Example 5: Evaluate the likelihood of gene expression based on folding free energy and codon metric

Gene sequences are uploaded into a prototype web application and the folding free energies of the gene sequences are calculated. The resulting free energies are used to estimate the probability of high expression (sample output in **Figure 23A**). To make the differences between native and engineered sequences clear, the pairing probabilities are plotted using the RNAbows visualization tool (sample output is shown in **Figure 23B**) (Aalberts DP and Jannen WK (2013) RNA 19, 475-478). The difference RNAbow diagram presents the original and synonymous sequences, with any substitutions highlighted with color. Paired bases are connected with arcs whose thickness is proportional to the probability of that pair. Unique base pairs have the same color highlighting as the sequence, to allow comparisons at a glance.

### Example 6: Create algorithms to engineer sequences with improved expression.

If the free energy of the sequence is stable enough to make high expression unlikely, a synonymous sequence with higher free energy and greater likelihood of high expression can be engineered.

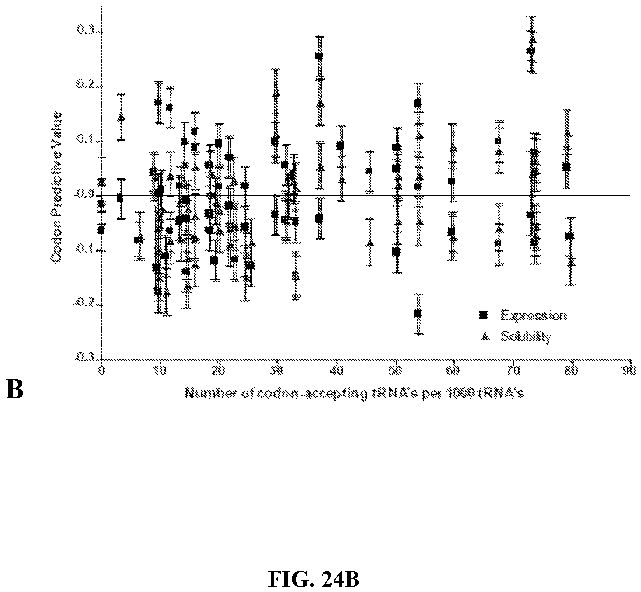

Simple sampling of 1000 sequences can typically identify a sequence with a free energy about 3 standard deviations higher than the mean. The prototype web-based tool currently uses simple sampling of synonymous sequences and chooses the best from among the samples. Sampling can be done from among all codons or "good" codons with positive expression (see, e.g., **Figure 24**). "Codon slope" relates the expression in the NESG data set to codon usage via ordinal logistic regression. Simple sampling 1000 is feasible, but relatively costly computationally.

A biased-sampling approach can improve the speed of sampling. **Figure 23B** highlights the paired bases and shows how some pairs can be eliminated in the synonymous sequence. One mismatch in the center of a stable duplex can increase the free energy of that structure by up to 7 kcal/mol. To increase the free energy, regions of high pairing will be disrupted.

The biased-sampling algorithm for the head is as follows. (1) Translate the native to the codon-optimized sequence and pre-compute the base positions where synonymous mutations can occur with good codons. (2) Compute the free energy and identify the base pairs of the sequence. Save any sequence with improved free energy. (3) At the positions where pairs are made and mutations can occur, use random sampling, biased to codon slopes, to replace the codons. Repeat (2) until satisfactory. (4) Report the synonymous sequence with the highest free energy. This biased-sampling strategy can reduce the number of iterations required to make a dramatic change to the free energy. In unpaired regions, codon usage remains optimal.

An improved sampling approach for the tail of the sequence will target an optimum free energy that is neither too high nor too low. Optimizing within a given window is straightforward, but neighboring windows may have unintended complementarity that could be far from optimal. The tail optimization procedure currently is as follows. (1) Use simple sampling of good codons to create synonymous subsequences: Select for free energies near the peak expression value. Assemble these fragments into a full tail sequence. (2) Evaluate the tail in overlapping windows (spanning adjacent design windows). (3) Tweak by hand, or resample from scratch. The tail algorithm can be improved if there are unacceptable free energies in overlapping regions from step (2). If so, repair by resampling that window and repeating step (2).

### Example 7: Optimizing codon usage and free energies

The optimal free energy density should be as high as possible in the head (first ~50 coding bases) and neither too low or too high in the tail. The roles of codons and folding free energy can be disentangled by evaluating expression of a few genes with different combinations of codon usage and folding free energy. Sequences can be engineered with desired free energies using all codons, or a subset. Synthetic sequences can be evaluated for expression in the NESG pipeline.

Codon and free energy effects on a few genes were studied. The following were compared: (1) WT wildtype sequences; (2) 6AA sequences, wherein the six most important codons were optimized (codons for Aspartate with GAT, for Glutamate with GAA, for Histidine with CAT, for Isoleucine with ATT, for Glutamine with CAA, and for Arginine with CGT); (3) 31C-FO in which the free energy is optimized using only good codons; (4) 31C-FD in which the free energy is made as stable as possible using only good codons.

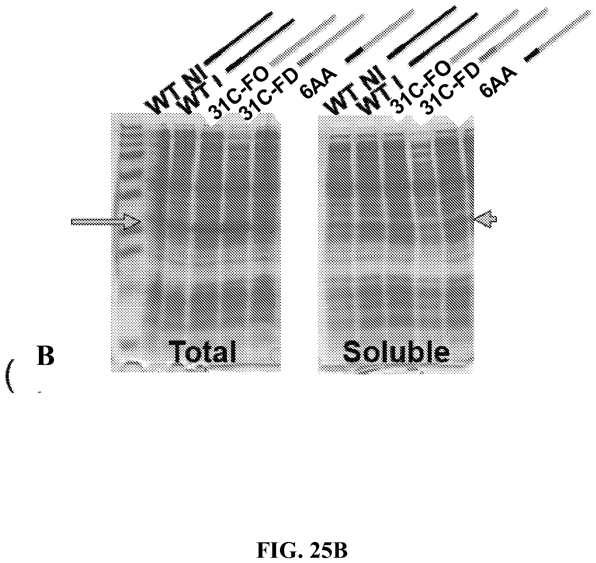

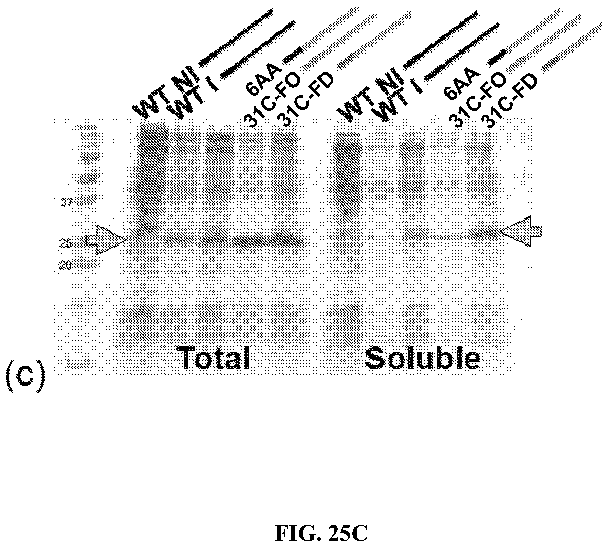

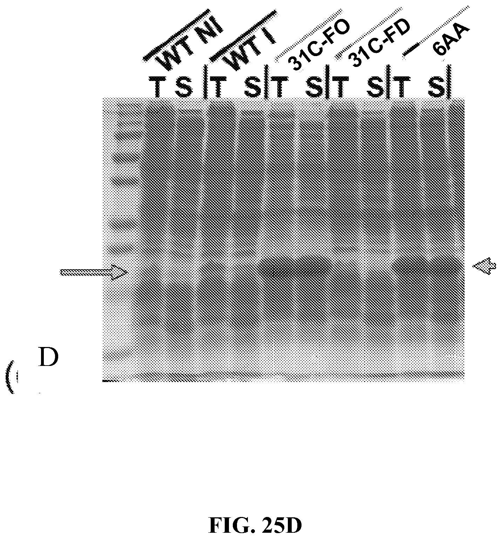

WT or 6AA tails were paired with WT, 31C-FO, or 31C-FD heads. The 6AA tails (Figure 25) are more highly expressed than WT in all 4 cases.

Optimized tails (6AA) increases the expression relative to WT. WT Not Induced and Induced are controls. In the head, codon optimization increases expression in all cases. In SCO1897, a 31C-FD head with low free energy can shut off expression. In other genes the 31C-FD free energy is not very low (Table 3). APE_0230.1 is a membrane protein so has low solubility.

**Table 3:**

| Head construct | G_{vec+51} | slope | Head construct | G_{vec+51} | Slope |
|---|---|---|---|---|---|
| APE_0230.1-WT | -30.2 | 9.3 | RSP_2139-WT | -39.4 | -53.7 |
| APE_0230.1-31C-FO | -27.6 | 108.3 | RSP_2139-31C-FO | -30.7 | 140.9 |
| APE_0230.1-31C-FD | -36.5 | 100.1 | RSP 2139-31C-FD | -40.7 | 112.6 |
| SRU_1983-WT | -35.1 | 34.6 | SCO1897-WT | -38.6 | -24.4 |
| SRU_1983-31C-FO | -34.5 | 104.5 | SCO1897-31C-FO | -32.8 | 89.5 |
| SRU_1983-31C-FD | -41.1 | 91.5 | SCO1897-31C-FD | -49.3 | 118.1 |

For the head constructs of the APE_0230.1, RSP 2139, SRU 1983, and SCO1897 genes, the free energy of the vector plus first 51 coding bases Gvec+51 in kcal/mol, and codon slope are listed in Table 3. It is clearly possible to design the free energy and codon properties simultaneously within the bounds of sequence constraints.

The 6AA tail sequences not only have better codon metric scores but also have free energy values closer to the 31C-FO targets: APE_0230.1: GWT = -311.1 kcal/mol, G6AA = -297.5 kcal/mol, Gtarget = -295.2 kcal/mol; SRU_1983: GWT = -362.6 kcal/mol, G6AA = -331.0 kcal/mol, Gtarget = -223.0 kcal/mol; RSP_2139: GWT = -406.3 kcal/mol, G6AA = -353.5 kcal/mol, Gtarget = -241.9 kcal/mol; SCO1897: GWT = -195.2 kcal/mol, G6AA = -158.4 kcal/mol, Gtarget = -138.5 kcal/mol.

Comparing the effect of the heads in these studies, it is observed that when the WT head is good (APE_0230.1), all are highly expressed. When the WT head has poor codon usage (RSP_2139), 31C-FO and 31C-FD increase expression. Even with good codon usage, very stable head free energy can abolish protein expression (SCO1897-31C-FD).

A reduction in toxicity was observed with 6AA optimized tails (**Figure 26**).

31C-FO heads and tails were also produced. In all five test genes (SRU_1983, APE_0230.1, SCO1897, RSP_2139, and ER449), expression was improved dramatically (**Figure 27**). The 31C-FO tails were built from 48mer fragments. The combination of 31C-FO optimized heads with 31C-FO optimized tails produced large increases in protein expression. Endogenous *E. coli* protein ER449 with 31C-FO optimization (**Figure 27****,** lanes 21.1 & 21.2) shows increased expression over wild type (WT).

### Example 8: Developing more predictive metrics

The combination of RNA folding reduction and good codon usage increases expression in the tested targets.

Modeling and algorithms can be improved to increase the understanding of the biology of translation and to produce better metrics for predicting whether constructs will be highly expressed. Metrics can then be used for optimizing sequence design.

Test current 31C-FO methods on a larger set of poorly expressed genes.

Determine if the bottleneck is the window with the lowest free energy, or a more global property like the average tail free energy. Test models against the NESG data set.

Optimize the window size for free energy optimization. Compare p-values for different window sizes.

While controlling for codon slope, design sequences with free energy densities spanning from high to low to probe that dependence. This kind of design can be performed with 31C-FO to 31C-FD constructions.

While controlling for free energy density, design sequences with codon slopes ranging from high to low to probe that dependence.

Looking at SRU_1983 (**Figure 25C**), both 31C-FO and 31C-FD express well, but 31C-FD has greater solubility. This may be an example where slightly lowering the translation rate increases usability of protein products..

Determine whether or not there are cases where ribosomal pausing facilitates protein folding (Watts et al., (2009) Nature, 460, 711-719) that should be engineered into sequences.

Test relative performance of specific codons (for example, test correlations with tRNA abundances).

Mine the NESG data set to study codon-codon correlations.

Evaluate whether long-range pairs create free energy bottlenecks, see **Example 9** below.

Explore how Shine-Dalgarno sequences impact translation, see **Example 10** below.

Overexpress proteins from the host organism, see **Figure 27****,** to try to better understand E. coli physiology and regulation.

These questions can be systematically explored by designing synthetic synonymous sequences and having them evaluated in the NESG pipeline.

### Example 9: Identifying long range pairs

Since preliminary indications are that high folding stability correlates with low gene expression, efficient methods will be developed for identifying complementary regions further apart than the window size. If the first 50 pairs well with the expression vector 5'-UTR or to the tail, initiation may be inhibited. Particularly stable stems elsewhere in the gene may slow the ribosome and decrease translational efficiency. To identify long-range pairs, it is not necessary to use an O(N³) RNA folding algorithm. Instead, a variation on the O(N²) Bindigo (Hodas NO and Aalberts DP (2004) Nucleic Acids Res., 32, 6636-6642) and BindigoNet algorithms can be used to identify the most stable complementary regions within an mRNA. Bindigo can be altered by identifying multiple local minima and setting the threshold for significance based on expected free energy density and Poisson statistics. The Bindigo-type run time will be hundreds of times faster than folding algorithms. Exemplary programs suitable for calculating free energy values in connection with the methods described herein include, but are not limited to RNAstructure, UNAFOLD, ViennaRNA, mFold, and Sfold. Default parameters for each of these programs can be used to perform calculations in connection with the methods described herein.

Correlations of global expression with the folding predictions in windows tiling the gene will be studied. It is possible that the most stable window is what most limits expression. Ordinal logistic regression and p-value will be used to identify best models and then tested experimentally. Other global effects will be studied by evaluating combinations of the folding free energies of different windows using neural net and other data mining techniques to seek key factors for high expression.

### Example 10: Locating Shine-Dalgarno compliments

The Shine-Dalgarno sequence has been affiliated with initiation (Etchegaray JP and Inouye M (1999) Journal of Biological Chemistry 274:10079-10085; Freischmidt A et al., (2012) Protein Expression Purif., 82, 26-31) and translational pausing (Li GW et al., (2012) Nature 484, 538-541). Genes can be evaluated for affinity with the Shine-Dalgarno sequence using the net binding free energy using the BindigoNet algorithm. Bindigo can also allow for the monitoring of whether otherwise optimal sequences contain potential translational pause sites, which can then be designed away. Likewise, to facilitate implementation in the NESG expression system, synonymous sequences will be monitored to ensure that commonly used restriction sites, etc do not appear.

### Example 11: Model how base composition affects RNA free energy

Building on the observation that the mean folding free energy depends on the length of the sequence (Hodas NO and Aalberts DP (2004) Nucleic Acids Res., 32, 6636-6642), the dependence of folding free energy on the composition of the RNA was studied.

(G+C) content is frequently proposed as a proxy of RNA folding stability (Biro, J.C. (2008) Theor Biol Med Model, 5:14; Gustafsson C et al., (2012) Protein Expression Purif., 83, 37-46). Better approximations can be made for RNA, which is not constrained to pair G and C equally as is required for DNA. Two, three, and five parameter models were considered:${G}_{2} = {g}_{0} + {g}_{N} N$${G}_{G + C} = {g}_{0} + {g}_{\left(G+C\right)} {N}_{\left(G+C\right)} + {g}_{\left(A+U\right)} {N}_{\left(A+U\right)}$${G}_{5} = {g}_{0} + {g}_{A} {N}_{A} + {g}_{C} {N}_{C} + {g}_{G} {N}_{G} + {g}_{U} {N}_{U} .$

All models include a penalty g₀ to initiate the fold or the unpaired region, plus terms that depend on the count Nx of bases of type ₓ. The Eq. (1) models thus explore the effect of length alone, of (G+C) composition, or of the composition of all 5 bases. Di- and tri-nucleotide correlations were extracted from the Human Exon Intron Database, and other specialized databases for tRNA, ribosomal RNA, and other types. These correlations were used to create synthetic sequences of fixed lengths 100, 200, 300, 400, 500nt. The folding and unpairing free energies were computed and then correlated those with the composition of the sequences. For the unpairing study, k-mers (k=3 to 21) were prohibited from pairing in longer sequences. Nₓ counts the number of x bases in the prohibited k-mer and where G now equals the free energy cost of imposing the constraint (i.e., the difference between the constrained and unconstrained folding free energies).

Model predictions were compared with explicit folding calculations (Zuker, M. (2003) Nucleic Acids Res., 31, 3406-3415; Mathews DH, et al., (2004) Proc. Natl. Acad. Sci. USA, 101, 7287-7292; Hofacker IL (2003) Nucleic Acids Res., 31, 3429-3431). Squared deviations between the computed folding energies and the model were minimized to obtain the optimal model parameters. Table 4 lists the optimized G₅ parameters. The parameters of model G₅ = g₀ + g_{A} N_{A} + g_{C} N_{C} + g_{G} N_{G} + g_{U} N_{U}, based on computing thousands of tri-nucleotide correlated random sequences. Folding refers to the minimum free energy of the fold, while unpairing refers to the free energy cost of prohibiting pairing in a k-mer. The large per-base free energy difference of Adenine and Guanine is notable, as is the destabilizing effect of Adenine.

**Table 4:**

| | **folding** | **unpairing** |
|---|---|---|
| **g₀** | +9.1 kcal/mol | +1.60 kcal/mol |
| **g_{A}** | +0.23 | -0.23 |
| **g_{C}** | -0.41 | +0.48 |
| **g_{G}** | -1.03 | +0.94 |
| **g_{U}** | -0.10 | +0.16 |

In **Figure 28****,** the scatter between explicit computation and models are plotted and the mean-squared residuals are listed.

Composition-dependent model G₅ significantly reduces the residuals, reflecting that the mean free energies of G and C bases differ, as do A and U. With model G₅, it is possible to capture most of the variation in the folding free energy and make reasonably accurate predictions in O(N) time, without resorting to an O(N3) folding computation.

Results from model G₅ that includes different per-base energies for each base show that the mean stability of Guanine and Adenine differs by greater than 1 kcal/mol (Table 4). It is notable in the lists of codon slopes from the NESG data set that typically the highest expression comes generally when an Adenine is in the wobble position and the least when a Guanine is in the wobble position.

The mean free energy cost G₅ for removing secondary structure in a region is potentially useful as a proxy for the more prohibitive explicit computation of the unpairing costs. To compute the unpairing costs explicitly takes O(N3) time, but the mean unfolding costs takes just O(k) time, where the length of the prohibited region k is much less than the length of the gene N.

These methods were developed using randomized sequences with mRNA correlations. The next steps are to test the model on the native sequences of the NESG data set to again study how well explicit free energy calculations correlate with the Eq. (1) models. In this way, whether or not G₅ is a useful approximation for modeling the accessibility of the ribosome binding-site or the local free energy costs as the ribosome processes along the gene can be explored.

The G₅ can also be used to model net tRNA-mRNA binding free energies, and the kinetics of translation. This may determine whether or not the net tRNA-codon binding free energies are well correlated with codon slopes.

Model G₅ measures the average properties of bases and does not include any correlations. Regions with greater-than-average complementarity will be most likely to bind. Using BindigoNet, the strong complementary substrings within a particular sequence can be identified in O(N2) time. The BindigoNet estimate of the cost to unpair a subsequence can be more accurate than using G5 alone because the specific features of the sequence in question are included. BindigoNet computations would be more expensive than using G5 alone, but take only a fraction of the time relative to a full O(N3) folding computation.

### Example 12: Cloning, Production and Detection

The *E. coli* strain DH5α was used for cloning, the other experiments used the strain BL21(λDE3 pMGK developed which was the same strain used for the high-through protein-expression (Acton, 2005). Bacteria were cultivated in LB medium (Affymetrix/USB). Ampicillin was added at 100 µg/ml for cultures harboring pET21-based plasmids. Kanamycin was added at 25 µg/ml to maintain the pMGK plasmid. Bacterial growth for protein expression and Northern blot experiments were done in the same media and conditions that were used to generate the high-through protein-expression dataset (Acton, 2005) minimum media under 250 rpm agitation at 37°C prior to induction and 17°C after induction).

The pET-21 clones of the gene APE_0230.1 (from *Aeropyrum pernix* K1), RSP_2139 from (*Rhodobacter sphaeroides*), SRU_1983 (from *Salinibacter ruber*), SCO1897 (from *Streptomyces coelicolor*) and *ycaQ* (from *E. coli*) were obtained from the NESG (those clones are respectively known as NESG targets: Xr92, RhR13, SrR141, RR162 and ER449). The 6AA_{T} and 31C-FO_{H}/31C-FO_{T} variant of the genes were DNA synthetized by GenScript. The head variants 31C-FO_{H} and 31C-FO_{H} were generated by PCR amplification using long forward primers comprising a NcoI site, the new head sequence and a sequence that hybridize after the head of the construct to amplify. The plasmid of the construct for which the head has to be replaced was used as DNA template for the PCR with the corresponding long forward primers and a reverse primer that hybridizing at the 3' end of the construct including the XhoI site. PCR products were cloned with In-Fusion kit in a pET-21 plasmid linearized with NcoI and XhoI. All the plasmids were verified by DNA sequencing and corrected when necessary using the QuikChange II Site-Directed Mutagenesis kit.

Starting cultures from a single colony were inoculated into 6mL of LB media containing 100ug/mL of Ampicillin and 30ug/mL Kanamycin. Cultures were grown at 37°C until highly turbid (4-6 hours). 40uL of the turbid media was used to inoculate 2mL of NESG MJ9 Minimal Media. This MJ9 preculture was grown overnight at 37C. The following day, OD₆₀₀ readings were taken of a 1:10 dilution of the turbid MJ9 preculture. This reading was used to calculate the volume of preculture necessary to normalize all cell samples to a starting culture reading of 0.1 in 6mL of media. This calculated volume was inoculated into 6mL of fresh MJ9 media and cells were grown at 37°C until OD₆₀₀ reached 0.5-0.7. Cells were then induced with 1mM IPTG, with one duplicate tube for each target WT left non-induced to act as a negative control. After induction, 200 µL x 2 of each culture was removed and placed into a sterile 96 well plate for growth curve monitoring. The remaining 5.6mL of induced samples were then transferred to 17°C and shaken overnight. The following day, sample tubes were removed from the shaker and placed on ice. Final OD600 measurements were taken using (insert instrument name here). Cells were centrifuged in 14 mL round bottom Falcon tubes at 4K rpm for 10 minutes and the supernatant discarded. Cells were resuspended in 1.2 mL of Lysis Buffer (50 mM NaH₂PO₄ pH 8.0, 30 mM NaCl, 10 mM 2-mercaptoethanol) and then transferred to 1.5 mL Eppendorf tubes on ice. Lysis was accomplished by sonication on ice, using a 40 V setting (∼12 Watt pulse) and pulsing 1 sec followed by a 2 sec rest, for a total of 40 pulses. 120 µL of each lysed sample was mixed with 40 µL of 4X Laemmli Buffer. Samples were then run on SDS-PAGE (Bio-Rad, Ready Gel, 15% Tris-HCl), with Bio-Rad Precision Plus All Blue Standard markers. Final OD₆₀₀ measurements were used to calculate the load volume for each individual sample, normalizing all samples to the density of the least turbid of each unique target.

Overnight cell growth was measured by transferring 200 µL of each induced culture to a 96-well sterile plate (insert plate type here) and covered with 50 µL of sterile paraffin oil. A negative control non-induced sample was loaded for each target WT. Duplicates of each sample were loaded to allot for any natural or human variation. Plates were placed into (insert name of instrument here) at room temperature, and shaken for 30 seconds. A start OD₆₀₀ reading was taken and then followed by 30 minutes of shaking until the next OD reading. Readings were repeated 27 more times for a total of 14.5 hours of growth analysis.

pET21 plasmids containing the optimized or unoptimized insert were digested with BlpI, phenol-chloroform purified and concentrated by ethanol precipitation. Of the digested samples, 2 µg were added to the RiboMax kit preparation, and *in vitro* transcribed as per protocol. Upon reaction completion, *in vitro* transcription samples were treated with DNAse then isopropanol precipitated and resuspended in The RNA Storage Solution. Transcript size and purity were verified by agarose gel electrophoresis with ethidium bromide staining. *In vitro* translation assays of the purified mRNAs were performed with the PURExpress system using L-[³⁵S]methionine premium. Each 25 µl reaction contained 10 µl of solution A, 7.5 µl of solution B and 2 µl of [³⁵S]methionine (10 µCi). The reactions were started by adding 2 µl of purified mRNA (4 µg/µl) and incubating at 37°C. Aliquot of 5 µl were withheld from the reaction at 15, 30, 60 and 90 min, stopped by adding 10 µl of 2X Laemmli and heating for 2 min at 60°C. Then 14 µl of each aliquot were run on a 4-20 % SDS-PAGE with Bio-Rad Precision Plus All Blue Standard markers. The gel was dried on Whathman as well as subjected to autoradiography, which is presented on this figure.

Northern blotting probe was designed as the reverse complement of the 71nt of the 5' UTR of the pET21 vector, and synthesized by Eurofins. The probe was labeled with biotin using the BrightStar Psoralen-Biotin Nonisotopic Labeling Kit. BL21 pMGK *E*. *coli* containing the plasmid of interest was grown overnight in LB at 37°C with shaking. Cultures were diluted 1:50 into MJ9 media and grown overnight at 37°C with shaking. Following day, the cultures were diluted to an OD₆₀₀ of 0.15 into MJ9 media and allowed to grow to an OD₆₀₀ of 0.6-0.7 prior to induction with 1 mM IPTG. Samples were taken at the indicated time points and RNAs were stabilized in 2 volumes of RNAProtect Bacteria Reagent. After pelleting, samples were lysozyme digested (15 mg/ml) for 15 minutes and RNAs were purified using the Direct-zol RNA Miniprep Kit and TRI-Reagent. Approximately 1-2 µg of total RNA per sample was separated on a 1.2% formaldehyde-agarose gel in MOPS-formaldehyde buffer. RNA integrity was verified by ethidium bromide staining. RNA was then transferred to a positively charged nylon membrane using downward capillary transfer with an alkaline transfer buffer (1 M NaCl, 10 mM NaOH, pH 9) for 2 h at room temperature. RNAs were cross-linked to the membrane using 1200 µJ UV. Membranes were pre-hybridized in Ultrahyb hybridization buffer for 1h at 42°C in a hybridization oven. Heat-denatured, biotin-labeled probe was then added to 10-20 pM final concentration and hybridized overnight at 42°C. Membranes were washed twice in wash buffer (0.2X SSC, 0.5% SDS) and probe signal was detected using the BrightStar BioDetect kit, as per protocol, with exposure to film.

### Example 13: CHGlir Codon Substitution

The methods described herein may relate to optimizing expression of a polypeptide by substituting one, or more codons in a sequence encoding the polypeptide according to CHGlir slope. The expression of protein can be increased by substituting at least one codon in a coding sequence with a synonymous codon having a higher CHGlir slope score. The expression of protein can be increased by substituting all codons in a coding sequence with synonymous codons having a higher CHGlir slope score. The expression of a protein can be increased by substituting some or all codons in a coding sequence with synonymous codons having a higher mean CHGlir slope score (*i.e*., CHGlir slope scores averaged over some window in the coding sequence). CHGlir slope scores are shown in Table 5.

**Table 5: CHGlir Slope Scores**

| codon | CHGlir slope | CHGlir SD | CHGlir-#obs |
|---|---|---|---|
| gcg | 5.70620 | 4.70345 | 3727 |
| gcc | -2.30824 | 4.30800 | 3727 |
| gca | -5.01519 | 5.04455 | 3727 |
| gct | -2.15397 | 5.06562 | 3727 |
| ^Ala | | | |
| aac | -1.03471 | 5.19279 | 3727 |
| aat | -6.26668 | 5.04062 | 3727 |
| ^Asn | | | |
| cgg | -16.52 | 5.57485 | 3727 |
| cgc | 0.73137 | 4.81903 | 3727 |
| cga | -16.16 | 7.91405 | 3727 |
| cgt | -8.00136 | 5.85346 | 3727 |
| agg | 8.10690 | 6.24158 | 3727 |
| aga | 1.25244 | 6.23697 | 3727 |
| ^Arg | | | |
| gac | 15.11992 | 4.51205 | 3727 |
| gat | 22.23124 | 4.66363 | 3727 |
| ^Asp | | | |
| tgc | -12.16 | 6.05460 | 3727 |
| tgt | -13.50 | 6.77429 | 3727 |
| ^Cys | | | |
| cag | -0.05862 | 4.89663 | 3727 |
| caa | 6.24499 | 4.77691 | 3727 |
| ^Gln | | | |
| gag | 13.01290 | 4.57617 | 3727 |
| gaa | 20.03292 | 4.36574 | 3727 |
| ^Glu | | | |
| ggg | 3.30392 | 5.58781 | 3727 |
| ggc | 3.40750 | 4.55601 | 3727 |
| gga | 6.08850 | 5.26724 | 3727 |
| ggt | 7.11991 | 5.23553 | 3727 |
| ^Gly | | | |
| cac | 2.65331 | 5.93934 | 3727 |
| cat | 9.77737 | 5.78082 | 3727 |
| ^His | | | |
| atc | -8.40023 | 4.80742 | 3727 |
| ata | -33.50 | 5.58263 | 3727 |
| att | -2.57433 | 4.84660 | 3727 |
| ^Ile | | | |
| ctg | -2.62368 | 4.25368 | 3727 |
| ctc | -1.46699 | 4.77372 | 3727 |
| cta | -17.47 | 7.05610 | 3727 |
| ctt | -10.70 | 5.31100 | 3727 |
| ttg | -12.05 | 5.08495 | 3727 |
| tta | -7.42526 | 4.85061 | 3727 |
| ^Leu | | | |
| aag | 3.81281 | 4.67490 | 3727 |
| aaa | 2.65751 | 4.44713 | 3727 |
| ^Lys | | | |
| atg | 0.00 | | 3727 |
| ^Met | | | |
| ttc | -4.59073 | 5.25262 | 3727 |
| ttt | 1.05422 | 4.86659 | 3727 |
| ^Phe | | | |
| ccg | 4.33983 | 5.30175 | 3727 |
| ccc | 9.36875 | 5.50275 | 3727 |
| cca | -8.12582 | 6.47161 | 3727 |
| cct | -9.91772 | 6.43434 | 3727 |
| ^Pro | | | |
| age | 2.41137 | 5.46194 | 3727 |
| agt | -1.63523 | 6.40751 | 3727 |
| tcg | -12.95 | 6.56715 | 3727 |
| tcc | -7.65339 | 6.32266 | 3727 |
| tca | 3.85079 | 6.52240 | 3727 |
| tct | -9.74631 | 6.32332 | 3727 |
| ^Ser | | | |
| acg | -1.14981 | 5.52607 | 3727 |
| acc | 6.92335 | 5.07432 | 3727 |
| aca | 1.40894 | 5.88977 | 3727 |
| act | -2.88385 | 6.09750 | 3727 |
| ^Thr | | | |
| tgg | -8.62889 | 5.29126 | 3727 |
| ^Trp | | | |
| tac | -6.16918 | 5.37694 | 3727 |
| tat | 1.50085 | 5.02836 | 3727 |
| ^Tyr | | | |
| gtg | 1.70020 | 4.77463 | 3727 |
| gtc | -2.74605 | 4.90204 | 3727 |
| gta | 8.54545 | 5.63133 | 3727 |
| gtt | 1.55914 | 5.01059 | 3727 |
| ^Val | | | |

### Example 14: BLOGIT Codon Substitution

The methods described herein relate to optimizing expression of a polypeptide by substituting one, or more codons in a sequence encoding the polypeptide according to BLOGIT coefficient or the strongly correlated OLOGIT coefficient. The expression of protein can be increased by substituting at least one codon in a coding sequence having a lower BLOGIT coefficient with a synonymous codon having a higher BLOGIT coefficient. The expression of protein can be increased by substituting all codons in a coding sequence having a lower BLOGIT coefficient with a synonymous codon having a higher BLOGIT coefficient. The expression of a protein can be increased by substituting some or all codons in a coding sequence with synonymous codons having a higher mean BLOGIT or OLOGIT slope score *(i.e.,* BLOGIT or OLOGIT slope scores averaged over some window in the coding sequence). BLOGIT and OLOGIT coefficients are shown in Table 6.

**Table 6: BLOGIT Coefficients**

| codon | BLOGIT-Coef | BLOGIT-std-err | BLOGIT-#obs | OLOGIT-Coef | OLOGIT-std-err | OLOGIT-#obs |
|---|---|---|---|---|---|---|
| gcg | -6.804633924 | 1.517522819 | 4316 | -4.823085492 | 1.0376422 | 7235 |
| gcc | -8.923701491 | 1.185485458 | 4316 | -6.164512157 | 0.819791891 | 7235 |
| gca | 10.08240206 | 2.476931083 | 4316 | 6.798210928 | 1.718198111 | 7235 |
| gct | 10.47436697 | 2.470050456 | 4316 | 7.193689576 | 1.703757551 | 7235 |
| ^Ala | | | | | | |
| aac | 3.360062447 | 2.705513191 | 4316 | 1.660800447 | 1.853643271 | 7235 |
| aat | 5.15522737 | 1.823703609 | 4316 | 2.664719782 | 1.194838973 | 7235 |
| ^Asn | | | | | | |
| cgg | -23.55346444 | 2.597970048 | 4316 | -14.23815969 | 1.627453439 | 7235 |
| cgc | -9.017054062 | 1.624950502 | 4316 | -5.809714476 | 1.099742903 | 7235 |
| cga | -0.061718663 | 5.271244991 | 4316 | 0.166309296 | 3.548675677 | 7235 |
| cgt | 11.6937581 | 3.119806794 | 4316 | 8.329631311 | 2.012120651 | 7235 |
| agg | -10.40899005 | 3.334371566 | 4316 | -6.42471655 | 2.029012638 | 7235 |
| aga | -5.685075249 | 3.051757248 | 4316 | -4.261034346 | 1.914654633 | 7235 |
| ^Arg | | | | | | |
| gac | -1.154898654 | 1.614484833 | 4316 | -0.125565519 | 1.124316712 | 7235 |
| gat | 19.18285167 | 1.793141744 | 4316 | 12.71485851 | 1.171875036 | 7235 |
| ^Asp | | | | | | |
| tgc | -21.24906837 | 3.51442846 | 4316 | -13.39307325 | 2.209140394 | 7235 |
| tgt | -9.837256839 | 3.955149232 | 4316 | -5.739787729 | 2.476479068 | 7235 |
| ^Cys | | | | | | |
| cag | -3.206326717 | 1.918384962 | 4316 | -1.443732047 | 1.319491452 | 7235 |
| caa | 14.73712063 | 2.04878977 | 4316 | 10.22264984 | 1.355310456 | 7235 |
| ^Gln | | | | | | |
| gag | -2.436534225 | 1.626751637 | 4316 | -1.56046089 | 1.086749822 | 7235 |
| gaa | 20.31120191 | 1.541485974 | 4316 | 13.35135348 | 0.973428606 | 7235 |
| ^Glu | | | | | | |
| ggg | -17.43194246 | 2.990130541 | 4316 | -13.027863 | 2.096077502 | 7235 |
| ggc | -7.684234515 | 1.392402242 | 4316 | -5.13248554 | 0.961300954 | 7235 |
| gga | 4.082426009 | 2.49702192 | 4316 | 1.354649841 | 1.660099247 | 7235 |
| ggt | 14.69294395 | 2.588868683 | 4316 | 10.24721563 | 1.757463377 | 7235 |
| ^Gly | | | | | | |
| cac | -0.813335191 | 1.659527761 | 4316 | -0.066917677 | 1.136580064 | 7235 |
| cat | 8.107615227 | 2.305995781 | 4316 | 6.498636571 | 1.583971228 | 7235 |
| ^His | | | | | | |
| atc | -1.574267134 | 2.095444867 | 4316 | -0.921429625 | 1.445725496 | 7235 |
| ata | -15.88559379 | 2.174033966 | 4316 | -10.73195961 | 1.315929509 | 7235 |
| att | 11.63321235 | 1.744915705 | 4316 | 7.380597237 | 1.167855382 | 7235 |
| ^Ile | | | | | | |
| ctg | -7.766415715 | 1.148993409 | 4316 | -5.038412095 | 0.781068683 | 7235 |
| ctc | -11.63039771 | 2.110745787 | 4316 | -7.990529398 | 1.431121944 | 7235 |
| cta | -2.745396069 | 4.24497583 | 4316 | -2.255217509 | 2.861993472 | 7235 |
| ctt | -1.874363783 | 2.690506422 | 4316 | -0.885995054 | 1.869029128 | 7235 |
| ttg | -0.08393207 | 2.725165832 | 4316 | 1.33338867 | 1.880629353 | 7235 |
| tta | 7.067607025 | 1.793256874 | 4316 | 4.227298284 | 1.159763323 | 7235 |
| ^Leu | | | | | | |
| aag | 1.413060132 | 1.836027179 | 4316 | 0.895631766 | 1.185469554 | 7235 |
| aaa | 10.13858192 | 1.236518791 | 4316 | 5.990830539 | 0.781023757 | 7235 |
| ^Lys | | | | | | |
| atg | 4.629102585 | 2.668254479 | 4316 | 3.401054555 | 1.807319231 | 7235 |
| ^Met | | | | | | |
| ttc | -10.28932181 | 2.401143141 | 4316 | -7.208508574 | 1.636593401 | 7235 |
| ttt | 9.011132751 | 1.906907625 | 4316 | 5.905270054 | 1.300740815 | 7235 |
| ^Phe | | | | | | |
| ccg | -11.91739138 | 2.189463708 | 4316 | -8.202058988 | 1.509455946 | 7235 |
| ccc | -18.6412822 | 2.607009147 | 4316 | -13.29145328 | 1.84547466 | 7235 |
| cca | 1.89544601 | 3.515420015 | 4316 | 1.370252194 | 2.33341725 | 7235 |
| cct | 0.44252667 | 3.539771828 | 4316 | -0.037884219 | 2.436757578 | 7235 |
| ^Pro | | | | | | |
| age | -3.385645438 | 2.696040794 | 4316 | -2.107784857 | 1.831520623 | 7235 |
| agt | 7.087140141 | 3.476358404 | 4316 | 3.591304574 | 2.353469404 | 7235 |
| tcg | -19.30672907 | 3.664759595 | 4316 | -13.11189159 | 2.533499935 | 7235 |
| tcc | -20.4434178 | 3.642338933 | 4316 | -13.6524053 | 2.458591664 | 7235 |
| tca | 9.520145 | 3.510338 | 4316 | 5.375291349 | 2.325186867 | 7235 |
| tct | 2.300125 | 3.366753 | 4316 | 0.690976027 | 2.277118463 | 7235 |
| ^Ser | | | | | | |
| acg | 2.847121 | 2.992774 | 4316 | 2.854065172 | 2.075921718 | 7235 |
| acc | -2.57334 | 2.151969 | 4316 | -1.387743362 | 1.470668151 | 7235 |
| aca | 16.42871 | 2.907795 | 4316 | 9.972327674 | 1.888290194 | 7235 |
| act | 12.39818 | 3.202234 | 4316 | 6.749903575 | 2.055207931 | 7235 |
| ^Thr | | | | | | |
| tgg | -14.1374 | 3.050839 | 4316 | -9.834768982 | 2.119050459 | 7235 |
| ^Trp | | | | | | |
| tac | -1.92715 | 2.92297 | 4316 | -1.104551549 | 2.011917361 | 7235 |
| tat | 7.701411 | 2.160332 | 4316 | 4.126555331 | 1.43647903 | 7235 |
| ^Tyr | | | | | | |
| gtg | -8.41942 | 1.91013 | 4316 | -5.5827516 | 1.284926136 | 7235 |
| gtc | -8.3496 | 2.155373 | 4316 | -6.053251471 | 1.467761102 | 7235 |
| gta | 16.0456 | 2.886918 | 4316 | 9.390947785 | 1.872023719 | 7235 |
| gtt | 14.56336 | 2.353019 | 4316 | 8.742370293 | 1.523291054 | 7235 |
| ^Val | | | | | | |
| tga | 9.217633 | 9.776924 | 4316 | 5.870561142 | 6.748731589 | 7235 |
| tag | -1.28783 | 12.94081 | 4316 | 3.767639585 | 9.165062039 | 7235 |
| taa | -1.28782593 | 12.94081185 | 4316 | 0 | 0 | 7235 |
| ^Stop | | | | | | |

### Example 15: Codon influence on large-scale protein expression correlates with E. coli mRNA levels

To investigate whether codon usage in the tail can influence protein expression, the native head sequences were retained and the codons optimized exclusively in the tails of four genes using the 6AA method (WT_{H}/6AA_{T} in **Figure 13B**). Tail optimization increases expression of all four of these target proteins, although the extent of improvement varies substantially. For two (RSP_2139 and SCO1897), protein expression is modestly improved due to reduced toxicity upon induction, which increases the cell mass in a given volume of culture, without increasing the yield of the target protein normalized to total cell protein. However, the other two target proteins show either significant (SRU 1983) or very large (APE_0230.1) increases in expression normalized to total cell protein, verifying the inference from the computational analyses that codon content in the tail can have a powerful influence on protein-expression level.

The relative influence of codon usage *vs*. mRNA folding in the head was also tested by constructing genes with identical tails but different heads that were codon-optimized using the 31C method while either optimizing (31C-FO_{H} with maximized ΔG_{UH}) or deoptimizing (31C-FD_{H} with minimized ΔG_{UH}) their calculated free energies of folding. The 31C-FO heads improved expression of all four proteins evaluated **(****Figure 13B**). The improvements were greatest for RSP_2139 and SCO1897, the proteins that improved only modestly in expression when their tails were optimized, suggesting that the principal obstacles to efficient translation of their native genes resides in their heads. Consistent with this inference, the 31C-FO heads for these proteins combined with either native or 6AA-optimized tails produce similarly high levels of expression **(****Figure 13B**). Deoptimizing head folding yielded different results for the four target proteins that paralleled their calculated free energies **(****Figure 13B**). There were large differences between these proteins in the lowest ΔG_{UH} that could be achieved in synonymous heads constructed using the A/U-rich 31C codon set, providing another example of coupling between codon usage and more global physicochemical properties of mRNA sequences. The most stably folded 31C-FD head (RSP_2139 with ΔG_{UH} = -47 kcal/mol) eliminates the very high expression produced by the synonymous 31C-FO head (ΔG_{UH} = -37 kcal/mol), verifying the conclusion from the modeling studies **(****Figure 29****)** and prior literature that stable head folding can block protein expression. The 31C-FD head for SRU_1983 (ΔG_{UH} = -41 kcal/mol) also decreases expression compared to the synonymous 31C-FO head (ΔG_{UH} = -34 kcal/mol), while the 31C-FD head for APE_0230.1 (ΔG_{UH} = -32 kcal/mol) produces equivalent expression to the synonymous 31C-FO head (ΔG_{UH} = -30 kcal/mol). However, these codon-optimized heads increase expression compared to the native heads with similar folding energies (ΔG_{UH} = -34 kcal/mol for SRU_1983 WT head and -34 kcal/mol for SRU_1983 31C-FO head), supporting the computational inference **(****Figure 29****)** that codon content in the head can strongly influence protein expression.

As described herein, the inferences from computational modeling were validated. Multi-parameter computational modeling is a powerful tool because it can, in principle, resolve the relative influence of cross-correlated parameters (*e.g*., codon content and predicted RNA-folding energy (Reuter, J. S. et al. (2010) BMC Bioinformatics 11, 129,)along with the other parameters evaluated in **Figures 17-18**). However, there can be noise in these estimates, and the apparent influence of some parameters can reflect the "hidden" influence of cross-correlated parameters not included in the analysis. For example, if evolution constrains more highly expressed proteins to be more soluble, there could be a positive correlation between protein-expression level and the frequency of codons for solubility-enhancing amino acids, even if these amino acids do not increase protein-translation efficiency. Therefore, it is essential to validate computational inferences using mechanistically informative experiments. The *in vitro* translation experiments **(****Figure 13C****)** described herein importantly verify that the most influential mRNA sequence features identified in the multi-parameter computational model **(****Figure 29****)** directly modulate translation, ruling out substantial interference from statistical noise, hidden variables, surrogate effects, or other latent systematic errors.

The experimental data presented in this paper strongly support the major conclusions from the computational modeling studies; however, the details of these studies require further validation, both to ensure their quantitative accuracy and to elucidate the underlying molecular mechanisms. A high priority in this area will be to evaluate whether the new codon-influence metric (colored symbols in **Figure 11B**) accurately describes the relative translation efficiencies of the different amino acids and the synonymous codons for the same amino acid. The broad features of this metric are validated by its strong correlation with global physiological protein and mRNA levels *in vivo* in *E*. *coli* **(****Figure 30****)**, but the differences in the values for some synonymous codon pairs are not themselves statistically significant. Protein expression experiments *in vivo* and high-resolution enzymological studies of protein synthesis *in vitro* (Caliskan, N. et al. (2014) Cell 157, 1619-1631; Ieong, K. W. et al. (2012) J Am Chem Soc 134, 17955-17962; Johansson, M. et al. (2012) Proc Natl Acad Sci USA 109, 131-136; Zaher, H. S. et al. (2009) Nature 457, 161-166) will be needed to critically evaluate the quantitative details of the new codon metric and to elucidate its mechanistic origin.

The results described herein lead to a coherent model for the influence of codon content on protein expression in *E*. *coli,* as well as several related mechanistic hypotheses. mRNAs with suboptimal codon usage that are transcribed equivalently *in vitro* **(****Figure 33****)** but translated inefficiently *in vitro* **(****Figure 13C****)** have strongly reduced concentrations *in vivo* **(****Figure 13D****)**. Furthermore, the new codon-influence metric derived from large-scale *in vivo* protein expression experiments in *E. coli* **(****Figures 11****,** **29****,** **34A****)** correlates with global protein levels, protein/mRNA ratios, and mRNA levels *in vivo* in this organism under physiological conditions **(****Figure 30****)**. Consequently, it is possible that inefficiently translated codons attenuate protein expression in two distinct but interrelated ways, first by reducing translation efficiency and thus the yield of protein from an mRNA molecule, and second by enhancing the rate of degradation of that mRNA molecule (Chevrier-Miller, M. et al. (1990), Nucleic Acids Res 18, 5787-5792; dos Reis, M. (2003) Nucleic Acids Research 31, 6976-6985; Leroy, A. et al. (2002) Molecular Microbiology 45, 1231-1243; Marchand, I. et al. (2001) Mol Microbiol 42, 767-776; Nogueira, T. et al. (2001) J Mol Biol 310, 709-722; lost, I. et al. (1995) Embo j 14, 3252-3261; Deana, A. et al. (1996) J Bacteriol 178, 2718-2720). Inefficiently translated codons could also promote premature termination of mRNAs synthesized by *E. coli* RNA polymerase (Cardinale, C. J. et al. (2008) Science 320, 935-938; Proshkin, S. et al. (2010) Science 328, 504-508), which would also lead to a reduction in steady-state concentration. Overall, the balance between the transcription-initiation rate of each mRNA, which should not depend directly on codon usage, and its premature termination and decay rates, which depend significantly on codon usage, controls its steady-state level. This dynamic creates the strong correlation that is described herein between physiological mRNA levels and codon content in *E*. *coli* **(****Figure 30****)**. The feedback between translation efficiency and mRNA level will amplify the influence of codon usage and perhaps also other translational regulatory phenomena on protein expression level, creating a physiologically important but heretofore under-appreciated linkage between translation efficiency and mRNA transcription/degradation.

Comparing the model to the results obtained in recent *in vivo* ribosome-profiling experiments, conducted in *E*. *coli,* have raised significant questions concerning the influence of codon usage on protein expression. This is likely because they have shown homogeneous occupancy of the mRNA within each open reading frame (ORF) and a strong correlation between the level of ribosome-occupied ORFs and the concentrations of the encoded proteins (Li, G.-W. et al. (2012) Nature 484, 538-541; Li, G. W. et al. (2014) Cell 157, 624-635), implying that ribosomes elongate proteins at a similar rate on most mRNA templates, irrespective of codon usage. However, changes in synonymous codon usage can clearly modulate protein expression level *in vivo* (Nogueira, T. et al. (2001) J Mol Biol 310, 709-722; Deana, A. et al. (1996) J Bacteriol 178, 2718-2720; Chen, G. T. et al. (1994) Genes Dev 8, 2641-2652; Dana, A. et al. (2014) Nucleic Acids Res 42, 9171-9181; Gingold, H. et al. (2011) Mol Syst Biol 7, 481; Goodman, D. B. et al. (2013) Science*;* Kimchi-Sarfaty, C. et al. (2007) Science 315, 525-528; Li, X. et al. (2006) RNA 12, 248-255; Plotkin, J. B. et al. (2011) Nat Rev Genet 12, 32-42; Quax, T. E. et al. (2013) Cell Rep 4, 938-944; .Spencer, P. S. et al. (2012) J Mol Biol 422, 328-335; Tuller, T. et al. (2010) Cell 141, 344-354; Tuller, T. et al. (2010) Proc Natl Acad Sci USA 107, 3645-3650; Vivanco-Dominguez, S. et al. (2012) J Mol Biol 417, 425-439; Zhang, F. et al. (2010) Science 329, 1534-1537; Chen, G. F. et al. (1990) Nucleic Acids Res 18, 1465-1473; Chiba, S. et al. (2012) Mol Cell 47, 863-872; Letzring, D. P. et al (2013) RNA 19, 1208-1217; Ramu, H. et al. (2011) Mol Cell 41, 321-330; Sorensen, M. A. et al. (2005) J Mol Biol 354, 16-24, (2005) and this phenomenon has been attributed in prior literature to codon-dependent variations in mRNA translation rate by ribosomes (Chen, G. T. et al. (1994) Genes Dev 8, 2641-2652; Li, X. et al. (2006) RNA 12, 248-255; Vivanco-Dominguez, S. et al. (2012) J Mol Biol 417, 425-439; Chiba, S. et al. (2012) Mol Cell 47, 863-872; Gao, W. et al. (1997) Mol Microbiol 25, 707-716; Ito, K. et al. (2013) Annu Rev Biochem 82, 171-202; Ivanova, N. et al. (2005) J Mol Biol 350, 897-905, (2005). This apparent inconsistency between contemporary genome-scale experimentation and a large body of prior literature in molecular biology remains unresolved. The mechanistic model presented above helps to resolve this conundrum, because the influence of codon usage on steady-state mRNA level can lead to a reduction in protein expression from an mRNA molecule irrespective of its translation-elongation rate. As long as most ORFs are translated many times before experiencing an internal codon-dependent event that leads to very rapid processive mRNA degradation, there can be relatively homogeneous ribosome occupancy within each ORF, as observed in the ribosome-profiling experiments ((Li, G.-W. et al. (2012) Nature 484, 538-541; Li, G. W. et al. (2014) Cell 157, 624-635). Because the level of each ribosome-occupied ORF captures the combined influence of the translation-initiation rate and the steady-state concentration of the corresponding mRNA, there is a close correspondence between the concentration of each protein and the level of the ribosome-occupied ORF (Li, G.-W. et al. (2012) Nature 484, 538-541; Li, G. W. et al. (2014) Cell 157, 624-635), but this level is lowered by codon-dependent reductions in mRNA concentration.

On the other hand, the correlation between the new codon-influence metric and global protein/mRNA ratios in *E*. *coli* (**Figure 30C**) raises questions about the accuracy of the ribosome-profiling results. The most straightforward explanation for the observed influence of codon content on protein/mRNA ratio, which should reflect the average number of protein molecules synthesized per mRNA molecule, is that there are significant codon-dependent variations in translation-elongation rate. This explanation is consistent with longstanding models for the influence of codon usage on protein synthesis but is at odds with some interpretations of ribosome-profiling results (Li, G.-W. et al. (2012) Nature 484, 538-541; Li, G. W. et al. (2014) Cell 157, 624-635). A less likely but plausible alternative explanation is that there is a strong evolutionary linkage between codon usage and the translation-initiation rate of ORFs in *E*. *coli,* in which case the correlation between codon content and protein/mRNA ratio could represent an indirect effect rather than a direct mechanistic coupling. While such an evolutionary linkage is possible, because codon usage and translation initiation jointly tune protein expression level, there is only a weak correlation between codon content and the mRNA folding properties in the heads of the genes in the dataset (**Figure 17A**), and these properties are likely to be key determinants of translation-initiation rate. The weakness of this correlation in a large set of naturally evolved genes from diverse organisms (**Figure 15**) lessens the probability that it is indirectly responsible for the correlation between the new codon-influence metric and global protein/mRNA ratios in *E*. *coli* (**Figure 30C**). Moreover, reduced translation-initiation rate should lead to reduced steady-state mRNA concentration due to enhanced degradation rate (Chevrier-Miller, M. et al. (1990), Nucleic Acids Res 18, 5787-5792; Nogueira, T. et al. (2001) J Mol Biol 310, 709-722; lost, I. et al. (1995) Embo j 14, 3252-3261; Deana, A. et al. (1996) J Bacteriol 178, 2718-2720), further complicating analyses of the observed correlations.

Although these considerations suggest that there are significant codon-dependent variations in translation-elongation rate, given the complexity of the biochemical and evolutionary processes that influence mRNA translation, carefully controlled experiments *in vivo* and *in vitro* will be required to achieve a reliable understanding of how variations in synonymous codon usage alter translation efficiency and mRNA stability. It was widely assumed in prior literature that these variations are attributable to slower accommodation on the ribosome of tRNAs present at lower concentrations in the cell (Chen, G. T. et al. (1994) Genes Dev 8, 2641-2652; Dana, A. et al. (2014) Nucleic Acids Res 42, 9171-9181; Caskey, C. T. et al. (1968) J Mol Biol 37, 99-118; Dong, H. et al. (1996) Journal of Molecular Biology 260, 649-663; Ikemura, T. (1981) J Mol Biol 151, 389-409), which causes slower execution of the translation-elongation cycle for the corresponding codons. The lack of a significant correlation between the new codon-influence metric and tRNA pool levels (**Figures 31C-E**) raises questions concerning this mechanistic model and suggests that the stereochemical features and allosteric consequences of codon-tRNA interaction are likely to make important contributions to codon-dependent variations in translation efficiency. Future research will be needed to elucidate these effects and also to establish whether codon-dependent variations in mRNA level are mediated by altered protection of mRNAs by translating ribosomes or instead by direct recruitment of RNases to ribosomes (Tsai, Y. C. et al. (2012) Nucleic Acids Res 40, 10417-10431) interacting with inefficiently translated codons or perhaps even by activation of an intrinsic RNase activity in the ribosome itself (Dreyfus, M. (2009) Chapter 11 Killer and Protective Ribosomes, 85, 423-466). Therefore, the results described herein highlight new problems to be investigated in addition to providing new insights and new tools for such studies that lie near the core of the central dogma of molecular biology.

### Example 16: Dissecting the biology of synonymous codon usage

A central feature of the genetic code is its degeneracy. The use of 61 different triplet nucleotide codons to direct synthesis of the 20 amino acids enables a vast number of synonymous DNA/RNA sequences to encode the same protein sequence, and this degeneracy is assumed to be exploited to control protein expression level in biological systems. However, uncertainty exists regarding the principles and mechanisms underlying this control. It is widely assumed that genomic codon usage frequency, which parallels the physiological concentration of the cognate tRNAs (Ikemura T. Journal of molecular biology (1981) 151(3):389-409; Dong H. et al. Journal of molecular biology (1996) 260(5):649-63), tracks the relative translation rate of the encoded amino acid and that the resulting differences in the translation rates of synonymous codons control protein-synthesis efficiency (Caskey CT et al. Journal of molecular biology (1968) 37(1):99-118; Chen GT et al. Genes & development (1994) 8(21):2641-52). However, recent "ribosome-profiling" using state-of-the-art genomics technology show that all protein-coding mRNA sequences in *E*. *coli* are translated at approximately the same rate (Li G-W et al. , Oh E, Weissman JS Nature 2012;484(7395):538-41; Li GW et al. Cell 2014;157(3):624-35). Other recent genomics studies have shown that the least frequently used (rarest) codons in *E*. *coli,* which attenuate protein expression in some contexts (Caskey CT et al. Journal of molecular biology (1968) 37(1):99-118; Chen GT et al. Genes & development (1994) 8(21):2641-52; Muramatsu T et al. Nature 1988;336(6195):179-81; Vivanco-Dominguez S et al. Journal of molecular biology 2012;417(5):425-39; Zhang SP et al. Gene 1991;105(1):61-72), instead increase protein expression when used near the start of a protein-coding sequence (Goodman DB et al. Science. 2013. doi: 10.1126/science.1241934). The literature (Li G-W et al. , Oh E, Weissman JS Nature 2012;484(7395):538-41; Li GW et al. Cell 2014;157(3):624-35; Goodman DB et al. Science 2013. doi: 10.1126/science.1241934) presenting these results have avoided discussion of their contradictions with prior literature, and hypotheses reconciling these contradictions have not yet been advanced elsewhere. Therefore, despite the fact that RNA translation into protein lies at the heart Central Dogma of Molecular Biology, uncertainty exists concerning fundamental biochemical and physiological features of this process.

A related problem concerns the biological function of the many non-essential but evolutionarily conserved enzymes that covalently modify components of the translation apparatus, including tRNAs (El Yacoubi B et al. Annual review of genetics 2012;46:69-95; Novoa EM et al. Cell 2012;149(1):202-13), ribosomal RNAs (Spenkuch F et al. RNA biology 2015:0. Epub 2015/01/27. doi: 10.4161/15476286.2014.992278; Dunkle JA et al. Proc Natl Acad Sci USA. 2014;111(17):6275-80; Popova AM et al. Journal of the American Chemical Society 2014;136(5):2058-69; Sergiev PV et al. Nucleic Acids Research 2012. doi: 10.1093/nar/gks219), and ribosomal proteins (Strader MB et al. Molecular & cellular proteomics : MCP 2011;10(3):M110.005199. Epub 2010/12/21. doi: 10.1074/mcp.M1 10.005199; Forouhar F et al. Nature chemical biology 2013;9(5):333-8). Many enzymes of this kind are expressed in *E*. *coli,* some of which have orthologs encoded in the human genome, but the physiological function is unknown for most of them, even though their biochemical activities have been elucidated (Arragain S et al. J Biol Chem. 2010;285(37):28425-33). It has been hypothesized that some of these enzymes regulate protein translation (El Yacoubi B et al. Annual review of genetics 2012;46:69-95; Novoa EM et al. Cell 2012;149(1):202-13; Sergiev PV et al. Nucleic Acids Research 2012. doi: 10.1093/nar/gks219; Fernandez-Vazquez J et al. PLoS genetics 2013;9(7):e1003647; Kirchner S et al. Nature reviews Genetics 2015;16(2):98-112) by changing the relative efficiency of translation of synonymous codons (Muramatsu T et al. Nature 1988;336(6195):179-81; Kruger MK et al. J Mol Biol. 1998;284(3):621-31). However, data supporting a regulatory activity of this kind has only been presented for one tRNA "hypermodification" enzyme in yeast (Phizicky EM et al. Genes & development 2010;24(17):1832-60; Laxman S et al. Cell 2013;154(2):416-29). Therefore, the physiological function remains undefined for the vast majority of the enzymes catalyzing covalent modification of the translation apparatus.

It is important to elucidate some of the "dark matter" of mRNA translation / protein synthesis. A new codon-influence metric for *E. coli* based on mathematical analysis of a large-scale experimental protein-overexpression dataset was recently derived (Boël G et al. Nature Submitted (under review)). This metric, which has substantial differences compared to previous literature, correlates only very weakly with genomic codon-usage frequency but very strongly with the physiological mRNA levels of all the genes encoded in the *E*. *coli* genome (Boël G et al. Nature Submitted (under review)). A variety of biochemical and molecular biological studies were conducted to validate the new metric and to begin to dissect the underlying molecular mechanisms. These studies revealed that mRNAs enriched in inefficiently translated codons have systematically reduced concentrations compared to synonymous mRNAs transcribed from the same promoter but enriched in efficiently translated codons, suggesting a tight coupling between mRNA translation efficiency and decay rate in *E. coli* (Boël G et al. Nature Submitted (under review)). The strength of this coupling, which explains the correlation demonstrated between the new codon-influence metric and global mRNA levels, is likely to have obscured analysis of some translational regulatory phenomena in *E. coli,* because observation of a strong influence on mRNA level has generally been assumed to reflect transcriptional regulation of gene expression rather than anything related to regulation of mRNA translation.

The affect of global measurements of mRNA level to infer codon efficiency was also investigated. This would open another approach to characterizing the factors influencing and regulating translation via analysis of readily obtainable microarray or RNAseq data. Applying the same mathematical model that was developed to analyze the large-scale protein-overexpression dataset to a single microarray dataset recapitulated key features of the codon-influence metric, supporting the utility of this approach.

Additional insight will be gained into the molecular mechanisms by which variations in synonymous codon usage control and regulate the process of mRNA translation by (1) evaluating the efficacy of alternative fluorescent-protein approaches for characterization of the relative expression efficiency of synonymous gene sequences in vivo in *E*. *coli,* (2) using existing biochemical methods and the methods developed under (1) to test the details of the new ***E. coli*** codon-influence metric, (3) analyzing RNAseq data from *E*. *coli* strains with knockouts of genes hypothesized to modulate synonymous codon usage, including those that covalently modify the translation apparatus, to evaluate their influence on relative codon efficiency under selected growth conditions, and (4) elucidating the biochemical systems controlling synonymous codon effects by quantifying the influence of all non-essential genes in *E*. *coli* on the relative expression level of proteins encoded by genes with defined differences in synonymous codon usage.

Translation, the final stage in the central dogma of molecular biology, involves ribosomes decoding mRNAs to synthesize proteins. Because proteins mediate the biochemical effects of most genes, translation is a critical determinant of the functional state of cells. A key feature of translation is the degeneracy of the genetic code, which uses 61 different triplet nucleotide codons to encode only 20 different amino acids. This degeneracy enables the same protein sequence to be translated from a vast number of synonymous mRNA sequences. Research in clinical genomics has revealed many examples of synonymous codon changes that alter human disease susceptibility (Kimchi-Sarfaty C et al. Science 2007;315(5811):525-8; Hunt RC et al. Trends in genetics : TIG 2014. Epub 2014/06/24. doi: 10.1016/j.tig.2014.04.006), and molecular biological studies have demonstrated that synonymous changes in mRNA sequence can produce both subtle and dramatic alterations in protein expression level (Hunt RC et al. Trends in genetics : TIG 2014. Epub 2014/06/24. doi: 10.1016/j.tig.2014.04.006; Steinthorsdottir V et al. Nature genetics 2007;39(6):770-5; Zhang F et al. Science 2010;329(5998):1534-7). While it is clear that variations in mRNA sequence play an important role in regulating protein expression in organisms from *E*. *coli* to humans, many different mechanistic hypotheses have been proposed to explain these effects (Spencer PS et al. Journal of molecular biology 2012;422(3):328-35), and their influence on translation efficiency remains unclear and in some cases controversial.

While there is widespread agreement that stable mRNA folding (Goodman DB et al. Science 2013. doi: 10.1126/science.1241934; Kozak M. Gene 2005;361:13-37; Shakin-Eshleman SH et al. Biochemistry 1988;27(11):3975-82; Castillo-Mendez MA et al. Biochimie 2012;94(3):662-72; Kudla G et al. Science 2009; 324(5924):255-8; Bentele K et al. Molecular systems biology 2013;9:675; Tuller T et al. Proceedings of the National Academy of Sciences of the United States of America 2010;107(8):3645-50) in the 5' region (head) of a gene can attenuate translation in *E*. *coli,* substantial uncertainty exists concerning the influence of synonymous codons on translation efficiency (Caskey CT et al. Journal of molecular biology (1968) 37(1):99-118; Chen GT et al. Genes & development (1994) 8(21):2641-52; Goodman DB et al. Science 2013. doi: 10.1126/science.1241934; Kudla G et al. Science 2009; 324(5924):255-8; Bentele K et al. Molecular systems biology 2013;9:675; Cannarozzi G et al. Cell 2010;141(2):355-67; Price WN et al. Microbial Informatics and Experimentation 2011;1(1):6; Wallace EW et al. Molecular biology and evolution 2013;30(6):1438-53; Elf J et al. Science 2003; 300(5626):1718-22; Ran W et al. mBio 2014;5(2):e00956-14; Quax TE et al. Cell reports 2013;4(5):938-44), the mechanistic basis of such effects, and their relationship to mRNA folding effects (Goodman DB et al. Science 2013. doi: 10.1126/science.1241934; Kozak M. Gene 2005;361:13-37; Shakin-Eshleman SH et al. Biochemistry 1988;27(11):3975-82; Castillo-Mendez MA et al. Biochimie 2012;94(3):662-72; Kudla G et al. Science 2009; 324(5924):255-8; Bentele K et al. Molecular systems biology 2013;9:675; Tuller T et al. Proceedings of the National Academy of Sciences of the United States of America 2010;107(8):3645-50). A ribosome-profiling study (Ingolia NT et al. Science 2009; 324(5924):218-23) concluded that the net translation-elongation rate is effectively constant for *E*. *coli* mRNAs, irrespective of codon usage (Li G-W et al. , Oh E, Weissman JS Nature 2012;484(7395):538-41; Li GW et al. Cell 2014;157(3):624-35). This finding challenges the assumption that differences in the translation rate of synonymous codons influence protein expression, an assumption underlying much of the codon-usage literature (Zhang F et al. Science 2010;329(5998): 1534-7; Spencer PS et al. Journal of molecular biology 2012;422(3):328-35; Gingold H et al. Molecular systems biology 2011;7:481. doi: 10.1038/msb.2011.14; Tuller T et al. Proceedings of the National Academy of Sciences of the United States of America 2010;107(8):3645-50; Quax TE et al. Cell reports 2013;4(5):938-44; Dana A et al. Nucleic Acids Res. 2014;42(14):9171-81; Sharp PM et al. Nucleic Acids Res. 1987;15(3):1281-95), but no alternative mechanism has been proposed to explain the many experiments in which changes in codon usage produce dramatic alterations in protein expression (Gingold H et al. Molecular systems biology 2011;7:481. doi: 10.1038/msb.2011.14). Uncertainty furthermore exists concerning which codon-related properties are beneficial *vs*. detrimental for protein expression (Gingold H et al. Molecular systems biology 2011;7:481. doi: 10.1038/msb.2011.14). For example, more homogeneous codon usage has been proposed alternatively to enhance (Cannarozzi G et al. Cell 2010;141(2):355-67; Quax TE et al. Cell reports 2013;4(5):938-44) or reduce (Zhang G et al. Nucleic Acids Res. 2010;38(14):4778-87) translation efficiency.

Much of the codon-usage literature focuses on inefficient translation of a set of rare codons (Zhang SP et al. Gene 1991;105(1):61-72) in the *E*. *coli* genome (Ikemura T. Journal of molecular biology 1981;151(3):389-409; Zhang SP et al. Gene 1991;105(1):61-72; Sharp PM et al. Nucleic Acids Res. 1987;15(3):1281-95), especially the AUA codon for ile (Caskey CT et al. Journal of molecular biology 1968;37(1):99-118; Muramatsu T et al. Nature 1988;336(6195):179-81) and the AGA, AGG, and CGG codons for arg (Chen GT, et al. Genes & development 1994;8(21):2641-52; Vivanco-Dominguez S et al. Journal of molecular biology 2012;417(5):425-39). On this basis, it is widely assumed that genomic codon-usage frequency, which parallels tRNA pool level, influences translation efficiency and that infrequent codons are translated inefficiently (Ikemura T. Journal of molecular biology 1981;151(3):389-409; Dong H et al. Journal of molecular biology 1996;260(5):649-63; Caskey CT et al. Journal of molecular biology 1968;37(1):99-118; Chen GT et al. Genes & development 1994;8(21):2641-52; Dana A et al. Nucleic Acids Res. 2014;42(14):9171-81). *In vitro* translation studies have demonstrated that the concentration of charged tRNA can influence the rate of protein elongation, with lower concentrations causing slower accommodation on the ribosome. The resulting reduction in protein-elongation rate is thought to cause infrequently used codons to be translated inefficiently *in vivo,* because the concentration of their cognate tRNAs is generally proportional to their codon-usage frequency (Ikemura T. Journal of molecular biology 1981;151(3):389-409; Dong H et al. Journal of molecular biology 1996;260(5):649-63). However, the expression of a fluorescent reporter protein is increased when the head of the gene contains the rare codons cited above as a barrier to translation¹¹. This effect was interpreted to reflect tolerance for inefficient codon usage in the head to prevent stable mRNA folding that would attenuate translation¹¹. However, no experiments were performed manipulating either parameter to verify this inference or to dissect their interplay, and alternative theories suggest that rare codons can directly enhance translation efficiency under some circumstances (Elf J et al. Science 2003;300(5626):1718-22; Dittmar KA et al. EMBO reports 2005;6(2):151-7; Tuller T et al. Cell 2010;141(2):344-54). The evolutionary biology literature focuses on a different correlate of genomic codon-usage frequency, which is accuracy in protein synthesis (Wallace EW et al. Molecular biology and evolution 2013;30(6):1438-53; Bulmer M. Genetics 1991;129(3):897-907; Akashi H. Genetics 1994;136(3):927-35). Biochemical studies suggest more frequent codons should be translated more accurately, because the levels of their cognate tRNAs are systematically higher, and competition from near-cognate tRNAs is the major cause of translational errors (Ikemura T. Journal of molecular biology 1981;151(3):389-409; Dong H et al. Journal of molecular biology 1996;260(5):649-63; Kramer EB et al. Rna 2007;13(1):87-96. doi: 10.1261/rna.294907; Zaher HS et al. Cell 2011;147(2):396-408). Usage of more frequent codons is enhanced at more conserved sites in proteins (Ran W et al. mBio. 2014;5(2):e00956-14; Akashi H. Genetics 1994;136(3):927-35), presumably because more accurate translation (Ninio J. FEBS letters. 1986;196(1):1-4) at such sites promotes greater fitness (Wallace EW et al. Molecular biology and evolution 2013;30(6):1438-53; Drummond DA et al. Cell 2008;134(2):341-52). While lower frequency codons also could be translated less efficiently (Dana A et al. Nucleic Acids Res. 2014;42(14):9171-81; Rocha EP. Genome research. 2004;14(11):2279-86), a systematic correlation between these parameters has yet to be demonstrated.

One factor complicating investigations of the influence of mRNA sequence on protein expression is that synonymous sequence changes simultaneously influence multiple mechanistic factors related to translation - codon identity, codon homogeneity, and mRNA folding as well as other potentially influential local and global sequence features that range from codon-pair effects to overall A/U/C/G content. Most previous studies have focused on individual parameters or pairs of parameters in a local region of mRNA (Li G-W et al. Nature 2012;484(7395):538-41; Goodman DB et al. Science 2013. doi: 10.1126/science.1241934; Kudla G et al. Science 2009;324(5924):255-8; Bentele K et al. Molecular systems biology 2013;9:675; Cannarozzi G et al. Cell 2010;141(2):355-67), and few mechanistic inferences from these studies have been tested using biochemical methods. To address these limitations, in a manuscript currently under review²⁶, statistical analyses of a large-scale experimental protein-expression dataset was performed as described herein, focusing on simultaneous evaluation of the influence of a wide variety of local and global mRNA sequence properties, and the resulting mechanistic inferences were tested using biochemical experiments. The combined computational and experimental studies described herein have provided new insight into the influence of mRNA sequence features on protein expression in *E*. *coli,* including the relative influence of codon content *vs*. mRNA-folding energy and the variation in the influence of these factors in different regions of the protein-coding sequence (Boël G, Letso R, Neely H, Price WN, Su M, Luff J, Valecha M, Everett JK, Acton T, Xiao R, Montelione GT, Aalberts DP, Hunt JF. Nature Submitted (under review)). They have also provided a codon-influence metric that is efficacious for engineering high-level protein expression but has major differences compared to past estimates (Li G-W et al. Nature 2012;484(7395):538-41; Li GW et al. Cell 2014;157(3):624-35; Goodman DB et al. Science 2013. doi: 10.1126/science.1241934; Kudla G et al. Science 2009;324(5924):255-8; Cannarozzi G et al. Cell 2010;141(2):355-67; Sharp PM et al. Nucleic Acids Res. 1987;15(3):1281-95). Furthermore, the biochemical experiments and computational analyses show that codon usage has a very strong influence on mRNA level *in vivo* in *E*. *coli,* paralleling results in yeast that have been reported at recent conference⁵⁸. The results suggest that the dynamics of the ribosomal elongation cycle exert a critical influence on mRNA stability that contributes to the biological effects of variations in synonymous codon usage. The extent of this connection will be explored and its biochemical mechanism elucidated (Boël G, Letso R, Neely H, Price WN, Su M, Luff J, Valecha M, Everett JK, Acton T, Xiao R, Montelione GT, Aalberts DP, Hunt JF. Nature Submitted (under review)).

This connection between codon usage and mRNA stability provides a possible explanation for the discrepancies alluded to above between recent genomic-scale studies of translation (Li G-W et al. Nature 2012;484(7395):538-41; Li GW et al. Cell 2014;157(3):624-35; Goodman DB et al. Science 2013. doi: 10.1126/science.1241934; Kudla G et al. Science 2009;324(5924):255-8) and longstanding hypotheses explaining the effects of variations in synonymous codon usage based on differences in ribosomal decoding rate (Zhang F et al. Science 2010;329(5998):1534-7; Spencer PS et al. Journal of molecular biology 2012;422(3):328-35; Gingold H, Pilpel Y. Molecular systems biology 2011;7:481; Tuller T, et al. Proceedings of the National Academy of Sciences of the United States of America. 2010;107(8):3645-50; Quax TE, et al. Cell reports 2013;4(5):938-44; Dana A et al. Nucleic Acids Res. 2014;42(14):9171-81; Sharp PM et al. Nucleic Acids Res. 1987; 15(3):1281-95). While it has proven difficult to rigorously link such differences to translational regulatory processes or functional changes in protein expression level *in vivo,* ribosome-profiling studies (Li G-W et al. Nature 2012;484(7395):538-41; Li GW et al. Cell 2014;157(3):624-35) have generated a more serious challenge to these hypotheses. Ribosome profiling⁴⁵ uses deep-sequencing technology to map comprehensively the ribosome locations on the full complement of mRNAs in living cells. Ribosome-profiling data suggest that the protein elongation rate is effectively constant for all mRNAs (Li G-W et al. Nature 2012;484(7395):538-41; Li GW et al. Cell 2014;157(3):624-35), irrespective of codon usage. Furthermore, they show at most minor differences in elongation rate at different locations within the mRNA encoding a given protein (Li G-W et al. Nature 2012;484(7395):538-41; Li GW et al. Cell 2014;157(3):624-35), and they fail to show any consistent difference in elongation rate at specific codons (Li G-W et al. Nature 2012;484(7395):538-41; Li GW et al. Cell 2014;157(3):624-35), contrary to expectations based on prior literature (Zhang F et al. Science 2010;329(5998):1534-7; Spencer PS et al. Journal of molecular biology 2012;422(3):328-35; Gingold H, Pilpel Y. Molecular systems biology 2011;7:481; Tuller T, et al. Proceedings of the National Academy of Sciences of the United States of America. 2010;107(8):3645-50; Quax TE, et al. Cell reports 2013;4(5):938-44; Dana A et al. Nucleic Acids Res. 2014;42(14):9171-81; Sharp PM et al. Nucleic Acids Res. 1987; 15(3):1281-95). Moreover, they have failed to provide any alternative explanation for how changes in codon usage can influence protein expression, even though there are many well-documented examples of this phenomenon (Dong H, et al. Journal of molecular biology 1996;260(5):649-63; Chen GT et al. Genes & development 1994;8(21):2641-52; Vivanco-Dominguez S at al. Journal of molecular biology 2012;417(5):425-39; Chevrier-Miller M et al. Nucleic Acids Res. 1990;18(19):5787-92; Deana A et al. Journal of bacteriology 1996;178(9):2718-20; lost I et al The EMBO journal 1995;14(13):3252-61; Rosano GL et al. Microbial cell factories. 2009;8:41; Chen GF et al. Nucleic Acids Res. 1990;18(6):1465-73; Goldman E et al. J Mol Biol. 1995;245(5):467-73; Ito K et al. PLoS One. 2011;6(12):e28413; Ito K et al. Annual review of biochemistry 2013;82:171-202; Sorensen MA et al. J Mol Biol. 2005;354(1):16-24).

The linkage between codon usage and mRNA stability suggested by the results described herein and by the parallel work in yeast (Vladimir Presnyak Y-HC et al. CSHL Translational Control; CSHL2014) could resolve this conundrum if codon-dependent translational obstacles limiting protein expression trigger sufficiently rapid degradation of the mRNA **(****Figure 36****)** to prevent it from being observed in ribosome profiling (Li G-W et al. Nature 2012;484(7395):538-41; Li GW et al. Cell 2014;157(3):624-35). There are indeed examples of such effects in individual genes in prior literature (Deana A et al. Journal of bacteriology 1996;178(9):2718-20; lost I et al The EMBO journal 1995;14(13):3252-61; Dreyfus M. Chapter 11 Killer and Protective Ribosomes2009;85:423-66; Richards J et al. Biochimica et biophysica acta. 2008;1779(9):574-82; dos Reis M. Nucleic Acids Research 2003; 31(23):6976-85). However, the model most frequently used to explain these effects assumes that they are mediated by enhanced sensitivity of mRNA to degradation when ribosome density drops due to upstream translational roadblocks (top of **Figure 36**). This mechanism would be expected to produce a reduction in ribosome density and ribosome occupancy between the start and the end of an mRNA subject to such codon-dependent degradation effects. However, ribosome profiling does not show such a tendency either in *E*. *coli* or in yeast. Furthermore, this mechanism can progressively reduce the expression-suppressing influence of inefficient codons throughout the length of a gene, and the results described herein do not show any such effect. These observations suggest that there could be a more directly connection between codon quality and mRNA degradation and that some codons could recruit mRNA degradation systems directly to a translating ribosome to mediate its rapid recycling coupled to degradation of its bound mRNA (bottom of **Figure 36**). This mechanism could explain codon-dependent variations in translation efficiency unrelated to tRNA concentration as well as those influenced by tRNA concentration if the allosteric couplings that mediate this process on the ribosome are influenced by the tRNA accommodation process. The studies described herein are designed to broaden and deepen understanding of the related molecular mechanisms that lie close to the heart Central Dogma of Molecular Biology.

A comprehensive and objective metric for the influence of codons on protein expression in E. coli has been generated. As described herein, the broad features of this metric, which has substantial differences compared to prior literature (Li G-W et al. Nature 2012;484(7395):538-41; Goodman DB et al. Science 2013. doi: 10.1126/science.1241934; Kudla G et al. Science 2009;324(5924):255-8; Bentele K et al. Molecular systems biology 2013;9:675; Cannarozzi G et al. Cell 2010;141(2):355-67), has been validated. The metric challenges widespread assumptions about the mechanism by which synonymous changes in codon usage influence protein expression. The examples described herein are designed to provide insight into the underlying biochemical mechanisms.

A mathematical approach to extract influential RNA sequence parameters from large-scale datasets with correlated sequence features simultaneously affecting multiple parameters has been developed. The results described herein show that a generalized multiple logistic regression modeling is efficacious in de-convoluting the complex relationships between features in large RNA sequence dataset.

A strong coupling between codon content and steady-state mRNA concentration in E. coli, suggesting mRNA decay rate is intimately coupled to translation efficiency, has been demonstrated. While couplings of this kind have been demonstrated for individual genes in prior literature, the strong genome-wide coupling demonstrated by the analyses suggests that changes in mRNA stability make an important mechanistic contribution to mediating the effects of synonymous changes in codon usage (Boël G, Letso R, Neely H, Price WN, Su M, Luff J, Valecha M, Everett JK, Acton T, Xiao R, Montelione GT, Aalberts DP, Hunt JF. Nature Submitted (under review)). This tight coupling could account for many difficulties encountered in characterizing translational regulatory phenomena.

The mathematical model described herein can infer codon efficiency from mRNA profiling data, opening a new approach to elucidating codon-related translational regulation. The key features of a comprehensive codon-influence metric from mathematical analysis of a single mRNA microarray dataset has been demonstrated and provides a new and exceedingly simple approach to characterizing codon-based translational regulatory effects *in vivo* (Boël G, Letso R, Neely H, Price WN, Su M, Luff J, Valecha M, Everett JK, Acton T, Xiao R, Montelione GT, Aalberts DP, Hunt JF. Nature Submitted (under review)).

The full complement of biochemical systems influencing synonymous codon usage in E. coli via quantitative genome-wide studies has also been elucidated.

### Example 17: High-Throughput protein-expression dataset

The expression of 6,348 protein-coding genes from a wide variety of phylogenetic sources were evaluated, which were transcribed from the bacteriophage T7 promoter in pET21, a 5.4 kb pBR322-derived plasmid harboring an ampicillin resistance marker (Acton TB, Gunsalus KC, Xiao R, Ma LC, Aramini J, Baran MC, Chiang YW, Climent T, Cooper B, Denissova NG, et al. Methods Enzymol. 2005; 394:210-43). Thanks to variations in codon-usage frequency in different organisms, this dataset provides broad sampling of codon-space. A bacteriophage polymerase was used to drive transcription to minimize potentially confounding effects from the coupling of translation to transcription by the native *E*. *coli* RNA polymerase (lost I, Dreyfus M. The EMBO journal. 1995; 14(13):3252-61; lost I, Guillerez J, Dreyfus M. Journal of bacteriology 1992;174(2):619-22). Protein expression (Acton TB, Gunsalus KC, Xiao R, Ma LC, Aramini J, Baran MC, Chiang YW, Climent T, Cooper B, Denissova NG, et al. Methods Enzymol. 2005; 394:210-43) was induced overnight in defined medium at 18 °C in *E*. *coli* strain BL21(DE3), which contains a single IPTG-inducible gene for T7 polymerase. This strain also contained pMGK, a 5.4 kb pACYC177-derived plasmid that harbors a kanamycin resistant gene, a single copy of the *lacI* gene, and a single copy of the *argU* gene encoding the tRNA cognate to the rare AGA codon for arg. The proteins were all expressed with an eight-residue C-terminal affinity tag (with sequence LEHHHHHH) that was omitted from computational analyses. The proteins in the dataset share less than 60% sequence identity. As previously described, protein expression level was scored from two isolates of the same plasmid on an integer scale from 0 (no expression) to 5 (highest expression), based on visual inspection of whole cell lysates on Coomassie-blue-stained SDS-PAGE gels. Scores rarely varied by more than ±1 between isolates (Figure S1 in Price WN, Handelman S, Everett J, Tong S, Bracic A, Luff J, Naumov V, Acton T, Manor P, Xiao R, Rost B, Montelione G, Hunt J. Microbial Informatics and Experimentation 2011;1(1):6). Roughly 30% of proteins gave a score of 0 (1,754 proteins) or 5 (1,973 proteins), while roughly 40% gave an intermediate score (2,621 proteins) (Price WN, Handelman S, Everett J, Tong S, Bracic A, Luff J, Naumov V, Acton T, Manor P, Xiao R, Rost B, Montelione G, Hunt J. Microbial Informatics and Experimentation 2011;1(1):6).

### Example 18: Characteristics of highly expressed genes

The distributions of a wide variety of mRNA sequence parameters in the genes were evaluated, giving each expression score in the large-scale dataset, which revealed many differences between those giving high *vs*. low expression. Histograms of the parameter distributions were examined for the genes giving each score (*e.g.,* as shown in **Figures 9A****,F**), which show roughly monotonic changes with increasing score. The "log-odds-ratio" plots of the natural logarithm of the ratio of the numbers of genes were also examined, giving scores of 5 *vs.* 0 as a function of each parameter value (*e.g*., as shown in **Figures 9E****,H**), which provide a graphical summary of the trends observed in the histograms. These plots also provide guidance for mathematical modeling of the relationship between mRNA sequence parameters and protein expression.

Increasing frequency of some codons correlates with higher or lower expression levels. The GAA codon for glutamate shows the strongest expression-enhancing effect **(****Figures 9A****,****E****)**, whereas the synonymous GAG codon shows an equivalent frequency distribution for all expression scores **(****Figure 9E****).** The AUA codon for ile shows one of the strongest expression-attenuating effects, whereas the synonymous AUC and AUU codons show neutral and slightly expression-enhancing effects, respectively **(****Figure 9E****).** While these trends naively suggest differences between the translation efficiencies of these codons, multivariate statistical analyses and biochemical analyses presented below indicate that their origin is more complex. However, adjacent pairs of AUA codons for ile have a very strong expression-attenuating effect that likely reflects inefficient translation. In contrast, the frequency of the AGGA motif (Ingolia NT, Ghaemmaghami S, Newman JR, Weissman JS. Science 2009;324(5924):218-23), which matches the Shine-Dalgarno ribosome-binding sequence, does not appear to have a significant influence on protein expression level.

The distributions of the predicted partition-function free energies of folding (Reuter JS, Mathews DH. BMC bioinformatics. 2010;11:129) of the mRNA transcripts also show systematic differences between proteins with different expression scores. Expression is attenuated by increasingly stable folding *(i.e.,* decreasing free energy of folding) in the first 48 nucleotides in the coding sequence (Kozak M. Gene. 2005;361:13-37; Shakin-Eshleman SH, Liebhaber SA Biochemistry 1988; 27(11):3975-82; Castillo-Mendez MA, Jacinto-Loeza E, Olivares-Trejo JJ, Guarneros-Pena G, Hernandez-Sanchez J. Biochimie. 2012;94(3):662-72), which is referred to as the head of the gene. Although this effect is consistent with observations made in previous studies, the data provide robust calibration of the probability of attenuating expression as a function of predicted free-energy of folding in the head (Δ*G_{H}*), and they show an ∼1/*e* reduction in the odds of high expression at *ΔG_{H}*= -15 kcal/mol. The strength of the correlation is increased modestly by including the 5' untranslated region (UTR) of the mRNA when calculating the free energy of folding of the head, Δ*G_{UH}* (**Figure 9F**). Unexpectedly, <Δ*G_{T}*>, the mean value of the predicted free energy of folding in the tail of the gene (*i.e*., nucleotides 49 through the stop codon), shows a non-linear influence on expression level, with both high and low values systematically attenuating expression **(****Figure 9H****)**. Roughly equivalent trends are observed when the mean is calculated in 50% overlapping windows with widths of 48, 96, or 144 nucleotides **(****Figure 9H****)**. Although these observations suggest that excessively stable or unstable mRNA folding in the tail attenuates expression, the analyses below indicate these effects also have more complex origins. Several additional global sequence parameters have a systematic relationship to protein expression level in the large-scale dataset (Boël G, Letso R, Neely H, Price WN, Su M, Luff J, Valecha M, Everett JK, Acton T, Xiao R, Montelione GT, Aalberts DP, Hunt JF. Nature Submitted (under review)).

The influence of nucleotide identity at individual positions at the start of the protein coding sequence on the log-odds-ratio of observing scores of 5 *vs.* 0 was examined. Nucleotide composition in this region has a very strong influence on protein expression, but its influence declines substantially after the sixth codon, which corresponds roughly to the region of the mRNA physically protected by the ribosome in the 70S initiation complex. Within the protected region, G bases reduce the probability of high expression, while A bases increase it, and C and U bases have intermediate effects. The rank-order of these effects matches the probability of base-pairing for each nucleotide in large ensembles of folded RNA structures (D.P. Aalberts, manuscript in preparation), suggesting the observed trend reflects a requirement for the mRNA bases in this region to be unpaired for efficient ribosome docking.

### Example 19: Multiparameter binary logistic regression analysis of mRNA features influencing protein expression level

The relative influence of different mRNA sequence parameters on protein expression level in the large-scale dataset using logistic regression was examined, which employs a generalized linear model to quantify the influence of continuous variables on binary or ordinal results. Results are modeled assuming that the log-odds-ratio for two mutually exclusive outcomes (*e.g.,* 5 *vs.* 0 scores in the dataset) increases linearly with the value of some function of a continuous variable (*e.g*., codon frequency). **Figures 9E****,H** illustrate the simplest form of binary logistic regression, in which the log-odds-ratio is assumed to be a linear function of the continuous variable. The solid lines show the most probable slopes for a linear relationship between the frequencies and the log-odds-ratio of proteins with 5 vs. 0 expression scores. This linear model accurately describes the beneficial influence of the GAA codon (green in **Figure 9E**), while it is less accurate in describing the deleterious influence of the AUA codon (red in **Figure 9E**). Logistic regression can be performed using different mathematical functions of the continuous variable to model more complex behavior of this kind. Nonetheless, "codon slopes" from linear logistic-regression analyses such as these provide a useful metric to quantify the influence of individual codons on protein expression.

Such single-variable analyses were conducted on all 61 non-stop codons using either binary (5 *vs.* 0 scores) or ordinal (5-0 scores) linear logistic regression. The relatively uniform variance in codon frequencies in the genes in the dataset enables regression parameters for all codons to be determined with similar precision. The codon-slopes determined this way show that codons ending in A or U are systematically enriched in genes giving the highest level of protein expression, while the synonymous codons ending in G or C are systematically depleted. These results provide guidance for engineering synthetic genes that enhance protein expression by emulating the properties of the best-expressed genes, a strategy demonstrated below to be successful. However, this computational approach does not provide reliable information on the influence of each codon because the frequencies of most codons ending in A or U are correlated with one another in the genes in the dataset, due at least in part to variations in AT *vs*. GC frequency in the genomes of the source organisms. Many parameters that vary systematically between genes giving different protein expression levels, including <Δ*G_{T}*>₉₆. A parameter that does not directly influence outcome can appear influential in a single-parameter regression when its value is correlated with that of a directly influential parameter. Therefore, to dissect the mechanistic contributions of the parameters, multi-parameter logistic-regression modeling was performed. This approach simultaneously analyzes the influence of all parameters, although the reliability with which differences between correlated parameters can be quantified depends on the extent to which they vary independently in genes in the dataset.

The final multi-parameter binary logistic-regression model combines the explanatory variables explored individually after eliminating those whose influence is captured by other correlated variables. The logarithm of the odds of observing the highest level of expression *vs*. no expression is given by: $θ = 3.8 + 0.046 ⁢ Δ {G}_{UH} - 1.5 ⁢ I + 6.6 {a}_{H} - 6.3 {a}_{H}^{2} - 1.9 {g}_{H}^{2} + 0.76 {u}_{3 ⁢ H} + 0.077 {s}_{7 - 16} + 0.059 {s}_{17 - 32} + 0.86 {\sum }_{c} {β}_{c} {f}_{c} - 18 {d}_{AUA} - 13 ⁢ r - 0.011 ⁢ L - 490 / L$ In this equation, Δ*G_{UH}* is the predicted free energy of folding of the head of the gene plus the 5'-UTR (in kcal/mol), *I* is a binary indicator variable that is 1 if Δ*G_{UH}* < -39 kcal and the GC content of nucleotides 2-6 is greater than 62% (and otherwise zero), *a_{H}* and *g_{H}* are respectively the frequencies of adenine and guanine in codons 2-6, *u_{3H}* is the frequency of uridine at 3^{rd} position in codons 2-6, *s*₇₋₁₆ and *s*₁₇₋₃₂ are respectively the mean slopes for codons 7-16 and 17-32, '*_{c}* and *f_{c}* are respectively the slopes and frequencies of each non-termination codon in the gene, *d_{AUA}* is a binary variable that assumes a value of 1 if there are any AUA-AUA di-codons, *r* is the codon repetition rate, and *L* is the sequence length.

Calculating the loss in the predictive power when terms are omitted gives the best estimate of their relative influence in the model and of different regions in the genes **(****Figures 29A****,****B****)**. The influence of the head is captured by the combination of the folding-energy and base-composition terms, which likely reflect accessibility of the translation-initiation site for ribosome docking (Duval M, Korepanov A, Fuchsbauer O, Fechter P, Haller A, Fabbretti A, Choulier L, Micura R, Klaholz BP, Romby P, Springer M, Marzi S. PLoS biology 2013;11(12):e1001731), together with the *s*₇₋₁₆ term. The influence of the tail is captured by the *s*₁₇₋₃₂ term together with the global terms, because the tail dominates these parameters (overall codon influence, *d_{AUA}, r,* and *L*). The computation modeling indicates that influential mRNA-folding energy effects are restricted to the head and that these effects are significant but weaker in overall influence than codon-related effects **(****Figure 29B****)**. The codon-related effects are ∼2.3 times stronger near the 5' end of the coding sequence and decline to a constant level after codon ∼32 (not shown), roughly matching the number of residues that fill the ribosomal exit channel (Lu J, Deutsch C. Journal of molecular biolog. 2008;384(1):73-86.)⁸¹. However, because the genes in the dataset have tails that are much longer than the head, codon content in the average tail is ∼7 times more influential than in the head. Control calculations show that in-frame codon models are superior to out-of-frame codon models. They also show that the mean predicted free energy of mRNA folding in the tail (*i.e.,* <*G_{T}*>₉₆) makes an insignificant contribution to the model when the codon slopes and codon-repetition rate *r* are included, indicating that the apparent influence of <*G_{T}*>₉₆ on expression **(****Figure 9H****)** is likely attributable to its correlation with these more influential parameters.

### Example 20: New codon-influence metric

The codon slopes from the multi-parameter logistic-regression model (Figure 11B) provide a new codon-influence metric quantifying the average effect of each codon on translation efficiency in *E*. *coli.* While some features of this metric match conclusions in previous literature, the broad trends do not. The AUA codon for ile, which is decoded by an unusual non-cognate tRNA (Forouhar F, Arragain S, Atta M, Gambarelli S, Mouesca JM, Hussain M, Xiao R, Kieffer-Jaquinod S, Seetharaman J, Acton TB, Montelione GT, Mulliez E, Hunt JF, Fontecave M. Nature chemical biology 2013;9(5):333-8; Spencer PS, Siller E, Anderson JF, Barral JM. Silent substitutions predictably alter translation elongation rates and protein folding efficiencies. Journal of molecular biology. 2012;422(3):328-35), has by far the strongest expression-attenuating effect, and adjacent pairs of AUA codons have a significantly stronger attenuating effect than two non-adjacent AUA codons. The other two codons for ile have an approximately neutral influence, indicating that the expression-attenuating effect of AUA is attributable to codon identity rather than amino acid structure. Similarly, the CGG and CGA codons for arg have a strong expression-attenuating effect, while the four synonymous codons have weaker effects that vary in direction. Among the eight rare codons emphasized in previous literature to be deleterious for expression (Strader MB, Costantino N, Elkins CA, Chen CY, Patel I, Makusky AJ, Choy JS, Court DL, Markey SP, Kowalak JA. Molecular & cellular proteomics : MCP. 2011;10(3):M110.005199; Forouhar F, Arragain S, Atta M, Gambarelli S, Mouesca JM, Hussain M, Xiao R, Kieffer-Jaquinod S, Seetharaman J, Acton TB, Montelione GT, Mulliez E, Hunt JF, Fontecave M. Nature chemical biology 2013; 9(5):333-8; Kruger MK, Pedersen S, Hagervall TG, Sorensen MA. J Mol Biol. 1998;284(3):621-31; Zhang F, Saha S, Shabalina SA, Kashina A. Science 2010;329(5998):1534-7; Dana A, Tuller T. Nucleic Acids Res. 2014;42(14):9171-81; Sharp PM, Li WH. Nucleic Acids Res. 1987;15(3):1281-95), only four attenuate expression in the dataset (the AUA/CGG/CGA codons cited above and the CUA codon for leu), while the other four are either neutral (the AGA codon for arg and the GGA codon for glycine) or weakly enhance expression (the AGG codon for arg and the CCC codon for pro). The apparent influence of AGA and possibly that of AGG may be biased by overexpression in the experiments of the *argU* tRNA cognate to AGA. Ignoring these two codons, which have the lowest frequencies in *E*. *coli,* the next three least frequent codons attenuate expression (**Figure 11C**). However, there is a wide variation in the magnitude of their influence, and codons with slightly higher frequencies are neutral or weakly enhance expression. Furthermore, there is no significant correlation between the frequencies of the remaining 56 non-stop codons and their influence on expression **(****Figure 11C****)**. Similarly, there is no significant correlation between the influence of all 61 non-stop codons and either the codon adaptation index (Sharp PM, Li WH. Nucleic Acids Res. 1987;15(3):1281-95), the codon sensitivity (Elf J, Nilsson D, Tenson T, Ehrenberg M. Science 2003;300(5626):1718-22), the tRNA adaptation index (Tuller T, Carmi A, Vestsigian K, Navon S, Dorfan Y, Zaborske J, Pan T, Dahan O, Furman I, Pilpel Y. Cell 2010;141(2):344-54), or an estimate of cognate tRNA concentration (Dong H, Nilsson L, Kurland CG. Journal of molecular biology 1996; 260(5):649-63).

The most strongly expression-enhancing codons in **Figure 11B** correspond to the three amino acids with sidechains that can act as general base catalysts (glu, asp, and his). For these three amino acids, the codons ending in A or U have a stronger expression-enhancing effect than the synonymous codons ending in G or C, indicating that codon structure is likely to modulate the efficiency of their translation. However, plotting the codon slopes from the multi-parameter logistic-regression model against amino acid hydrophobicity reveals a strong correlation **(****Figure 11D****)*,*** with charged amino acids having systematically higher slopes than polar or hydrophobic amino acids. Therefore, the analyses suggest that translation efficiency varies systematically with amino acid structure. The correlation of the new codon-influence metric with hydrophobicity is so strong that integral membrane proteins in *E*. *coli* can be identified with ∼80% accuracy based on its mean value in their gene sequences **(****Figure 37****)**. This observation suggests that the evolution of the decoding properties of the ribosome may have been influenced by the greater challenges involved in the biogenesis of membrane proteins compared to soluble proteins. In contrast, analyzing the codon slopes as a function of the identity of the nucleotide base at each codon position indicates that differences in the translation efficiency of synonymous codons **(****Figure 11B****)** are unlikely to have a systematic relationship to base content.

### Example 21: Design and testing of efficiently translated genes

The validity and predictive value of the analyses presented above were tested by evaluating the expression properties of synthetic genes encoding 22 unrelated proteins **(****Figure 13****)**. Sequences were designed using two different methods that emulate the codon-usage and mRNA-folding properties of the genes giving the highest level of protein expression in the large-scale dataset. In the "six amino acid" (6AA) method, all codons for arg, asp, glu, gln, his, and ile were substituted with the synonymous codon with the highest slope in **Figure 11B**. The resulting mRNAs are enriched in codons ending in A or U bases, which have lower mean folding energies than G or C bases, and they tend to have mRNA-folding properties and other properties that match those of the genes giving the highest protein expression in the dataset, providing a concrete example of the influence of the parameter cross-correlations. In the "31 codon folding optimization" (31C-FO) method, the calculated free energy of mRNA folding was explicitly optimized using just 31 codons with the highest slopes for each amino acid in the single-variable logistic regressions in **Figure 11B**. Folding energy in the head (Δ*G_{UH}*) was maximized *(i.e.,* minimizing folding stability), while the folding energy in the tail (<Δ*G_{T}*>₄₈) was adjusted to be near -10 kcal/mol. In some experiments, the head but not the tail was engineered, or *vice versa,* to evaluate the reliability of the inferences from multi-parameter computational modeling concerning their relative contributions. In brief, these experiments demonstrate that folding effects in the head, codon usage in the head, and codon usage in the tail all have a significant influence on protein expression, supporting the validity of the computational inferences **(****Figures 29****,** **11B-D****)**.

### Example 22: Biochemical analyses of optimized synthetic genes show a strong linkage between codon efficiency and mRNA level

For five native *vs*. optimized bacterial genes from the large-scale dataset, cellular growth-rates **(****Figure 13A****)**, protein expression levels **(****Figure 13B****)**, and mRNA levels **(****Figure 13D****)** after induction *in vivo* in *E*. *coli* were compared. The products of *in vitro* transcription and translation **(****Figure 13C****)** reactions were also compared. For one target, inhibition of cell growth upon induction of protein expression is eliminated by optimization of the gene sequence even though it greatly increases protein expression **(****Figure 13A-B****)**, suggesting that mRNA features impeding translation can cause physiological toxicity in *E*. *coli.* Although *in vitro* transcription of the native or optimized genes using purified T7 RNA yields equivalent amounts of mRNA (*in vitro* translation of the resulting mRNAs using purified ribosomes and translation factors yields substantially higher levels of protein synthesis for all of the optimized sequences **(****Figure 13C**)). Notably, the sites of translational pausing are different in some of the optimized mRNAs *vs*. native mRNAs. Essentially equivalent results were observed when all of these experiments were performed on native *vs*. optimized variants of the other four proteins (Boël G, Letso R, Neely H, Price WN, Su M, Luff J, Valecha M, Everett JK, Acton T, Xiao R, Montelione GT, Aalberts DP, Hunt JF. Nature Submitted (under review)). These observations demonstrate that translation efficiency in *E*. *coli* is improved by the codon-optimization methods derived from the computational analyses of the large-scale expression dataset **(****Figures 29****,** **11B-D****)**.

Consistently lower levels of mRNA *in vivo* were observed after induction of the inefficiently translated native genes compared to the optimized genes **(****Figure 13D****)**, suggesting that mRNA-sequence-dependent translational obstacles can strongly influence steady-state mRNA level. Notably, 5 min after induction, full-length mRNA is detected for all of the optimized but none of the native genes. This observation suggests the inefficiently translated native mRNAs are rapidly degraded, because T7 polymerase transcribes them with equivalent efficiency *in vitro.* To evaluate the physiological relevance of this inference, the results from the multi-parameter logistic-regression model were used to calculate *s_{ALL},* the average codon-slope **(****Figure 11B****),** for every endogenous gene in *E*. *coli.* This parameter derived from the large-scale expression dataset correlates strongly with *in vivo* protein levels in *E*. *coli* quantified using mass spectrometry **(****Figure 30B****),** supporting the validity of the new codon-influence metric. Strikingly, *s_{ALL}* correlates almost as strongly with *in vivo* mRNA levels of all predicted cytoplasmic proteins **(****Figures 30A-B****)**, indicating that codon content significantly influences steady-state mRNA concentration. For proteins detected in mass spectrometric profiling, which are generally more abundant, *s_{ALL}* correlates with both their mRNA levels and protein/mRNA ratios, which can reflect translation efficiency. These global correlations support codon content exerting an important influence not only on the efficiency of mRNA translation but also on mRNA stability.

### Example 23: Multiparameter logistic regression analysis of a single mRNA microarray dataset produces a similar codon-influence metric as the large-scale protein-expression dataset

Based on the strong correlation that was observed between the new codon-influence metric and global mRNA concentrations in *E*. *coli* **(****Figure 30****),** similar multiparameter regression methods were investigated to determine whether they could be applied to infer codon influence *directly from computational analysis of mRNA microarray data (i.e.,* without including any data related to protein-expression level). The methods will be optimized, but the codon slopes that have been determined from a multiparameter logistic regression analysis on mRNA microarray values are strongly correlated with those inferred from the large-scale expression dataset **(****Figure 38****)**. This analysis used a similar computational model to that described above, which was applied to the most strongly and weakly expressed 30% of the 2,817 genes predicted to encode cytoplasmic proteins. The analyzed microarray dataset came from *E*. *coli* MG1655 rather than the BL21(DE3) strain overexpressing the *argU* tRNA that was used to generate the large-scale dataset, and there were also substantial difference in growth conditions. Therefore, the difference between the codon influence inferred from these two analyses could be real. While the details of this analysis will be evaluated, it is clear that it generates some reliable information on codon effects. The most beneficial (GAA) and detrimental (AUA) codons for protein expression in the large-scale dataset give very similar slopes in the microarray analysis **(****Figure 38****)**. Notably, three of the four codons showing the strongest differences between their slopes inferred from the protein-expression *vs*. microarray datasets encode arginine (as highlighted by the white regions in **Figure 38**). Notably, the influence of the AGA and AGG codons, which are cognate to the *argU* tRNA, is strongly negative in the microarray dataset but modestly positive in the protein-expression dataset, as would be expected from prior literature showing that "codon supplementation" improves their translation efficiency. Intriguingly, the codon showing the strongest change in the opposite direction is the CGU codon for arginine, suggesting that the charging dynamics of its cognate tRNA or some other factor influencing its translation efficiency is perturbed by competition from the *argU* tRNA. While the analysis methods and results will be analyzed, the data in **Figure 38** demonstrate that multiparameter regression analysis of mRNA concentration levels provides significant information on codon effects. This new and facile approach to characterizing codon influence on protein expression merits further exploration.

### Example 24: Genome-scale correlations

The genome-scale correlations described above indicate that codon content is an important determinant of both the translation efficiency and stability of mRNA in *E*. *coli* and that these parameters are tightly coupled, as suggested in some prior literature (Dana A, Tuller T. Nucleic Acids Res. 2014;42(14):9171-81; Dittmar KA, Sorensen MA, Elf J, Ehrenberg M, Pan T EMBO reports. 2005;6(2):151-7; Drummond DA, Wilke CO. Cell 2008;134(2):341-52; Rocha EP. Genome research 2004;14(11):2279-86; Vladimir Presnyak Y-HC, Sophie Martin, Najwa Al Husaini, David Weinberg, Sara Olson, Kristian E. Baker, Brenton Graveley, Jeff Coller. CSHL Translational Control; CSHL2014). Several molecular mechanisms could explain the observed coupling of codon content to steady-state mRNA concentration. It is possible that it is mediated by a kinetic competition between protein elongation and mRNA degradation that is modulated by ribosomal elongation dynamics (*i.e.,* the sequential binding and conformational processes involved in amino-acyl-tRNA selection, peptide-bond synthesis, and tRNA/mRNA translocation). The bacteriophage T7 RNA polymerase used in the experiments synthesizes mRNA too rapidly for translating ribosomes to keep up, making the resulting transcripts insensitive to transcription-translation coupling but more sensitive to endonuclease cleavage (lost I, Dreyfus M. The EMBO journal 1995;14(13):3252-61; Cardinale CJ, Washburn RS, Tadigotla VR, Brown LM, Gottesman ME, Nudler E. Science 2008;320(5878):935-8). Therefore, the observation that inefficiently translated mRNAs produced by T7 polymerase are fragmented and have lower concentrations *in vivo* **(****Figure 13D****)** is likely to reflect enhanced degradation. This reasoning, as well as the tendency of expression-attenuating codons to eliminate protein expression entirely in the large-scale dataset **(****Figures 9A****,****F****)**, suggests that mRNA degradation is controlled in part by ribosomal elongation dynamics (Zaher HS, Green R. Cell 2011;147(2):396-408; Deana A, Ehrlich R, Reiss C. Journal of bacteriology 1996;178(9):2718-20; dos Reis M. Nucleic Acids Research 2003;31(23):6976-85; Li X, Yokota T, Ito K, Nakamura Y, Aiba H Molecular microbiology 2007;63(1):116-26; Nogueira T, de Smit M, Graffe M, Springer M. Journal of molecular biology 2001;310(4):709-22; Li X, Hirano R, Tagami H, Aiba H Rna 2006;12(2):248-55; Leroy A, Vanzo NF, Sousa S, Dreyfus M, Carpousis AJ. Molecular Microbiology. 2002;45(5):1231-43). Several biochemical systems mediate recycling of ribosomes stalled due to protein synthesis/folding problems (Richards J, Sundermeier T, Svetlanov A, Karzai AW. Biochimica et biophysica acta. 2008;1779(9):574-82; Li X, Hirano R, Tagami H, Aiba H. Rna. 2006;12(2):248-55) or mRNA truncation (Drummond DA, Wilke CO. Cell 2008;134(2):341-52; Deana A, Ehrlich R, Reiss C. Journal of bacteriology 1996;178(9):2718-20). In eukaryotes, this "No-Go" decay pathway involves the Dom34, Hbs1 (Shoemaker CJ, Green R. Nat Struct Mol Biol. 2012;19(6):594-601; Shoemaker CJ, Eyler DE, Green R. Science 2010;330(6002):369-72), and ABCE1 (Becker T, Franckenberg S, Wickles S, Shoemaker CJ, Anger AM, Armache JP, Sieber H, Ungewickell C, Berninghausen O, Daberkow I, et al. Nature 2012;482(7386):501-6) proteins, whereas in *E*. *coli,* similar activities are mediated by unrelated systems including the tmRNA pathway (Vivanco-Dominguez S, Bueno-Martinez J, Leon-Avila G, Iwakura N, Kaji A, Kaji H, Guarneros G. Journal of molecular biology 2012;417(5):425-39; Richards J, Sundermeier T, Svetlanov A, Karzai AW. Biochimica et biophysica acta. 2008;1779(9):574-82; Ivanova N, Pavlov MY, Ehrenberg M. Journal of molecular biology 2005;350(5):897-905; Christensen SK, Gerdes K. Molecular Microbiology 2003;48(5):1389-400), ArfA, YaeJ (Chadani Y, Ono K, Kutsukake K, Abo T. Molecular microbiology 2011;80(3):772-85), and RF3 (Vivanco-Dominguez S, Bueno-Martinez J, Leon-Avila G, Iwakura N, Kaji A, Kaji H, Guarneros G. Journal of molecular biology 2012;417(5):425-39; Zaher HS, Green R. Cell 2011; 147(2):396-408). These prokaryotic mRNA quality-control systems (Shoemaker CJ, Green R. Nat Struct Mol Biol. 2012;19(6):594-601) are candidates to participate in the mRNA decay process that is hypothesized to be coupled to codon-dependent variations in ribosomal elongation dynamics.

The codon-influence metric **(****Figure 11B****)** has significant differences compared to previous inferences. It shows that amino-acid identity influences translation efficiency **(****Figures 11D** **&** **37****)** but that, despite longstanding assumptions (Li G-W, Oh E, Weissman JS. Nature 2012;484(7395):538-41; Li GW, Burkhardt D, Gross C, Weissman JS. Cell 2014;157(3):624-35), genomic codon-usage frequency is not directly related. The 3^{rd}, 4^{th}, and 5^{th} least frequent codons in *E*. *coli* have the most deleterious influence on expression in the large-scale dataset **(****Figure 11B****)**. However, these codons attenuate expression to widely varying extents, and slightly more frequent codons have a neutral or expression-enhancing influence **(****Figure 11B****)**. Furthermore, the frequencies of the other 58 non-stop codons are not significantly correlated with expression level **(****Figure 11B****)**. Codon-usage frequency has been assumed to influence translation *in vivo* because it is correlated with the concentration of the cognate tRNA (Ikemura T. Journal of molecular biology 1981;151(3):389-409; Dong H, Nilsson L, Kurland CG. Journal of molecular biology 1996;260(5):649-63; Caskey CT, Beaudet A, Nirenberg M. Journal of molecular biology 1968;37(1):99-118; Muramatsu T, Nishikawa K, Nemoto F, Kuchino Y, Nishimura S, Miyazawa T, Yokoyama S. Nature 1988;336(6195):179-81), which can clearly influence protein-elongation rate *in vitro* (Forouhar F, Arragain S, Atta M, Gambarelli S, Mouesca JM, Hussain M, Xiao R, Kieffer-Jaquinod S, Seetharaman J, Acton TB, Montelione GT, Mulliez E, Hunt JF, Fontecave M. Nature chemical biology. 2013;9(5):333-8; Spencer PS, Siller E, Anderson JF, Barral JM. Journal of molecular biology 2012;422(3):328-35) and protein yield *in vivo* (Chen GT, Inouye M. Genes & development 1994;8(21):2641-52; Vivanco-Dominguez S, Bueno-Martinez J, Leon-Avila G, Iwakura N, Kaji A, Kaji H, Guarneros G. Journal of molecular biology 2012;417(5):425-39; Deana A, Ehrlich R, Reiss C. Journal of bacteriology 1996;178(9):2718-20; Li X, Hirano R, Tagami H, Aiba H. Rna 2006;12(2):248-55). Indeed, the ArgU tRNA in the experiments were overexpressed to promote higher expression of proteins enriched in AGA/AGG codons (Chen GT, Inouye M. Genes & development 1994;8(21):2641-52), which may bias the influence of these codons in the dataset **(****Figure 11B****)**. Further research will be required to understand the factors determining when tRNA concentration influences ribosomal elongation dynamics. Nonetheless, the analyses suggest that ribosomal elongation dynamics exert a stronger influence on protein expression than cognate tRNA concentration. This inference is consistent with the demonstration that the translation factor EFP aids elongation of proline-rich sequences (Ude S, Lassak J, Starosta AL, Kraxenberger T, Wilson DN, Jung K. Science 2013;339(6115):82-5). Furthermore, it suggests that translational regulatory effects could operate via modification of ribosomal elongation dynamics, mediated for example by covalent modification of tRNAs or the ribosome (Muramatsu T, Nishikawa K, Nemoto F, Kuchino Y, Nishimura S, Miyazawa T, Yokoyama S. Nature 1988;336(6195):179-81). Complicating related mechanistic studies (Deana A, Ehrlich R, Reiss C. Journal of bacteriology 1996;178(9):2718-20; lost I, Dreyfus M. The EMBO journal 1995;14(13):3252-61; dos Reis M. Nucleic Acids Research 2003;31(23):6976-85; Nogueira T, de Smit M, Graffe M, Springer M. Journal of molecular biology 2001;310(4):709-22), the results also suggest that such regulatory effects could be manifested via alterations in mRNA levels. The following examples are designed to (*i*) validate more extensively the details of the new codon-influence metric in **Figure 11B****,** (*ii*) elucidate the molecular mechanisms underlying these effects and the others observed, and (*iii*) generate deeper insight into the biological implications of variations in synonymous codon usage.

### Example 25: Evaluate the efficacy of alternative fluorescent-protein approaches for characterization of the relative expression efficiency of synonymous gene sequences in vivo in E. coli.

Fluorescent protein methods for rapid quantification of the influence of synonymous codon variations on protein expression *in vivo* will be developed. Fluorescent methods, including the use of genetically encoded fluorescent proteins, will be used. Genomics tools that will be used include a plasmid collection containing in-frame translational fusions (Kitagawa M, Ara T, Arifuzzaman M, Ioka-Nakamichi T, Inamoto E, Toyonaga H, Mori H. DNA research: an international journal for rapid publication of reports on genes and genomes. 2005;12(5):291-9; Rajagopala SV, Yamamoto N, Zweifel AE, Nakamichi T, Huang HK, Mendez-Rios JD, Franca-Koh J, Boorgula MP, Fujita K, Suzuki K, Hu JC, Wanner BL, Mori H, Uetz P. BMC Genomics. 2010;11:470; Nakahigashi K, Toya Y, Ishii N, Soga T, Hasegawa M, Watanabe H, Takai Y, Honma M, Mori H, Tomita M. Molecular systems biology 2009;5:306) of yellow fluorescent protein (YFP) to almost every protein-coding gene in *E*. *coli.* A derivative of this collection has been used to quantify an ∼1.5-fold change in the expression of a specific protein during log-phase growth in *E*. *coli* cells in which the EttA translation factor was genetically knocked (Datsenko KA, Wanner BL. Proceedings of the National Academy of Sciences of the United States of America. 2000;97(12):6640-5; Baba T, Ara T, Hasegawa M, Takai Y, Okumura Y, Baba M, Datsenko KA, Tomita M, Wanner BL, Mori H. Molecular systems biology. 2006;2:2006 0008. doi: 10.1038/msb4100050; Otsuka Y, Muto A, Takeuchi R, Okada C, Ishikawa M, Nakamura K, Yamamoto N, Dose H, Nakahigashi K, Tanishima S, et al. Nucleic Acids Res. 2015; 43(Database issue):D606-17. Epub 2014/11/17. doi: 10.1093/nar/gku1164) **(****Figure 39****)**. This experiment employed a chromosomally encoded in-frame translational fusion to the AceB (Nakahigashi K, Toya Y, Ishii N, Soga T, Hasegawa M, Watanabe H, Takai Y, Honma M, Mori H, Tomita M Molecular systems biology. 2009;5:306) protein expressed under the control of the endogenous *E*. *coli* promoter for that protein. The data in **Figure 39** demonstrate that real-time measurements of fluorescent fusion protein expression *in vivo* using a microplate reader provide very sensitive accurate quantification of protein expression at physiological levels. This technology will be harnessed for robust quantification of the effects of variations in synonymous codon usage on protein expression in *E*. *coli.*

The efficacy of alternative technical approaches to quantifying synonymous codon effects *in vivo* using fluorescent proteins will be systematically evaluated. These studies will compare results obtained using each of the candidate fluorescent protein methods to those obtained in the results described herein on protein expression from synonymous genes. Protein levels quantified via Coomasie Blue staining or SDS-PAGE gels or quantitative immunoblotting will be compared to fluorescence emission intensity signals *in vivo,* and the corresponding mRNA levels will be examined in using Northern blotting or real-time PCR (RT-PCR). Results from these fluorescent protein systems will be compared to those obtained on the same synonymous gene pairs in the results described herein. Key variables to be examined include the following:
(1) Comparison of single *vs*. dual fluorescence reporter approaches for their robustness and accuracy in quantifying protein expression differences *in vivo:* The data shown in **Figure 39** demonstrate that observation of the emission from single fluorescent reporter proteins in carefully controlled experiments can reliably quantify an expression difference on the order of 1.5-fold. These data suggest that single fluorescence reporters may be sufficient to characterize many important codon effects. However, increased robustness may be achieved in some experiments using dual fluorescent-protein reporter systems enabling simultaneous measurement of the emission from two proteins with different spectral characteristics. The ratiometric fluorescence measurements from systems of this kind will be evaluated to determine whether they provide superior performance to single-channel measurements from one reporter, based on modeling the signal to noise characteristics. The performance of ratiometric systems constructed using different colored variants (Chudakov DM, Lukyanov S, Lukyanov KA. Trends in biotechnology. 2005;23(12):605-13) of GFP (Heim R, Cubitt AB, Tsien RY. Nature. 1995;373(6516):663-4), Superfolder GFP (Pedelacq JD, Cabantous S, Tran T, Terwilliger TC, Waldo GS. Nat Biotechnol. 2006;24(1):79-88), and Superfast GFP (Fisher AC, DeLisa MP. PLoS One. 2008;3(6):e2351) *(i.e.,* with blue *vs*. cyan *vs*. green *vs*. yellow emissions) will also be compared.
(2) Compare two approaches to constructing the fluorescent reporter genes **(****Figure 40****)**: One will involve in-frame translational fusions that produce a covalent fusion between the test protein and the fluorescent reporter protein, while the other will involve a transcriptional or "operon" fusion in which the two proteins are translated independently from the same polycistronic message. In the latter approach, the test protein will have a stop codon that will be followed by a short linker (∼5-25 nucleotides), which will be followed by an AUG initiation codon at the start of the coding sequence for the fluorescent protein. The results from such operon fusions will be compared either with (as shown on the bottom in **Figure 40**) or without a ribosome-binding site (Shine Dalgarno sequence) in the linker region. Covalent fusion protein constructs will be engineered without the N-terminal methionine in the fluorescent protein to avoid internal translation re-initiation.
(3) Compare results obtained with the same synonymous genes and reporters transcribed either from T7 RNA polymerase, as used for the results described herein, or from *E*. *coli* RNA polymerase (which were used in the study on the physiology of integral membrane protein overexpression in *E*. *coli* in Boël G, Letso R, Neely H, Price WN, Su M, Luff J, Valecha M, Everett JK, Acton T, Xiao R, Montelione GT, Aalberts DP, Hunt JF. Nature Submitted (under review)). In the latter case, the results obtained from a *lac-*derived promoter under IPTG control will be controlled to those obtained with a variably inducible *ara*-derived promoter under arabinose control.
(4) Compare results obtained when reporters are expressed on a high copy-number pBR322-derived plasmid, a low copy number pACYC184 derived plasmid, or inserted in single copy on the chromosome using either the CRIM plasmid method or the λ *red* recombination method (Datsenko KA, Wanner BL. Proceedings of the National Academy of Sciences of the United States of America. 2000;97(12):6640-5).
(5) Compare results obtained when equivalent synonymous codon changes are introduced directly into a GFP variant rather than an upstream fusion partner. These studies will be performed in parallel with the evaluation of the translational and transcriptional fusion systems described above, because this approach may offer a technical short-cut simplifying implementation of the approach. Codon effects have some degree of context-dependence, so this simpler approach may not work. To evaluate if it does, gene-optimization studies equivalent to those described above will be performed, using the same set of biochemical and molecular biological assay methods.

The systematic studies will establish the most robust and efficient optical method to quantify the influence of synonymous codon variations on protein expression level in *E. coli.*

### Example 26: Use the existing biochemical methods and the methods developed to test the details of the new E. coli codon-influence metric coli.

The broad features of the new codon-influence metric have been validated experimentally but the details will be explored in follow-up studies. For many pairs of synonymous codons, the differences between their influence scores derived from multiple logistic regression analysis are not large enough to be statistically significant when considered individually. However, the high predictive value in many analyses of the mean codon-influence score based on the metric suggests that many of these differences are likely to be real and mechanistically significant. The tools and assays will be used to analyze the details of the new codon metric and related mechanistic phenomena. Examples of experiments to be conducted include the following:
(1) Synthesize sets of synonymous genes in which all occurrences of one specific amino acid are encoded either by the same codon, by a random mixture of two wobble-related codons, by a random mixture of two non-wobble-related codons, or by a random mixture of all codons. The resulting data quantifying the relative translational efficiency of each synonymous codon will be compared to the values in the codon-influence metric, and this experimental design will also critically evaluate claims in previous literature that homogeneity or inhomogeneity in codon usage can have a significant influence on protein expression level. In the case of leucine, as one specific example, the metric indicates that the CUG and CUC codons are most efficient and roughly equivalent to one another, CUU and UUG and UUA are intermediate and roughly equivalent to one another, and CUA is least efficient. In this case, eight variants for at least two different proteins will be synthesized. Six variants would each use exactly one of the codons, one variant would use a random mixture of the CUG and CUC codons, and one variant would use a random mixture of the CUU and UUG and UUA codons. The proteins used in these studies will initially be drawn from the set included in the results described herein, although the same experimental design can be applied directly to a GFP variant if the calibration studies demonstrate that it exhibits equivalent behavior.
(2) In cases where significant differences are observed in the influence of two synonymous codons on expression, overexpression of the cognate tRNAs can be tested to determine whether they significantly modulate the observed differences. These studies will employ variants of the pMGK plasmid in which the *argU* gene (Saxena P, Walker JR. Journal of bacteriology. 1992;174(6):1956-64) is replaced by one or more copies of the genes encoding the relevant tRNAs. Similar experiments will explore whether overexpression of selected tRNA synthetases (Krishnakumar R, Ling J. FEBS letters. 2014;588(3):383-8) influence observed effects. These studies will explore more deeply the influence of tRNA pool level on protein expression efficiency. Possible effects of supplementation of the medium with the corresponding amino acid will also be explored.

Protein expression levels produced by the synonymous genes both *in vivo* and *in vitro* as well steady-state levels of the corresponding mRNAs *in vivo* assayed via Northern blotting or RT-PCR will be compared. In this manner, codon influence on *in vitro* translation will be evaluated to determine whether it always parallels its influence on mRNA level or whether some codons differentially influence these two properties.

### Example 27: Generate/analyze RNAseq data from E. coli strains with knockouts of genes hypothesized to modulate synonymous codon usage, including those that covalently modify the translation apparatus, to evaluate their influence on relative codon efficiency under selected growth conditions.

The influence of a set of candidate genes/proteins **(****Figure 12****)** on selected synonymous codon effects identified in the above results and in the studies conducted example 26 will be evaluated. These studies will focus initially on proteins known to be involved in mRNA degradation, translational quality control, and covalent modification of the translation apparatus. The results indicate that at least some mRNA-sequence-dependent translational obstacles are tightly coupled to mRNA degradation in *E*. *coli.* Several biochemical systems in *E*. *coli* are known to contribute to recycling of stalled ribosomes due to protein synthesis/folding problems (Richards J, Sundermeier T, Svetlanov A, Karzai AW. Biochimica et biophysica acta. 2008;1779(9):574-82; dos Reis M. Nucleic Acids Research. 2003;31(23):6976-85; Li X, Hirano R, Tagami H, Aiba H. Rna. 2006;12(2):248-55; Leroy A, Vanzo NF, Sousa S, Dreyfus M, Carpousis AJ. Molecular Microbiology. 2002;45(5):1231-43), including the tmRNA pathway (Vivanco-Dominguez S, Bueno-Martinez J, Leon-Avila G, Iwakura N, Kaji A, Kaji H, Guarneros G. Journal of molecular biology. 2012;417(5):425-39; Richards J, Sundermeier T, Svetlanov A, Karzai AW. Biochimica et biophysica acta. 2008;1779(9):574-82; Ivanova N, Pavlov MY, Ehrenberg M. Journal of molecular biology. 2005;350(5):897-905; Christensen SK, Gerdes K. Molecular Microbiology. 2003;48(5):1389-400) and the ArfA, YaeJ (Chadani Y, Ono K, Ozawa S, Takahashi Y, Takai K, Nanamiya H, Tozawa Y, Kutsukake K, Abo T. Molecular microbiology. 2010;78(4):796-808), and RF3 (Vivanco-Dominguez S, Bueno-Martinez J, Leon-Avila G, Iwakura N, Kaji A, Kaji H, Guarneros G. Journal of molecular biology. 2012;417(5):425-39; Zaher HS, Green R. Nature. 2009;457(7226):161-6) proteins. These systems could potential help link codon-dependent translational obstacles and allosteric signals on the ribosome to mRNA degradation. Finally, covalent modifications of the translational apparatus, especially non-essential modifications of tRNAs (Arragain S, Handelman SK, Forouhar F, Wei FY, Tomizawa K, Hunt JF, Douki T, Fontecave M, Mulliez E, Atta M. J Biol Chem. 2010;285(37):28425-33; Phizicky EM, Hopper AK. Genes & development. 2010;24(17):1832-60.; Sergeeva OV, Bogdanov AA, Sergiev PV. Biochimie. 2014. Epub 2014/12/17. doi: 10.1016/j.biochi.2014.11.019), could contribute to the differential influence of synonymous codons. A variety of assays will be performed on a set of strains harboring knockouts of individual candidate genes constructed (Baba T, Ara T, Hasegawa M, Takai Y, Okumura Y, Baba M, Datsenko KA, Tomita M, Wanner BL, Mori H. Molecular systems biology. 2006;2:2006 0008; Mori H, Baba T, Yokoyama K, Takeuchi R, Nomura W, Makishi K, Otsuka Y, Dose H, Wanner BL Methods in molecular biology. 2015;1279:45-65; Otsuka Y, Muto A, Takeuchi R, Okada C, Ishikawa M, Nakamura K, Yamamoto N, Dose H, Nakahigashi K, Tanishima S, et al. Nucleic Acids Res. 2015;43(Database issue):D606-17. Epub 2014/11/17. doi: 10.1093/nar/gku1164). These assays will focus on characterizing and quantifying the effects of the gene knockouts on pairs of synonymous genes showing strong differences in expression levels in the studies described above. The assays will employ the biochemical methods described above as well as the fluorescence methods developed under example 25.

In parallel, global changes in the influence of synonymous codons on mRNA levels in these *E*. *coli* knockout strains using RNAseq transcriptomic profiling will be probed (Sharma CM, Hoffmann S, Darfeuille F, Reignier J, Findeiss S, Sittka A, Chabas S, Reiche K, Hackermuller J, Reinhardt R, Stadler PF, Vogel J. Nature. 2010;464(7286):250-5). Refined versions of the generalized linear multiparameter logistic regression modeling methods described above **(****Figure 38****)** will be applied to evaluate whether there are changes in the correlation between specific codons and global mRNA levels in *E*. *coli.* Statistically significant changes in the influence of individual codons will be evaluated in follow-up experiments in which the standard biochemical and fluorescence assays are applied to synonymous gene pairs differing in the content of one those codons. Transcriptomic data will be collected and analyzed (Conway T, Creecy JP, Maddox SM, Grissom JE, Conkle TL, Shadid TM, Teramoto J, San Miguel P, Shimada T, Ishihama A, Mori H, Wanner BL. mBio. 2014;5(4):e01442-14).

### Example 28: Elucidate the biochemical systems controlling synonymous codon effects by quantifying the influence of all non-essential genes in E. coli on the relative expression level of proteins encoded by genes with defined differences in synonymous codon usage

Genomics tools (Baba T, Ara T, Hasegawa M, Takai Y, Okumura Y, Baba M, Datsenko KA, Tomita M, Wanner BL, Mori H. Molecular systems biology. 2006;2:2006 0008. doi: 10.1038/msb4100050; Mori H, Baba T, Yokoyama K, Takeuchi R, Nomura W, Makishi K, Otsuka Y, Dose H, Wanner BL Methods in molecular biology. 2015;1279:45-65; Otsuka Y, Muto A, Takeuchi R, Okada C, Ishikawa M, Nakamura K, Yamamoto N, Dose H, Nakahigashi K, Tanishima S, et al. Nucleic Acids Res. 2015;43(Database issue):D606-17. Epub 2014/11/17. doi: 10.1093/nar/gku1164; Takeuchi R, Tamura T, Nakayashiki T, Tanaka Y, Muto A, Wanner BL, Mori H BMC microbiology. 2014;14:171) will be used in conjunction with the fluorescent-reporter protein systems developed under example 25 to globally quantify the influence of all non-essential *E*. *coli* genes on selected synonymous codon effects. These studies will employ a molecularly "barcoded" single-gene knockout collection (Otsuka Y, Muto A, Takeuchi R, Okada C, Ishikawa M, Nakamura K, Yamamoto N, Dose H, Nakahigashi K, Tanishima S, et al. Nucleic Acids Res. 2015;43(Database issue):D606-17. Epub 2014/11/17. doi: 10.1093/nar/gku1164; Yong HT, Yamamoto N, Takeuchi R, Hsieh YJ, Conrad TM, Datsenko KA, Nakayashiki T, Wanner BL, Mori H. Genes & genetic systems. 2013;88(4):233-40) in which each mutant strain harbors a unique PCR-amplifiable nucleotide sequence tag. A fluorescent protein construct or constructs reporting on a specific synonymous codon variation will be introduced into a mixed population of cells containing every strain in this comprehensive knock-out collection (Baba T, Ara T, Hasegawa M, Takai Y, Okumura Y, Baba M, Datsenko KA, Tomita M, Wanner BL, Mori H. Molecular systems biology. 2006;2:2006 0008. doi: 10.1038/msb4100050). Several methods will be evaluated to introduce the reporter construct(s) into these mixed populations including transformation of the high or low copy-number plasmids described in example 25, as well as single-copy integration of a CRIM plasmid (Haldimann A, Wanner BL. Journal of bacteriology. 2001;183(21):6384-93) bearing the reporter(s) into the *E*. *coli* chromosome. Following induction of expression of the proteins with specific variations in synonymous codon usage, a fluorescence-activated cell sorter (FACS) will be used to measure single-channel or dual-channel fluorescence emission intensity from single *E*. *coli* cells in the mixed population (Francisco JA, Campbell R, Iverson BL, Georgiou G. Proceedings of the National Academy of Sciences. 1993; 90(22):10444-8; Mazor Y, Van Blarcom T, Mabry R, Iverson BL, Georgiou G. Nature biotechnology. 2007;25(5):563-5; Yoo TH, Pogson M, Iverson BL, Georgiou G. ChemBioChem. 2012;13(5):649-53). The cells showing the largest changes in the influence of the synonymous codon variations will be isolated and grown out for sequencing of their genetic barcodes, which will identify the single gene knocked out in each strain. The barcoding technology is so efficient that it is straightforward to use this approach to characterize hundreds of strains producing defined alteration in the influence of synonymous codons on protein expression as quantified via FACS analysis. Strains identified this way will be validated and characterized in depth using the established biochemical and molecular biological assays and the methods described in examples 25-27.

### Example 29: Large-scale protein expression methods and dataset

The methods for the large-scale protein expression experiments have been previously described (Acton, T. B. et al. (2005) Methods Enzymol 394, 210-243; Xiao, R. et al. (2010) J Struct Biol 172, 21-33; Acton, T. B. et al. (2011) Methods Enzymol 493, 21-60,) and are similar to those described below for protein expression *in vivo* except that induction was performed in 0.5 ml cultures in 96 well plates. The dataset analyzed herein was culled from that described in a previous report analyzing correlations between amino acid sequence and protein expression/solubility levels (Price, W. N. et al. (2011) Microbial Informatics and Experimentation 1, 6). The new dataset was restricted to non-redundant proteins expressed with a C-terminal LEHHHHHH tag that are encoded by genes that do not contain any codons affected by an alternative translation table in the source organism. Homologous sequences were culled by an iterative procedure that reduced the level of amino acid sequence identity between any pair to less than 60%, which results in a substantially lower level of nucleic acid sequence identity. At each step, all pairs of proteins sharing at least 60% identical amino acid sequence identity were transitively grouped together into a set, and the shortest sequence was eliminated from each set before reinitiating the same set-assignment procedure on all remaining proteins.

### Example 30: Computational Modeling

The binary multi-parameter logistic regression model gives *θ*, the logarithm of the ratio of the probabilities of obtaining the highest level of protein expression (*p_{E5}*) *vs*. none (p*_{E0}*) from an mRNA sequence in the large-scale dataset, as a linear function of generalized variables *xᵢ*: $θ = Ln \left[{p}_{E 5}/{p}_{E 0}\right] = A + {\sum }_{i} {β}_{i} {x}_{i}$ The probability of obtaining the highest level (E = 5) *vs*. no (E = 0) protein expression from a given sequence is therefore given by: $π \left(θ\right) = \frac{{p}_{E 5}}{{p}_{E 0} + {p}_{E 5}} = \frac{exp \left\{θ\right\}}{1 + exp \left\{θ\right\}}$ To capture non-linear relationships between mRNA sequence parameters and outcome, the generalized variables *xᵢ* can represent mathematical functions of mRNA sequence parameters as well as those parameters themselves. The *R* statistics program (Team, R. C. R: A language and environment for statistical computing. (2012)) was used to compute the most probable values of the model parameters (*A*,*βᵢ*). Logistic-regression slopes *βᵢ* > 0 indicate that the probability of high expression increases as the associated variable increases in numerical value. Because ΔG increases in numerical value as folding stability decreases, a positive slope for free-energy terms indicates an increase in the probability of high expression as predicted folding stability decreases, while a negative slope for these terms indicates an increase in the probability of high expression as predicted folding stability increases. The final model, which is called M **(****Figure 34A** and **Figure 29****),** is given in the main text, and the codon slopes *β_{c}* from this model are depicted in **Figure 11B****.** In principle, the probability of high protein expression can be increased by manipulating mRNA sequence properties to maximize the value of *θ* and thus *π* in the equations above using the parameters (*A*,*βᵢ*) from model M.

Inclusion of parameters in this model was guided by the Likelihood Ratio test and the Akaike Information Criterion (Akaike, H. (1974) Automatic Control, IEEE Transactions on 19, 716-723) (AIC), a standard measure of whether an improvement in model quality exceeds that expected at random from increasing the number of degrees of freedom (*d*.*f*.). The Likelihood Ratio χ² (LR χ²) is asymptotic to the χ² distribution and is defined as the reduction in the deviance *D* of the observed data from the predictions of the model compared to the null model containing just the constant term *A* (as defined above). The deviance is defined as: $D = - 2 {\sum }_{j = 1}^{n} \left[{E}_{j}ln\left({π}_{j}\right)+\left(1-{E}_{j}\right)ln\left(1-{π}_{j}\right)\right]$ This sum is conducted over the *n* = 3,727 proteins giving expression scores of 0 or 5 among the 6,348 in the large-scale protein expression dataset, and the variable *Eⱼ* assumes values of 0 or 1 if protein *j* is expressed at the E = 0 or E = 5 levels, respectively. The variable *πⱼ* = *π*(*θⱼ*) gives the predicted probability of obtaining expression of protein *j* at the E = 5 rather than E = 0 level according to the equations given above describing the multi-parameter binary logistic model. For the dataset analyzed herein, the deviance has values of 5,154 and 3,952 for the null model and the final model M, respectively **(****Figure** 34A). Bootstrap validation was also performed using the 'rms' package in *R* to ensure that the final model is not over-fit.

The sequence parameters explored in the course of model development **(****Figure 34****)** included the length of the gene, the individual codon frequencies in-frame or out-of-frame in the entire gene, the individual codon frequencies in-frame separately in the head and the tail, di-codon frequencies, the statistical entropy of the codon sequence, the codon-repetition rate (defined below), the frequencies of the nucleotide bases at each codon position in the entire gene and in defined windows within its sequence, and a variety of predicted mRNA folding-energy parameters including those shown in **Figures 9** **&** **16****,** which were evaluated individually and as statistical aggregates. The codon repetition rate is defined as $r = < {d}_{i}^{- 1} > ,$ where *dᵢ* is the distance from any codon to the next occurrence of the same codon moving towards the 3' end of the gene. The value of ${d}_{i}^{- 1}$ is set to zero if the codon does not occur again, so the value of *r* for the sequence AAA.CGT.CCG.CGT.AAA is the average of (1/4, 1/2, 0, 0, 0)=3/20. The number of degrees of freedom for codon variables is one fewer than the number of non-stop codons because their frequencies *f_{c}* in a sequence must sum to 1 (*i.e.,* ∑*f_{c}* = 1). Therefore, for the analyses shown in **Figures 11** **and** **29****,** ATG was removed, effectively forcing its slope *β_{ATG}* = 0 and its contribution to the model to be absorbed into the constant *A*

The inclusion of mean codon-slope variables s₇₋₁₆ and s₁₇₋₃₂ in model M uniformly reduces the individual codon slopes *β_{c}* to ∼86% of their values when no mean-slope terms are included in the model, reflecting the disproportionate influence of codons near the 5' terminus compared to those in the rest of the gene (**Figure 32**). More complex models were tested that include variables such as the frequencies of individual codons plus either the next base or the previous base, but these were ruled out based on bootstrap validation criteria. Introducing additional variables into model M (**Figure 34B**) was also examined. Adding the mean slope of codons 2-6 does not produce a statistically significant improvement, and using this term instead of the base-composition terms in this region yields inferior results, consistent with the analyses shown in **Figure 32**. Adding the frequency of the Shine-Dalgarno consensus AGGA in any frame (*f_{AGGA}* in **Figures 16G-H**) also fails to produce a statistically significant improvement. Similarly, adding terms for the mean value of the predicted free energy of mRNA folding in the tail does not significantly improve the model, even though unstable folding in the tail correlates with reduced protein expression (**Figures 9G-H**). This correlation as well as those of the overall A, T, G, and C content in the gene (**Figures 16A-E**) must be captured more effectively by the cross-correlated sequence parameters (**Figures 17-18**) that are included in the model, suggesting that these other parameters are more influential mechanistically

### Example 31: Design of synonymous mRNA sequences

In the 6AA method, codons for six amino acids were changed to the single codon specified in **Figure 35**, which has a larger slope than that of any synonymous codon in the single-parameter binary logistic regression analyses (dark gray symbols in **Figure 11B**). Although no explicit free energy optimization was performed with the 6AA method, it produced genes in which the predicted free energies of mRNA folding were more favorable than those in the naturally occurring starting sequences. In the 31C-FO method, predicted mRNA folding energy was optimized while selecting codons from the 31 listed in **Figure 35**, which have slopes greater than zero in the single-parameter binary logistic regression analyses (dark gray symbols in **Figure 11B**). The predicted free energy of folding of the head plus 5' UTR (Δ*G_{UH}*) was maximized numerically (*i.e.,* to yield the least stable folding), while the predicted free energy of the folding in the tail was optimized to be near -10 kcal/mol in windows of 48 nucleotides. The 31C-FD used the same set of codons to produce genes in which the predicted free energy of folding was minimized numerically (*i.e.,* to yield the most stable folding).

### Example 32: Bacterial strains and growth media

The *E*. *coli* strain DH5α was used for cloning. Expression experiments used *E*. *coli* strain BL21(DE3) pMGK (Acton, T. B. et al. (2005) Methods Enzymol 394, 210-243). Bacteria were cultivated in LB medium (Affymetrix/USB). Ampicillin was added at 100 µg/ml for cultures harboring pET21-based plasmids. Kanamycin was added at 25 µg/ml to maintain the pMGK plasmid. Bacterial growth for protein expression and Northern blot experiments were done in the same media and conditions that were used to generate the high-through protein-expression dataset (Acton, T. B. et al. (2005) Methods Enzymol 394, 210-243) (*i.e.,* MJ9 minimum medium (Jansson, M. et al. (1996) J Biomol NMR 7, 131-141) with 250 rpm agitation at 37 °C prior to induction at 17 °C).

### Example 33: Plasmids

The pET-21 clones of the genes APE_0230.1 (*Aeropyrum pernix* K1), RSP_2139 from (*Rhodobacter sphaeroides*), SRU_1983 (*Salinibacter ruber*), SCO1897 (*Streptomyces coelicolor)* and *ycaQ* (*E. coli*) were obtained from the protein-production laboratory of the Northeast Structural Genomics Consortium (www.nesg.org) at Rutgers University (NESG targets Xr92, RhR13, SrR141, RR162, and ER449, respectively). The 6AA_{T} and 31 C-FO_{H}/31C-FO_{T} variant of the genes were DNA synthetized by GenScript. The head variants 31C-FO_{H} and 31C-FO_{H} were generated by PCR amplification using long forward primers comprising an NcoI restriction site, the new head sequence, and a sequence complementary to the downstream region in the target gene. A plasmid containing the starting construct was used as DNA template for the PCR with the corresponding long forward primers and a reverse primer hybridizing at the 3' end of the construct including the XhoI restriction site. The resulting PCR products were cloned using the In-Fusion kit (Clontech) into a pET-21 derivative linearized with NcoI and XhoI. The full protein-coding sequence in every plasmid was verified by DNA sequencing (Genewiz and Eton Bioscience) and corrected when necessary using the QuikChange II Site-Directed Mutagenesis kit (Agilent Technologies). DNA sequences of the final constructs are provided in the Supplementary Information file BoelEtA12014SequenceData.csv.

### Example 34: E. coli growth curves

Overnight cell growth was measured by transferring 200 µl of each induced culture to a 96-well sterile plate (Greiner bio-one) and covered with 50 µl of sterile paraffin oil. A negative control non-induced sample was loaded for each target WT. Duplicates of each sample were loaded to allot for any natural or human variation. Plates were placed into a plate reader (Biotek Synergy) at room temperature, and shaken for 30 seconds. A start OD₆₀₀ reading was taken and then followed by 30 minutes of shaking until the next OD reading. Readings were repeated for a total of 9 hours of growth analysis.

### Example 35: Analysis of protein expression in vivo

Starting cultures from a single colony were inoculated into 6 ml of LB media containing 100 µg/ml of Ampicillin and 30 µg/ml Kanamycin. Cultures were grown at 37°C until highly turbid (4-6 hours). 40 µl of the turbid media was used to inoculate 2 ml of MJ9 minimal medium (Jansson, M. et al. (1996) J Biomol NMR 7, 131-141). This MJ9 preculture was grown overnight at 37 °C. The following day, OD₆₀₀ readings were taken of a 1:10 dilution of the turbid MJ9 preculture. This reading was used to calculate the volume of preculture necessary to normalize all cell samples to a starting culture reading of 0.1 in 6 ml of media. This calculated volume was inoculated into 6 ml of fresh MJ9 media and cells were grown at 37 °C until OD₆₀₀ reached 0.5-0.7. Cells were then induced with 1mM IPTG, with one duplicate tube for each target WT left non-induced to act as a negative control. After induction, 200 µl x 2 of each culture was removed and placed into a sterile 96 well plate for growth curve monitoring (see above). The remaining 5.6 ml of induced samples were then transferred to 17 °C and shaken overnight. The following day, sample tubes were removed from the shaker and placed on ice. Final OD₆₀₀ measurements were taken. Cells were centrifuged in 14 ml round bottom Falcon tubes at 4K rpm for 10 minutes and the supernatant discarded. Cells were resuspended in 1.2 ml of Lysis Buffer (50 mM NaH₂PO₄ pH 8.0, 30 mM NaCl, 10 mM 2-mercaptoethanol) and then transferred to 1.5 ml Eppendorf tubes on ice. Lysis was accomplished by sonication on ice, using a 40 V setting (∼12 Watt pulse) and pulsing 1 sec followed by a 2 sec rest, for a total of 40 pulses. 120 µl of each lysed sample was mixed with 40 µl of 4X Laemmli Buffer. Samples were then run on SDS-PAGE (Bio-Rad, Ready Gel, 15% Tris-HCl), with Bio-Rad Precision Plus All Blue Standard markers. Final OD₆₀₀ measurements were used to calculate the load volume for each individual sample, normalizing all samples to the density of the least turbid of each unique target. Te integrity of the plasmids were verified after growth and induction by DNA sequencing (Genewiz and Eton Bioscience).

### Example 36: In vitro transcription and translation

pET21 plasmids containing the optimized or unoptimized insert were digested with BlpI, phenol-chloroform purified and concentrated by ethanol precipitation. Of the digested samples, 2 µg were added to the RiboMax kit (Promega) preparation, and *in vitro* transcribed as per protocol. Upon reaction completion, *in vitro* transcription samples were treated with DNAse (Promega) then isopropanol precipitated and resuspended in The RNA Storage Solution (Ambion). Transcript size and purity were verified by agarose gel electrophoresis with ethidium bromide staining. For the time point kinetic 20 µl T7 reactions were assembled and started with 1 µg of DNA template. At time 0, 5, 10 and 30 minutes 4.5 µl of each reaction were run on denaturing formaldehyde-agarose gel.

*In vitro* translation assays of the purified mRNAs were performed with the PURExpress system (New England Biolabs) using L-[³⁵S]methionine premium (PerkinElmer). Each 25 µl reaction contained 10 µl of solution A, 7.5 µl of solution B and 2 µl of [³⁵S]methionine (10 µCi). The reactions were started by adding 2 µl of purified mRNA (4 µg/µl) and incubating at 37 °C. Aliquot of 5 µl were withheld from the reaction at 15, 30, 60 and 90 min, stopped by adding 10 µl of 2X Laemmli and heating for 2 min at 60 °C. Then 14 µl of each aliquot were run on a 4-20 % SDS-PAGE (Bio-Rad) with Bio-Rad Precision Plus All Blue Standard markers. The gel was dried on Whatman as well as subjected to autoradiography.

### Example 37: Northern blot analyses

Northern blotting probe was designed as the reverse complement of the 71nt of the 5' UTR of the pET21 vector, and synthesized by Eurofins. The probe was labeled with biotin using the BrightStar Psoralen-Biotin Nonisotopic Labeling Kit. BL21(DE3) pMGK *E. coli* containing the plasmid of interest was grown overnight in LB at 37 °C with shaking. Cultures were diluted 1:50 into MJ9 media and grown overnight at 37 °C with shaking. Following day, the cultures were diluted to an OD₆₀₀ of 0.15 into MJ9 media and allowed to grow to an OD₆₀₀ of 0.6-0.7 prior to induction with 1 mM IPTG. Samples were taken at the indicated time points and RNAs were stabilized in 2 volumes of RNAProtect Bacteria Reagent. After pelleting, samples were lysozyme digested (15 mg/ml) for 15 minutes and RNAs were purified using the Direct-zol RNA Miniprep Kit and TRI-Reagent. Approximately 1-2 µg of total RNA per sample was separated on a 1.2% formaldehyde-agarose gel in MOPS-formaldehyde buffer. RNA integrity was verified by ethidium bromide staining. RNA was then transferred to a positively charged nylon membrane using downward capillary transfer with an alkaline transfer buffer (1 M NaCl, 10 mM NaOH, pH 9) for 2 h at room temperature. RNAs were crosslinked to the membrane using 1200 µJ UV (Stratalinker). Membranes were pre-hybridized in Ultrahyb hybridization buffer for 1h at 42°C in a hybridization oven. Heat-denatured, biotin-labeled probe was then added to 10-20 pM final concentration and hybridized overnight at 42°C. Membranes were washed twice in wash buffer (0.2X SSC, 0.5% SDS) and probe signal was detected using the BrightStar BioDetect kit, as per protocol, with exposure to film.

### Example 38: RNA extraction and microarray analyses

*E*. *coli* MG1655 cells were cultured in M9 0.4 % glucose minimum media to a final OD₆₀₀ of 1.0. Cells were treated with RNA Protect Bacteria Reagent (Qiagen), and RNA extracted using the RNeasy Mini Kit (Qiagen) was reverse transcribed using SuperScript II Reverse Transcriptase (Invitrogen) followed by treatment with RNaseH (Invitrogen) and RNaseA (EpiCentre). The resulting cDNA preparation was purified using the MinElute Purification Kit (Qiagen) and then fragmented into 50-200 bp fragments using DNaseI (EpiCentre). Biotinylation was performed with Terminal Deoxynucleotidyl Transferase (New England Biolabs) and Biotin-N⁶-ddATP (Enzo Life Sciences). Biotinylated cDNA was hybridized on Affymetrix *E*. *coli* 2.0 arrays by the Gene Expression Center at the University of Wisconsin Biotechnology Center. Raw data (.cel) files were analyzed using the RMA (Robust Multi-chip Average) algorithm in the Affymetrix Expression Console.

### Example 39: Classification of cytoplasmic proteins in E. coli MG1655

All predicted proteins in the version of the genome in the Ecocyc database (Keseler, I. M. et al. (2013) Nucleic Acids Research 41, D605-D612) were analyzed using the programs LipoP (Juncker, A. S. et al. (2003) Protein Sci 12, 1652-1662) and TMHMM (Krogh, A., Larsson, B., von Heijne, G. & Sonnhammer, E. L. (2001) J Mol Biol 305, 567-580), and those without a predicted transmembrane helix or a predicted signal peptide were classified as cytoplasmic proteins and included in the analyses in **Figure 30**.

### Example 40: Analysis of related datasets

The Plotkin dataset quantifying fluorescence levels observed *in vivo* in *E*. *coli* from expression of a set of recoded eGFP genes were reanalyzed. The sequence correlations in this dataset are generally consistent with the expectations based on the results described herein. To put the observed trends in perspective, it is important to note two factors regarding the experimental design used to generate the Plotkin dataset.

First, in order to avoid sequence features reputed to make mRNAs susceptible to cleavage by RNAseE, Plotkin and co-workers used a limited set of synonymous codon substitutions rather than systematically sampling codon space. The sequence features that they tried to avoid turn out not to have a significant influence in the *E*. *coli* mRNA decay dataset recently reported by Xie and co-workers and reanalyzed herein. The unnecessary restrictions they imposed on codon substitution prevented them from sampling many of the strongest synonymous codon substitution effects inferred from the dataset described herein, which provides a substantially broader and deeper sampling of codon space than theirs. Therefore, codon content is expected to have a substantially weaker influence on their dataset than on the dataset described herein.

A second factor about the experimental design underlying the Plotkin dataset is that it quantifies protein expression via fluorescence emission intensity from natively folded eGFP, even though this GFP variant is known to be aggregation-prone and to fold inefficiently under some conditions *in vivo* in *E*. *coli.* Subsequent papers from two different groups have reported isolation of mutations that improve folding of this variant and prevent a loss in fluorescence yield due to protein aggregation at elevated eGFP expression levels *in vivo* in *E*. *coli.* Plotkin and co-workers performed little validation of protein expression using other methods and did not provide any calibration establishing the range of eGFP expression levels over which fluorescence yield scales linearly with the amount of protein synthesized. Therefore, the later papers reporting isolation of stabilized variants of eGFP in *E*. *coli* raise the possibility that some expression-enhancing effects could be obscured in the Plotkin dataset by increased misfolding coupled to aggregation in some regimes of increasing eGFP expression.

Simultaneous multiparameter linear regression modeling of the Plotkin dataset was performed using similar methods to those used to model the large-scale protein-expression dataset. These analyses show that the predicted free energy of mRNA folding and base composition and in the head of the gene have significant influences on eGFP fluorescence level in the Plotkin dataset that parallel their influences in the protein-expression dataset. Plotkin and co-workers detected the former effect but not the latter effect, which is a novel finding of the work presented herein. While the base-composition effects inferred from the Plotkin dataset differ in some details from those inferred from the dataset described herein, which seems likely to derive from the specific sequence context in their eGFP expression construct, the broad trends match. It is observed that *s_{All}*, the mean value of the new codon-influence metric, has a weak but significant influence on eGFP fluorescence level in the Plotkin dataset, but this effect goes in the opposite direction from that observed in the protein-expression dataset. Given the inefficient in vivo folding properties of eGFP, the most likely explanation for the observed effect is that increased translation efficiency leads to a reduction in eGFP fluorescence yield due to increased misfolding coupled to aggregation in some genes included in the Plotkin dataset. Further investigation will be required to rigorously dissect this effect.

The dataset of Goodman *et al*. quantifying fluorescence levels observed *in vivo* in *E. coli* from expression of a single superfolder GFP (sfGFP) gene sequence fused to a 10-residue N-terminal extension of varying sequence *(i.e.,* comprising codons 2-11 of the expressed gene) was also reanalyzed. Notably, this GFP variant is one of the two mentioned above that were isolated to fold more efficiently *in vivo* in *E. coli* than the eGFP protein used by Plotkin and co-workers . Based on the analyses, the region of the gene varied in the Goodman dataset contains only five codons where synonymous substitution influences expression level *(i.e.,* codons 7-11), because base-composition effects dominate codon-usage effects for codons 2-6, so strong codon-usage effects are not expected. Simultaneous multiparameter linear regression modeling of the Goodman dataset was performed using similar methods to those used to model the large-scale protein-expression dataset. The results of these analyses are consistent with both the computational model the qualitative conclusions presented herein. The predicted free energy of mRNA folding and base composition and in the head of the gene have significant influences on sfGFP fluorescence level in the Goodman dataset that parallel their influences in the protein-expression dataset described herein. Like Plotkin and co-workers, Goodman *et al*. detected the former effect but not the latter effect. The base-composition effects inferred from the Goodman dataset differ in some details from those inferred from the dataset described herein, which seems likely to derive from the specific sequence context in their sfGFP expression construct, but the broad trends once again match. It is observed that *s_{All}* has a weak but significant influence on sfGFP fluorescence level in the same direction as that observed in the protein-expression dataset but the opposite direction from that observed in the Plotkin dataset. This difference likely reflects the more efficient *in vivo* folding of the sfGFP construct used by Goodman *et al*. compared to the eGFP construct used by Plotkin and co-workers.

Recently published experimental datasets quantifying *E. coli* mRNA decay rates were also reanalyzed. This paper, which was published by Xie and coworkers, used RNAseq for global quantification of mRNA decay rates following inhibition of transcription initiation by the antibiotic rifampicin during growth in either logarithmic phase or early stationary phase in LB medium. While these datasets provide the most comprehensive characterization to date of mRNA decay in *E. coli,* they cover a relatively small fraction of the genes in *E. coli* (<25%), and the set of genes that is covered is strongly biased towards abundant mRNAs with high steady-state concentrations, one of several factors making analysis of these datasets non-trivial. Initial analyses support several interpretations advanced in the results that are described herein:

There is a significant positive correlation between mRNA lifetime and steady-state level in both the exponential and stationary-phase datasets reported by Xie and co-workers. In other words, more abundant mRNAs have systematically slower decay rates than less abundant mRNAs. It is inferred that the existence of such a relationship to explain the systematically higher steady-state levels in *E. coli* of mRNAs with higher values of *s_{All}* or mean codon-influence score, which was hypothesized to reflect slower decay of mRNAs with better codon usage. The abundance-lifetime relationship demonstrated by the mRNA decay datasets from Xie and coworkers supports the logic underlying the interpretation of this effect.

Furthermore, two different computational analyses demonstrate that the mRNAs for which decay rates were measured are systematically depleted of codons that correlate with reduced protein expression. The distribution of *s_{All}* is significant higher for the genes in *E. coli* for which mRNA decay rates were measured than for those for which they were not measured. Second, codons with lower codon-influence score (*s*) inferred from the large-scale protein-expression dataset have systematically lower frequencies in the set of mRNAs for which decay rates were measured than in the entirety of the *E. coli* genome. These observations, combined with the observation of a significant positive correlation between mRNA lifetime and steady-state level, provide experimental support for the hypothesis that the correlation between *s_{All}* and genome-wide physiological steady-state mRNA concentration in *E. coli* reflects at least in part preferential degradation of mRNAs with sub-optimal codon usage. Therefore, a large-scale dataset produced by another group applying orthogonal methods under physiological conditions in *E. coli* supports the inference based on the experiments on mRNAs transcribed by T7 polymerase that are described herein.

Additional analyses show that the codon-influence score has a significant relationship of the kind predicted with the mRNA lifetimes measured by Xie and co-workers. First, the codon-influence score (*s*) for each codon inferred from the large-scale protein-expression dataset shows a significant positive correlation with the Spearman rank-order correlation coefficient between the frequency of that codon and the experimentally measured mRNA lifetimes *(i.e.,* more optimal codon usage according to the metric correlates with longer measured mRNA lifetime). Second, simultaneous multiparameter linear regression modeling shows that *s_{All}* is a significant predictor of measured mRNA lifetime even when considered simultaneously with other sequence parameters, including nucleotide base composition. Other noteworthy features of this analysis are that the base-preferences previously inferred to control susceptibility to RNAseE, which were believed to be major determinants of mRNA lifetime in *E. coli,* are not in fact correlated with lifetime. Similarly, the features that Plotkin and co-workers avoided in their codon-substitution scheme are not in fact correlated with lifetime, as mentioned above. Finally, the tRNA-adaptation index (tAI) has no significant relationship with the measured mRNA lifetimes, while the codon-adaptation index (CAI) has an influence that captures some but not all of the influence of *s_{All}*. Notably, the CAI, which reflects the sequence characteristics of the mRNAs encoding the most abundant proteins expressed under physiological conditions, does not have a significant influence on the large-scale protein-expression dataset when considered simultaneously with *s_{All}*. Therefore, this metric historically assumed to reflect translation efficiency may instead reflect primarily mRNA decay effects. Future research will be required to rigorously deconvolute and quantify the relative influence of mRNA sequence features on transcription *vs*. translation *vs*. mRNA decay in *E. coli.* However, numerous analyses of the mRNA decay dataset recently published by Xie and co-workers uniformly support the hypothesis that sub-optimal codon usage as measured by the new codon-influence metric correlates with more rapid mRNA decay in *E. coli.*

### References

Aalberts DP and Jannen WK (2013) RNAbows: an intuitive tool for visualizing RNA secondary structures. RNA 19, 475-478.
Acton TB et al. (2005) Robotic cloning and polypeptide production platform of the Northeast Structural Genomics Consortium. Methods in Enzymology 394:210-243.
Akaike H (1974) A new look at the statistical model identification. IEEE transactions on automatic control 19:716-723.
Appel RD, Bairoch A, Hochstrasser DF (1994) A new generation of information retrieval tools for biologists: the example of the ExPASy WWW server. Trends in Biochemical Sciences 19:258.
Bentele K, Saffert P, Rauscher R, Ignatova Z, Bluthgen N (2013) Efficient translation initiation dictates codon usage at gene start. Molecular systems biology 9, 675.
Bertone P et al. (2001) SPINE: an integrated tracking database and data mining approach for identifying feasible targets in high-throughput structural proteomics. Nucleic acids research 29:2884.
Biro, J.C. (2008) Correlation between nucleotide composition and folding energy of coding sequences with special attention to wobble bases. Theor Biol Med Model, 5:14.
Brant R (1990) Assessing proportionality in the proportional odds model for ordinal logistic regression. Biometrics 46:1171-1178.
Bulmer M (1991) The selection-mutation-drift theory of synonymous codon usage. Genetics 129, 897-907.
Campbell JW et al. (1972) X-ray diffraction studies on enzymes in the glycolytic pathway. Cold Spring Harb. Symp. Quant. Biol 36:165-170.
Cannarozzi G et al. (2010) A role for codon order in translation dynamics. Cell 141, 355-367.
Caskey CT, Beaudet A, Nirenberg M (1968) RNA codons and protein synthesis. 15. Dissimilar responses of mammalian and bacterial transfer RNA fractions to messenger RNA codons. J Mol Biol 37, 99-118.
Carstens CP (2003) Use of tRNA-supplemented host strains for expression of heterologous genes in E. coli. Methods in Molecular Biology 205:225-234.
Chen GT, Inouye M (1994) Role of the AGA/AGG codons, the rarest codons in global gene expression in Escherichia coli. Genes & development 8, 2641-2652.
Chen J, Acton TB, Basu SK, Montelione GT, Inouye M (2002) Enhancement of the solubility of polypeptides overexpressed in Escherichia coli by heat shock. Journal of molecular microbiology and biotechnology 4:519-524.
Chen L, Oughtred R, Berman HM, Westbrook J (2004) TargetDB: a target registration database for structural genomics projects (Oxford Univ Press).
Christen EH et al. (2009) A general strategy for the production of difficult-to-express inducer-dependent bacterial repressor polypeptides in Escherichia coli. Polypeptide Expression and Purification.
Creamer TP (2000) Side-chain conformational entropy in polypeptide unfolded states. Polypeptides: Structure, Function, and Genetics 40.
Crombie T, Swaffield JC, Brown AJ (1992) Polypeptide folding within the cell is influenced by controlled rates of polypeptide elongation. J. Mol. Biol 228:7-12.
Dale GE, Broger C, Langen H, Arcy AD, Stüber D (1994) Improving polypeptide solubility through rationally designed amino acid replacements: solubilization of the trimethoprim-resistant type S1 dihydrofolate reductase. Polypeptide Engineering Design and Selection 7:933-939.
Davis GD, Elisee C, Newham DM, Harrison RG (1999) New fusion polypeptide systems designed to give soluble expression in Escherichia coli. Biotechnology and bioengineering 65.
De Bernardez Clark E (1998) Refolding of recombinant polypeptides. Current Opinion in Biotechnology 9:157-163.
Derewenda ZS (2004) Rational polypeptide crystallization by mutational surface engineering. Structure 12:529-535.
Elf J, Nilsson D, Tenson T, Ehrenberg M (2003) Selective charging of tRNA isoacceptors explains patterns of codon usage. Science 300, 1718-1722.
Etchegaray JP, Inouye M (1999) Translational enhancement by an element downstream of the initiation codon in Escherichia coli. Journal of Biological Chemistry 274:10079-10085.
Freischmidt A, Liss M, Wagner R, Kalbitzer HR, Horn G (2012) RNA secondary structure and in vitro translation efficiency. Protein Expression Purif., 82, 26-31.
Georgiou G, Valax P (1996) Expression of correctly folded polypeptides in Escherichia coli. Current Opinion in Biotechnology 7:190-197.
Goh CS et al. (2003) SPINE 2: a system for collaborative structural proteomics within a federated database framework. Nucleic acids research 31:2833.
Goh CS et al. (2004) Mining the structural genomics pipeline: identification of polypeptide properties that affect high-throughput experimental analysis. Journal of molecular biology 336:115-130.
Goodman DB, Church GM, Kosuri S (2013) Causes and Effects of N-Terminal Codon Bias in Bacterial Genes. Science, doi:10.1126/science.1241934.
Gottesman S (1990) Minimizing proteolysis in Escherichia coli: genetic solutions. Methods in enzymology 185:119.
Gustafsson C, Govindarajan S, Minshull J (2004) Codon bias and heterologous polypeptide expression. Trends in biotechnology 22:346-353.
Gustafsson C, Minshull J, Govindarajan S, Ness J, Villalobos A and Welch M (2012) Engineering genes for predictable protein expression. Protein Expression Purif., 83, 37-46.
Hatfield GW, Roth DA (2007) Optimizing scaleup yield for polypeptide production: Computationally Optimized DNA Assembly (CODA) and Translation Engineering. Biotechnol Annu Rev 13:27-42.
Hodas NO and Aalberts DP. (2004) Efficient computation of optimal oligo-RNA binding. Nucleic Acids Res., 32, 6636-6642.
Hofacker IL (2003) Vienna RNA secondary structure server. Nucleic Acids Res., 31, 3429-3431.
Hosmer DW, Lemeshow S (2004) Applied logistic regression (Wiley-Interscience).
Hunt RC, Simhadri VL, Iandoli M, Sauna ZE, Kimchi-Sarfaty C (2014) Exposing synonymous mutations. Trends in genetics : TIG, doi:10.1016/j.tig.2014.04.006.
Idicula-Thomas S, Balaji PV (2005) Understanding the relationship between the primary structure of polypeptides and its propensity to be soluble on overexpression in Escherichia coli. Polypeptide Science: A Publication of the Polypeptide Society 14:582.
Idicula-Thomas S, Kulkarni AJ, Kulkarni BD, Jayaraman VK, Balaji PV (2006) A support vector machine-based method for predicting the propensity of a polypeptide to be soluble or to form inclusion body on overexpression in Escherichia coli. Bioinformatics 22:278-284.
Kapust RB, Waugh DS (1999) Escherichia coli maltose-binding polypeptide is uncommonly effective at promoting the solubility of polypeptides to which it is fused. PRS 8:1668-1674.
Kefala G, Kwiatkowski W, Esquivies L, Maslennikov I, Choe S (2007) Application of Mistic to improving the expression and membrane integration of histidine kinase receptors from Escherichia coli. Journal of Structural and Functional Genomics 8:167-172.
Kim CH, Oh Y, Lee TH (1997) Codon optimization for high-level expression of human erythropoietin (EPO) in mammalian cells. Gene 199:293-301.
Komar AA (2009) A pause for thought along the co-translational folding pathway. Trends Biochem. Sci 34:16-24.
Kozak M (2005) Regulation of translation via mRNA structure in prokaryotes and eukaryotes. Gene 361, 13-37.
Krogh A, Larsson B, Von Heijne G, Sonnhammer ELL (2001) Predicting transmembrane polypeptide topology with a hidden Markov model: application to complete genomes. J Mol Biol 305:567-580.
Krüger MK, Pedersen S, Hagervall TG, Sorensen MA (1998) The modification of the wobble base of tRNAGlu modulates the translation rate of glutamic acid codons in vivo. Journal of molecular biology 284:621-631.
Kudla G, Murray AW, Tollervey D, Plotkin JB (2009) Coding-sequence determinants of gene expression in Escherichia coli. science 324:255.
Kyte J, Doolittle RF (1982) A simple method for displaying the hydropathic character of a polypeptide. Journal of Molecular Biology 157:105.
Lee C et al. (2008) An improved SUMO fusion polypeptide system for effective production of native polypeptides. Polypeptide Sci. 17:1241-1248.
Lewis HA et al. (2005) Impact of the {Delta} F 508 mutation in first nucleotide-binding domain of human cystic fibrosis transmembrane conductance regulator on domain folding and structure. Journal of Biological Chemistry 280:1346-1353.
Li GW, Oh E, Weissman JS (2012) The anti-Shine-Dalgarno sequence drives translational pausing and codon choice in bacteria. Nature 484, 538-541.
Liu G et al. (2005) NMR data collection and analysis protocol for high-throughput polypeptide structure determination. Proceedings of the National Academy of Sciences of the United States of America 102:10487.
Luft JR et al. (2003) A deliberate approach to screening for initial crystallization conditions of biological macromolecules. Journal of Structural Biology 142:170-179.
Magnan CN, Randall A, Baldi P (2009) SOLpro: accurate sequence-based prediction of polypeptide solubility. Bioinformatics.
Makrides SC (1996) Strategies for achieving high-level expression of genes in Escherichia coli. Microbiology and Molecular Biology Reviews 60:512.
Mathews DH, Disney MD, Childs JL, Schroeder SJ, Zuker M and Turner DH (2004) Incorporating chemical modification constraints into a dynamic programming algorithm for prediction of RNA secondary structure. Proc. Natl. Acad. Sci. USA, 101, 7287-7292.
Muramatsu T et al. (1988) Codon and amino-acid specificities of a transfer RNA are both converted by a single post-transcriptional modification. Nature 336, 179-181.
Nakamura Y, Gojobori T, Ikemura T (2000) Codon usage tabulated from international DNA sequence databases: status for the year 2000. Nucleic Acids Res 28:292.
Pédelacq JD et al. (2002) Engineering soluble polypeptides for structural genomics. Nature biotechnology 20:927-932.
Pedersen S (1984) Escherichia coli ribosomes translate in vivo with variable rate. The EMBO Journal 3:2895.
Plotkin JB, Kudla G (2011) Synonymous but not the same: the causes and consequences of codon bias. Nature reviews. Genetics 12, 32-42.
Price WN et al. (2009) Understanding the physical properties that control polypeptide crystallization by analysis of large-scale experimental data. Nat. Biotechnol 27:51-57.
Rice P, Longden I, Bleasby A (2000) EMBOSS: the European molecular biology open software suite. Trends in genetics 16:276-277.
Rost B (2005) How to use polypeptide 1D structure predicted by PROFphd. The proteomics protocols handbook. Totowa (New Jersey): Humana:875-901.
Rost B, Yachdav G, Liu J (2004) The predictpolypeptide server. Nucleic Acids Research 32:W321.
Sanbonmatsu KY, Joseph S, Tung C (2005) Simulating movement of tRNA into the ribosome during decoding. Proceedings of the National Academy of Sciences of the United States of America 102:15854-15859.
Schauder B and McCarthy JEG (1989) The role of bases upstream of the Shine-Dalgarno region and in the coding sequence in the control of gene-expression in Escherichia coli:translation and stability of messenger-RNAs in vivo. Gene, 78, 59-72.
Shakin-Eshleman SH, Liebhaber SA (1988) Influence of duplexes 3' to the mRNA initiation codon on the efficiency of monosome formation. Biochemistry 27, 3975-3982.
Slabinski, L., L. Jaroszewski, et al. (2007). "The challenge of polypeptide structure determination--lessons from structural genomics." Polypeptide Sci 16(11): 2472-82.
Smialowski P et al. (2007) Polypeptide solubility: sequence based prediction and experimental verification. Bioinformatics 23:2536.
Sorensen HP, Mortensen KK (2005) Advanced genetic strategies for recombinant polypeptide expression in Escherichia coli. Journal of biotechnology 115:113-128.
Spencer PS, Siller E, Anderson JF, Barral JM (2012) Silent substitutions predictably alter translation elongation rates and protein folding efficiencies. J Mol Biol 422, 328-335.
Steinthorsdottir V et al. (2007) A variant in CDKAL1 influences insulin response and risk of type 2 diabetes. Nature genetics 39, 770-775.
Tanha J et al. (2006) Improving solubility and refolding efficiency of human V(H)s by a novel mutational approach. Polypeptide Eng. Des. Sel 19:503-509.
Tartaglia GG, Pechmann S, Dobson CM, Vendruscolo M (2009) A Relationship between mRNA Expression Levels and Polypeptide Solubility in E. coli. Journal of Molecular Biology.
Tresaugues L et al. (2004) Refolding strategies from inclusion bodies in a structural genomics project. Journal of Structural and Functional Genomics 5:195-204.
Trevino SR, Scholtz JM, Pace CN (2007) Amino acid contribution to polypeptide solubility: Asp, Glu, and Ser contribute more favorably than the other hydrophilic amino acids in RNase Sa. J. Mol. Biol 366:449-460.
Vivanco-Dominguez S et al. (2012) Protein synthesis factors (RF1, RF2, RF3, RRF, and tmRNA) and peptidyl-tRNA hydrolase rescue stalled ribosomes at sense codons. J Mol Biol 417, 425-439.
Wagner S et al. (2008) Tuning Escherichia coli for membrane polypeptide overexpression. Proc. Natl. Acad. Sci. U.S.A 105:14371-14376.
Waldo GS (2003) Genetic screens and directed evolution for polypeptide solubility. Current opinion in chemical biology 7:33-38.
Wang and Dunbrack, Jr. (2003). "PISCES: a polypeptide sequence culling server." Bioinformatics 19:1589-1591.
Ward JJ, McGuffin LJ, Bryson K, Buxton BF, Jones DT (2004) The DISOPRED server for the prediction of polypeptide disorder (Oxford Univ Press).
Watts JM, Dang KK, Gorelick RJ, Leonard CW, Bess JW, Jr., Swanstrom R, Burch CL, Weeks, KM (2009) Architecture and secondary structure of an entire HIV-1 RNA genome. Nature, 460, 711-719.
Wigley WC, Stidham RD, Smith NM, Hunt JF, Thomas PJ (2001) Polypeptide solubility and folding monitored in vivo by structural complementation of a genetic marker polypeptide. Nat. Biotechnol 19:131-136.
Wilkinson DL, Harrison RG (1991) Predicting the solubility of recombinant polypeptides in Escherichia coli. Nature Biotechnology 9:443-448.
Wu X, Jörnvall H, Berndt KD, Oppermann U (2004) Codon optimization reveals critical factors for high level expression of two rare codon genes in Escherichia coli: RNA stability and secondary structure but not tRNA abundance. Biochemical and Biophysical Research Communications 313:89-96.
Yadava A, Ockenhouse CF (2003) Effect of Codon Optimization on Expression Levels of a Functionally Folded Malaria Vaccine Candidate in Prokaryotic and Eukaryotic Expression Systems Editor: WA Petri, Jr. Infection and immunity 71:4961-4969.
Zuker, M. (2003) Mfold web server for nucleic acid folding and hybridization prediction. Nucleic Acids Res., 31, 3406-3415.

## Claims

1. A computer implemented method to increase the expression of a recombinant polypeptide in an in vitro or in vivo expression system, comprising making one or more synonymous substitutions in the protein-coding nucleic acid sequence by selecting a good codon for every amino acid according to a generalized linear multiparameter modeling of the influence of a comprehensive set of RNA sequence parameters on measurable experimental values correlated with protein expression level, wherein the comprehensive set of RNA sequence parameters includes (i) in-frame single codon frequencies, (ii) in-frame ATA-ATA dicodon frequency, (iii) linear, parabolic, and inverse-linear functions of nucleotide base composition codons 2-6, (iv) nucleotide base composition in the remainder of the protein-coding sequence, (v) the partition-function free-energy of RNA folding calculated by the program RNAstructure with default parameters for the first 48 nucleotides in the protein-coding sequence plus the 5'-UTR sequence if that sequence is consistent in the modeled dataset, (vi) the average value of the partition-function free-energy of RNA folding calculated by the program RNAstructure with default parameters for 50% overlapping windows of 96 nucleotides after nucleotide 48 in the protein-coding sequence, (vii) the length in nucleotides of the protein-coding sequence, (viii) the amino acid repetition rate, and (ix) the codon repetition rate.

2. The method of claim 1 in which the generalized linear multiparameter model is a linear regression model.

3. The method of claim 1 in which the generalized linear multiparameter model is a logistic regression model based on observations with the highest vs. lowest of the measurable experimental values correlated with protein expression level, with the highest and lowest sets typically each comprising one-third of the observations in the dataset.

4. The method of claim 1 in which the table of allowed codons includes the codon for each amino acid with the most positive effect on the measurable experimental values correlated with protein expression level, as quantified by the slope of that codon in the generalized linear multiparameter model, plus all synonymous codons within the range of the uncertainty in the slope of the codon with the most positive effect.

5. The method of claim 1 in which the table of allowed codons includes the codon for each amino acid with the most positive effect on the measurable experimental values correlated with protein expression level, as quantified by the slope of that codon in the generalized linear multiparameter model, plus all synonymous codons within twice the range of the uncertainty in the slope of the codon with the most positive effect.

6. The method of claim 1 in which the parameters positively correlated with protein expression level that are used for generalized linear multiparameter modeling are experimentally measured protein-expression levels under expression conditions in a host strain.

7. The method of claim 6 in which the experimentally measured protein-expression levels are obtained from a dataset acquired using bacteriophage T7 RNA polymerase to express 6,348 proteins with maximally 30% pairwise amino acid identity in E. coli BL21(DE3) cells growing in chemically defined liquid medium with glucose as a carbon source.

8. The method of claim 1 in which the parameters positively correlated with protein expression level that are used for generalized linear multiparameter modeling are global steady-state mRNA levels measured under expression conditions in a host strain.

9. The method of claim 1 in which the parameters positively correlated with protein expression level that are used for generalized linear multiparameter modeling are mRNA lifetimes measured under expression conditions in a host strain [0421, 0419].

10. A computer implemented method to increase the expression of a recombinant polypeptide in an in vitro or in vivo expression system that comprises providing a nucleic acid sequence with a protein-coding sequence functionally linked to a 5'-untranslated region (5'-UTR) containing a ribosome-binding site, making one or more synonymous substitutions in codons 2, 3, 5, 4, and 6 of the protein-coding sequence that lower guanine content or raise adenine content, and making synonymous substitutions in the resulting coding sequence that produce a partition-function free-energy of RNA folding calculated by the program RNAstructure with default parameters that is as close as achievable to being greater than -10 kcal/mol for the first 48 nucleotides in the protein-coding sequence and, if using a pET vector 5'-UTR, that is -30.0 kcal/mol or higher for the first 48 nucleotides in the coding sequence plus the 5'-UTR.

11. A computer implemented method to increase the expression of a recombinant polypeptide in an in vitro or in vivo expression system by making one or more synonymous substitutions so that the partition-function free-energy of RNA folding as calculated by the program RNAstructure with default parameters is as close as achievable to being in the range from 10 kcal/mol less than to 5 kcal/mol greater than (-0.32^{∗}(W-18)) kcal/mol where W is the number of nucleotides in the tail sequence window downstream of nucleotide 48 in the protein-coding sequence, with W typically being 96 nucleotides.

12. The method of claims 10 or 11 further comprising optimization of the protein-coding sequence by making one or more synonymous substitutions that replace every in-frame ATA codon for isoleucine with either ATT or ATC.

13. The method of claims 10 or 11 further comprising optimization of the protein-coding sequence according to the 6AA method, wherein CGT is used to encode all arginine residues, GAT is used to encode all aspartate residues, GAA is used to encode all glutamate residues, CAA is used to encode all glutamine residues, CAT is used to encode all histidine residues, and ATT is used to encode all isoleucine residues.

14. The method of claims 10 or 11 further comprising optimization of the protein-coding sequence according to according to the 31C method, wherein AAT is used to encode all asparagine residues, GAT is used to encode all aspartate residues, TGT is used to encode all cysteine residues, GAA is used to encode all glutamate residues, GGT is used to encode all glycine residues, AAA is used to encode all lysine residues, ATG is used to encode all methionine residues, TTT is used to encode all phenylalanine residues, TGG is used to encode all tryptophan residues, TAT is used to encode all tyrosine residues, a random selection of GCT or GCA is used to encode all alanine residues, a random selection of CGT or CGA is used to encode all arginine residues, a random selection of CAA or CAG is used to encode all glutamine residues, a random selection of CAT or CAC is used to encode all histidine residues, a random selection of ATT or ATC is used to encode all isoleucine residues, a random selection of TTA or TTG or CTA is used to encode all leucine residues, a random selection of CCT or CCA is used to encode all proline residues, a random selection of AGT or TCA is used to encode all serine residues, a random selection of ACA or ACT is used to encode all threonine residues, and a random selection of GTT or GTA is used to encode all valine residues.

## Patentansprüche

1. Computerimplementiertes Verfahren zum Erhöhen der Expression eines rekombinanten Polypeptids in einem in vitro- oder in vivo-Expressionssystem, umfassend das Vornehmen einer oder mehrerer synonymer Substitutionen in der Protein-codierenden Nukleinsäuresequenz durch Auswählen eines guten Codons für jede Aminosäure gemäß einem generalisierten linearen Multiparameter-Modell des Einflusses eines umfassenden Satzes von RNA-Sequenz-Parametern auf messbare experimentelle Werte, die mit dem Proteinexpressionsspiegel korrelieren, wobei der umfassende Satz von RNA-Sequenz-Parametern Folgendes beinhaltet (i) Einzelcodonhäufigkeiten im Leserahmen, (ii) die Häufigkeit des Dicodons ATA-ATA im Leserahmen, (iii) lineare, parabolische und invers-lineare Funktionen der Nukleotidbasenzusammensetzung Codons 2-6, (iv) die Nukleotidbasenzusammensetzung in der übrigen Protein-codierenden Sequenz, (v) die freie Energie gemäß Verteilungsfunktion der RNA-Faltung, die durch das Programm RNAstructure mit Standardparametern für die ersten 48 Nukleotide in der Protein-codierenden Sequenz plus der 5'-UTR-Sequenz, wenn diese Sequenz im modellierten Datensatz konsistent ist, berechnet wird, (vi) den Durchschnittswert der freien Energie gemäß Verteilungsfunktion der RNA-Faltung, die durch das Programm RNAstructure mit Standardparametern für 50% überlappende Fenster von 96 Nukleotiden nach Nukleotid 48 in der Protein-codierenden Sequenz berechnet wird, (vii) die Länge der Protein-codierenden Sequenz in Nukleotiden, (viii) die Aminosäurewiederholungsrate und (ix) die Codonwiederholungsrate.

2. Verfahren nach Anspruch 1, wobei das generalisierte lineare Multiparameter-Modell ein lineares Regressionsmodell ist.

3. Verfahren nach Anspruch 1, wobei das generalisierte lineare Multiparameter-Modell ein logistisches Regressionsmodell auf der Basis von Beobachtungen mit den höchsten vs. den niedrigsten mit dem Proteinexpressionsspiegel korrelierenden messbaren experimentellen Werten ist, wobei der höchste und der niedrigste Satz jeweils üblicherweise ein Drittel der Beobachtungen in dem Datensatz ausmacht.

4. Verfahren nach Anspruch 1, wobei die Tabelle zulässiger Codons das Codon für jede Aminosäure mit der stärksten positiven Wirkung auf die mit dem Proteinexpressionsspiegel korrelierenden messbaren experimentellen Werte, wie sie anhand der Steigung dieses Codons in dem generalisierten linearen Multiparameter-Modell quantifiziert wird, plus sämtliche synonymen Codons im Bereich der Unsicherheit in der Steigung des Codons mit der am stärksten positiven Wirkung einschließt.

5. Verfahren nach Anspruch 1, wobei die Tabelle zulässiger Codons das Codon für jede Aminosäure mit der stärksten positiven Wirkung auf die mit dem Proteinexpressionsspiegel korrelierenden messbaren experimentellen Werte, wie sie anhand der Steigung dieses Codons in dem generalisierten linearen Multiparameter-Modell quantifiziert wird, plus sämtliche synonymen Codons innerhalb des Doppelten des Bereichs der Unsicherheit in der Steigung des Codons mit der am stärksten positiven Wirkung einschließt.

6. Verfahren nach Anspruch 1, wobei die positiv mit dem Proteinexpressionsspiegel korrelierenden Parameter, die für die generalisierte lineare Multiparameter-Modellierung verwendet werden, experimentell unter Expressionsbedingungen in einem Wirtsstamm gemessene Proteinexpressionsspiegel sind.

7. Verfahren nach Anspruch 6, wobei die experimentell gemessenen Proteinexpressionsspiegel aus einem Datensatz erhalten werden, der unter Verwendung von Bakteriophage-T7-RNA-Polymerase zur Expression von 6348 Proteinen mit maximal 30% paarweiser Aminosäureidentität in E. coli-BL21(DE3)-Zellen, die in chemisch definiertem flüssigem Medium mit Glucose als Kohlenstoffquelle wuchsen, erfasst wird.

8. Verfahren nach Anspruch 1, wobei die positiv mit dem Proteinexpressionsspiegel korrelierenden Parameter, die für die generalisierte lineare Multiparameter-Modellierung verwendet werden, globale Fließgleichgewichts-mRNA-Spiegel sind, die unter Expressionsbedingungen in einem Wirtsstamm gemessen wurden.

9. Verfahren nach Anspruch 1, wobei die positiv mit dem Proteinexpressionsspiegel korrelierenden Parameter, die für die generalisierte lineare Multiparameter-Modellierung verwendet werden, mRNA-Lebensdauern sind, die unter Expressionsbedingungen in einem Wirtsstamm gemessen wurden [0421, 0419].

10. Computerimplementiertes Verfahren zum Erhöhen der Expression eines rekombinanten Polypeptids in einem in vitro- oder in vivo-Expressionssystem, umfassend das Bereitstellen einer Nukleinsäuresequenz mit einer Protein-codierenden Sequenz, funktionell verknüpft mit einer 5'-untranslatierten Region (5'-UTR), die eine Ribosomenbindungsstelle enthält, das Vornehmen einer oder mehrerer synonymer Substitutionen in den Codons 2, 3, 5, 4 und 6 der Protein-codierenden Nukleinsäuresequenz, die den Guaningehalt senken oder den Adeningehalt erhöhen, und das Vornehmen synonymer Substitutionen in der übrigen Protein-codierenden Sequenz, die zu einer durch das Programm RNAstructure mit Standardparametern berechneten freien Energie gemäß Verteilungsfunktion der RNA-Faltung führen, die so nah wie erreichbar bei einem Wert über -10 kcal/mol für die ersten 48 Nukleotide in der Protein-codierenden Sequenz liegt, und, wenn eine pET-Vektor-5'-UTR verwendet wird, die -30,0 kcal/mol oder mehr für die ersten 48 Nukleotide in der Protein-codierenden Sequenz plus der 5'-UTR beträgt.

11. Computerimplementiertes Verfahren zum Erhöhen der Expression eines rekombinanten Polypeptids in einem in vitro- oder in vivo-Expressionssystem durch Vornehmen einer oder mehrerer synonymer Substitutionen, so dass die freie Energie gemäß Verteilungsfunktion der RNA-Faltung, wie sie durch das Programm RNAstructure mit Standardparametern berechnet wird, so nah wie erreichbar im Bereich von 10 kcal/mol kleiner als bis 5 kcal/mol größer als (-0,32*(W-18)) kcal/mol liegt, wobei W die Anzahl an Nukleotiden im Schwanzsequenzfenster stromabwärts von Nukleotid 48 in der Protein-codierenden Sequenz ist, wobei W üblicherweise 96 Nukleotide beträgt.

12. Verfahren nach den Ansprüchen 10 oder 11, das ferner die Optimierung der Protein-codierenden Sequenz durch Vornehmen einer oder mehrerer synonymer Substitutionen, die jedes ATA-Codon für Isoleucin im Leserahmen durch entweder ATT oder ATC ersetzen, umfasst.

13. Verfahren nach den Ansprüchen 10 oder 11, das ferner die Optimierung der Protein-codierenden Sequenz gemäß dem 6AA-Verfahren umfasst, wobei CGT zum Codieren aller Argininreste verwendet wird, GAT zum Codieren aller Aspartatreste verwendet wird, GAA zum Codieren aller Glutamatreste verwendet wird, CAA zum Codieren aller Glutaminreste verwendet wird, CAT zum Codieren aller Histidinreste verwendet wird und ATT zum Codieren aller Isoleucinreste verwendet wird.

14. Verfahren nach den Ansprüchen 10 oder 11, das ferner die Optimierung der Protein-codierenden Sequenz gemäß dem 31C-Verfahren umfasst, wobei AAT zum Codieren aller Asparaginreste verwendet wird, GAT zum Codieren aller Aspartatreste verwendet wird, TGT zum Codieren aller Cysteinreste verwendet wird, GAA zum Codieren aller Glutamatreste verwendet wird, GGT zum Codieren aller Glycinreste verwendet wird, AAA zum Codieren aller Lysinreste verwendet wird, ATG zum Codieren aller Methioninreste verwendet wird, TTT zum Codieren aller Phenylalaninreste verwendet wird, TGG zum Codieren aller Tryptophanreste verwendet wird, TAT zum Codieren aller Tyrosinreste verwendet wird, eine Zufallsauswahl aus GCT oder GCA zum Codieren aller Alaninreste verwendet wird, eine Zufallsauswahl aus CGT oder CGA zum Codieren aller Argininreste verwendet wird, eine Zufallsauswahl aus CAA oder CAG zum Codieren aller Glutaminreste verwendet wird, eine Zufallsauswahl aus CAT oder CAC zum Codieren aller Histidinreste verwendet wird, eine Zufallsauswahl aus ATT oder ATC zum Codieren aller Isoleucinreste verwendet wird, eine Zufallsauswahl aus TTA oder TTG oder CTA zum Codieren aller Leucinreste verwendet wird, eine Zufallsauswahl aus CCT oder CCA zum Codieren aller Prolinreste verwendet wird, eine Zufallsauswahl aus AGT oder TCA zum Codieren aller Serinreste verwendet wird, eine Zufallsauswahl aus ACA oder ACT zum Codieren aller Threoninreste verwendet wird und eine Zufallsauswahl aus GTT oder GTA zum Codieren aller Valinreste verwendet wird.

## Revendications

1. Procédé mis en oeuvre par ordinateur pour augmenter l'expression d'un polypeptide recombiné dans un système d'expression *in vitro* ou *in vivo*, comprenant la réalisation d'une ou plusieurs substitutions synonymes dans la séquence d'acide nucléique codant pour la protéine par la sélection d'un bon codon pour chaque acide aminé en fonction d'une modélisation linéaire généralisée à plusieurs paramètres de l'incidence d'un jeu complet de paramètres de séquence d'ARN sur des valeurs expérimentales mesurables corrélées avec le taux d'expression de la protéine, dans lequel le jeu complet de paramètres de séquence d'ARN comprend (i) les fréquences de codons individuels en phase, (ii) la fréquence du dicodon ATA-ATA en phase, (iii) les fonctions linéaires, paraboliques et linéaires inverses des codons 2-6 de la composition de bases nucléotidiques, (iv) la composition de bases nucléotidiques dans le reste de la séquence codant pour la protéine, (v) l'énergie libre de la fonction de partition du repliement d'ARN calculée par le programme RNAstructure avec les paramètres par défaut pour les 48 premiers nucléotides de la séquence codant pour la protéine auxquels s'ajoute la séquence 5'-UTR si cette séquence est constante dans le jeu de données modélisé, (vi) la valeur moyenne de l'énergie libre de la fonction de partition du repliement d'ARN calculée par le programme RNAstructure avec les paramètres par défaut pour la fenêtre de chevauchement à 50 % de 96 nucléotides venant après le nucléotide 48 dans la séquence codant pour la protéine, (vii) la longueur en nombre de nucléotides de la séquence codant pour la protéine, (viii) le taux de répétition d'acides aminés et (ix) le taux de répétition de codons.

2. Procédé selon la revendication 1 dans lequel le modèle linéaire généralisé à plusieurs paramètres est un modèle de régression linéaire.

3. Procédé selon la revendication 1 dans lequel le modèle linéaire généralisé à plusieurs paramètres est un modèle de régression logistique basé sur des observations, les plus élevées par rapport aux plus basses des valeurs expérimentales mesurables étant corrélées avec le taux d'expression de la protéine, les jeux les plus élevés et les plus bas constituant d'ordinaire chacun un tiers des observations présentes dans le jeu de données.

4. Procédé selon la revendication 1 dans lequel le tableau de codons permis comprend le codon pour chaque acide aminé présentant l'effet le plus positif sur les valeurs expérimentales mesurables corrélées avec le taux d'expression de la protéine, tel que quantifié par la pente de ce codon dans le modèle linéaire généralisé à plusieurs paramètres, auquel s'ajoutent tous les codons synonymes dans les limites de l'intervalle d'incertitude sur la pente du codon présentant l'effet le plus positif.

5. Procédé selon la revendication 1 dans lequel le tableau de codons permis comprend le codon pour chaque acide aminé présentant l'effet le plus positif sur les valeurs expérimentales mesurables corrélées avec le taux d'expression de la protéine, tel que quantifié par la pente de ce codon dans le modèle linéaire généralisé à plusieurs paramètres, auquel s'ajoutent tous les codons synonymes dans les limites de deux fois l'intervalle d'incertitude sur la pente du codon présentant l'effet le plus positif.

6. Procédé selon la revendication 1 dans lequel les paramètres positivement corrélés avec le taux d'expression de la protéine qui sont utilisés pour la modélisation linéaire généralisée à plusieurs paramètres sont des taux d'expression de la protéine mesurés expérimentalement dans des conditions d'expression dans une souche hôte.

7. Procédé selon la revendication 6 dans lequel les taux d'expression de la protéine mesurés expérimentalement sont obtenus à partir d'un jeu de données acquis à l'aide de l'ARN polymérase du bactériophage T7 pour exprimer 6 348 protéines présentant une identité des acides aminés par paires d'au maximum 30 % dans des cellules d'*E. coli* BL21(DE3) croissant dans du milieu liquide chimiquement défini avec du glucose en tant que source de carbone.

8. Procédé selon la revendication 1 dans lequel les paramètres positivement corrélés avec le taux d'expression de la protéine qui sont utilisés pour la modélisation linéaire généralisée à plusieurs paramètres sont des taux d'ARNm en régime stationnaire globaux mesurés dans des conditions d'expression dans une souche hôte.

9. Procédé selon la revendication 1 dans lequel les paramètres positivement corrélés avec le taux d'expression de la protéine qui sont utilisés pour la modélisation linéaire généralisée à plusieurs paramètres sont des durées de vie d'ARNm mesurées dans des conditions d'expression dans une souche hôte [0421, 0419] .

10. Procédé mis en oeuvre par ordinateur pour augmenter l'expression d'un polypeptide recombiné dans un système d'expression *in vitro* ou *in vivo* qui comprend la fourniture d'une séquence d'acide nucléique comportant une séquence codant pour la protéine liée de manière fonctionnelle à une région 5' non traduite (5'-UTR) contenant un site de liaison au ribosome, la réalisation dans les codons 2, 3, 5, 4 et 6 de la séquence codant pour la protéine d'une ou plusieurs substitutions synonymes qui abaissent la teneur en guanine ou élèvent la teneur en adénine et la réalisation dans la séquence codante résultante de substitutions synonymes qui produisent une énergie libre de la fonction de partition du repliement d'ARN calculée par le programme RNAstructure avec les paramètres par défaut qui se rapproche autant que possible d'une valeur supérieure à -10 kcal/mol pour les 48 premiers nucléotides de la séquence codant pour la protéine et, si on utilise une 5'-UTR de vecteur pET, qui est supérieure ou égale à -30,0 kcal/mol pour les 48 premiers nucléotides de la séquence codant pour la protéine auxquels s'ajoute la 5'-UTR.

11. Procédé mis en oeuvre par ordinateur pour augmenter l'expression d'un polypeptide recombiné dans un système d'expression *in vitro* ou *in vivo* par la réalisation d'une ou plusieurs substitutions synonymes pour que l'énergie libre de la fonction de partition du repliement d'ARN telle que calculée par le programme RNAstructure avec les paramètres par défaut se rapproche autant que possible d'une valeur dans l'intervalle de 10 kcal/mol de moins à 5 kcal/mol de plus que (-0,32*(W-18)) kcal/mol, W étant le nombre de nucléotides dans la fenêtre de séquence de queue en aval du nucléotide 48 dans la séquence codant pour la protéine, W étant d'ordinaire de 96 nucléotides.

12. Procédé selon les revendications 10 ou 11 comprenant en outre l'optimisation de la séquence codant pour la protéine par la réalisation d'une ou plusieurs substitutions synonymes qui remplacent chaque codon ATA en phase pour l'isoleucine par soit ATT soit ATC.

13. Procédé selon les revendications 10 ou 11 comprenant en outre l'optimisation de la séquence codant pour la protéine selon la méthode 6AA, dans lequel CGT est utilisé pour coder tous les résidus d'arginine, GAT est utilisé pour coder tous les résidus d'aspartate, GAA est utilisé pour coder tous les résidus de glutamate, CAA est utilisé pour coder tous les résidus de glutamine, CAT est utilisé pour coder tous les résidus d'histidine et ATT est utilisé pour coder tous les résidus d'isoleucine.

14. Procédé selon les revendications 10 ou 11 comprenant en outre l'optimisation de la séquence codant pour la protéine selon la méthode 31C, dans lequel AAT est utilisé pour coder tous les résidus d'asparagine, GAT est utilisé pour coder tous les résidus d'aspartate, TGT est utilisé pour coder tous les résidus de cystéine, GAA est utilisé pour coder tous les résidus de glutamate, GGT est utilisé pour coder tous les résidus de glycine, AAA est utilisé pour coder tous les résidus de lysine, ATG est utilisé pour coder tous les résidus de méthionine, TTT est utilisé pour coder tous les résidus de phénylalanine, TGG est utilisé pour coder tous les résidus de tryptophane, TAT est utilisé pour coder tous les résidus de tyrosine, une sélection aléatoire de GCT ou GCA est utilisée pour coder tous les résidus d'alanine, une sélection aléatoire de CGT ou CGA est utilisée pour coder tous les résidus d'arginine, une sélection aléatoire de CAA ou CAG est utilisée pour coder tous les résidus de glutamine, une sélection aléatoire de CAT ou CAC est utilisée pour coder tous les résidus d'histidine, une sélection aléatoire de ATT ou ATC est utilisée pour coder tous les résidus d'isoleucine, une sélection aléatoire de TTA ou TTG ou CTA est utilisée pour coder tous les résidus de leucine, une sélection aléatoire de CCT ou CCA est utilisée pour coder tous les résidus de proline, une sélection aléatoire de AGT ou TCA est utilisée pour coder tous les résidus de sérine, une sélection aléatoire de ACA ou ACT est utilisée pour coder tous les résidus de thréonine et une sélection aléatoire de GTT ou GTA est utilisée pour coder tous les résidus de valine.
